# EUROPÄISCHE PATENTANMELDUNG

(11) **EP 2 236 505 A1**
(43) Veröffentlichungstag der Anmeldung: **06.10.2010**
(21) Anmeldenummer: 09157253.7
(22) Anmeldetag: 03.04.2009
(51) Int. Cl.: C07D 401/12, C07D 401/14, C07D 213/75, C07D 237/20, A01N 43/40

(54) **Acylierte Aminopyridine und - pyridazine als Insektizide**

(71) Anmelder: Bayer CropScience AG, 40789 Monheim (DE)
(72) Erfinder: Die Erfindernennung liegt noch nicht vor

(57) **Zusammenfassung**

Die vorliegende Erfindung betrifft Aminopyridin- und Aminopyridazin-Derivate der allgemeinen Formel (I), 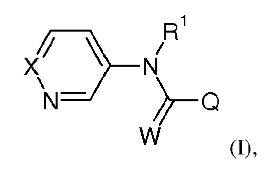 - in welcher R¹, X, W und Q die in der Beschreibung angegebenen Bedeutungen haben - mehrere Verfahren zu ihrer Herstellung und ihre Verwendung als Insektizide.

## Beschreibung

Die vorliegende Erfindung betrifft neue Schädlingsbekämpfungsmittel, mehrere Verfahren zu ihrer Herstellung und ihre Verwendung als Wirkstoffe, insbesondere ihre Verwendung als Insektizide.

In der Literatur sind bestimmte 3-substituierte Pyridine als Insektizide beschrieben, siehe beispielsweise EP314615 oder WO 2009/027393. Es wurde nun überraschend gefunden, dass bestimmte acylierte Aminopyridine und -pyridazine starke insektizide Eigenschaften besitzen.

Gegenstand der vorliegenden Erfindung sind daher Aminopyridin- und Aminopyridazin-Derivate der allgemeinen Formel (I), 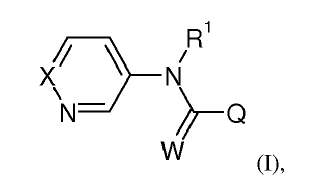 wobei
- R¹: für
Wasserstoff, für gegebenenfalls einfach oder mehrfach, gleich oder verschieden substituiertes C₁- C₆-Alkyl, C₂-C₆-Alkenyl, C₂-C₆-Alkinyl oder C₃-C₆-Cycloalkyl steht,
wobei die Substituenten unabhängig voneinander ausgewählt sein können aus Halogen, Cyano, Nitro, Hydroxy, C₁-C₆-Alkyl, C₁-C₆-Alkoxy, C₃-C₆-Cycloalkyl, C₁-C₆-Halogenalkoxy, C₁-C₆- Alkylthio, Aryl, Hetaryl, Arylalkyl, Hetarylalkyl,
wobei die Substituenten Aryl, Hetaryl, Arylalkyl, Hetarylalkyl gegebenenfalls einfach oder mehrfach, gleich oder verschieden durch Halogen, Cyano, Nitro, Hydroxy, C₁-C₆-Alkyl, C₂-C₆- Alkenyl, C₂-C₆-Alkinyl, C₃-C₆-Cycloalkyl, C₁-C₆-Alkoxy, C₁-C₆-Halogenalkyl, C₁-C₆- Halogenalkoxy oder C₁-C₆-Alkylthio substituiert sein können,
- X: für CH oder N steht,
- Q: für einen aromatischen, gegebenenfalls durch 1-bis 3 Heteroatome aus der Reihe N, S, O substituierten, 5-6 gliedrigen Ring Q steht, welcher gegebenenfalls durch die Reste R²-R⁴ und den Rest A-Y unabhängig voneinander substituiert sein kann,
falls X für N steht
- Q: für einen aromatischen, gegebenenfalls durch 1-bis 3 Heteroatome aus der Reihe N, S, O substituierten, 6 gliedrigen Ring Q steht, welcher gegebenenfalls durch die Reste R²-R⁴ und den Rest A-Y unabhängig voneinander substituiert sein kann; für einen aromatischen, gegebenenfalls durch 1-bis 3 Heteroatome aus der Reihe N, S, O substituierten, 5 gliedrigen Ring Q steht, wobei höchstens eines der Heteroatome N ist, welcher gegebenenfalls durch die Reste R²-R⁴ und den Rest A-Y unabhängig voneinander substituiert sein kann;
oder für die folgenden Ringsysteme steht, ausgewählt aus Q₈ₐ-Q_{8d} 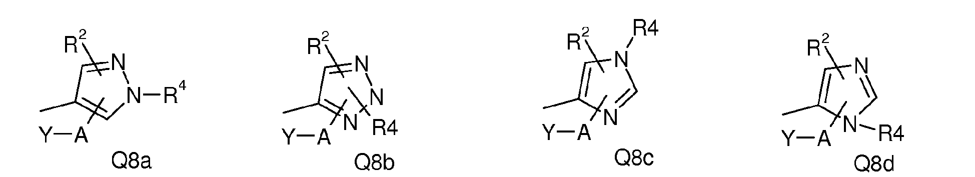
- falls X: für CH steht:
- W: für O oder S steht,
- R² und R³: unabhängig voneinander für Wasserstoff, Hydroxy, Halogen, Nitro, für gegebenenfalls einfach oder mehrfach, gleich oder verschieden substituiertes Amino, C₁-C₆-Alkyl, C₁-C₆- Alkoxy, C₁-C₆-Alkoxy-C₁-C₆-Alkyl, C₂-C₆-Alkenyl, C₂-C₆-Alkinyl oder C₃-C₆-Cycloalkyl stehen, wobei die Substituenten unabhängig voneinander ausgewählt sein können aus Halogen, C₁-C₆-Alkyl, C₁-C₆-Alkylcarbonyl, C₁-C₆-Alkoxy, C₁-C₆-Halogenalkoxy, C₃-C₆- Cycloalkyl, C₃-C₆-Cycloalkylcarbonyl, Cyano, Nitro, Hydroxy, C₁-C₆-Alkylthio, C₁-C₆- Alkylsulfinyl, C₁-C₆-Alkylsulfonyl, (C₁-C₆-Alkoxy)carbonyl, C₁-C₆-Alkylamino, C₃-C₆- Cycloalkylamino, (C₁-C₆-Alkyl)C₃-C₆-cycloalkylamino oder Aryl, Hetaryl, das gegebenenfalls einfach oder mehrfach gleich oder verschieden durch Halogen, Cyano, Nitro, Hydroxy, C₁-C₆-Alkyl, C₁-C₆-Halogenalkyl, C₂-C₆-Alkenyl, C₂-C₆-Alkinyl, C₃-C₆- Cycloalkyl, C₁-C₆-Alkoxy, C₁-C₆-Halogenalkoxy C₁-C₆-Alkylthio, C₁-C₆-Alkylsulfinyl, C₁-C₆-Alkylsulfonyl substituiert sein kann,
- R² und R³: ebenfalls zusammen einen 5-bis 7-gliedrigen, 1-3 Heteroatome aus der Reihe N,S,O enthaltenden, heteroaromatischen Ring ausbilden können, welcher gegebenenfalls ein-oder mehrfach durch C₁-C₆-Alkyl substituiert sein kann,
- R⁴: für Wasserstoff, für gegebenenfalls einfach oder mehrfach, gleich oder verschieden substituiertes, C₁-C₆-Alkyl, C₁-C₆-Alkoxy-C₁-C₆-Alkyl, C₂-C₆-Alkenyl, C₂-C₆-Alkinyl, C₃- C₆-Cycloalkyl, Aryl, Hetaryl steht, wobei die Substituenten unabhängig voneinander ausgewählt sein können aus Halogen, C₁-C₆-Alkyl, C₁-C₆-Alkylcarbonyl, C₁-C₆-Alkoxy, C₁-C₆-Halogenalkoxy, C₃-C₆-Cycloalkyl, C₃-C₆-Cycloalkylcarbonyl, Cyano, Nitro, Hydroxy, C₁-C₆-Alkylthio, C₁-C₆-Alkylsulfinyl, C₁-C₆-Alkylsulfonyl, (C₁-C₆- Alkoxy)carbonyl, C₁-C₆-Alkylamino, C₃-C₆-Cycloalkylamino, (C₁-C₆-Alkyl)C₃-C₆- cycloalkylamino, Aryl oder Hetaryl, das gegebenenfalls einfach oder mehrfach gleich oder verschieden durch Halogen, Cyano, Nitro, Hydroxy, C₁-C₆-Alkyl, C₁-C₆-Halogenalkyl, C₂- C₆-Alkenyl, C₂-C₆-Alkinyl, C₃-C₆-Cycloalkyl, C₁-C₆-Alkoxy, C₁-C₆-Halogenalkoxy C₁-C₆- Alkylthio, C₁-C₆-Alkylsulfinyl, C₁-C₆-Alkylsulfonyl substituiert sein kann,
- A: für eine direkte Bindung oder für C₁-C₆-Alkylen, C₁-C₆-Alkylenoxy, Oxy(C₁-C₆)-alkylen, Thio(C₁-C₆)-alkylen, C₁-C₆-alkylenthio, -O- -S-, C₁-C₄-Alkylencarboxy, Carboxy(C₁-C₄)- Alkylen, -NR⁵, -C=N-O-, C₁-C₄-Alkylencarbamido, Carbamido(C₁-C₄)-Alkylen, - NR⁵(C=O)O-, NR⁵(SO₂)- -NR⁵(C=O)NR⁵-, -(SO₂)- steht,
- R⁵: für C₁-C₆-Alkyl, C₃-C₆-Cycloalkyl oder für Wasserstoff steht,
- Y: für Wasserstoff, für gegebenenfalls ein- oder mehrfach, gleich oder verschieden substituiertes C₁-C₆-Alkyl, C₃-C₆-Cycloalkyl, für ein gegebenenfalls ein- oder mehrfach, gleich oder verschieden substituiertes, 5-10 gliederiges, 0-4 Heteroatome enthaltendes, gegebenenfalls annelliertes Ringsystem steht, wobei die Heteroatome ausgewählt sein können aus der Reihe N,S,O,
wobei die Substituenten ausgewählt sein können aus Halogen, Nitro, Cyano, aus gegebenenfalls einfach oder mehrfach, gleich oder verschieden substituiertem Amino, C₁-C₆-Alkyl, C₁-C₆-Alkoxy, C₁-C₆-Alkoxy-C₁-C₆-Alkyl, C₂-C₆-Alkenyl, C₂-C₆-Alkinyl, C₃-C₆-Cycloalkyl, Aryl, Hetaryl, C₁-C₆-Arylalkyl, C₁-C₆-Hetarylalkyl, Aryloxy, Hetaryloxy, Sulfonyl, C₁-C₆-Thioalkyl, C₁-C₆-Carboxyalkyl,
wobei die Substituenten unabhängig voneinander ausgewählt sein können aus Halogen, C₁-C₆-Alkyl, C₁-C₆-Alkylcarbonyl, C₁-C₆-Halogenalkyl, C₁-C₆-Alkoxy, C₁-C₆-Halogenalkoxy, C₃-C₆-Cycloalkyl, C₃-C₆-Cycloalkylcarbonyl, Cyano, Nitro, C₁-C₆-Alkylthio, C₁-C₆-Alkylsulfinyl, C₁-C₆-Alkylsulfonyl, (C₁-C₆-Alkoxy)carbonyl, C₂-C₆-Alkenyl, C₂-C₆-Alkinyl, C₁-C₆-Alkylamino, C₃-C₆-Cycloalkylamino, (C₁-C₆-Alkyl)C₃C₆-cycloalkylamino, Di(C₁-C₄)alkylamino,
sowie Salze von Verbindungen der Formel (I) und deren Anwendung zur Bekämpfung von tierischen Schädlingen

Die Verbindungen der Formel (I) können gegebenenfalls in verschiedenen polymorphen Formen oder als Mischung verschiedener polymorpher Formen vorliegen. Sowohl die reinen Polymorphe als auch die Polymorphgemische sind Gegenstand der Erfindung und können erfindungsgemäß verwendet werden.

Die Verbindungen der Formel (I) umfassen gegebenenfalls Diastereomere oder Enantiomere.

Die Aminopyridine und Aminopyridazine sind durch die Formel (I) allgemein definiert. Bevorzugte Restedefinitionen der vorstehenden und nachfolgend genannten Formeln sind im Folgenden angegeben. Diese Definitionen gelten für die Endprodukte der Formel (I) wie für alle Zwischenprodukte gleichermaßen.

Als bevorzugt, besonders bevorzugt und ganz besonders bevorzugt gelten Verbindungen der Formel (I), sowie ein Vefahren zur Bekämpfung von Schädlingen unter Anwendung der Verbindungen der Formel (I), wobei
R¹ bevorzugt für
Wasserstoff, für gegebenenfalls einfach oder mehrfach, gleich oder verschieden substituiertes C₁-C₄-Alkyl, C₂-C₄-Alkenyl, C₂-C₄-Alkinyl oder C₃-C₄-Cycloalkyl steht,
wobei die Substituenten unabhängig voneinander ausgewählt sein können aus Halogen, Cyano, Nitro, Hydroxy, C₁-C₄-Alkyl, C₁-C₄-Alkoxy, C₃-C₄-Cycloalkyl, C₁-C₄-Halogenalkoxy, Aryl, Hetaryl, Arylalkyl, Hetarylalkyl,
wobei die Substituenten Aryl, Hetaryl, Arylalkyl, Hetarylalkyl gegebenenfalls einfach oder mehrfach, gleich oder verschieden durch Halogen, Cyano, Nitro, Hydroxy, C₁-C₄-Alkyl, C₂-C₄-Alkenyl, C₂-C₄-Alkinyl, C₃-C₄-Cycloalkyl, C₁-C₄-Alkoxy, C₁-C₄-Halogenalkyl oder C₁-C₄-Halogenalkoxy substituiert sein können,
R¹ besonders bevorzugt für
Wasserstoff, für gegebenenfalls einfach oder mehrfach, gleich oder verschieden substituiertes C₁-C₄-Alkyl, C₂-C₄-Alkenyl oder C₂-C₄-Alkinyl steht,
wobei die Substituenten unabhängig voneinander ausgewählt sein können aus Halogen, C₁-C₄-Alkyl, Aryl, Arylalkyl,
wobei die Substituenten Aryl, Arylalkyl gegebenenfalls einfach oder mehrfach, gleich oder verschieden durch Halogen, C₁-C₄-Alkyl, C₁-C₄-Halogenalkyl, C₁-C₄-Halogenalkoxy substituiert sein können,
R¹ ganz besonders bevorzugt für
Wasserstoff, Methyl, Ethyl, Propyl, Allyl, Propargyl, gegebenenfalls durch Methyl, Halogen, C₁-C₄-Halogenalkyl oder C₁-C₄-Halogenalkoxy substituiertes Benzyl steht,
- Q: bevorzugt für einen Ring steht, ausgewählt aus der Gruppe Q1 bis Q 14 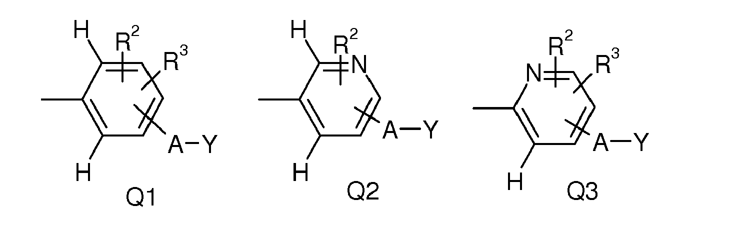 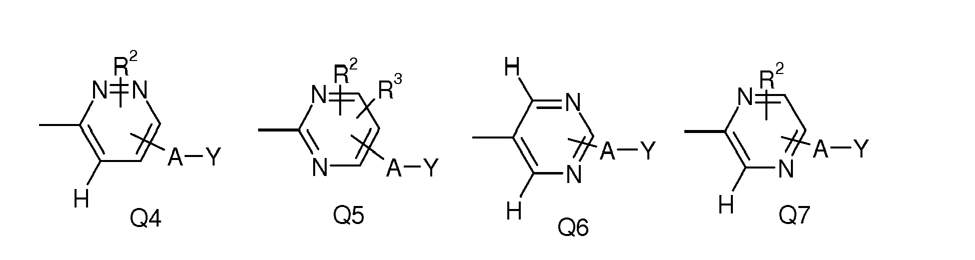 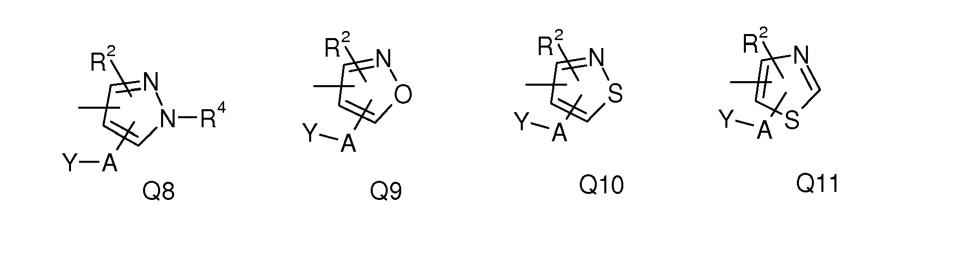 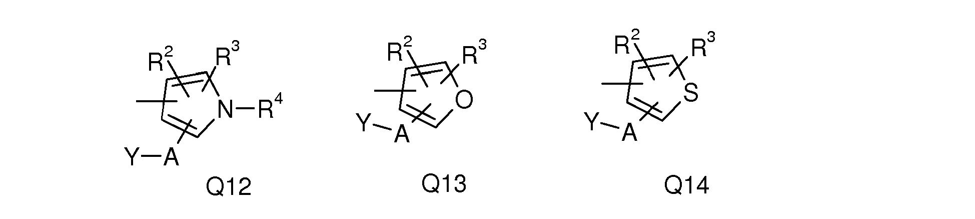
Falls X für N steht
- Q: besonders bevorzugt für einen der folgenden Ringe steht, 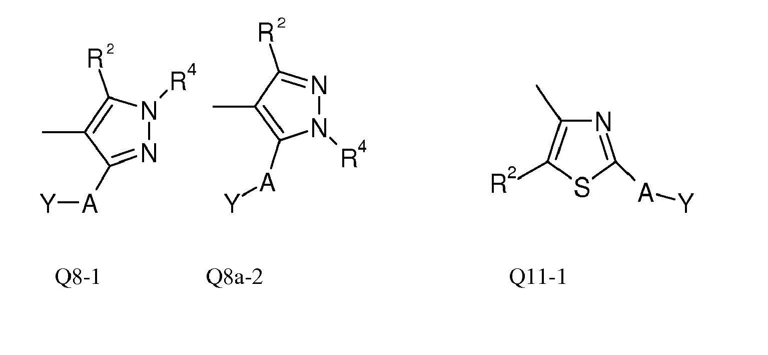 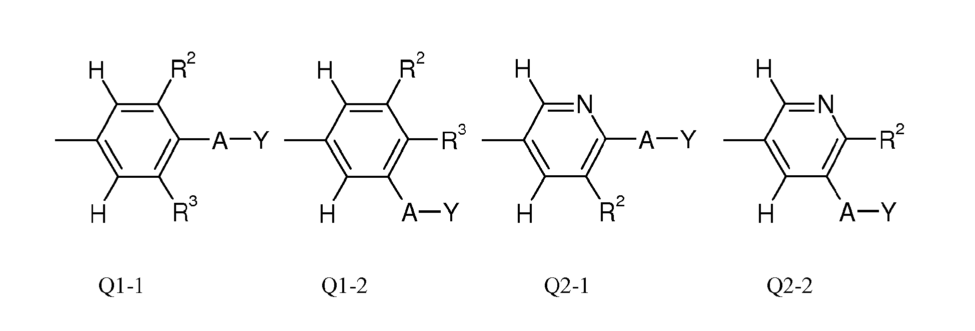
Falls X für N steht
- Q: ganz besonders bevorzugt für einen der folgenden Ringe steht, 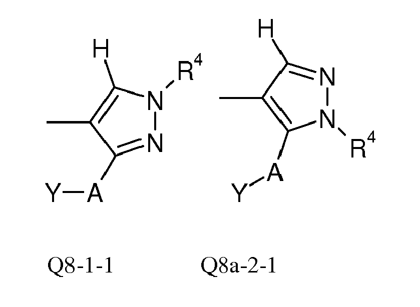 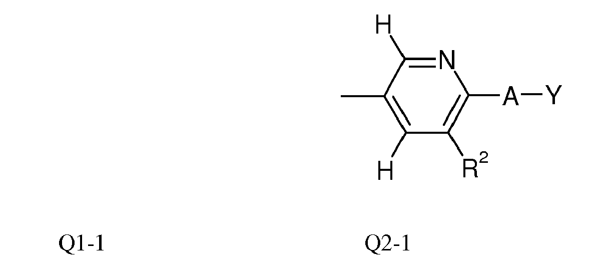
Falls X für N steht
- Q: bevorzugt für einen Ring steht, ausgewählt aus der Gruppe Q1 bis Q 14 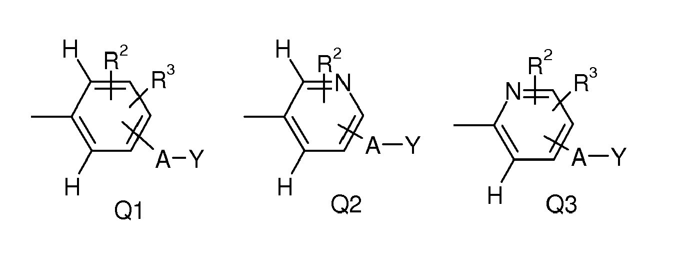 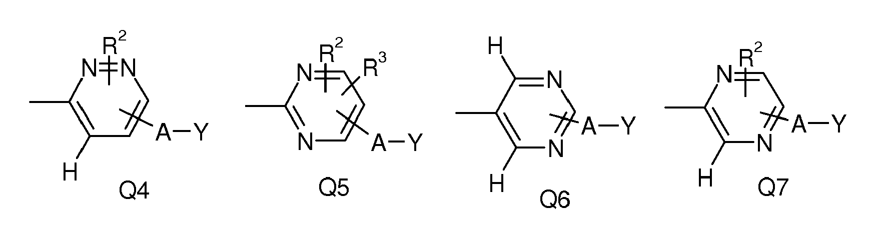 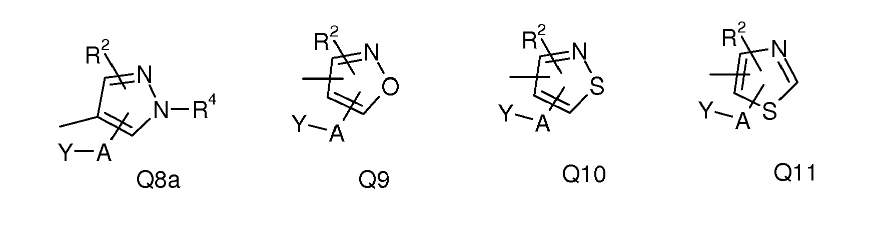 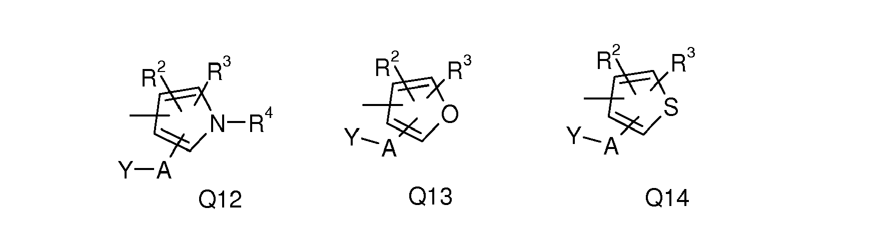
Falls X für CH steht
- Q: besonders bevorzugt für einen der folgenden Ringe steht, 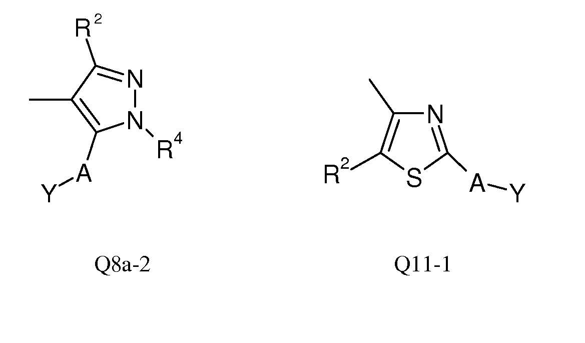 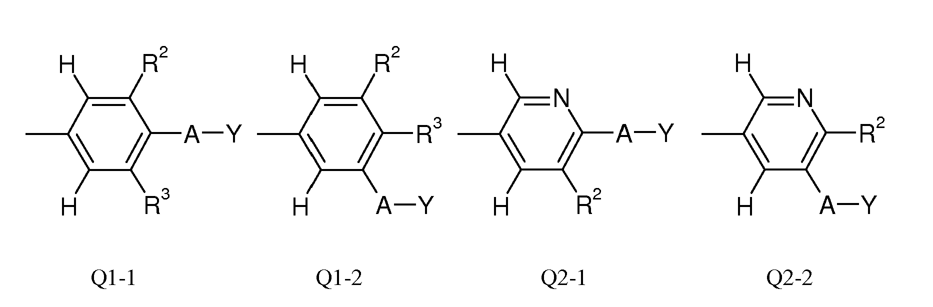
Falls X für CH steht
- Q: ganz besonders bevorzugt für einen der folgenden Ringe steht, 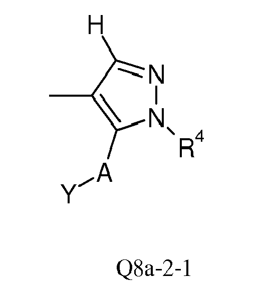 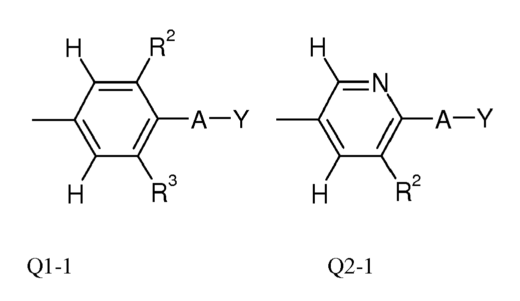
Falls X für CH steht
R² und R³ unabhängig voneinander bevorzugt für Wasserstoff, Hydroxy, Halogen, Nitro, für gegebenenfalls einfach oder mehrfach, gleich oder verschieden substituiertes Amino, C₁-C₄-Alkyl, C₁-C₄-Alkoxy, C₁-C₄-Alkoxy-C₁-C₄-Alkyl, C₂-C₄-Alkenyl, C₂-C₄-Alkinyl oder C₃-C₄-Cycloalkyl stehen, wobei die Substituenten unabhängig voneinander ausgewählt sein können aus Halogen, C₁-C₄-Alkyl, C₁-C₄-Alkylcarbonyl, C₁-C₄-Alkoxy, C₁-C₄-Halogenalkoxy, C₃-C₄-Cycloalkyl, C₃-C₄-Cycloalkylcarbonyl, Cyano, Nitro, Hydroxy, C₁-C₄-Alkylthio, C₁-C₄-Alkylsulfinyl, C₁-C₄-Alkylsulfonyl, (C₁-C₄₋Alkoxy)carbonyl, C₁-C₄-Alkylamino, C₃-C₄-Cycloalkylamino, (C₁-C₄-Alkyl)C₃-C₄-cycloalkylamino oder Aryl, Hetaryl, das gegebenenfalls einfach oder mehrfach gleich oder verschieden durch Halogen, Cyano, Nitro, Hydroxy, C₁-C₄-Alkyl, C₁-C₄-Halogenalkyl, C₂-C₄-Alkenyl, C₂-C₄-Alkinyl, C₃-C₄-Cycloalkyl, C₁-C₄-Alkoxy, C₁-C₄-Halogenalkoxy C₁-C₄-Alkylthio, C₁-C₄-Alkylsulfinyl, C₁-C₄-Alkylsulfonyl substituiert sein kann,
R² und R³ ebenfalls bevorzugt zusammen einen 5-gliedrigen, gegebenenfalls ein-oder mehrfach durch C₁-C₆-Alkyl substituierten, N enthaltenden, heteroaromatischen Ring ausbilden können,
R² und R³ unabhängig voneinander besonders bevorzugt für Wasserstoff, Halogen, Nitro, für gegebenenfalls einfach oder mehrfach, gleich oder verschieden substituiertes Amino, C₁-C₄-Alkyl, C₁-C₄-Alkoxy, C₁-C₄-Alkoxy-C₁-C₄-Alkyl oder C₃-C₄-Cycloalkyl stehen, wobei die Substituenten unabhängig voneinander ausgewählt sein können aus Halogen, C₁-C₄-Alkyl, C₁-C₄-Alkoxy, C₁-C₄-Halogenalkoxy, C₃-C₄-Cycloalkyl, Cyano, Nitro,
R² und R³ unabhängig voneinander ganz besonders bevorzugt für Wasserstoff, Halogen, Nitro, Amino, C₁-C₄-Alkyl, C₁-C₄-Halogenalkyl, C₁-C₄-Alkoxy oder C₁-C₄-Alkoxy-C₁-C₄-Alkyl stehen.
R⁴ bevorzugt für Wasserstoff, für gegebenenfalls einfach oder mehrfach, gleich oder verschieden substituiertes, C₁-C₄-Alkyl, C₁-C₄-Alkoxy-C₁-C₄-Alkyl, C₂-C₄-Alkenyl, C₂-C₄-Alkinyl, C₃-C₄-Cycloalkyl, Aryl, Hetaryl steht, wobei die Substituenten unabhängig voneinander ausgewählt sein können aus Halogen, C₁-C₄-Alkyl, C₁-C₄-Alkoxy, C₁-C₄-Halogenalkoxy, C₃-C₄-Cycloalkyl, Cyano, Nitro, C₁-C₄-Alkylthio, (C₁-C₆-Alkoxy)carbonyl oder Aryl, Hetaryl, das gegebenenfalls einfach oder mehrfach gleich oder verschieden durch Halogen, Cyano, Nitro, C₁-C₄-Alkyl, C₁-C₄-Halogenalkyl, C₃-C₄-Cycloalkyl, C₁-C₄-Alkoxy, C₁-C₄-Halogenalkoxy substituiert sein kann,
R⁴ besonders bevorzugt für Wasserstoff, für gegebenenfalls einfach oder mehrfach, gleich oder verschieden substituiertes, C₁-C₄-Alkyl, C₁-C₄-Alkoxy-C₁-C₄-Alkyl, C₂-C₄-Alkenyl, C₂-C₄-Alkinyl, C₃-C₄-Cycloalkyl, Aryl, Hetaryl steht, wobei die Substituenten unabhängig voneinander ausgewählt sein können aus Halogen, C₁-C₄-Alkyl, C₁-C₄-Alkoxy, C₁-C₄-Halogenalkoxy, C₃-C₄-Cycloalkyl, Cyano, Nitro, (C₁-C₄₋Alkoxy)carbonyl oder Aryl, Hetaryl, das gegebenenfalls einfach oder mehrfach gleich oder verschieden durch Halogen, Cyano, Nitro, C₁-C₄-Alkyl, C₁-C₄-Halogenalkyl, C₁-C₄-Alkoxy, C₁-C₄-Halogenalkoxy substituiert sein kann,
R⁴ ganz besonders bevorzugt für Wasserstoff, für gegebenenfalls einfach oder mehrfach, gleich oder verschieden substituiertes, C₁-C₄-Alkyl, C₁-C₄-Alkoxy-C₁-C₄-Alkyl, C₂-C₄-Alkenyl, C₃-C₄-Cycloalkyl, Aryl, Hetaryl steht, wobei die Substituenten unabhängig voneinander ausgewählt sein können aus Halogen, C₁-C₄-Alkyl, C₁-C₄-Alkoxy, oder Aryl, Hetaryl, das gegebenenfalls einfach oder mehrfach gleich oder verschieden durch Halogen, C₁-C₄-Halogenalkyl substituiert sein kann,
A bevorzugt für eine direkte Bindung oder für C₁-C₄-Alkylen, C₁-C₄-Alkylenoxy, Oxy(C₁-C₄)-alkylen, Thio(C₁-C₄)-alkylen, C₁-C₄-alkylenthio, -O- -S-, C₁-C₄-Alkylencarboxy, Carboxy(C₁-C₄)-Alkylen, -NR⁵, -C=N-O-, -NR⁵(C=O)O-, NR⁵(SO₂)-, -NR⁵(C=O)NR⁵-, gegebenenfalls durch R⁵ substituiertes Carbamido, Amidocarbonyl, C₁-C₄-Alkylencarbamido, Carbamido(C₁-C₄)-Alkylen, - (SO₂)- steht,
A besonders bevorzugt für eine direkte Bindung oder für C₁-C₄-Alkylen, -CH₂-O-, CH₂-S-, -O- -S-, -O-CH₂ , -S-CH₂-, -C(=O)O-, OC(=O)-, -NR⁵-, -C=N-O-, - R⁵NC(=O)-, - (CH₂)R⁵NC(=O)-, - C(=O)NR⁵, -NR⁵(C=O)O-, NR⁵(SO₂)-, -NR⁵(C=O)NR⁵-, -(SO₂)- steht,
A ganz besonders bevorzugt für eine direkte Bindung oder für -CH₂-, -CH₂-O-, CH₂-S-, -O- -S-, - O-CH₂-, -S-CH₂-, -C(=O)O-, OC(=O)-, -NR⁵-, -C=N-O-, - R⁵NC(=O)-, -C(=O)NR⁵-, - (CH₂)R⁵NC(=O)-, -NR⁵(C=O)O-, NR⁵(SO₂)-, -NR⁵(C=O)NR⁵-, -(SO₂)- steht,
R⁵ bevorzugt für C₁-C₄-Alkyl, C₃-C₆-Cycloalkyl oder für Wasserstoff steht,
R⁵ besonders bevorzugt für Methyl, Ethyl, Cyclopentyl, Isobutyl, Wasserstoff steht,
Y bevorzugt für Wasserstoff, für gegebenenfalls ein- oder mehrfach gleich oder verschieden substituiertes C₁-C₄-Alkyl, C₃-C₄-Cycloalkyl, für einen gegebenenfalls ein- oder mehrfach gleich oder verschieden substituierten, 5-6 gliederiges, 0-4 Heteroatome enthaltendes Ringsystem steht, wobei die Heteroatome ausgewählt sein können aus der Reihe N, O, sowie für Naphthalin, Chinolin steht,
wobei die Substituenten ausgewählt sein können aus Halogen, Nitro, Cyano, aus gegebenenfalls einfach oder mehrfach, gleich oder verschieden substituiertem Amino, C₁-C₄-Alkyl, C₁-C₄-Alkoxy, C₁-C₄-Alkoxy-C₁-C₄-Alkyl, C₂-C₄-Alkenyl, C₂-C₄-Alkinyl, C₃-C₄-Cycloalkyl, Aryl, Hetaryl, C₁-C₄-Arylalkyl, C₁-C₄-Hetarylalkyl, Aryloxy, Hetaryloxy, Sulfonyl, C₁-C₄-Thioalkyl, C₁-C₄-Carboxyalkyl,
wobei die Substituenten unabhängig voneinander ausgewählt sein können aus Halogen, C₁-C₄-Alkyl, C₁-C₄-Alkylcarbonyl, C₁-C₄-Halogenalkyl, C₁-C₄-Alkoxy, C₁-C₄-Halogenalkoxy, C₃-C₄-Cycloalkyl, C₃-C₄-Cycloalkylcarbonyl, Cyano, Nitro, (C₁-C₄-Alkoxy)carbonyl, C₁-C₄-Alkylthio, Di(C₁-C₄)alkylamino,
Y besonders bevorzugt für gegebenenfalls ein- oder mehrfach, gleich oder verschieden substituiertes C₁-C₄-Alkyl, C₃-C₄-Cycloalkyl, für einen gegebenenfalls ein- oder mehrfach, gleich oder verschieden substituierten, 5-6 gliederigen, 0-3 Stickstoffatome enthaltenden Ring steht, sowie für Naphthalin, Chinolin steht,
wobei die Substituenten ausgewählt sein können aus Halogen, Nitro, Cyano, aus gegebenenfalls einfach oder mehrfach, gleich oder verschieden substituiertem Amino, C₁-C₄-Alkyl, C₁-C₄-Alkoxy, C₁-C₄-Alkoxy-C₁-C₄-Alkyl, C₃-C₄-Cycloalkyl, Aryl, Hetaryl, C₁-C₄-Arylalkyl, C₁-C₄-Hetarylalkyl, Aryloxy, Hetaryloxy, Sulfonyl, C₁-C₄-Thioalkyl, C₁-C₄₋ Carboxyalkyl,
wobei die Substituenten unabhängig voneinander ausgewählt sein können aus Halogen, C₁-C₄-Alkyl, C₁-C₄-Halogenalkyl, C₁-C₄-Alkoxy, C₁-C₄-Halogenalkoxy, C₁-C₄-Alkylthio, C₃-C₄-Cycloalkyl, Di(C₁-C₄)alkylamino,
Y ganz besonders bevorzugt für gegebenenfalls ein- oder mehrfach, gleich oder verschieden substituiertes C₁-C₄-Alkyl, C₃-C₄-Cycloalkyl, für gegebenenfalls ein- oder mehrfach, gleich oder verschieden substituiertes Phenyl, Pyridyl, Pyrimidinyl, Pyrazyl, Triazolyl, Pyrrolidinyl, Morpholinyl, Piperidinyl steht,
wobei die Substituenten ausgewählt sein können aus Halogen, Nitro, Cyano, aus gegebenenfalls einfach oder mehrfach, gleich oder verschieden substituiertem C₁-C₄-Alkyl, C₁-C₄-Alkoxy, Phenyl, Triazol, Phenyloxy, Sulfonyl, C₁-C₄-Thioalkyl, C₁-C₄₋ Carboxyalkyl,
wobei die Substituenten unabhängig voneinander ausgewählt sein können aus Halogen, C₁-C₄-Alkyl, C₁-C₄-Halogenalkyl, C₁-C₄-Alkoxy, C₁-C₄-Halogenalkoxy, C₁-C₄-Alkylthio, C₃-C₄-Cycloalkyl, Dimethylamino,
- X: bevorzugt und besonders bevorzugt für CH oder N steht,
- X: ganz besonders bevorzugt für CH steht,
- X: ebenfalls ganz besonders bevorzugt für N steht,
- W: bevorzugt für O oder S steht,
- W: besonders bevorzugt und ganz besonders bevorzugt für O steht,

Die oben angegebenen einzelnen allgemeinen, bevorzugten, besonders bevorzugten und ganz besonders bevorzugten Bedeutungen für die Substituenten R¹-R⁵, X, W und A,Y,Q können beliebig miteinander kombiniert werden.

Gegenstand der vorliegenden Erfindung sind vorzugsweise auch die Lithium-, Natrium-, Kalium-, Magnesium-, Calcium-, Ammonium-, C₁-C₄-Alkyl-ammonium, Di-(C₁-C₄-alkyl)-ammonium-, Tri-(C₁-C₄-alkyl)-ammonium-, Tetra-(C₁-C₄-alkyl)-ammonium-, Tri-(C₁-C₄-alkyl)-sulfonium, C₅- oder C₆-Cycloalkyl-ammonium-, Di-(C₁-C₂-alkyl)-benzyl-ammonium und Tri-(C₁-C₂-alkyl)-benzyl-ammonium-Salze von Verbindungen der Formel (I), in welcher R¹ X, W, und A die oben allgemeinen, bevorzugten, besonders bevorzugten und insbesondere bevorzugten Bedeutungen aufweisen und die nach allgemein üblichen Verfahren hergestellt werden können.

Die vorliegenden Verbindungen der allgemeinen Formel (I) können gegebenenfalls ein chirales Kohlenstoffatom aufweisen.

Gemäß den Regeln nach Cahn, Ingold und Prelog (CIP-Regeln) können diese Substituenten sowohl eine (R)- als auch eine (S)-Konfiguration aufweisen.

Von der vorliegenden Erfindung werden Verbindungen der allgemeinen Formel (I) sowohl mit (S)-als auch mit (R)-Konfiguration an den jeweiligen chiralen Kohlenstoffatomen umfasst, d.h., dass die vorliegende Erfindung die Verbindungen der allgemeinen Formel (I) erfasst, in welchen die betreffenden Kohlenstoffatome jeweils unabhängig voneinander
(1) eine (R)-Konfiguration; oder
(2) eine (S)-Konfiguration
aufweisen. Wenn mehrere Chiralitätszentren in den Verbindungen der allgemeinen Formel (I) vorliegen, sind beliebige Kombinationen der Konfigurationen der Chiralitätszentren möglich, d.h. dass
(1) ein Chiralitätszentrum die (R)-Konfiguration und das andere Chiralitätszentrum die (S)-Konfiguration;
(2) ein Chiralitätszentrum die (R)-Konfiguration und das andere Chiralitätszentrum die (R)-Konfiguration; und
(3) ein Chiralitätszentrum die (S)-Konfiguration und das andere Chiralitätszentrum die (S)-Konfiguration
aufweist.

Erfindungsgemäß umfasst sind daher insbesondere die folgenden Verbindungen der Formeln (I-1), (1-2) und (1-3)
(I-1) RS -Verbindung
(1-2) RR-Verbindung
(1-3) SS-Verbindung

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:

Aus der Ordnung der Anoplura (Phthiraptera) z.B. Damalinia spp., Haematopinus spp., Linognathus spp., Pediculus spp., Trichodectes spp..

Aus der Klasse der Arachnida z.B. Acarus siro, Aceria sheldoni, Aculops spp., Aculus spp., Amblyomma spp., Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Hemitarsonemus spp., Hyalomma spp., Ixodes spp., Latrodectus mactans, Metatetranychus spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.

Aus der Klasse der Bivalva z.B. Dreissena spp..

Aus der Ordnung der Chilopoda z.B. Geophilus spp., Scutigera spp..

Aus der Ordnung der Coleoptera z.B. Acanthoscelides obtectus, Adoretus spp., Agelastica alni, Agriotes spp., Amphimallon solstitialis, Anobium punctatum, Anoplophora spp., Anthonomus spp., Anthrenus spp., Apogonia spp., Atomaria spp., Attagenus spp., Bruchidius obtectus, Bruchus spp., Ceuthorhynchus spp., Cleonus mendicus, Conoderus spp., Cosmopolites spp., Costelytra zealandica, Curculio spp., Cryptorhynchus lapathi, Dermestes spp., Diabrotica spp., Epilachna spp., Faustinus cubae, Gibbium psylloides, Heteronychus arator, Hylamorpha elegans, Hylotrupes bajulus, Hypera postica, Hypothenemus spp., Lachnosterna consanguinea, Leptinotarsa decemlineata, Lissorhoptrus oryzophilus, Lixus spp., Lyctus spp., Meligethes aeneus, Melolontha melolontha, Migdolus spp., Monochamus spp., Naupactus xanthographus, Niptus hololeucus, Oryctes rhinoceros, Oryzaephilus surinamensis, Otiorrhynchus sulcatus, Oxycetonia jucunda, Phaedon cochleariae, Phyllophaga spp., Popillia japonica, Premnotrypes spp., Psylliodes chrysocephala, Ptinus spp., Rhizobius ventralis, Rhizopertha dominica, Sitophilus spp., Sphenophorus spp., Sternechus spp., Symphyletes spp., Tenebrio molitor, Tribolium spp., Trogoderma spp., Tychius spp., Xylotrechus spp., Zabrus spp..

Aus der Ordnung der Collembola z.B. Onychiurus armatus.

Aus der Ordnung der Dermaptera z.B. Forficula auricularia.

Aus der Ordnung der Diplopoda z.B. Blaniulus guttulatus.

Aus der Ordnung der Diptera z.B. Aedes spp., Anopheles spp., Bibio hortulanus, Calliphora erythrocephala, Ceratitis capitata, Chrysomyia spp., Cochliomyia spp., Cordylobia anthropophaga, Culex spp., Cuterebra spp., Dacus oleae, Dermatobia hominis, Drosophila spp., Fannia spp., Gastrophilus spp., Hylemyia spp., Hyppobosca spp., Hypoderma spp., Liriomyza spp.. Lucilia spp., Musca spp., Nezara spp., Oestrus spp., Oscinella frit, Pegomyia hyoscyami, Phorbia spp., Stomoxys spp., Tabanus spp., Tannia spp., Tipula paludosa, Wohlfahrtia spp.

Aus der Klasse der Gastropoda z.B. Arion spp., Biomphalaria spp., Bulinus spp., Deroceras spp., Galba spp., Lymnaea spp., Oncomelania spp., Succinea spp..

Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp., Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Weiterhin lassen sich Protozoen, wie Eimeria, bekämpfen.

Aus der Ordnung der Heteroptera z.B. Anasa tristis, Antestiopsis spp., Blissus spp., Calocoris spp., Campylomma livida, Cavelerius spp., Cimex spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Nezara spp., Oebalus spp., Pentomidae, Piesma quadrata, Piezodorus spp., Psallus seriatus, Pseudacysta persea, Rhodnius spp., Sahlbergella singularis, Scotinophora spp., Stephanitis nashi, Tibraca spp., Triatoma spp.

Aus der Ordnung der Homoptera z.B. Acyrthosipon spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Doralis spp., Drosicha spp., Dysaphis spp., Dysmicoccus spp., Empoasca spp., Eriosoma spp., Erythroneura spp., Euscelis bilobatus, Geococcus coffeae, Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva fimbriolata, Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes vaporariorum, Trioza spp., Typhlocyba spp., Unaspis spp., Viteus vitifolii.

Aus der Ordnung der Hymenoptera z.B. Diprion spp., Hoplocampa spp., Lasius spp., Monomorium pharaonis, Vespa spp..

Aus der Ordnung der Isopoda z.B. Armadillidium vulgare, Oniscus asellus, Porcellio scaber.

Aus der Ordnung der Isoptera z.B. Reticulitermes spp., Odontotermes spp..

Aus der Ordnung der Lepidoptera z.B. Acronicta major, Aedia leucomelas, Agrotis spp., Alabama argillacea, Anticarsia spp., Barathra brassicae, Bucculatrix thurberiella, Bupalus piniarius, Cacoecia podana, Capua reticulana, Carpocapsa pomonella, Cheimatobia brumata, Chilo spp., Choristoneura fumiferana, Clysia ambiguella, Cnaphalocerus spp., Earias insulana, Ephestia kuehniella, Euproctis chrysorrhoea, Euxoa spp., Feltia spp., Galleria mellonella, Helicoverpa spp., Heliothis spp., Hofmannophila pseudospretella, Homona magnanima, Hyponomeuta padella, Laphygma spp., Lithocolletis blancardella, Lithophane antennata, Loxagrotis albicosta, Lymantria spp., Malacosoma neustria, Mamestra brassicae, Mocis repanda, Mythimna separata, Oria spp., Oulema oryzae, Panolis flammea, Pectinophora gossypiella, Phyllocnistis citrella, Pieris spp., Plutella xylostella, Prodenia spp., Pseudaletia spp., Pseudoplusia includens, Pyrausta nubilalis, Spodoptera spp., Thermesia gemmatalis, Tinea pellionella, Tineola bisselliella, Tortrix viridana, Trichoplusia spp..

Aus der Ordnung der Orthoptera z.B. Acheta domesticus, Blatta orientalis, Blattella germanica, Gryllotalpa spp., Leucophaea maderae, Locusta spp., Melanoplus spp., Periplaneta americana, Schistocerca gregaria.

Aus der Ordnung der Siphonaptera z.B. Ceratophyllus spp., Xenopsylla cheopis.

Aus der Ordnung der Symphyla z.B. Scutigerella immaculata.

Aus der Ordnung der Thysanoptera z.B. Baliothrips biformis, Enneothrips flavens, Frankliniella spp., Heliothrips spp., Hercinothrips femoralis, Kakothrips spp., Rhipiphorothrips cruentatus, Scirtothrips spp., Taeniothrips cardamoni, Thrips spp..

Aus der Ordnung der Thysanura z.B. Lepisma saccharina.

Zu den pflanzenparasitären Nematoden gehören z.B. Anguina spp., Aphelenchoides spp., Belonoaimus spp., Bursaphelenchus spp., Ditylenchus dipsaci, Globodera spp., Heliocotylenchus spp., Heterodera spp., Longidorus spp., Meloidogyne spp., Pratylenchus spp., Radopholus similis, Rotylenchus spp., Trichodorus spp., Tylenchorhynchus spp., Tylenchulus spp., Tylenchulus semipenetrans, Xiphinema spp..

Die Verbindungen der Formel (I) können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Die Herstellung der Formulierungen erfolgt entweder in geeigneten Anlagen oder auch vor oder während der Anwendung.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie N-Alkylpyrrolidone) und Lactone, der Sulfone und Sulfoxide (wie Dimethylsysulfoxid).

Im Falle der Benutzung von Wasser als Streckmittel können z.B. auch organische Lösemittel als Hilfslösungsmittel verwendet werden. Als flüssige Lösemittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Als feste Trägerstoffe kommen in Frage:
z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP- POE-Ester, AlkylAryl- und/oder POP- POE-Ether, Fett- und/oder POP- POE-Addukte, POE- und/oder POP-Polyol Derivate, POE- und/oder POP-Sorbitan- oder-Zucker-Addukte, Alky- oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel.

Die Formulierungen enthalten im Allgemeinen zwischen 0,01 und 98 Gew.-% Wirkstoff, vorzugsweise zwischen 0,5 und 90 %.

Der Wirkstoff kann in seinen handelsüblichen Formulierungen sowie in den aus diesen Formulierungen bereiteten Anwendungsformen in Mischung mit anderen Wirkstoffen wie Insektiziden, Lockstoffen, Sterilantien, Bakteriziden, Akariziden, Nematiziden, Fungiziden, wachstumsregulierenden Stoffen, Herbiziden, Safenern, Düngemitteln,Semiochemicals oder auch mit Mitteln zur Verbesserung der Pflanzeneigenschaften vorliegen.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepaßten üblichen Weise.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren, Angießen und bei Vermehrungsmaterial, insbesondere bei Saatgut, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CrylIA, CrylIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid-tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel I bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ekto- und Endoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acari (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:

Käfer wie Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;

Hautflügler wie Sirex juvencus, Urocerus gigas, Urocerus gigas taignus, Urocerus augur;

Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;

Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Die anwendungsfertigen Mittel können gegebenenfalls noch weitere Insektizide und gegebenenfalls noch ein oder mehrere Fungizide enthalten.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden. Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:

Aus der Ordnung der Scorpionidea z.B. Buthus occitanus.

Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.

Aus der Ordnung der Araneae z.B. Aviculariidae, Araneidae.

Aus der Ordnung der Opiliones z.B. Pseudoscorpiones chelifer, Pseudoscorpiones cheiridium, Opiliones phalangium.

Aus der Ordnung der Isopoda z.B. Oniscus asellus, Porcellio scaber.

Aus der Ordnung der Diplopoda z.B. Blaniulus guttulatus, Polydesmus spp..

Aus der Ordnung der Chilopoda z.B. Geophilus spp..

Aus der Ordnung der Zygentoma z.B. Ctenolepisma spp., Lepisma saccharina, Lepismodes inquilinus.

Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.

Aus der Ordnung der Saltatoria z.B. Acheta domesticus.

Aus der Ordnung der Dermaptera z.B. Forficula auricularia.

Aus der Ordnung der Isoptera z.B. Kalotermes spp., Reticulitermes spp.

Aus der Ordnung der Psocoptera z.B. Lepinatus spp., Liposcelis spp.

Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.

Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.

Aus der Ordnung der Lepidoptera z.B. Achroia grisella, Galleria mellonella, Plodia interpunctella, Tinea cloacella, Tinea pellionella, Tineola bisselliella.

Aus der Ordnung der Siphonaptera z.B. Ctenocephalides canis, Ctenocephalides felis, Pulex irritans, Tunga penetrans, Xenopsylla cheopis.

Aus der Ordnung der Hymenoptera z.B. Camponotus herculeanus, Lasius fuliginosus, Lasius niger, Lasius umbratus, Monomorium pharaonis, Paravespula spp., Tetramorium caespitum.

Aus der Ordnung der Anoplura z.B. Pediculus humanus capitis, Pediculus humanus corporis, Pemphigus spp., Phylloera vastatrix, Phthirus pubis.

Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neonicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

### Erläuterung der Verfahren und Zwischenprodukte

Beispielhaft und ergänzend wird die Herstellung von Verbindungen der Formel (I) in den folgenden Formelschemata erläutert. An dieser Stelle sei auch auf die Herstellungsbeispiele verwiesen.

Die erfindungsgemäßen Verbindungen der Formel (I) mit W = O und R¹ = H oder Methyl werden gemäß Formelschema 1 erhalten. Die erfindungsgemäßen Verbindungen der Formel (I) mit W = S können aus diesen durch Umsetzung mit einem Schwefelungsreagenz wie Lawessons Reagenz erhalten werden.

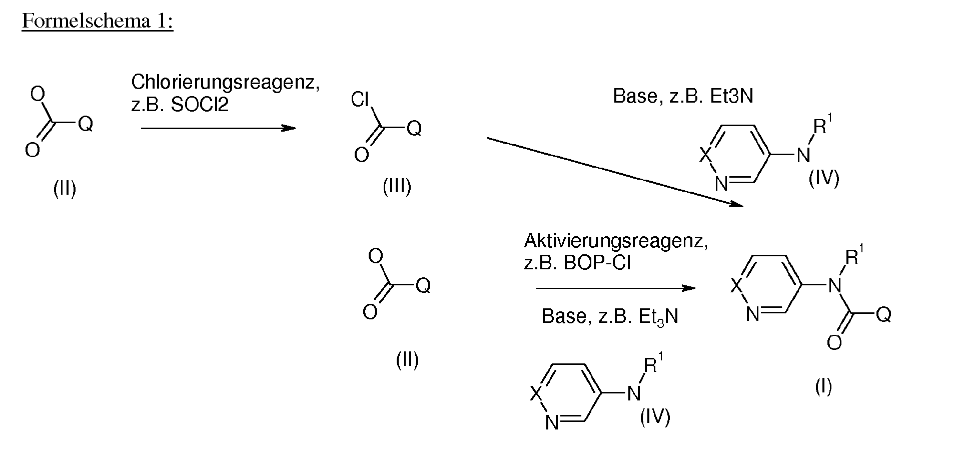

Die Carbonsäuren der Formel (II) werden entweder durch Umsetzung mit einem Chlorierungsreagenz wie beispielweise Thionylchlorid in ihre Säurechloride (III) überführt und danach mit den Aminen der Formel (IV) mit R¹ =H oder Methyl in Gegenwart einer Hilfsbase wie Triethylamin zu den erfindungsgemäßen Verbindungen der Formel (I) umgesetzt oder zunächst mit einem Aktivierungsreagenz wie BOP-C1 in Gegenwart einer Hilfsbase wie Triethylamin und danach mit den Aminen der Formel (IV) mit R¹ =H oder Methyl zu den erfindungsgemäßen Verbindungen der Formel (I) umgesetzt.

Die für die Herstellung der erfindungsgemäßen Verbindungen der Formel (I) benötigten Aminopyridine der Formel (IV) und Aminopyridazine mit R¹ = H oder Methyl sind beschrieben.

Siehe für 3-Aminopyridin Chemische Berichte 1936, 1534; für 4-Aminopyridazin Pharmaceutical Bulletin 4, 1956, 137, 497; für 4-Methylaminopyridazin Journal of the Chemical Society C, 1967, 23, 2473, für 3-Methylaminopyridin Journal of the Chemical Society 1957, 442.

Die erfindungsgemäßen Verbindungen der Formel (I) mit W = O und R¹ ≠ H oder Methyl werden gemäß Formelschema 2 erhalten.

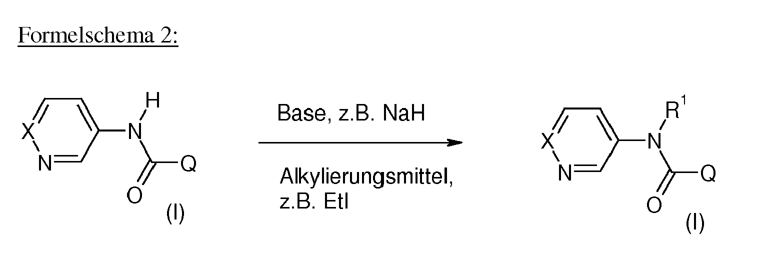

Die erfindungsgemäßen Verbindungen der Formel (I) mit W = O und R¹ ≠ H oder Methyl werden in Gegenwart einer Hilfsbase wie beispielsweise Natriumhydrid mit einem Alkylierungsmittel wie beispielsweise Ethyliodid umgesetzt.

Die erfindungsgemäßen Verbindungen der Formel (I) mit W = S können danach durch Umsetzung mit einem Schwefelungsreagenz wie Lawessons Reagenz erhalten werden.

Die für die Herstellung der erfindungsgemäßen Verbindungen der Formel (I) benötigten Carbonsäuren der Formel (II) lassen sich nach im Prinzip bekannten Methoden herstellen oder sind bekannt.

Für 3-Difluoromethyl-1-methyl-1*H*-pyrazol-4-carbonsäure siehe beispielsweise WO2005123690.

Für 1-Methyl-3-trifluoromethyl-1*H*-pyrazol-4-carbonsäure siehe beispielsweise US5438070.

Für 1-Ethyl-3-trifluoromethyl-1*H*-pyrazol-4-carbonsäure siehe beispielsweise US5480587.

Für 3-Cyclopropyl-1-methyl-1*H*-pyrazol-4-carbonsäure siehe beispielsweise JP03190862.

Für 3-Methoxy-1-methyl-1*H*-pyrazol-4-carbonsäure siehe beispielsweise US2007/196406.

Für 3-(Chloro-difluoro-methyl)-1-methyl-1*H*-pyrazol-4-carbonsäure siehe beispielsweise US5223526.

Für 5-Methyl-1-(2,2,2-trifluoro-ethyl)-1*H*-pyrazol-4-carbonsäure siehe beispielsweise EP1176140.

Für 1,3-Dimethyl-1*H*-pyrazol-4-carbonsäure siehe beispielsweise EP1176140.

Für 3-(4,6-Dimethoxy-pyrimidin-2-yloxy)-benzoesäure siehe beispielsweise WO2004100946.

Für 4-(4,6-Dimethyl-pyrimidin-2-yloxy)-benzoesäure siehe beispielsweise DE3602016.

Für 3-Bromo-4-methoxy-benzoesäure siehe beispielsweise Liebeigs Annalen, 1989, 863

Die besonders bevorzugten Carbonsäuren der Formel (II) mit Q8-1 (Formel (11-1), und Q8a-2 (Formel (II-2) lassen sich allgemein gemäß Formelschema 3 herstellen.

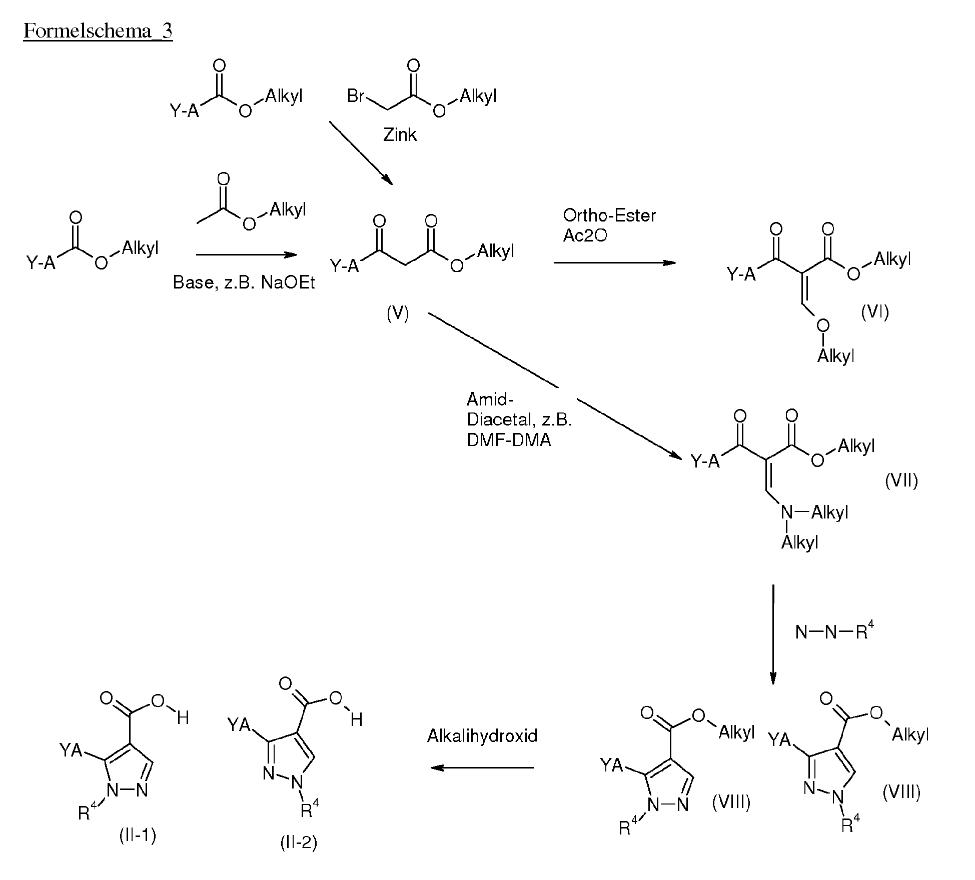

Die beta-Ketocarbonsäureester der Formel (V) werden entweder durch Umsetzung eines Carbonsäure- esters mit Alkylacetat in Gegenwart einer Hilfsbase wie Natriumethanolat, siehe dazu beispielsweise Journal of the American Chemical Society, 1953. 3152 oder Tetrahedron 2005, 2169 oder Tetrahedron Letters 2001. 2689, oder durch Bildung eines Zinkenolates aus einem Bromacetat und Umsetzung mit einem Carbonsäureester, siehe dazu Bioorganic and Medicinal Chemistry 2004, 233, oder Journal of the American Chemical Society 1954, 5391 erhalten. Die Ester der Formel (V) werden dann mit Orthoestern in Gegenwart von Essigsäureanhydrid zu den Enolethern der Formel (VI), siehe dazu Journal of the American Chemical Society 1951, 3684 oder mit Amiddiacetalen wie beispielsweise DMF-Dimethylacetal zu den Dialkylenaminen der Formel (VII), siehe dazu beispielsweise Bioorganic and Medicinal Chemistry Letters, 2005, 4370 umgesetzt. Aus den Verbindungen der Formel (VI) und (VII) werden dann durch Umsetzung mit Hydrazinen die Pyrazolcarbonsäureester der Formel (VIII) erhalten, die durch Umsetzung mit einer Base wie Alkalihydroxid in die Carbonsäuren der Formel (II) überführt werden können.

Die Thiazolcarbonsäuren der Formel (II-3) mit Q11-1 sind bekannt oder lassen sich nach im Prinzip bekannten Methoden gemäß Formelschema 4 herstellen.

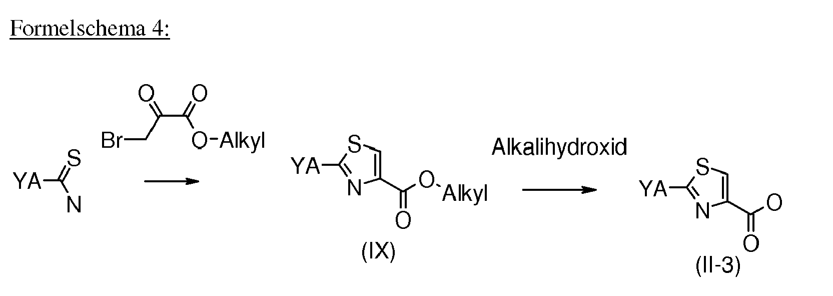

Durch Umsetzung von Thioamiden mit Brompyruvaten sind die Thiazolcarbonsäureester der Formel (IX) zu erhalten; die benötigten Thioamide lassen sich beispielsweise durch Umsetzung der entsprechenden Nitrile mit Ammoniumsulfid erhalten. Aus den Carbonsäureestern der Formel (IX) werden die Carbonsäuren der Formel (II-3) durch Umsetzung mit Alkalihydroxid erhalten.

Die erfindungsgemäßen Verbindungen der Formel (I) mit Q1 lassen sich ebenfalls durch Umsetzung von Verbindungen der Formel (I) mit einer reagierenden Gruppe F wie beispielsweise Chlor oder Brom entsprechend Formelschema 5 erhalten. Die benötigten Verbindungen der Formel (I) mit einer reagierenden Gruppe F lassen sich gemäß Formelschema 1 herstellen.

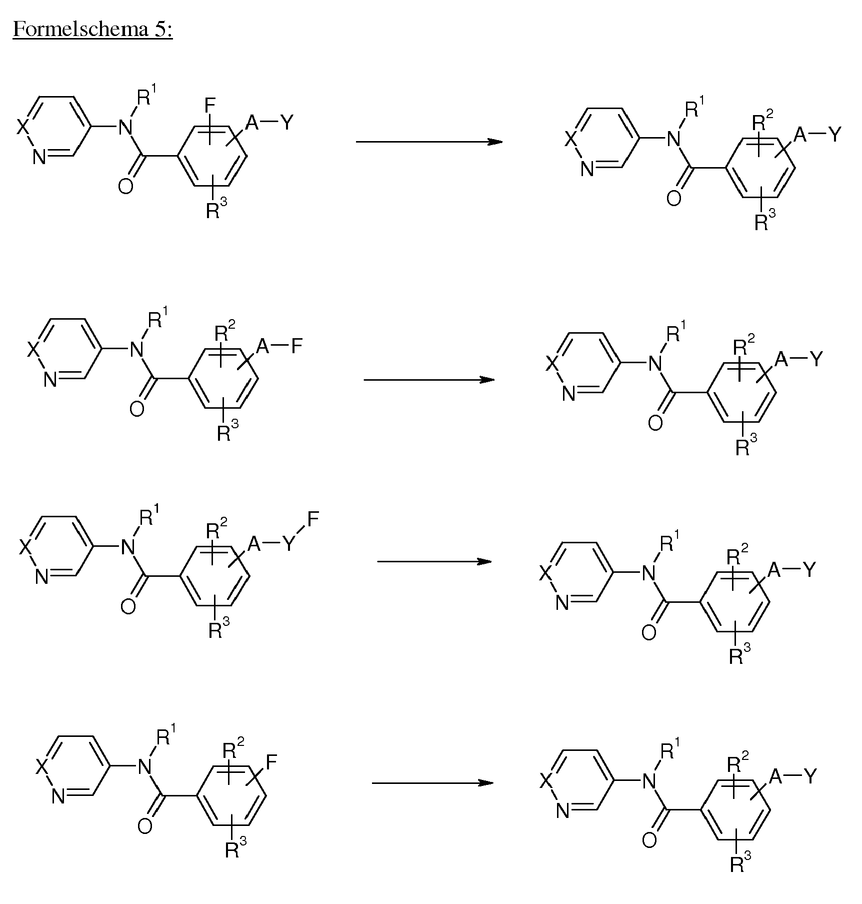

Die erfindungsgemäßen Verbindungen der Formel (I) mit Q2 lassen sich ebenfalls durch Umsetzung von Verbindungen der Formel (I) mit einer reagierenden Gruppe F wie beispielsweise Chlor oder Brom entsprechend Formelschema 6 erhalten. Die benötigten Verbindungen der Formel (I) mit einer reagierenden Gruppe F lassen sich gemäß Formelschema 1 herstellen.

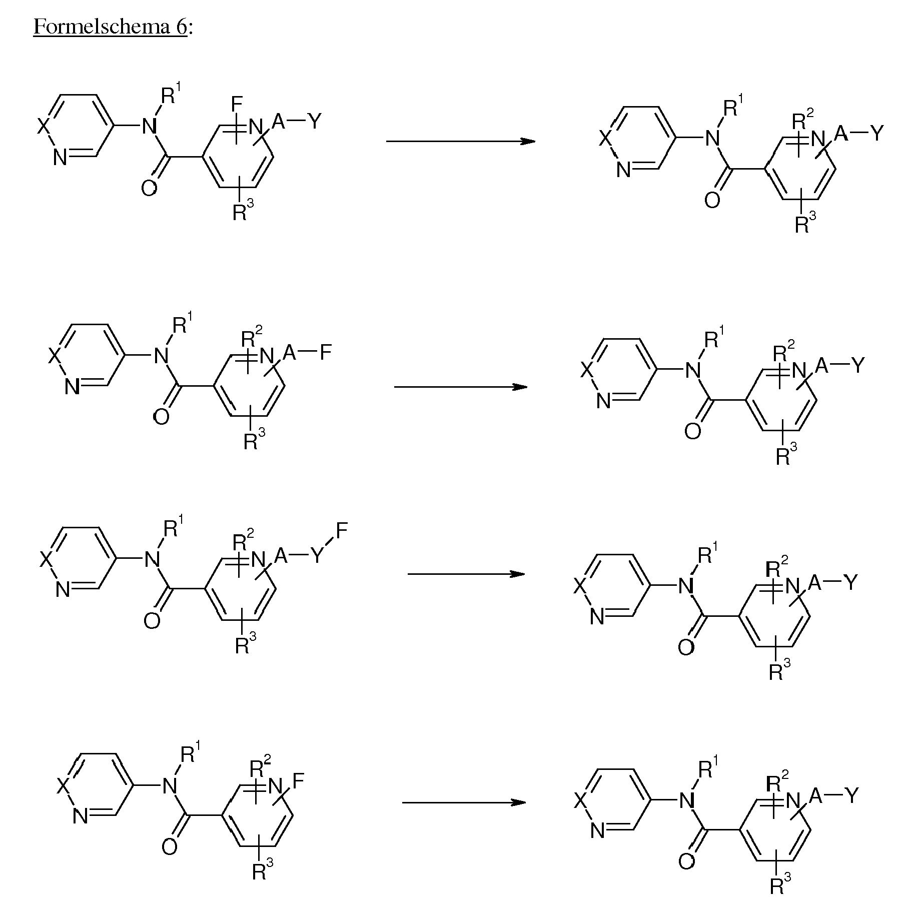

Zur genaueren Erklärung siehe die beispielhaften Umsetzungen in den Formelschemata 7, 8 und 9.

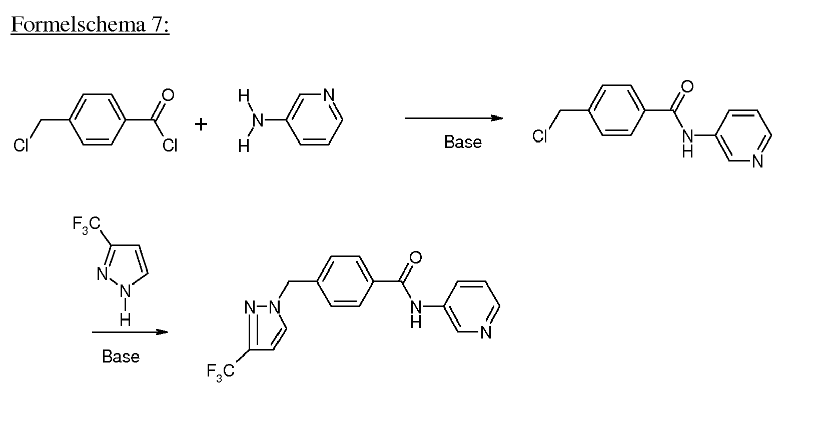

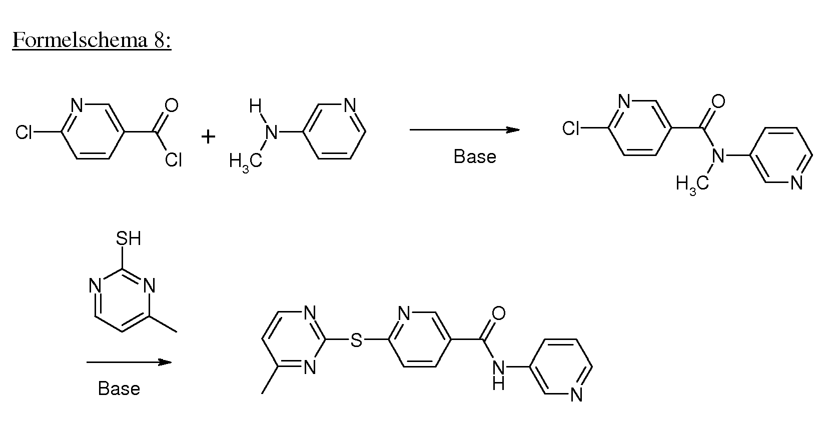

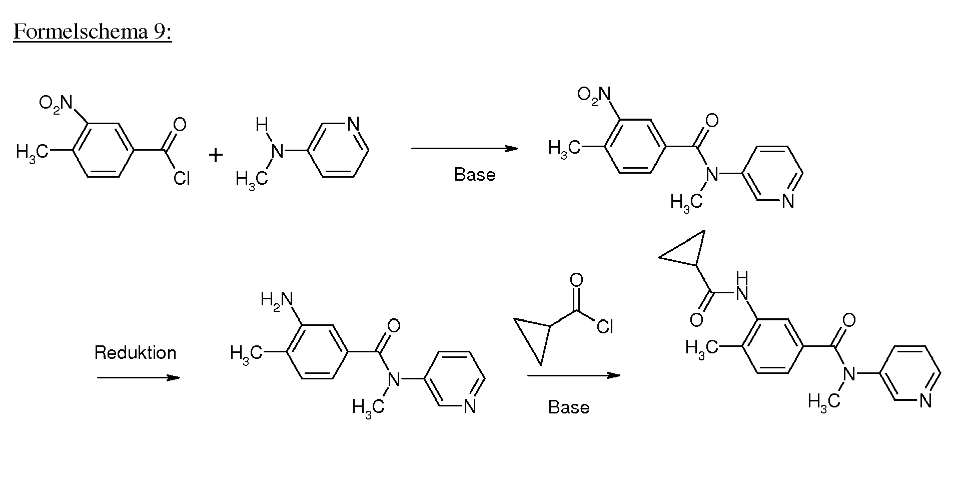

Die folgenden Herstellungs- und Verwendungsbeispiele illustrieren die Erfindung, ohne sie zu beschränken.

### Herstellungsbeispiele

### Beispiel 1

### Stufe 1:

### Methyl-pyridin-3-yl-amin

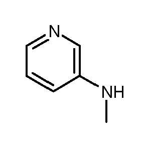

100,1g (633mmol) 3-Brompyridin wurden mit 160ml (1280mmol) Methylamin in Ethanol 8M, 4g (27,5mmol) 8-Hydroxychinolin, 2,2g (11,5mmol) Kupfer(I)iodid 16h bei 120° im Autoklaven gerührt. Man saugte das Gemisch über Sand/Kieselgel ab, engte ein, versetzte den Rückstand mit wäßriger Zitronensäure und Cyclohexan, sättigte die wäßrige Phase mit Natriumchlorid, versetzte mit verdünnter Natronlauge bis pH=10, extrahierte 6 mal mit Ethylacetat, trocknete die vereinigten organischen Phasen mit MgSO4 und dampfte ein. Der Rückstand wurde im Membranpumpenvakuum im Kugelrohr destilliert.

Ausbeute: 46,8g(63% d. Th), 1H-NMR (CD3CN) 2,75 (s, 3H), 4,3 (br, 1H), 6,85 (m, 1H), 7,05(m, 1H), 7,8 (m, 1H), 7,95 (m, 1H)

### Stufe 2

### 2-[1-Ethoxy-methyliden]-4,4,4-trifluoro-3-oxo-buttersäureethylester

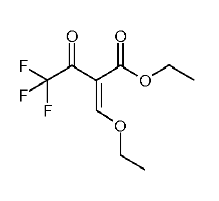

101g (548,5mmol) 4,4,4-Trifluoro-3-oxo-buttersäureethylester, 164g (1106mmol) Orthoameisensäuretriethylester, 114,1g (1118mmol) Essigsäureanhydrid wurden in einer Destillationsapparatur 1h bei 120°C gerührt, dann wurde die Temperatur innerhalb von 30 min auf 140°C erhöht. Nach teilweisem Abkühlen wurde im Membranpumpenvakuum destilliert.

Ausbeute: 103,6g (75% d. Th) 1H-NMR (CD3CN) 1,2-1,4 (m, 6H), 4,2-4,4 (m, 4H), 7,75-7,95 (1H)

### Stufe 3

### 1-Pyrimidin-2-yl-5-trifluoromethyl-1H-pyrazol-4-carbonsäureethylester

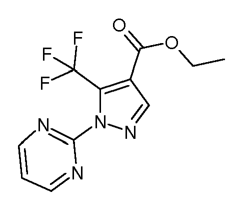

6,5g (27mmol) 2-[1-Ethoxy-methyliden]-4,4,4-trifluoro-3-oxo-buttersäureethylester wurden in Ethanol/Dioxan gelöst und bei Eisbadkühlung mit 2,99g(27,1mmol) 2-Hydrazino-pyrimidin versetzt. Man rührte 16h bei RT, dann wurde die Mischung noch 1.5h bei Rückfluß erhitzt, dann eingedampft und der Rückstand chromatographisch (cyclohexan/Aceton) gereinigt.

Ausbeute: 6,08g (77%d.Th) 1H-NMR (D6-DMSO) 1,3 (t, 3H), 4,35 (q, 2H), 7,85 (t, 1H), 8,4 (s, 1H), 9,1 (d, 2H)

### Stufe 4

### Pyrimidin-2-yl-5-trifluoromethyl-1H-pyrazol-4-carbonsäure

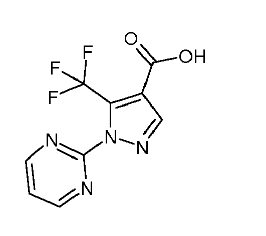

5,89g (20,5mmol) 1-Pyrimidin-2-yl-5-trifluoromethyl-1*H*-pyrazol-4-carbonsäureethylester wurden in 50 ml Tetrahydrofuran gelöst und mit Natronlauge versetzt.Bei Verdünnen mit 100 ml Ethanol bildet sich ein Niederschlag. Man versetzte mit wäßriger Zitronensäure, verdünnt mit Wasser und Ethylacetat, versetzte mit wäßrigem Natriumchlorid und extrahierte 4 mal mit Ethylacetat. Die vereinigten organischen Phasen wurden mit MgSO4 getrocknet und eingedampft.

Ausbeute: 5,07g (88% d. Th), 1H-NMR (D6-DMSO) 7,75 (m, 1H), 8,25 (m, 1H), 9,05 (m, 2H).

### Stufe 5

### 1-Pyrimidin-2-yl-5-trifluoromethyl-1H-pyrazole-4-carbonsäure- methyl-pyridin-3-yl-amid (1-268)

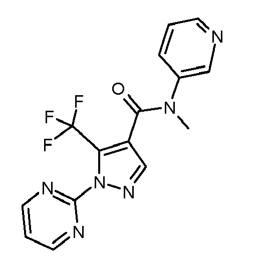

1g (3,8mmol) 1-Pyrimidin-2-yl-5-trifluoromethyl-1*H*-pyrazol-4-carbonsäure wurden in 40 ml Dichlormethan mit 1,5g (11,6mmol) Diisopropylethylamin und 1g (4mmol) BOP-C1 versetzt, 1h gerührt und dann mit 0,68g (5,8mmol) Methyl-pyridin-3-yl-amin versetzt und 16h gerührt. Man versetzte mit Wasser, die organische Phase wurde abgetrennt, getrocknet und eingedampft. Der Rückstand wurde chromatographisch (Cyclohexan/Aceton) gereinigt.

### Ausbeute: 1g (74% d. Th), 1H-NMR siehe Präparateliste

### Beispiel 2

### Stufe1:

### 3-Bromo-4-methoxy-N-methyl-N-pyridin-3-yl-benzamid

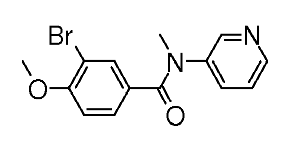

5g (22mmol) 3-Brom-4-methoxy-benzoesäure wurden in 50 ml Dichlormethan mit 8,3g (65mmol) Diidospropylethalamin gelöst und mit 5,8g (23mmol) BOP-C1 und dann mit 2,5g (24mmol) Methyl-pyridin-3-yl-amin versetzt und 2d gerührt. Die Mischung wurde eingeengt, der Rückstand mit Wasser, wäßrigem Natriumchlorid versetzt, dreimal mit Ethylacetat extrahiert, die vereinigten organischen Phasen getrocknet und eingedampft. Der Rückstand wurde chromatographisch an Kieselgel (Cyclohexan/Aceton) gereinigt.

Ausbeute: 5,49g (74%d. Th), 1H-NMR (D6-DMSO) 3,35 (s, 3H), 3,8 (s, 3H), 6,95 (d, 1H), 7,25 (m, 1H), 7,35 (m, 1H), 7,5 (m, 1H), 7,7 (m, 1H), 8,35 (m, 2H)

### Stufe 2

### 4-Methoxy-N-methyl-3-pyrazol-1-yl-N-pyridin-3-yl-benzamid (1-280)

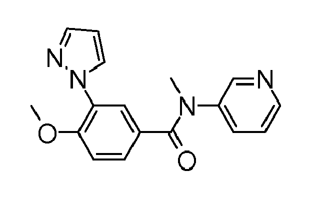

0,8g (2mmol) (3-Bromo-4-methoxy-*N*-methyl-*N*-pyridin-3-yl-benzamid, 1g (15mmol) Pyrazol, 2g (15mmol) Kaliumcarbonat, 38mg (0,2mmol) Kupfer(I)iodid, 36mg (0,24mmol) 8-Hydroxychinolin wurden in20 ml DMF mit 5 Tropfen Polyethylenglykol 400 15h bei 140° unter Argon gerührt, dann eingeengt. Der Rückstand wurde mit wäßriger Zitronensäure, Natronlauge, waßrigem Natriumchlorid versetzt, bei pH=9 mit Ethylacetat extrahiert. Die organische Phase wurde getrocknet und eingedampft. Der Rückstand wurd chormatographisch an Kieselgel (Cyclohexan/Aceton) gereinigt.

Ausbeute: 0,25g (32% d. Th) 1H-NMR siehe Präparateliste.

### Beispiel 3

### Stufe 1

### 4-(Chlormethyl)-N-(pyridin-3-yl)benzolcarboxamid

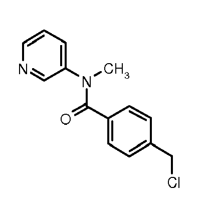

1.22g (6,4mmol) 4-Chloromethyl-benzoylchlorid in Dichlormethan wurde zu 0,7g (6,4mmol) Methyl-pyridin-3-yl-amin in Pyridin/Dichlormethan bei Eisbadkühlung gegeben. Man versetzte mit Wasser, Dichlormethan, die organische Phase wurde eingeengt. Das erhaltene Rohprodukt wurde direkt weiter umgesetzt.

### Stufe 2

### N-Methyl-N-(pyridin-3-yl)-4-{[3-(trifluormethyl)-1H-pyrazol-1-yl]methyl}benzolcarboxamid (1-27)

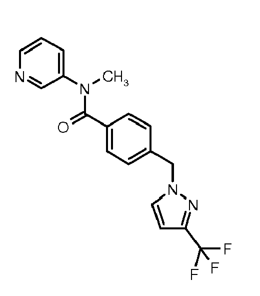

0,25g (0,9mmol) 4-(Chlormethyl)-N-(pyridin-3-yl)benzolcarboxamid und 3-Trifluormethylpyrazol wurden mit 0,17g(1,2mmol) Kaliumcarbonat in 3ml DMF 2h bei 65° gerührt, dann mit Wasser/MTBE versetzt. Die organische Phase wurde eingedampft und der Rückstand chromatographisch gereinigt.

1H-NMR siehe Präparateliste.

### Beispiel 4

### Stufe 1

### 6-Chlor-N-methyl-N-(pyridin-3-yl)pyridin-3-carboxamid

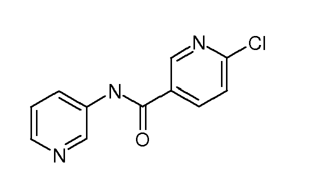

5g (31,7mmol) 6-Chloro-nicotinsäure wurden in 50 ml Dichlormethan mit einer katalytischen Menge Dimethylformamid und 10ml (38mmol) Oxalylchlorid versetzt, 2h gerührt und eingeengt. Der Rückstand wurde mit 50ml Dichlormethan versetzt und bei Eisbadkühlung mit 2,98g (31,75mmol) 3-Aminopyridin und 6,6ml Triethylamin versetzt. Man rührte bei RT, dann wurde mit Wasser versetzt, der entstandene Niederschlag abfiltriert, das Filtrat mit Dichlormethan extrahiert, die organische Phase getrocknet und eingedampft. Der Rückstand wurde an Kieselgel durch Chromatographie gereinigt.

Ausbeute: 1,62g (21%d. Th).

### Stufe 2

### 6-(3-Chloro-phenoxy)-N-pyridin-3-yl-nicotinamid (1-83)

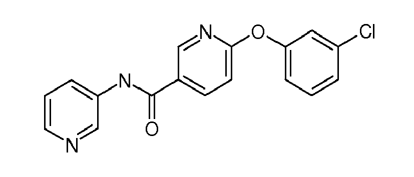

250mg (1,07mmol) 6-Chlor-N-methyl-N-(pyridin-3-yl)pyridin-3-carboxamid wurden mit 137,5mg (1,07mmol) 3-Chlorphenol und 177mg (1,28mmol) Kaliumcarbonat in 10ml DMF 3h bei 100°C gerührt, dann bei 120°C 4h stehen gelassen. Nach Abkühlen wurd mit Wasser, Ethylacetat versetzt, die organischen Phase getrocknet und eingedampft. Der Rückstand wurde mit Chromatographie an Kieselgel gereinigt.

Ausbeute: 230mg (65%d. Th). 1H-NMR siehe Präparateliste.

### Beispiel 5

### Stufe 1

### 4,N-Dimethyl-3-nitro-N-pyridin-3-yl-benzamid

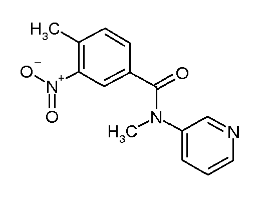

1,1g (5,51mmol) 4-Methyl-3-nitro-benzoylchlorid wurden in 10 ml Chloroform gelöst, bei Eisbadkühlung mit 715mg (6,6mmol) Methyl-pyridin-3-yl-amin in 10 ml Chloroform mit 1ml Triethylamin versetzt und 16h bei RT gerührt. Man verdünnte mit Chloroform, Wasser, die organische Phase wurde mit verdünnter Essigsäure und Wasser gewaschen, mit MgSO4 getrocknet und eingedampft.

Ausbeute: 1,6g (101% d. Th). 1H-NMR (D6-DMSO) 2,4 (s, 3H), 3,4 (s, 3H), 7,35 (m, 2H), 7,45 (d, 1H), 7,7 (d, 1H), 7,85 (s, 1H), 8,4 (m, 2H)

### Stufe 2

### 3-Amino-4,N-dimethyl-N-pyridin-3-yl-benzamid

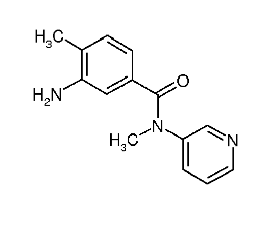

1,5g (5,2mmol) 4,*N*-Dimethyl-3-nitro-*N*-pyridin-3-yl-benzamid wurden in 70ml Ethanol gelöst und innerhalb von 2h mit 3,9g (20mmol) Zinn(II)chlorid in 65ml EtOH versetzt und 2h unter Rückfluß erwärmt. Die Mischung wurde eingedampft, mit Wasser und Natronlauge bis ph=9 versetzt, mit Ethylacetat extrahiert, die organische Phase mit MgSO4 getrocknet und eingedampft.

Ausbeute: 0,95g (71% d. Th). 1H-NMR (D6-DMSO): 2 (s, 3H), 3,3 (s, 3H), 4,7 (s, 2H), 6,25 (d, 1H), 6,65 (s, 1H), 6,75 (d, 1H), 7,25 (m, 1H), 7,6 (m, 1H), 8,3 (m, 2H)

### Stufe 3

### 3-Isobutyrylamino-4,N-dimethyl-N-pyridin-3-yl-benzamid (1-300)

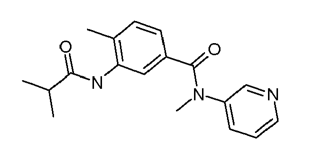

100mg (0,4mmol) 3-Amino-4,*N*-dimethyl-*N*-pyridin-3-yl-benzamid in 1 ml Chloroform mit 52mg (0,5mmol) Triethylamin wurden zu einer Lösung von 42mg (0,4mmol) Isobutansäurechlorid in 1.5ml Chloroform (1mmol) EDC in 2ml Dioxan gelöst bei Eisbadkühlung gegeben. Man rührt 16h bei RT und noch 16h bei 50°C. Die Mischung wurde mit Chloroform und Wasser verdünnt, die organische Phase mit MgSO4 getrocknet und eingedampft.

Ausbeute: 89mg (64%d. Th) 1H-NMR (D&-DMSO) 1,05 (m, 6H), 2,2 (s, 3H), 2,6 (sep, 1H), 3,35 (s, 3H), 6,9 (d, 1H), 7,05 (d, 1H), 7,3 (m, 1H), 7,4 (s, 1H), 7,65 (m, 1H), 8,3 (m, 2H), 8,9 (s, 1H)

Die in der Tabelle 1 beschriebenen Verbindungen der allgemeinen Formel (I) erhält man gemäß oder analog zu den oben beschriebenen Synthesebeispielen: 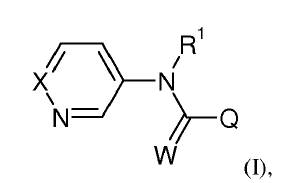 wobei die Reste R¹, Q, X und W die in der Tabelle 1 angegebenen Bedeutungen aufweisen

**Tabelle 1:**

| (I-) | Structure | MS | 1H-NMR |
|---|---|---|---|
| 1 | 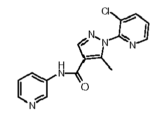 | | (D6-DMSO) δ:9.95 ppm (NH); 8.88 (d,1 H); 8.63-8.62 (m,1 H); 8.33 (s,1 H); 8.29-8.26 (m,2 H); 8.14-8.11 (m,1 H); 7.71-7.68 (m,1 H); 7.37-7.34 (m,1 H); 2.41 (s,CH3). |
| 2 | 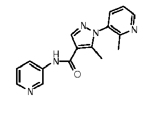 | | (D6-DMSO) δ:9.91 ppm (NH); 8.63-8.62 (m,1 H); 8.12 (s,1 H); 7.98 (s,1 H); 7.80-7.77 (m,2 H); 7.44-7.41 (m,2 H); 2.29 (s,CH3); 2.18 (s,CH3). |
| 3 | 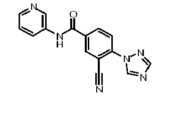 | | (D6-DMSO) δ: 7.41-7.44 (1H, m), 8.08-8.11 (1H, m), 8.18-8.20 (1H, m), 8.34-8.35 (1H, m), 8.42-8.43 (2H, m), 8.65 (1H, d, J = 1.5 Hz), 8.93 (1H, d, J = 2.0 Hz), 9.31 (1H, s), 10.71 (1H, s). |
| 4 | 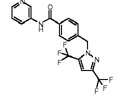 | | (CDCl3) δ: 5.55 (2H, s), 6.95 (1H, s), 7.29-7.34 (3H, m), 7.86-7.89 (2H, m), 7.99-8.02 (1H, m), 8.26-8.29 (1H, m), 8.38-8.40 (1H, m), 8.65-8.66 (1H, m). |
| 5 | 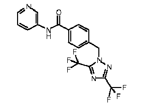 | | (CDCl3) δ: 5.60 (2H, s), 7.31-7.34 (1H, m), 7.43-7.45 (2H, m), 7.91-7.93 (2H, m), 8.12-8.15 (1H, m), 8.25-8.28 (1H, m), 8.37-8.39 (1H, m), 8.66-8.67 (1H, m). |
| 6 | 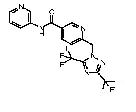 | | (CDCl3) δ: 5.77 (2H, s), 7.33-7.36 (2H, m), 8.25-8.28 (2H, m), 8.38-8.40 (2H, m), 8.68 (1H, d, J = 2.4 Hz), 9.04 (1H, s). |
| 7 | 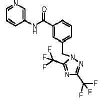 | | (CDCl3) δ: 5.59 (2H, s), 7.16-7.34 (3H, m), 7.52-7.53 (2H, m), 7.90-7.93 (2H, m), 8.25-8.28 (1H, m), 8.36-8.39 (2H, m), 8.67-8.67 (1H, m). |
| 8 | 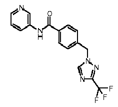 | | (CDCl3) δ: 5.45 (2H, s), 7.39-7.41 (3H, m), 7.88-8.03 (3H, m), 8.16 (1H, s), 8.26-8.29 (1H, m), 8.39-8.41 (1H, m), 8.68 (1H). |
| 9 | 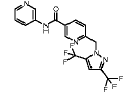 | | (CDCl3) δ: 5.67 (2H, d, J = 23.1 Hz), 6.96-7.00 (1H, m), 7.06-7.09 (1H, m), 7.32-7.35 (1H, m), 8.21-8.26 (2H, m), 8.38-8.39 (1H, m), 8.46-8.49 (1H, m), 8.67-8.68 (1H, m), 9.05-9.06 (1H, m). |
| 10 | 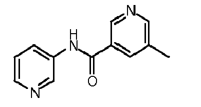 | | (CDCl3) δ: 2.38 (3H, s), 7.27-7.29 (1H), 8.03 (1H), 8.29-8.30 (2H), 8.52 (1H), 8.71 (1H), 8.91 (1H). |
| 11 | 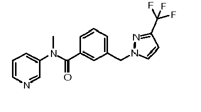 | | (CDCl3) δ: 3.50 (5H, s), 5.22 (3H, s), 6.51 (2H, d, J = 2.0 Hz), 7.17-7.30 (13H, m), 8.25 (1H, d, J = 2.2 Hz), 8.35-8.37 (1H, m). |
| 12 | 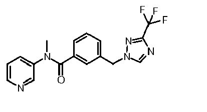 | | (CDCl3) δ: 3.56 (3H, t, J = 2.4 Hz), 4.47 (2H, t, J = 2.4 Hz), 7.22-7.43 (6H, m), 8.39-8.40 (1H, m), 8.43-8.44 (1H, m). |
| 13 | 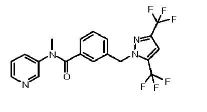 | | (CDCl3) δ: 3.50 (3H, s), 5.33 (2H, s), 6.90 (1H, s), 7.17-7.25 (6H, m), 8.27-8.28 (1H, m), 8.34-8.35 (1H, m). |
| 14 | 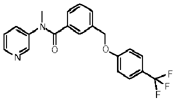 | | (CDCl3) δ: 3.52 (3H, s), 4.99 (2H, s), 6.90-6.93 (2H, m), 7.13-7.16 (1H, m), 7.23-7.27 (2H, m), 7.31-7.38 (3H, m), 7.51-7.54 (2H, m), 8.33-8.35 (2H, m). |
| 15 | 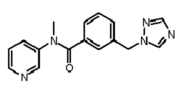 | | (CDCl3) δ: 3.50 (3H, s), 5.22 (2H, s), 7.14-7.33 (6H, m), 7.88 (1H, s), 7.94 (1H, s), 8.29 (1H, d, J = 2.4 Hz), 8.39 (1H, dd, J = 4.8, 1.5 Hz). |
| 16 | 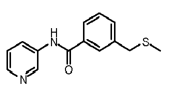 | | (CDCl3) δ: 1.98 (3H, s), 3.72 (2H, s), 7.30-7.33 (1H, m), 7.45-7.50 (2H, m), 7.76-7.80 (1H, m), 7.84-7.85 (1H, m), 8.29-8.36 (2H, m), 8.47 (1H, s), 8.68-8.69 (1H, m). |
| 17 | 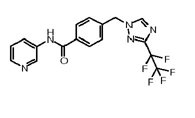 | | (CDCl3) δ: 5.50 (2H, s), 7.33-7.36 (1H, m), 7.43 (2H, d, J = 8.4 Hz), 7.93 (3H, d, J = 8.4 Hz), 8.21 (1H, s), 8.26-8.29 (1H, m), 8.40-8.42 (1H, m), 8.67-8.68 (1H, m). |
| 18 | 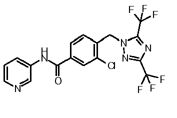 | | (CDCl3) δ: 5.73 (2H, s), 7.08-7.11 (1H, m), 7.32-7.34 (1H, m), 7.78-7.81 (1H, m), 8.01-8.01 (1H, m), 8.24-8.27 (1H, m), 8.37-8.39 (1H, m), 8.46 (1H, br s), 8.67-8.68 (1H, m). |
| 19 | 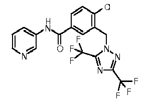 | | (CDCl3) δ: 5.72 (2H, s), 7.33-7.36 (1H, m), 7.61 (1H, d, J = 8.2 Hz), 7.74-7.75 (1H, m), 7.85 (1H, d, J = 8.3 Hz), 7.92 (1H, s), 8.22-8.25 (1H, m), 8.41-8.42 (1H, m), 8.65-8.66 (1H, m). |
| 20 | 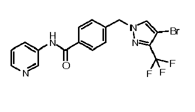 | | (CDCl3) δ: 5.37 (2H, s), 7.30-7.36 (3H, m), 7.49 (1H, s), 7.87-7.91 (2H, m), 8.23-8.25 (2H, m), 8.37-8.39 (1H, m), 8.67-8.68 (1H, m). |
| 21 | 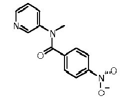 | | (CDCl3) δ: 3.53 (3H, s), 7.25 (1H, dd), 7.44 (2H, d), 8.07 (2H, d), 8.33 (1H, d), 8.43 (1H, dd). |
| 22 | 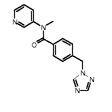 | | (CDCl3) δ: 3.51 (3H, s), 5.28 (2H, s), 7.06-7.09 (2H, m), 7.19-7.23 (1H, m), 7.27-7.31 (2H, m), 7.37-7.38 (1H, m), 7.96 (1H, s), 8.01 (1H, s), 8.32-8.33 (1H, m), 8.39-8.41 (1H, m). |
| 23 | 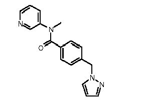 | | (CDCl3) δ: 3.51 (3H, s), 5.26 (2H, s), 6.27-6.27 (1H, m), 6.99 (2H, d, J = 8.4 Hz), 7.17-7.39 (6H, m), 7.52-7.53 (1H, m), 8.33-8.34 (1H, m), 8.38-8.39 (1H, m). |
| 24 | 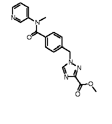 | | (CDCl3) δ: 3.51 (3H, s), 3.99 (3H, s), 5.36 (2H, s), 7.10-7.13 (2H, m), 7.20-7.23 (1H, m), 7.30-7.31 (2H, m), 7.38-7.38 (1H, m), 8.08 (1H, s), 8.32-8.33 (1H, m), 8.39-8.41 (1H, m). |
| 25 | 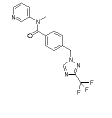 | | (CDCl3) δ: 3.52 (3H, s), 5.32 (2H, s), 7.11-7.14 (2H, m), 7.21-7.24 (1H, m), 7.32-7.38 (3H, m), 8.04 (1H, s), 8.32-8.33 (1H, m), 8.40-8.41 (1H, m). |
| 26 | 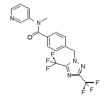 | | (CDCl3) δ: 3.51 (3H, s), 5.45 (2H, s), 7.14-7.36 (6H, m), 8.33-8.34 (1H, m), 8.39-8.40 (1H, m). |
| 27 | 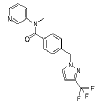 | | (CDCl3) δ: 3.51 (3H, s), 5.28 (2H, s), 6.53 (1H, t), 7.04 (2H, d), 7.18-7.33 (4H, m), 7.38-7.38 (1H, m), 8.32 (1H, d), 8.39 (1H, dd). |
| 28 | 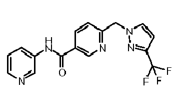 | | (CDCl3) δ: 5.46 (2H, s), 6.56 (1H, s), 6.95-7.05 (2H, m), 7.59 (1H, s), 7.90-7.94 (1H, m), 8.15 (2H, dd), 8.29 (1H, d), 8.63 (1H, s), 9.01 (1H, s), 9.43 (1H, s). |
| 29 | 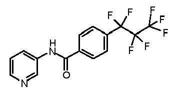 | | (CDCl3) δ: 7.28-7.31 (1H, m), 7.67 (2H, d, J = 8.4 Hz), 8.02 (2H, d, J = 8.6 Hz), 8.26-8.31 (2H, m), 8.70-8.70 (1H, m), 9.13 (1H, s). |
| 30 | 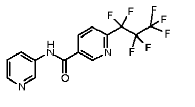 | | (CDCl3) δ: 7.35-7.37 (1H, m), 7.83-7.85 (1H, m), 8.27-8.30 (1H, m), 8.44-8.45 (2H, m), 8.67-8.72 (2H, m), 9.25 (1H, s). |
| 31 | 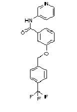 | | (CDCl3) δ: 5.22 (2H, s), 7.20-7.21 (1H, m), 7.34-7.37 (1H, m), 7.45-7.47 (2H, m), 7.56-7.60 (3H, m), 7.67-7.70 (2H, m), 8.30-8.33 (1H, m), 8.42-8.44 (1H, m), 8.69-8.70 (1H, m). |
| 32 | 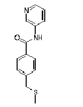 | | (CDCl3) δ: 2.01 (3H, s), 3.73 (2H, s), 7.32-7.35 (1H, m), 7.44-7.47 (2H, m), 7.84-7.87 (3H, m), 8.29-8.33 (1H, m), 8.39-8.41 (1H, m), 8.67-8.67 (1H, m). |
| 33 | 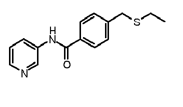 | | (CDCl3) δ: 1.24 (3H, t), 2.44 (2H, q ), 3.78 (2H, s), 7.32-7.35 (1H, m), 7.44-7.47 (2H, m), 7.86-7.90 (3H, m), 8.29-8.32 (1H, m), 8.39-8.40 (1H, m), 8.66-8.67 (1H, m). |
| 34 | 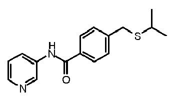 | | (CDCl3) δ: 1.24-1.26 (6H, m), 2.77-2.79 (1H, m), 3.76 (2H, s), 7.26-7.28 (1H, m), 7.39-7.42 (2H, m), 7.82-7.85 (2H, m), 8.25-8.31 (3H, m), 8.66-8.67 (1H, m), 8.76 (1H, s). |
| 35 | 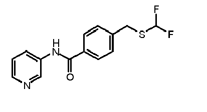 | | (CDCl3) δ: 4.05 (2H, s), 6.75 (1H, t, J = 56.1 Hz), 7.26-7.29 (1H, m), 7.41-7.44 (2H, m), 7.84-7.87 (2H, m), 8.25-8.30 (2H, m), 8.66-8.67 (1H, m), 8.74-8.79 (1H, m). |
| 36 | 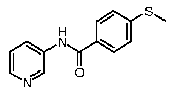 | | (CDCl3) δ: 2.54 (3H, s), 7.30-7.33 (3H, m), 7.79-7.83 (2H, m), 8.00 (1H, s), 8.28-8.30 (1H, m), 8.37-8.39 (1H, m), 8.66 (1H, d, J = 2.6 Hz). |
| 37 | 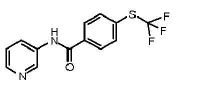 | | (CDCl3) δ: 7.33-7.36 (1H, m), 7.78-7.80 (2H, m), 7.92-7.95 (2H, m), 8.06 (1H, s), 8.27-8.30 (1H, m), 8.40-8.42 (1H, m), 8.68-8.68 (1H, m). |
| 38 | 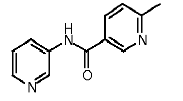 | | (CDCl3) δ: 2.66 (3H, s), 7.26-7.37 (2H, m), 8.08-8.14 (2H, m), 8.27-8.29 (1H, m), 8.41-8.42 (1H, m), 8.70 (1H, d, J = 2.6 Hz), 9.01 (1H, d, J = 2.2 Hz). |
| 39 | 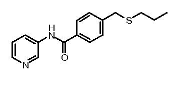 | | (CDCl3) δ: 0.95-0.98 (3H, m), 1.56-1.63 (2H, m), 2.40 (2H, t, J = 7.2 Hz), 3.76 (2H, s), 7.32-7.35 (1H, m), 7.46 (2H, d, J = 8.4 Hz), 7.84-7.85 (3H, m), 8.29-8.33 (1H, m), 8.39-8.41 (1H, m), 8.67-8.67 (1H, m). |
| 40 | 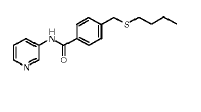 | | (CDCl3) δ: 0.89 (3H, t, J = 7.2 Hz), 1.35-1.40 (2H, m), 1.54-1.56 (2H, m), 2.42 (2H, t, J = 7.3 Hz), 3.75 (2H, s), 7.31-7.34 (1H, m), 7.45 (2H, d, J = 8.4 Hz), 7.84 (2H, d, J = 8.4 Hz), 8.04 (1H, s), 8.29-8.32 (1H, m), 8.38-8.39 (1H, m), 8.66-8.67 (1H, m). |
| 41 | 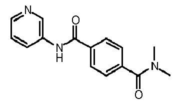 | | (CDCl3) δ: 2.94 (3H, s), 3.15 (3H, s), 7.29-7.32 (5H, m), 7.81-7.83 (2H, m), 8.39-8.42 (2H, m), 8.87-8.88 (1H, m), 9.39 (1H, s). |
| 42 | 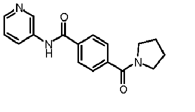 | | (CDCl3) δ: 1.91-1.98 (4H, m), 3.35-3.38 (2H, m), 3.66-3.68 (2H, m), 7.32-7.35 (1H, m), 7.41-7.42 (2H, m), 7.84-7.86 (2H, m), 8.39-8.44 (2H, m), 8.88-8.89 (1H, m), 9.12 (1H, s). |
| 43 | 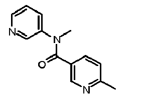 | | (CDCl3) δ: 2.49 (3H, s), 3.53 (3H, s), 7.01-7.04 (1H, m), 7.23-7.26 (2H, m), 7.43-7.46 (1H, m), 7.52-7.55 (1H, m), 8.34-8.36 (2H, m), 8.42-8.44 (1H, m). |
| 44 | 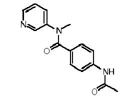 | | (CDCl3) δ: 2.13 (3H, s), 3.52 (3H, s), 7.20-7.25 (3H, m), 7.33-7.41 (3H, m), 7.59 (1H, br s), 8.33-8.33 (1H, m), 8.38-8.40 (1H, m). |
| 45 | 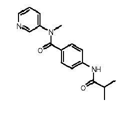 | | (CDCl3) δ: 1.22 (6H, d, J = 7.0 Hz), 2.46 (1H, d, J = 7.0 Hz), 3.51 (3H, s), 7.21-7.26 (4H, m), 7.37-7.40 (3H, m), 8.34 (1H, d, J = 2.0 Hz), 8.38-8.39 (1H, m). |
| 46 | 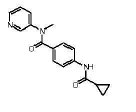 | | (CDCl3) δ: 0.81-0.86 (2H, m), 1.04-1.07 (2H, m), 1.46-1.48 (1H, m), 3.51 (3H, s), 7.19-7.25 (3H, m), 7.34-7.40 (3H, m), 7.66 (1H, s), 8.34 (1H, d, J = 2.0 Hz), 8.38-8.39 (1H, m). |
| 47 | 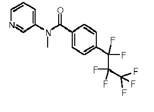 | | (CDCl3) δ: 3.54 (3H, s), 7.20-7.22 (1H, m), 7.38-7.43 (5H, m), 8.37 (1H, d, J = 2.4 Hz), 8.42-8.43 (1H, m). |
| 48 | 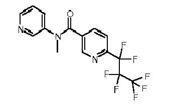 | | (CDCl3) δ: 3.56 (3H, s), 7.26-7.28 (2H, m), 7.41-7.44 (1H, m), 7.57-7.60 (1H, m), 7.84-7.86 (1H, m), 8.36 (1H, d, J = 2.2 Hz), 8.47-8.49 (1H, m), 8.58 (1H, s). |
| 49 | 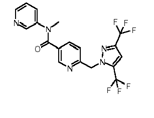 | | (CDCl3) δ: 3.52 (3H, s), 5.56 (2H, s), 6.81 (1H, d, J = 8.1 Hz), 6.95 (1H, s), 7.23-7.25 (1H, m), 7.37-7.38 (1H, m), 7.61-7.63 (1H, m), 8.36 (1H, d, J = 2.4 Hz), 8.43-8.45 (2H, m). |
| 50 | 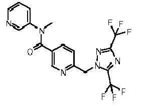 | | (CDCl3) δ: 3.53 (3H, s), 5.61 (2H, s), 7.05-7.08 (1H, m), 7.24-7.26 (1H, m), 7.40-7.42 (1H, m), 7.66-7.69 (1H, m), 8.33-8.34 (1H, m), 8.40-8.41 (1H, m), 8.45-8.46 (1H, m). |
| 51 | 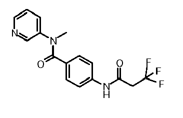 | | (CDCl3) δ: 3.20 (2H, q, J = 10.4 Hz), 3.50 (3H, s), 7.14 (2H, d, J = 8.8 Hz), 7.24-7.28 (3H, m), 7.44-7.47 (1H, m), 8.28 (1H, d, J = 2.4 Hz), 8.40 (1H, dd, J = 4.8, 1.3 Hz), 9.15 (1H, s). |
| 52 | 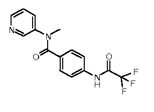 | | (CDCl3) δ: 3.53 (3H, s), 7.21-7.33 (5H, m), 7.40-7.44 (3H, m), 8.13 (1H, s), 8.32 (1H, d, J = 2.2 Hz), 8.41 (1H, dd, J = 4.7, 1.6 Hz). |
| 53 | 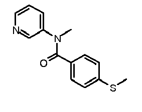 | | (CDCl3) δ: 2.41 (3H, s), 3.50 (3H, s), 7.01-7.04 (2H, m), 7.18-7.23 (3H, m), 7.37-7.38 (1H, m), 8.36-8.39 (2H, m). |
| 54 | 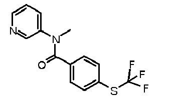 | | (CDCl3) δ: 3.53 (3H, s), 7.19-7.23 (1H, m), 7.33-7.35 (3H, m), 7.50 (2H, d, J = 8.2 Hz), 8.36 (1H, d, J = 2.4 Hz), 8.42 (1H, dd, J = 4.8, 1.5 Hz). |
| 55 | 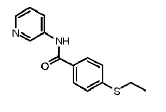 | | (CDCl3) δ: 1.38 (3H, t, J = 14.8 Hz), 3.04 (2H, q, J = 7.4 Hz), 7.31-7.39 (3H, m), 7.79-7.83 (3H, m), 8.28-8.31 (1H, m), 8.39 (1H, dd, J = 4.8, 1.5 Hz), 8.66 (1H, d, J = 2.7 Hz). |
| 56 | 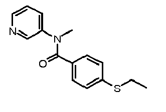 | | (CDCl3) δ: 1.28 (3H, t), 2.90 (2H, q), 3.51 (3H, s), 7.07-7.09 (2H, m), 7.18-7.21 (3H, m), 7.37-7.39 (1H, m), 8.36-8.39 (2H, m). |
| 57 | 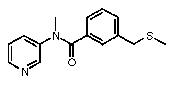 | | (CDCl3) δ: 1.77 (3H, s), 3.51 (2H, s), 3.52 (3H, s), 7.17-7.24 (5H, m), 7.40-7.42 (1H, m), 8.34-8.37 (2H, m). |
| 58 | 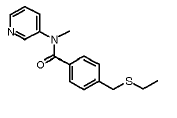 | | (CDCl3) δ: 1.16 (3H, t), 2.33 (2H, q), 3.51 (3H, s), 3.62 (2H, s), 7.14-7.28 (5H, m), 7.38 (1H, dq), 8.35-8.38 (2H, m). |
| 59 | 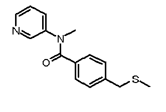 | | (CDCl3) δ: 1.89 (3H, s), 3.51 (3H, s), 3.57 (2H, s), 7.19-7.23 (5H, m), 7.38 (1H, dt, J = 5.8, 2.2 Hz), 8.35-8.38 (2H, m). |
| 60 | 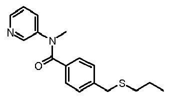 | | (CDCl3) δ: 0.91 (3H, t), 1.49-1.52 (2H, m), 2.28 (2H, t), 3.52 (3H, s), 3.60 (2H, s), 7.14-7.26 (5H, m), 7.35-7.39 (1H, m), 8.36-8.38 (2H, m). |
| 61 | 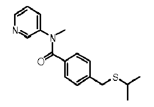 | | (CDCl3) δ: 1.19 (6H, dd), 2.66-2.69 (1H, m), 3.52 (3H, s), 3.65 (2H, s), 7.18-7.25 (7H, m), 7.35-7.39 (1H, m), 8.36-8.38 (2H, m). |
| 62 | 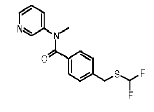 | | (CDCl3) δ: 3.52 (3H, s), 3.92 (2H, s), 6.67 (1H, t), 7.20-7.26 (5H, m), 7.36 (1H, q), 8.35 (1H, d), 8.40 (1H, dd). |
| 63 | 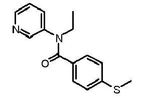 | | (CDCl3) δ: 1.23 (3H, t), 2.42 (3H, s), 3.99 (2H, q), 7.02 (2H, d), 7.18-7.22 (3H, m), 7.35-7.37 (1H, m), 8.34 (1H, d), 8.41 (1H, dd). |
| 64 | 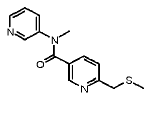 | | (CDCl3) δ: 1.96 (2H, s), 3.53 (3H, s), 3.71 (2H, s), 7.24-7.27 (2H, m), 7.43 (1H, dt), 7.62 (1H, dd), 8.34 (1H, d), 8.39 (1H, d), 8.44 (1H, dd). |
| 65 | 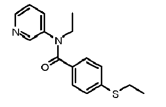 | | (CDCl3) δ: 1.21-1.30 (6H, m), 2.90 (2H, q, J = 7.4 Hz), 3.98 (2H, q, J = 7.1 Hz), 7.07 (2H, d, J = 4.2 Hz), 7.17-7.22 (3H, m), 7.34-7.37 (1H, m), 8.34 (1H, d, J = 2.6 Hz), 8.40 (1H, dd, J = 4.8, 1.5 Hz). |
| 66 | 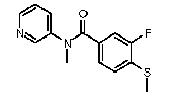 | | (CDCl3) δ: 2.42 (3H, s), 3.51 (3H, s), 7.01 (2H, d), 7.23-7.25 (2H, m), 7.39-7.42 (1H, m), 8.35 (1H, d), 8.43 (1H, dd). |
| 67 | 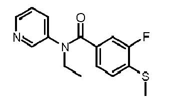 | | (CDCl3) δ: 1.23 (3H, t), 2.40 (3H, s), 3.98 (2H, q), 6.98-7.01 (3H, m), 7.25 (1H, dd), 7.41 (1H, ddd), 8.33 (1H, d), 8.44 (1H, dd). |
| 68 | 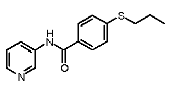 | | (CDCl3) δ: 1.07 (3H, t), 1.73-1.76 (2H, m), 2.99 (2H, t), 7.31-7.36 (4H, m), 7.79 (2H, d), 8.30 (1H, d), 8.40 (1H, dd), 8.66 (1H, d). |
| 69 | 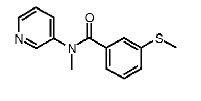 | | (CDCl3) δ: 2.35 (3H, s), 3.53 (3H, s), 6.99 (1H, d), 7.09 (1H, t), 7.17-7.20 (3H, m), 7.39 (1H, dt), 8.36 (1H, d), 8.40 (1H, dd). |
| 70 | 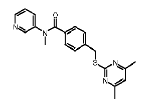 | | (CDCl3) δ: 2.37 (6H, s), 3.50 (3H, s), 4.30 (2H, s), 6.67 (1H, s), 7.16-7.35 (6H, m), 8.37 (2H). |
| 71 | 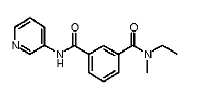 | | (CDCl3) δ: 1.14-1.25 (3H, m), 2.92-3.10 (3H), 3.36-3.54 (2H), 7.27-7.34 (1H, m), 7.41-7.43 (2H, m), 7.85-7.90 (2H, m), 8.37-8.41 (2H, m), 8.83 (1H), 9.54 (1H, s). |
| 72 | 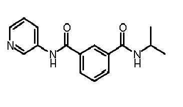 | | (acetone-d6) δ: 1.34 (6H, d), 4.30-4.32 (1H, m), 7.47 (1H), 7.71 (1H), 8.09 (1H), 8.17 (1H), 8.29 (1H), 8.45 (2H), 8.96 (1H), 9.03 (1H). |
| 73 | 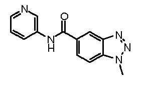 | | (CDCl3) δ: 4.28 (6H), 7.28-7.33 (2H, m), 7.55 (1H), 7.83 (1H), 7.98 (1H), 8.11 (1H), 8.19 (1H), 8.34-8.36 (4H, m), 8.70 (1H), 8.76 (1H), 8.90 (1H), 9.30 (1H), 9.77 (1H). |
| 74 | 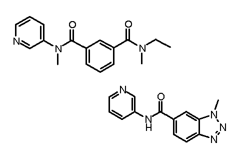 | | (CDCl3) δ: 1.09-1.21 (3H), 2.70-3.01 (5H), 3.53 (3H, s), 7.19-7.41 (6H, m), 8.38-8.40 (2H, m). |
| 75 | 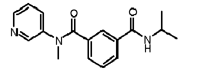 | | (CDCl3) δ: 1.25 (6H, d), 3.53 (3H, s), 4.21-4.25 (1H, m), 5.88-5.90 (1H, m), 7.19-7.31 (4H, m), 7.39-7.42 (1H, m), 7.70-7.74 (2H, m), 8.36 (1H, d), 8.40 (1H, dd). |
| 76 | 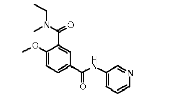 | | (CDCl3) δ: 1.12-1.24 (3H, m), 2.81 (2H ), 3.16 (3H, d), 3.60 (3H), 6.80-6.83 (1H), 7.27-7.30 (1H, m), 7.73 (1H, dd), 7.83-7.87 (1H), 8.33-8.36 (2H), 8.85-8.86 (1H), 9.46 (1H). |
| 77 | 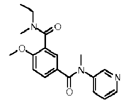 | | (CDCl3) δ: 0.91-1.28 (3H, m), 2.96-2.99 (3H, m), 3.50 (3H, s), 3.78 (3H, s), 6.74 (1H, dd), 7.16-7.39 (4H, m), 8.36-8.40 (2H, m). |
| 78 | 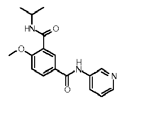 | | (CDCl3) δ: 1.14 (6H, d), 4.01 (3H), 4.07-4.15 (1H, m), 7.04-7.07 (1H, m), 7.26-7.28 (1H, m), 7.62 (1H, d), 8.15 (1H), 8.29-8.33 (2H, m), 8.83-8.84 (1H, m), 8.87-8.88 (1H, m), 9.63 (1H, s). |
| 79 | 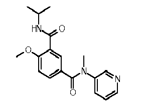 | | (CDCl3) δ: 7.09-7.12 (1H, m), 7.32-7.41 (2H, m), 7.58-7.59 (1H, m), 8.00 (1H, br s), 8.24-8.51 (5H, m), 8.83-8.91 (2H, m). |
| 80 | 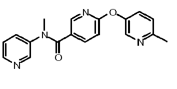 | | (CDCl3) δ: 2.57 (3H, s), 3.53 (3H, s), 6.81-6.84 (1H, m), 7.16-7.47 (4H, m), 7.67-7.70 (1H, m), 8.01-8.02 (1H, m), 8.29-8.42 (3H, m). |
| 81 | 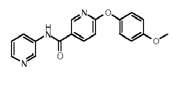 | | (CDCl3) δ: 3.83 (3H, s), 6.94-7.11 (4H, m), 7.33-7.36 (1H, m), 8.02 (1H, br s), 8.19-8.43 (4H, m), 8.66-8.71 (2H, m). |
| 82 | 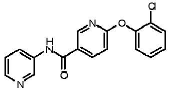 | | (CDCl3) δ: 7.04 (1H, d, J = 8.6 Hz), 7.28-7.40 (5H, m), 8.19-8.37 (3H, m), 8.66-8.67 (2H, m), 8.80-8.84 (1H, m). |
| 83 | 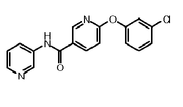 | | (CDCl3) δ: 7.06-7.08 (2H, m), 7.19-7.26 (2H, m), 7.32-7.40 (2H, m), 7.88 (1H, br s), 8.24-8.27 (2H, m), 8.41-8.42 (1H, m), 8.67-8.69 (2H, m). |
| 84 | 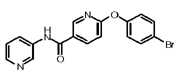 | | (CDCl3) δ: 7.05-7.08 (3H, m), 7.30-7.37 (2H, m), 7.54-7.57 (1H, m), 7.80 (1H, br s), 8.21-8.27 (2H, m), 8.41-8.43 (1H, m), 8.67-8.67 (2H, m). |
| 85 | 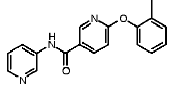 | | (CDCl3) δ: 2.17 (3H, s), 6.98-7.36 (6H, m), 7.84 (1H, br s), 8.21-8.42 (3H, m), 8.67-8.67 (2H, m). |
| 86 | 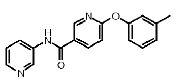 | | (CDCl3) δ: 2.39 (3H, s), 6.98-7.07 (3H, m), 7.26-7.36 (3H, m), 7.92 (1H, br s), 8.25-8.37 (3H, m), 8.67-8.70 (2H, m). |
| 87 | 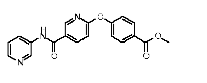 | | (CDCl3) δ: 3.93 (3H, s), 7.05-7.08 (1H, m), 7.31-7.54 (3H, m), 7.86-7.92 (2H, m), 8.08 (1H, br s), 8.21-8.44 (3H, m), 8.66-8.71 (2H, m). |
| 88 | 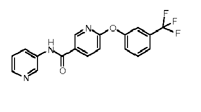 | | (CDCl3) δ: 3.93 (3H, s), 7.05-7.08 (1H, m), 7.31-7.54 (3H, m), 7.82-7.83 (1H, m), 7.93-7.95 (1H, m), 8.08 (1H, br s), 8.22-8.28 (2H, m), 8.40-8.43 (1H, m), 8.67-8.70 (2H, m). |
| 89 | 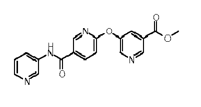 | | (CDCl3) δ: 7.08-7.10 (1H, m), 7.32-7.59 (5H, m), 8.02-8.06 (1H, m), 8.24-8.42 (3H, m), 8.67-8.68 (2H, m). |
| 90 | 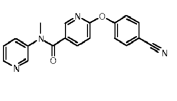 | | (CDCl3) δ: 3.52 (3H, s), 6.84-6.87 (1H, m), 7.16-7.74 (7H, m), 8.02-8.05 (1H, m), 8.32-8.48 (2H, m). |
| 91 | 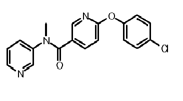 | | (CDCl3) δ: 3.53 (3H, s), 6.77-6.80 (1H, m), 7.00-7.03 (2H, m), 7.23-7.48 (4H, m), 7.67-7.69 (1H, m), 8.04-8.04 (1H, m), 8.34-8.43 (2H, m). |
| 92 | 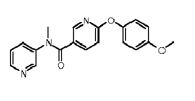 | | (CDCl3) δ: 3.51 (3H, s), 3.79 (3H, s), 6.71-7.08 (5H, m), 7.20-7.47 (1H, m), 7.61-7.65 (1H, m), 8.05-8.06 (1H, m), 8.32-8.48 (1H, m). |
| 93 | 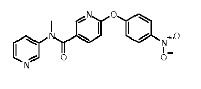 | | (CDCl3) δ: 3.53 (3H, s), 6.86-6.91 (1H, m), 7.17-7.77 (4H, m), 8.05-8.48 (5H, m). |
| 94 | 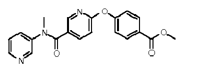 | | (CDCl3) δ: 3.53 (3H, s), 3.90 (3H, s), 6.79-6.82 (1H, m), 7.20-7.48 (5H, m), 7.68-7.72 (1H, m), 7.90-8.04 (2H, m), 8.35-8.48 (2H, m). |
| 95 | 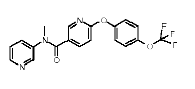 | | (CDCl3) δ: 3.50 (3H, s), 6.78-6.81 (1H, m), 7.07-7.70 (7H, m), 8.13-8.38 (3H, m). |
| 96 | 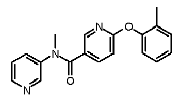 | | (CDCl3) δ: 2.06 (3H, s), 3.50 (3H, s), 6.73 (1H, dd, J = 8.6, 0.7 Hz), 7.14-7.30 (6H, m), 7.66-7.69 (1H, m), 8.03-8.03 (1H, m), 8.35-8.42 (2H, m). |
| 97 | 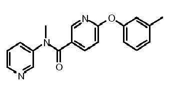 | | (CDCl3) δ: 2.34 (3H, s), 3.52 (3H, s), 6.78-6.98 (4H, m), 7.23-7.30 (2H, m), 7.43-7.46 (1H, m), 7.64-7.67 (1H, m), 8.07-8.07 (1H, m), 8.35-8.43 (2H, m). |
| 98 | 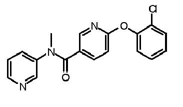 | | (CDCl3) δ: 3.52 (3H, s), 6.84 (1H, d, J = 8.6 Hz), 7.23-7.36 (6H, m), 7.69 (1H, dd, J = 8.5, 2.5 Hz), 8.03 (1H, d, J = 2.4 Hz), 8.40 (2H, dt, J = 20.0, 7.5 Hz). |
| 99 | 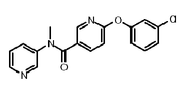 | | (CDCl3) δ: 3.52 (3H, s), 6.79 (1H, d, J = 8.6 Hz), 6.96-7.03 (1H, m), 7.09-7.10 (1H, m), 7.21-7.29 (3H, m), 7.43-7.47 (1H, m), 7.67-7.70 (1H, m), 8.06-8.07 (1H, m), 8.34-8.34 (1H, m), 8.44-8.46 (1H, m). |
| 100 | 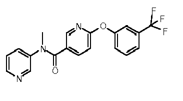 | | (CDCl3) δ: 3.52 (3H, s), 6.81-6.84 (1H, m), 7.25-7.72 (7H, m), 8.05-8.06 (1H, m), 8.34-8.34 (1H, m), 8.44-8.46 (1H, m). |
| 101 | 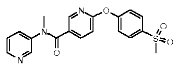 | | (CDCl3) δ: 3.06 (3H, s), 3.53 (3H, s), 6.86-6.89 (1H, m), 7.23-7.50 (4H, m), 7.71-7.74 (1H, m), 7.93-8.08 (3H, m), 8.35-8.45 (2H, m). |
| 102 | 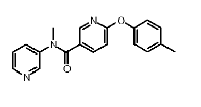 | | (CDCl3) δ: 3.52 (3H, s), 6.84 (1H, d, J = 8.6 Hz), 7.23-7.36 (6H, m), 7.68-7.70 (1H, m), 8.02-8.03 (1H, m), 8.36-8.43 (2H, m). |
| 103 | 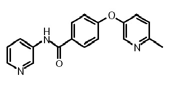 | | (CDCl3) δ: 2.59 (3H, s), 6.96-7.43 (6H, m), 7.88-7.90 (2H, m), 8.03 (1H, br s), 8.27-8.40 (2H, m), 8.67-8.68 (1H, m). |
| 104 | 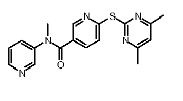 | | (CDCl3) δ: 2.39 (6H, s), 3.53 (3H, s), 6.79 (1H, s), 7.25-7.42 (2H, m), 7.61-7.66 (1H, m), 7.81-7.84 (1H, m), 8.38-8.50 (3H, m). |
| 105 | 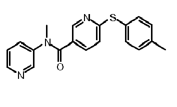 | | (CDCl3) δ: 2.39 (3H, s), 3.49 (3H, s), 6.64-6.66 (1H, m), 7.20-7.44 (7H, m), 8.24-8.46 (3H, m). |
| 106 | 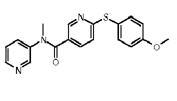 | | (CDCl3) δ: 3.53 (3H, s), 3.83 (3H, s), 6.59-6.66 (1H, m), 6.95-6.98 (2H, m), 7.20-7.56 (5H, m), 8.27-8.44 (3H, m). |
| 107 | 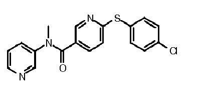 | | (CDCl3) δ: 3.52 (3H, s), 6.72-6.75 (1H, m), 7.20-7.49 (7H, m), 8.26-8.33 (2H, m), 8.44-8.48 (1H, m). |
| 108 | 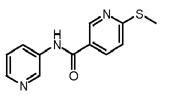 | | (CDCl3) δ: 2.62 (3H, s), 7.29-7.35 (2H, m), 7.97-8.49 (4H, m), 8.67-8.70 (1H, m), 8.91-8.93 (1H, m). |
| 109 | 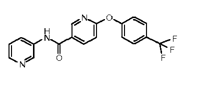 | | (CDCl3) δ: 7.09-7.12 (1H, m), 7.30-7.34 (3H, m), 7.69-7.72 (2H, m), 8.00 (1H, br s), 8.18-8.41 (3H, m), 8.67-8.69 (2H, m). |
| 110 | 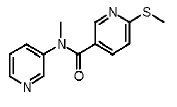 | | (CDCl3) δ: 2.48 (3H, s), 3.51 (3H, s), 7.00-7.03 (1H, m), 7.20-7.45 (3H, m), 8.33-8.45 (3H, m). |
| 111 | 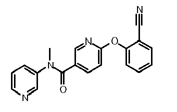 | | (CDCl3) δ: 3.53 (3H, s), 6.93-6.96 (1H, m), 7.18-7.73 (7H, m), 8.02-8.06 (1H, m), 8.42-8.47 (2H, m). |
| 112 | 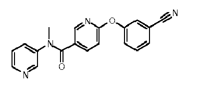 | | (CDCl3) δ: 3.52 (3H, s), 6.83-6.86 (1H, m), 7.26-7.73 (7H, m), 8.03-8.04 (1H, m), 8.36-8.45 (2H, m). |
| 113 | 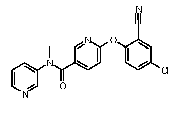 | | (CDCl3) δ: 3.51 (3H, s), 6.94-6.96 (1H, m), 7.19-7.73 (5H, m), 8.03-8.04 (1H, m), 8.24-8.48 (3H, m). |
| 114 | 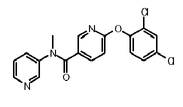 | | (CDCl3) δ: 3.52 (3H, s), 6.85-6.88 (1H, m), 7.17-7.36 (5H, m), 7.69-7.72 (1H, m), 7.99-8.00 (1H, m), 8.35-8.43 (2H, m). |
| 115 | 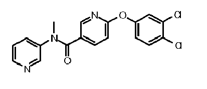 | | (CDCl3) δ: 3.52 (3H, s), 6.81-6.95 (2H, m), 7.21-7.48 (4H, m), 7.68-7.71 (1H, m), 8.04-8.04 (1H, m), 8.35-8.44 (2H, m). |
| 116 | 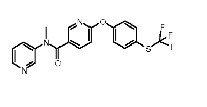 | | (CDCl3) δ: 3.53 (3H, s), 6.81-6.84 (1H, m), 7.08-7.48 (4H, m), 7.59-7.74 (3H, m), 8.06-8.48 (3H, m). |
| 117 | 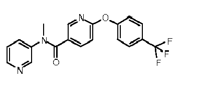 | | (CDCl3) δ: 3.52 (3H, s), 6.82-6.85 (1H, m), 7.17-7.49 (4H, m), 7.66-7.70 (3H, m), 8.05-8.06 (1H, m), 8.32-8.48 (2H, m). |
| 118 | 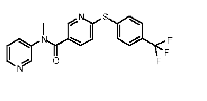 | | (CDCl3) δ: 3.53 (3H, s), 6.85-6.88 (1H, m), 7.25-7.67 (7H, m), 8.29-8.45 (3H, m). |
| 119 | 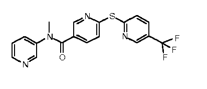 | | (CDCl3) δ: 3.53 (3H, s), 7.23-7.27 (2H, m), 7.45-7.59 (4H, m), 7.79-7.81 (1H, m), 8.35-8.48 (3H, m), 8.72-8.72 (1H, m). |
| 120 | 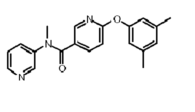 | | (CDCl3) δ: 2.30 (6H, s), 3.52 (3H, s), 6.71-6.80 (4H, m), 7.24-7.67 (3H, m), 8.07-8.08 (1H, m), 8.36-8.43 (2H, m). |
| 121 | 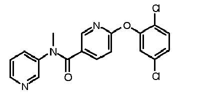 | | (CDCl3) δ: 3.52 (3H, s), 6.85-6.88 (1H, m), 7.14-7.45 (5H, m), 7.69-7.72 (1H, m), 8.02-8.02 (1H, m), 8.37-8.44 (2H, m). |
| 122 | 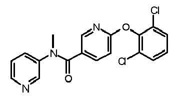 | | (CDCl3) δ: 3.50 (3H, s), 6.91-6.94 (1H, m), 7.09-7.43 (5H, m), 7.69-7.73 (1H, m), 7.99-8.00 (1H, m), 8.38-8.46 (2H, m). |
| 123 | 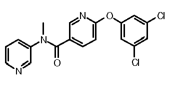 | | (CDCl3) δ: 3.52 (3H, s), 6.80-6.82 (1H, m), 6.99-7.03 (2H, m), 7.19-7.48 (3H, m), 7.68-7.71 (1H, m), 8.06-8.07 (1H, m), 8.36-8.45 (2H, m). |
| 124 | 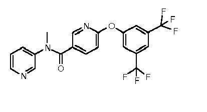 | | (CDCl3) δ: 3.55 (3H, s), 6.87-6.90 (1H, m), 7.26-7.31 (1H, m), 7.45-7.75 (5H, m), 8.04-8.05 (1H, m), 8.36-8.45 (2H, m). |
| 125 | 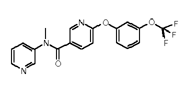 | | (CDCl3) δ: 3.52 (3H, s), 6.79-6.82 (1H, m), 7.00-7.05 (3H, m), 7.31-7.43 (4H, m), 7.68-7.71 (1H, m), 8.06-8.07 (1H, m), 8.36-8.44 (2H, m). |
| 126 | 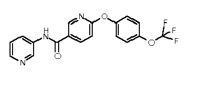 | | (CDCl3) δ: 7.05-7.36 (6H, m), 7.92 (1H, br s), 8.24-8.26 (2H, m), 8.40-8.42 (1H, m), 8.66-8.69 (2H, m). |
| 127 | 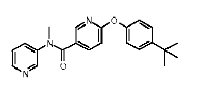 | | (CDCl3) δ: 1.31 (9H, s), 3.49 (3H, s), 6.72-6.75 (1H, m), 6.97-7.01 (2H, m), 7.23-7.67 (5H, m), 8.08-8.09 (1H, m), 8.36-8.43 (2H, m). |
| 128 | 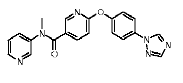 | | (CDCl3) δ: 3.52 (3H, s), 6.83-6.86 (1H, m), 7.21-7.30 (3H, m), 7.46-7.49 (1H, m), 7.68-7.71 (3H, m), 8.07-8.09 (2H, m), 8.32-8.51 (3H, m). |
| 129 | 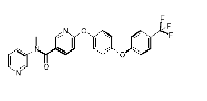 | | (CDCl3) δ: 3.52 (3H, s), 6.78-6.80 (1H, m), 7.03-7.11 (4H, m), 7.27-7.29 (3H, m), 7.49-7.65 (4H, m), 8.08-8.09 (1H, m), 8.36-8.44 (2H, m). |
| 130 | 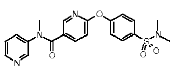 | | (CDCl3) δ: 2.75 (6H, s), 3.53 (3H, s), 6.84-6.86 (1H, m), 7.22-7.49 (4H, m), 7.71-7.79 (3H, m), 8.08-8.09 (1H, m), 8.36-8.45 (2H, m). |
| 131 | 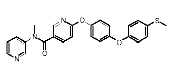 | | (CDCl3) δ: 1.27 (3H, t, J = 18.8 Hz), 3.99 (2H, q, J = 7.1 Hz), 6.76-6.79 (1H, m), 7.06-7.47 (6H, m), 7.66-7.69 (1H, m), 8.04-8.05 (1H, m), 8.32-8.44 (2H, m). |
| 132 | 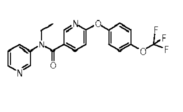 | | (CDCl3) δ: 0.97 (3H, t, J = 20.1 Hz), 1.61-1.72 (2H, m), 3.89 (2H, q, J = 15.2 Hz), 6.77-6.80 (1H, m), 7.06-7.29 (5H, m), 7.43-7.46 (1H, m), 7.65-7.67 (1H, m), 8.03-8.04 (1H, m), 8.30-8.45 (2H, m). |
| 133 | 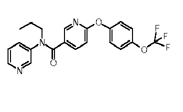 | | (CDCl3) δ: 4.52-4.54 (2H, m), 5.18-5.22 (2H, m), 5.89-6.02 (1H, m), 6.78-6.81 (1H, m), 7.06-7.28 (5H, m), 7.43-7.46 (1H, m), 7.68-7.71 (1H, m), 8.07-8.07 (1H, m), 8.32-8.42 (2H, m). |
| 134 | 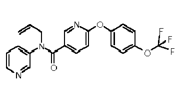 | | (CDCl3) δ: 4.51-4.54(2H,m),5.15-5.24(2H,m),5.91-6.00(1H,m),6.79(1H,d),7.07-7.28(5H,m),7.44-8.45(5H,m) |
| 135 | 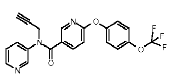 | | (CDCl3) δ: 2.30 (1H, t, J = 2.5 Hz), 4.68 (2H, d, J = 2.6 Hz), 6.78-6.81 (1H, m), 7.08-7.33 (5H, m), 7.56-7.75 (2H, m), 8.09-8.10 (1H, m), 8.41-8.49 (2H, m). |
| 136 | 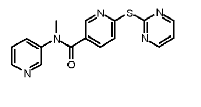 | | (CDCl3) δ: 3.51 (3H, s), 7.20-7.30 (5H, m), 7.42-7.46 (1H, m), 7.60-7.62 (1H, m), 8.24-8.48 (3H, m). |
| 137 | 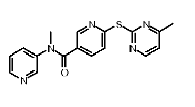 | | (CDCl3) δ: 2.44 (3H, s), 3.52 (3H, s), 6.91-6.92 (1H, m), 7.24-7.27 (1H, m), 7.43-7.46 (1H, m), 7.67-7.81 (2H, m), 8.39-8.44 (4H, m). |
| 138 | 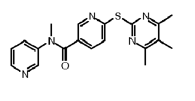 | | (CDCl3) δ: 2.20 (3H, s), 2.41 (6H, s), 3.51 (3H, s), 7.23-7.45 (2H, m), 7.61-7.64 (1H, m), 7.76-7.78 (1H, m), 8.37-8.44 (3H, m). |
| 139 | 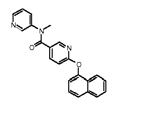 | | (CDCl3) δ: 3.52 (4H, s), 6.80-6.83 (1H, m), 7.17-7.52 (6H, m), 7.67-7.88 (4H, m), 8.03-8.04 (1H, m), 8.35-8.42 (2H, m). |
| 140 | 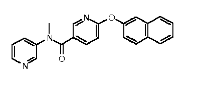 | | (CDCl3) δ: 3.51 (3H, s), 6.80-6.83 (1H, m), 7.19-7.87 (10H, m), 8.07-8.07 (1H, m), 8.36-8.43 (2H, m). |
| 141 | 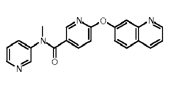 | | (CDCl3) δ: 3.52 (3H, s), 6.88-6.91 (1H, m), 7.29-7.38 (4H, m), 7.73-7.82 (3H, m), 8.07-8.08 (2H, m), 8.38-8.44 (2H, m), 8.89-8.91 (1H, m). |
| 142 | 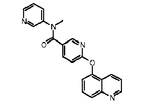 | | (CDCl3) δ: 3.53 (3H, s), 6.88-6.91 (1H, m), 7.28-7.44 (4H, m), 7.68-7.74 (2H, m), 8.00-8.01 (2H, m), 8.20-8.40 (3H, m), 8.92-8.93 (1H, m). |
| 143 | 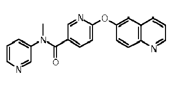 | | (CDCl3) δ: 3.52 (3H, s), 6.86-6.89 (1H, m), 7.25-7.52 (5H, m), 7.71-7.73 (1H, m), 8.06-8.14 (3H, m), 8.36-8.44 (2H, m), 8.88-8.89 (1H, m). |
| 144 | 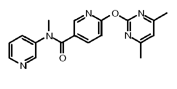 | | (CDCl3) δ: 2.54 (6H, s), 3.50 (3H, s), 6.30-6.33 (1H, m), 7.01-7.51 (4H, m), 7.90-7.95 (1H, m), 8.51-8.53 (2H, m). |
| 145 | 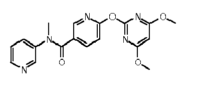 | | (CDCl3) δ: 3.48 (3H, s), 3.92 (6H, s), 6.06 (1H, s), 6.31-6.34 (1H, m), 7.05-7.09 (1H, m), 7.31-7.48 (2H, m), 7.88-7.91 (1H, m), 8.49-8.51 (2H, m). |
| 146 | 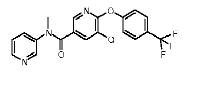 | | (CDCl3) δ: 3.50 (3H, s), 7.19-7.33 (3H, m), 7.47-7.84 (5H, m), 8.35-8.46 (2H, m). |
| 147 | 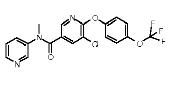 | | (CDCl3) δ: 3.51 (3H, s), 7.10-7.50 (6H, m), 7.81-7.82 (2H, m), 8.35-8.46 (2H, m). |
| 148 | 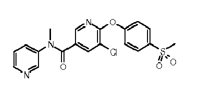 | | (CDCl3) δ: 3.07 (3H, s), 3.51 (3H, s), 7.26-7.52 (4H, m), 7.86-7.94 (4H, m), 8.35-8.47 (2H, m). |
| 149 | 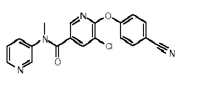 | | (CDCl3) δ: 7.19-7.52 (5H, m), 7.67-7.84 (4H, m), 8.31-8.32 (1H, m), 8.48-8.48 (1H, m). |
| 150 | 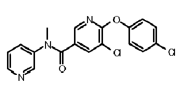 | | (CDCl3) δ: 3.50 (3H, s), 7.01-7.05 (2H, m), 7.26-7.50 (4H, m), 7.79-7.82 (2H, m), 8.32-8.47 (2H, m). |
| 151 | 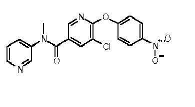 | | (CDCl3) δ: 3.52 (3H, s), 7.24-7.52 (4H, m), 7.81-7.86 (2H, m), 8.33-8.44 (4H, m). |
| 152 | 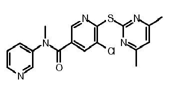 | | (CDCl3) δ: 2.39 (6H, s), 3.54 (3H, s), 6.80 (1H, s), 7.27-7.29 (1H, m), 7.42-7.45 (1H, m), 7.74-7.74 (1H, m), 8.19-8.20 (1H, m), 8.43-8.47 (2H, m). |
| 153 | 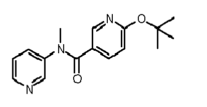 | | (CDCl3) δ: 1.58 (9H, s), 3.53 (3H, s), 6.46-6.49 (1H, m), 7.37-7.39 (1H, m), 7.61-7.64 (1H, m), 7.88-7.91 (1H, m), 8.52-8.59 (2H, m), 8.82-8.83 (1H, m). |
| 154 | 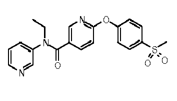 | | (CDCl3) δ: 1.25 (3H, t, J = 7.1 Hz), 3.07 (3H, s), 4.00 (2H, q, J = 7.1 Hz), 6.85-6.87 (1H, m), 7.25-7.30 (3H, m), 7.45-7.47 (1H, m), 7.70-7.73 (1H, m), 7.96-8.04 (3H, m), 8.33-8.45 (2H, m). |
| 155 | 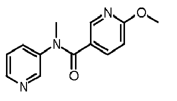 | | (CDCl3) δ: 3.52 (3H, s), 3.88 (3H, s), 6.55-6.58 (1H, m), 7.25-7.28 (1H, m), 7.41-7.53 (2H, m), 8.09-8.10 (1H, m), 8.37-8.43 (2H, m). |
| 156 | 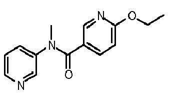 | | (CDCl3) δ: 1.34 (3H, t, J = 7.1 Hz), 3.51 (3H, s), 4.30 (2H, q, J = 7.1 Hz), 6.50-6.55 (1H, m), 7.24-7.26 (1H, m), 7.44-7.49 (2H, m), 8.08-8.09 (1H, m), 8.35-8.45 (2H, m). |
| 157 | 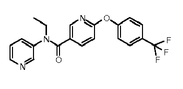 | | (CDCl3) δ: 1.25 (3H, t, J = 7.1 Hz), 3.99 (2H, q, J = 7.1 Hz), 6.81-6.84 (1H, m), 7.16-7.30 (3H, m), 7.44-7.47 (1H, m), 7.64-7.70 (3H, m), 8.04-8.05 (1H, m), 8.32-8.44 (2H, m). |
| 158 | 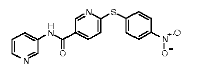 | | (CDCl3) δ: 7.29-7.34 (2H, m), 7.73-7.76 (3H, m), 7.83 (1H, br s), 8.09-8.12 (1H, m), 8.24-8.28 (2H, m), 8.42-8.44 (1H, m), 8.68-8.71 (1H, m), 8.90-8.91 (1H, m). |
| 159 | 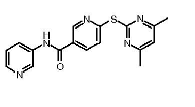 | | (CDCl3) δ: 2.40 (6H, s), 6.83 (1H, s), 7.31-7.39 (1H, m), 8.03-8.06 (1H, m), 8.17-8.40 (3H, m), 8.70-8.72 (2H, m), 9.01-9.02 (1H, m). |
| 160 | 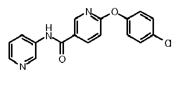 | | (CDCl3) δ: 7.06-7.12 (3H, m), 7.40 (2H, d, J = 8.4 Hz), 7.79 (1H, br s), 8.02-8.10 (1H, m), 8.23-8.25 (2H, m), 8.42 (1H, d, J = 4.4 Hz), 8.66-8.69 (2H, m). |
| 161 | 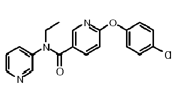 | | (CDCl3) δ: 1.25 (3H, t, J = 21.4 Hz), 3.99 (2H, q, J = 7.1 Hz), 6.75-6.78 (1H, m), 6.99-7.02 (2H, m), 7.25-7.46 (4H, m), 7.66-7.68 (1H, m), 8.02-8.03 (1H, m), 8.32-8.43 (2H, m). |
| 162 | 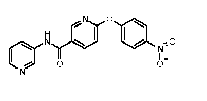 | | (CDCl3) δ: 7.16-7.38 (3H, m), 7.79 (1H, br s), 8.29-8.40 (5H, m), 8.68-8.69 (2H, m). |
| 163 | 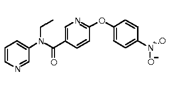 | | (CDCl3) δ: 1.25 (3H, t, J = 7.1 Hz), 4.00 (2H, q, J = 7.1 Hz), 6.86-6.89 (1H, m), 7.20-7.75 (5H, m), 8.04-8.48 (5H, m). |
| 164 | 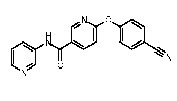 | | (CDCl3) δ: 7.13-7.37 (4H, m), 7.72-7.77 (3H, m), 8.24-8.44 (3H, m), 8.68-8.69 (2H, m). |
| 165 | 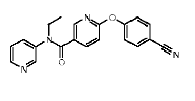 | | (CDCl3) δ: 1.26 (3H, s), 4.00 (2H, q, J = 7.1 Hz), 6.83-6.86 (1H, m), 7.15-7.48 (4H, m), 7.67-7.71 (3H, m), 8.03-8.04 (1H, m), 8.28-8.29 (1H, m), 8.46-8.47 (1H, m). |
| 166 | 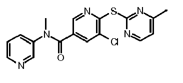 | | (CDCl3) δ: 2.42 (3H, s), 3.53 (3H, s), 6.91-6.93 (1H, m), 7.26-7.47 (2H, m), 7.77-7.77 (1H, m), 8.21-8.50 (4H, m). |
| 167 | 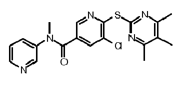 | | (CDCl3) δ: 2.18 (3H, s), 2.37 (6H, s), 3.52 (3H, s), 7.29-7.37 (2H, m), 7.68-7.68 (1H, m), 8.14-8.15 (1H, m), 8.43-8.47 (2H, m). |
| 168 | 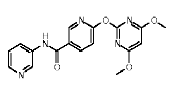 | | (CDCl3) δ: 3.97 (6H, s), 6.12 (1H, s), 6.68-6.71 (2H, m), 7.27-7.33 (3H, m), 8.42-8.61 (3H, m). |
| 169 | 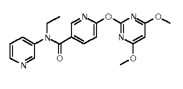 | | (CDCl3) δ: 1.24 (3H, t, J = 12.2 Hz), 3.91-3.97 (5H, m), 6.06 (1H, s), 6.30-6.33 (1H, m), 7.03-7.07 (1H, m), 7.31-7.51 (2H, m), 7.89-7.90 (1H, m), 8.48-8.51 (2H, m). |
| 170 | 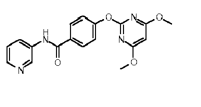 | | (CDCl3) δ: 3.53 (3H, s), 7.20-7.46 (5H, m), 7.57-7.63 (1H, m), 8.24-8.52 (3H, m). |
| 171 | 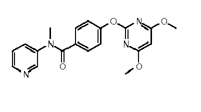 | | (CDCl3) δ: 3.53 (3H, s), 3.78 (6H, s), 5.77 (1H, s), 7.04-7.07 (2H, m), 7.17-7.20 (1H, m), 7.31-7.42 (3H, m), 8.37-8.38 (2H, m). |
| 172 | 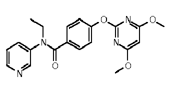 | | (CDCl3) δ: 1.26 (3H, t, J = 7.1 Hz), 3.76 (6H, s), 4.01 (2H, q, J = 7.1 Hz), 5.78 (1H, s), 7.02-7.05 (2H, m), 7.17-7.40 (4H, m), 8.35-8.38 (2H, m). |
| 173 | 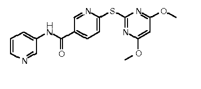 | | (CDCl3) δ: 3.84(6H,s),5.84(1H,s),7.26-7.38(1H,m),7.92(1H,bs),8.09-9.06(6H,m) |
| 174 | 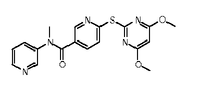 | | (CDCl3) δ: 3.52 (3H, s), 3.78 (6H, s), 5.78 (1H, s), 7.24-7.26 (1H, m), 7.45-7.78 (3H, m), 8.35-8.48 (3H, m). |
| 175 | 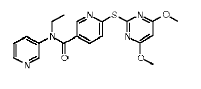 | | (CDCl3) δ: 1.27 (3H, t, J =7.1 Hz), 3.76 (6H, s), 4.01 (2H, q, J =7.1 Hz), 5.77 (1H, s), 7.21-7.48 (2H, m), 7.64-7.73 (2H, m), 8.32-8.46 (3H, m). |
| 176 | 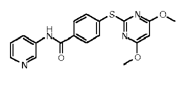 | | (CDCl3) δ: 3.77 (6H, s), 5.77 (1H, s), 7.32-7.35 (1H, m), 7.75-7.78 (2H, m), 7.89-7.92 (2H, m), 8.17 (1H, br s), 8.31-8.39 (2H, m), 8.70-8.70 (1H, m). |
| 177 | 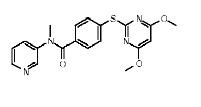 | | (CDCl3) δ: 3.54 (3H, s), 3.71 (6H, s), 5.74 (1H, s), 7.19-7.33 (3H, m), 7.40-7.51 (3H, m), 8.35-8.39 (2H, m). |
| 178 | 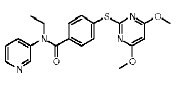 | | (CDCl3) δ: 1.26 (3H, t, J = 7.1 Hz), 3.68 (6H, s), 4.00 (2H, q, J = 7.1 Hz), 5.71 (1H, s), 7.19-7.49 (6H, m), 8.33-8.39 (2H, m). |
| 179 | 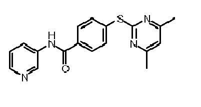 | | (CDCl3) δ: 2.36 (6H, s), 6.76 (1H, s), 7.33-7.36 (1H, m), 7.78-7.90 (5H, m), 8.30-8.33 (1H, m), 8.41-8.43 (1H, m), 8.68-8.69 (1H, m). |
| 180 | 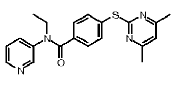 | | (CDCl3) δ: 1.26 (3H, t, J = 7.1 Hz), 2.33 (6H, s), 4.01 (2H, q, J = 7.1 Hz), 6.69 (1H, s), 7.19-7.47 (6H, m), 8.38-8.41 (2H, m). |
| 181 | 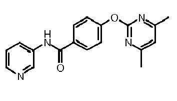 | | (CDCl3) δ: 2.44(6H,s),6.82(1H,s),7.26-8.68(9H,m) |
| 182 | 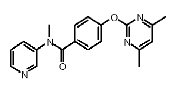 | | (CDCl3) δ: 2.38(6H,s),3.54(3H,s),6.76(1H,s),7.02-7.40(5H,m),8.40-8.54(2H,m) |
| 183 | 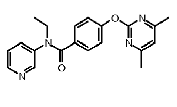 | | (CDCl3) δ:1.25(3H,t), 2.37(6H,s),4.00(2H,q),6.76(1H,s),7.02-7.48(6H,m),8.37-8.43(2H,m) |
| 184 | 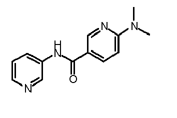 | | (CDCl3) δ: 3.17 (6H, s), 6.54 (1H, dd, J = 9.0, 0.5 Hz), 7.26-7.32 (1H, m), 7.86 (1H, br s), 7.97 (1H, dd, J = 9.0, 2.6 Hz), 8.25-8.37 (2H, m), 8.65-8.71 (2H, m). |
| 185 | 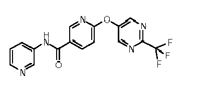 | | (CDCl3) δ: 7.22-7.34 (2H, m), 8.18-8.41 (4H, m), 8.70-8.88 (4H, m). |
| 186 | 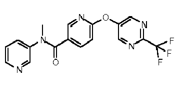 | | (CDCl3) δ: 3.53 (3H, s), 6.95-6.97 (1H, m), 7.31-7.47 (2H, m), 7.77-8.00 (2H, m), 8.33-8.47 (2H, m), 8.76 (2H, s). |
| 187 | 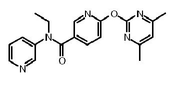 | | (CDCl3) δ: 1.22 (3H, q, J = 7.1 Hz), 2.18 (6H, s), 3.95 (1H, q, J = 7.1 Hz), 6.30 (1H, d, J = 9.7 Hz), 7.02-7.09 (2H, m), 7.26-7.46 (2H, m), 7.93-7.94 (1H, m), 8.51-8.53 (2H, m). |
| 188 | 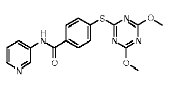 | | (CDCl3) δ: 3.94(6H,s),7.34-8.70(8H,m) |
| 189 | 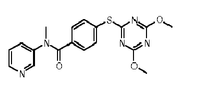 | | (CDCl3) δ:3.53(3H,s), 3.93(6H,s),7.20-8.41(9H,m) |
| 190 | 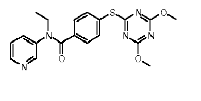 | | (CDCl3) δ:1.25(3H,t), 3.87(6H,s),4.02(2H,q),7.20-8.41(9H,m) |
| 191 | 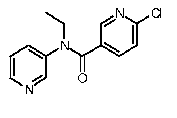 | | (CDCl3) δ: 1.25 (3H, t, J = 7.1 Hz), 4.00 (2H, q, J = 7.1 Hz), 7.18-7.61 (3H, m), 8.26-8.46 (3H, m). |
| 192 | 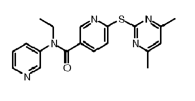 | | (CDCl3) δ: 1.26 (3H, t, J = 7.1 Hz), 2.38 (6H, s), 4.01 (1H, q, J = 7.1 Hz), 6.78 (1H, s), 7.24-7.44 (2H, m), 7.66-7.78 (2H, m), 8.37-8.47 (2H, m). |
| 193 | 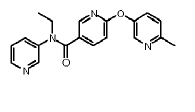 | | (CDCl3) δ: 1.25 (3H, q, J = 7.1 Hz), 2.51 (3H, s), 3.99 (2H, q, J = 7.1 Hz), 6.81 (1H, dd, J = 8.6, 0.7 Hz), 7.15-7.70 (5H, m), 8.00-8.01 (1H, m), 8.29-8.47 (3H, m). |
| 194 | 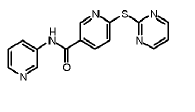 | | (CDCl3) δ: 7.11-7.12 (1H, m), 7.35-7.38 (1H, m), 7.95 (1H, br s), 8.03-8.29 (3H, m), 8.47-8.68 (4H, m), 9.07 (1H, t, J = 1.2 Hz). |
| 195 | 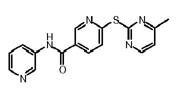 | | (CDCl3) δ: 2.50 (3H, s), 6.97 (1H, d, J = 5.1 Hz), 7.35-7.38 (1H, m), 7.89 (1H, br s), 8.05-8.45 (5H, m), 8.71 (1H, d, J = 2.6 Hz), 9.04 (1H, d, J = 2.4 Hz). |
| 196 | 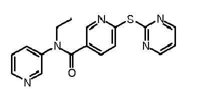 | | (CDCl3) δ: 1.26 (3H, t, J = 7.1 Hz), 4.01 (2H, q, J = 7.1 Hz), 7.04-7.06 (1H, m), 7.25-7.46 (2H, m), 7.66-7.79 (2H, m), 8.36-8.53 (5H, m). |
| 197 | 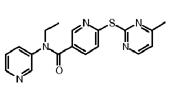 | | (CDCl3) δ: 1.26 (3H, t, J = 7.1 Hz), 2.43 (3H, s), 4.01 (2H, q, J = 7.1 Hz), 6.91 (1H, d, J = 5.1 Hz), 7.23-7.80 (4H, m), 8.39-8.44 (4H, m). |
| 198 | 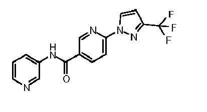 | | CDCl3) δ: 6.77 (1H, d, J = 2.6 Hz), 7.36-7.39 (1H, m), 7.87 (1H, br s), 8.18-8.46 (4H, m), 8.70-8.72 (2H, m), 8.97-8.98 (1H, m). |
| 199 | 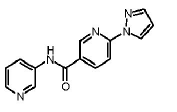 | | (CDCl3/DMSO-d6) δ: 7.30-7.90(2H,m),8.20-9.10(8H,m),10.38(1H,bs) |
| 200 | 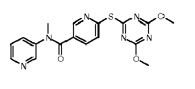 | | (CDCl3) δ: 3.53 (3H, s), 3.90 (6H, s), 7.26-7.47 (2H, m), 7.71-7.77 (2H, m), 8.36-8.46 (3H, m). |
| 201 | 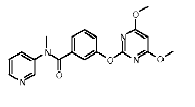 | | (CDCl3) δ: 3.52 (3H, s), 3.81 (6H, s), 5.77 (1H, s), 7.10-7.41 (6H, m), 8.35-8.38 (2H, m). |
| 202 | 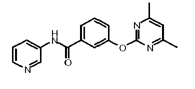 | | (CDCl3) δ: 2.39 (6H, s), 6.78 (1H, s), 7.26-7.76 (5H, m), 8.16 (1H, br s), 8.40-8.58 (3H, m). |
| 203 | 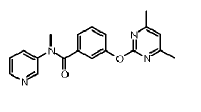 | | (CDCl3) δ: 2.38 (6H, s), 3.49 (3H, s), 6.78 (1H, s), 7.11-7.55 (5H, m), 8.40-8.53 (3H, m). |
| 204 | 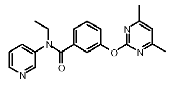 | | (CDCl3) δ: 1.23 (3H, t, J = 7.1 Hz), 2.40 (6H, s), 3.97 (2H, q, J = 7.1 Hz), 6.76 (1H, s), 7.09-7.43 (5H, m), 8.35-8.40 (3H, m). |
| 205 | 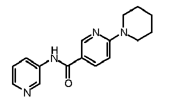 | | (CDCl3) δ: 1.63-1.70 (8H, m), 3.67-3.69 (4H, m), 6.65 (1H, d, J = 8.6 Hz), 7.26-7.37 (2H, m), 7.74 (1H, br s), 7.93-7.96 (1H, m), 8.19-8.72 (4H, m). |
| 206 | 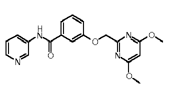 | | (CDCl3) δ: 3.87 (6H, s), 5.19 (2H, s), 5.95 (1H, s), 7.10-7.30 (4H, m), 7.82-7.85 (3H, m), 8.37-8.55 (3H, m). |
| 207 | 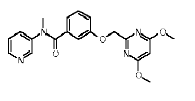 | | (CDCl3) δ: 3.49 (3H, s), 3.86 (6H, s), 5.06 (2H, s), 5.93 (1H, s), 6.77-6.81 (2H, m), 7.15-7.39 (4H, m), 8.35-8.38 (2H, m). |
| 208 | 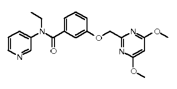 | | (CDCl3) δ: 1.24 (3H, t, J = 7.1 Hz), 3.84 (6H, s), 3.98 (2H, q, J = 7.1 Hz), 5.06 (2H, s), 5.93 (1H, s), 6.76-6.79 (2H, m), 7.16-7.39 (4H, m), 8.32-8.38 (2H, m). |
| 209 | 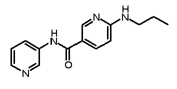 | | (CDCl3) δ: 1.24 (3H, t, J = 7.1 Hz), 3.84 (6H, s), 3.98 (2H, q, J = 7.1 Hz), 5.06 (2H, s), 5.93 (1H, s), 6.76-6.79 (2H, m), 7.16-7.39 (4H, m), 8.32-8.38 (2H, m). |
| 210 | 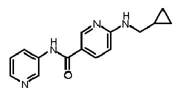 | | (CDCl3) δ: 0.28-0.30 (2H, m), 0.56-0.62 (2H, m), 1.08-1.13 (1H, m), 3.21-3.23 (2H, m), 5.11 (1H, br s), 6.43 (1H, d, J = 8.8 Hz), 7.30-7.33 (1H, m), 7.66 (1H, br s), 7.94-7.97 (1H, m), 8.26-8.37 (2H, m), 8.64-8.65 (2H, m). |
| 211 | 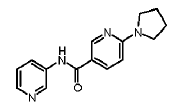 | | (CDCl3) δ: 2.07-2.13 (4H, m), 3.52-3.55 (4H, m), 6.39-6.42 (1H, m), 7.25-7.30 (1H, m), 7.76 (1H, br s), 7.93-8.71 (5H, m). |
| 212 | 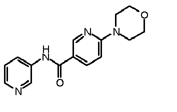 | | (CDCl3) δ: 3.66-3.83 (8H, m), 6.66 (1H, d, J = 9.2 Hz), 7.26-7.34 (1H, m), 7.74 (1H, br s), 8.12-8.60 (5H, m). |
| 213 | 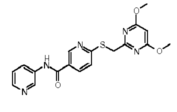 | | (CDCl3) δ: 3.90 (6H, s), 4.57 (2H, s), 5.91 (1H, s), 7.26-7.48 (2H, m), 7.84 (1H, br s), 8.21-8.68 (5H, m). |
| 214 | 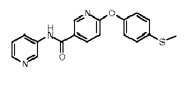 | | (CDCl3) δ: 2.51 (3H, s), 7.01-7.36 (6H, m),8.25(1H,bs) 8.24-8.69 (5H, m). |
| 215 | 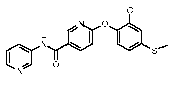 | | (CDCl3) δ: 2.52 (3H, s), 7.09-7.36 (5H, m), 7.74 (1H, br s), 8.31-8.58 (5H, m). |
| 216 | 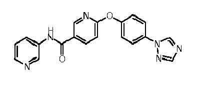 | | (CDCl3/DMSO-d6) δ: 7.07-7.35(4H,m),7.86-8.84(6H,m),8.82-8.94(7H,m),10.32(1H,bs) |
| 217 | 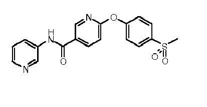 | | (CDCl3) δ: 3.10 (2H, s), 7.15 (1H, d, J = 8.4 Hz), 7.35-7.37 (4H, m), 7.87 (1H, br s), 8.00-8.71 (7H, m). |
| 218 | 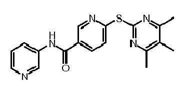 | | (CDCl3) δ: 2.24(3H,s),2.45(6H,s),7.30-7.38(1H,m),8.00-8.98(7H,m) |
| 219 | 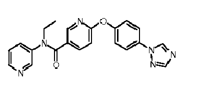 | | (CDCl3) δ: 1.25 (3H, q, J = 7.1 Hz), 3.99 (2H, q, J = 7.1 Hz), 6.80 (1H, dd, J = 8.6, 0.7 Hz), 7.15-7.71(7H, m), 8.00-8.47 (5H, m) |
| 220 | 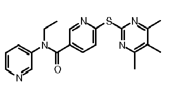 | | (CDCl3) δ: 1.25 (3H, t, J = 7.1 Hz), 2.17 (3H, s), 2.41 (6H, s), 4.00 (2H, q, J = 7.1 Hz), 7.45-7.70 (4H, m), 8.36-8.46 (3H, m). |
| 221 | 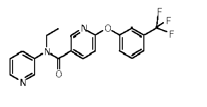 | | (CDCl3) δ: 1.25 (3H, q, J = 7.1 Hz), 3.99 (2H, q, J = 7.1 Hz), 6.80 (1H, dd, J = 8.6, 0.7 Hz), 7.15-7.71(7H, m), 8.00-8.47 (3H, m) |
| 222 | 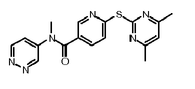 | | (CDCl3) δ: 2.41(6H, s), 3.58(3H,s), 6.83 (1H, s), 7.21-7.26(1H, m),7.70(1H,dd),7.98(1H,d),8.52(1H,d),9.07-9.09(2H,m) |
| 223 | 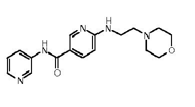 | | (CDCl3) δ: 2.50-2.51 (4H, m), 2.64-2.66 (2H, m), 3.42-3.46 (2H, m), 3.73-3.74 (4H, m), 5.60 (1H, br s), 6.46 (1H, d, J = 9.0 Hz), 7.26-7.33 (1H, m), 7.85 (1H, br s), 7.94-8.38 (3H, m), 8.65-8.66 (2H, m). |
| 224 | 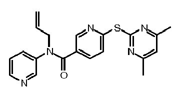 | | (CDCl3) δ: 2.42(6H,s),4.54(2H,d),5.91-6.02(1H,m),6.78(1H,s),7.20-8.46(7H,m) |
| 225 | 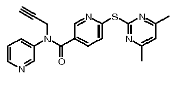 | | (CDCl3) δ: 2.35(1H,t),2.41(6H,s),4.71(2H,d),6.79(1H,s),7.27-8.52(7H,m) |
| 226 | 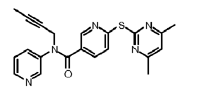 | | (CDCl3) δ: 1.80(3H,t),2.38(6H,s),4.63-4.65(1H,m),6.78(1H,s),7.27-8.50(7H,m) |
| 227 | 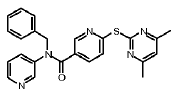 | | (CDCl3) δ:2.37(6H,s),5.14(2H,s),6.78(1H,s),7.14-8.41(12H,m) |
| 228 | 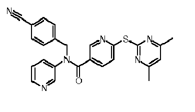 | | (CDCl3) δ:2.39(6H,s),5.19(2H,s),6.79(1H,s),7.18-8.45(11H,m) |
| 229 | 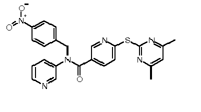 | | (CDCl3) δ:2.38(6H,s),5.23(2H,s),6.79(1H,s),7.18-8.4s(11H,m) |
| 230 | 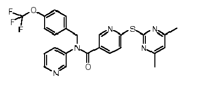 | | (CDCl3) δ:2.37(6H,s),5.13(2H,s),6.78(1H,s),7.14-8.44(11H,m) |
| 231 | 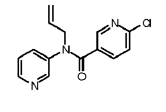 | | (CDCl3) δ:4.51-4.54(2H,m),5.17-5.26(2H,m),5.88-6.02(1H,m),7.19-8.47(7H,m) |
| 232 | 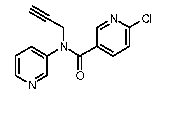 | | (CDCl3) δ:2.32(1H,t),4.69(2H,d),5.88-6.02(1H,m),7.20-8.54(7H,m) |
| 233 | 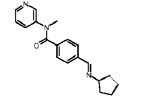 | | (CDCl3) δ: 1.52-1.72 (4H, br), 1.72-1.92 (4H, br), 3.52 (3H, s), 3.64-3.82 (1H, m), 7.14-7.46 (4H, m), 7.73 (2H, d, J=8.4 Hz), 8.19 (1H, s), 8.28-8.46 (2H, m) |
| 234 | 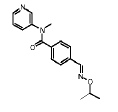 | | (CDCl3) δ: 1.27 (6H, d, J=6.3 Hz), 3.52 (3H, s), 4.32-4.48 (1H, m), 7.13-7.46 (6H, m), 7.94 (1H, s), 8.28-8.45 (2H, m) |
| 235 | 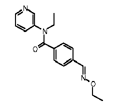 | | (CDCl3) δ: 1.14-1.34 (6H, m), 4.00 (2H, q, J=7.2 Hz), 4.19 (2H, q, J=6.9 Hz), 7.13-7.46 (8H, m), 7.96 (1H, s), 8.29-8.45 (2H, m) |
| 236 | 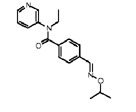 | | (CDCl3) δ: 1.18-1.34 (9H, m), 3.99 (2H, q, J=7.2 Hz), 4.32-4.48 (1H, m), 7.14-7.46 (6H, m), 7.93 (1H, s), 8.30-8.45 (2H, m) |
| 237 | 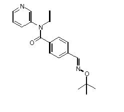 | | (CDCl3) δ: 1.23 (3H, t, J=7.2 Hz), 1.32 (9H, s), 4.00 (2H, q, J=7.2 Hz), 7.14-7.45 (5H, m), 7.92 (1H, s), 8.30-8.45 (2H, m) |
| 238 | 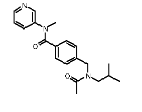 | LC/MS APCI [M+H]+ :340 | (CDCl3) δ: 3.97 (3H, s), 7.32-7.39 (1H, m), 7.86-8.02 (3H, m), 8.18 (2H, d, J= 8.4 Hz), 8.27-8.35 (1H, m), 8.40-8.44 (1H, m), 8.70 (1H, d) |
| 239 | 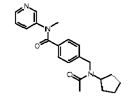 | LC/MS APCI [M+H]+ :352 | |
| 240 | 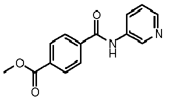 | | (CDCl3) δ: 3.97 (3H, s), 7.32-7.39 (1H, m), 7.86-8.02 (3H, m), 8.18 (2H, d, J= 8.4 Hz), 8.27-8.35 (1H, m), 8.40-8.44 (1H, m), 8.70 (1H, d) |
| 241 | 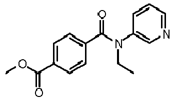 | | (CDCl3) δ: 1.24 (3H, t, J=7.2 Hz), 3.85 (3H, s), 4.00 (2H, q, J=7.2 Hz), 7.16-7.44 (4H, m), 7.86 (2H, d, J=6.9 Hz), 8.29-8.45 (2H, m) |
| 242 | 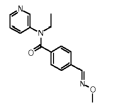 | | (CDCl3) δ: 1.24 (3H, t, J=7.2 Hz), 3.94 (3H, s), 3.99 (2H, q, J=7.2 Hz), 7.14-7.48 (5H, m), 7.94 (1H, s), 8.29--8.45 (2H, m) |
| 243 | 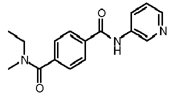 | LC/MS APCI [M+H]+ :284 | |
| 244 | 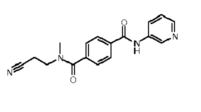 | | (CDCl3) δ: 2.83 (2H, br s), 3.13 (3H, br s), 3.81 (2H, br), 7.31-7.40 (1H, m), 7.42-7.58 (3H, m), 7.92 (2H, d, J=8.4 Hz), 8.33-8.44 (2H, m), 8.73-8.84 (1H, br) |
| 245 | 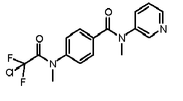 | | (CDCl3) δ: 3.29 (3H, br s), 3.53 (3H, s), 7.13-7.40 (6H, m), 8.37-8.43 (2H, m) |
| 246 | 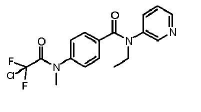 | | (CDCl3) δ: 1.26 (3H, t, J=7.2 Hz), 3.30 (3H, br s), 4.01 (2H, q, J=7.2 Hz), 7.05-7.40 (6H, m), 8.33-8.45 (2H, m) |
| 247 | 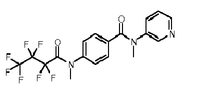 | | (CDCl3) δ: 3.30 (3H, br s), 3.54 (3H, s), 7.07-7.40 (6H, m), 8.41(2H, d) |
| 248 | 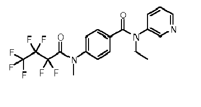 | | (CDCl3) δ: 1.26 (3H, t, J=7.2 Hz), 3.29 (3H, br s), 4.01 (2H, q, J=7.2 Hz), 6.98-7.44 (6H, m), 8.33-8.47 (2H, m) |
| 249 | 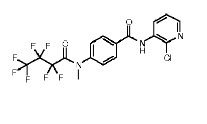 | LC/MS APCI [M+H]+ :458 | |
| 250 | 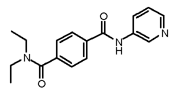 | | (CDCl3) δ: 1.08 (3H, br t), 1.26 (3H, br t), 3.19 (2H, br q), 3.55 (2H, br q), 7.11-7.33 (3H, m), 7.65-7.80 (2H, m), 8.29-8.44 (2H, m), 8.76-8.87 (1H, d), 9.78 (1H, s) |
| 251 | 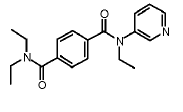 | | (CDCl3) δ: 1.03 (3H, br s), 1.21 (3H, br s), 1.24 (3H, t, J=7.2 Hz), 3.22 (2H, br s), 3.42 (2H, br s), 3.97 (2H, q, J=7.2 Hz), 7.16-7.42 (6H, m), 8.36-8.49 (2H, m) |
| 252 | 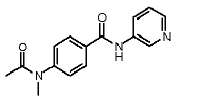 | | (CDCl3) δ: 1.96 (3H, br s), 3.32 (3H, br), 7.31-7.40 (4H, m), 7.97 (2H, d, J=8.1 Hz), 8.27-8.45 (2H, m), 8.67 (1H, d) |
| 253 | 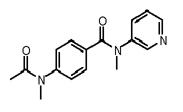 | | (CDCl3) δ: 1.80 (3H, br s), 3.20 (3H, s), 3.54 (3H, s), 7.05 (2H, d, J=8.7 Hz), 7.21-7.46 (4H, m), 8.33-8.43 (2H,m ) |
| 254 | 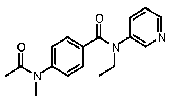 | | (CDCl3) δ: 1.26 (3H, t, J=7.2 Hz), 3.18 (3H, s), 4.01 (2H, q, J=7.2 Hz), 7.03 (2H, d, J=8.4 Hz), 8.29-8.47 (2H, m) |
| 255 | 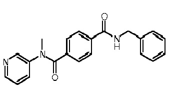 | | (CDCl3) δ: 3.47 (3H, s), 4.56 (2H, d, J=5.7 Hz), 6.84-7.02 (1H, br), 7.11-7.40 (9H, m), 7.62 (2H, d, J=8.4 Hz), 8.22-8.36 (2H, m) |
| 256 | 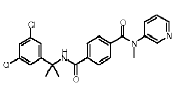 | | (CDCl3) δ: 3.51 (3H, s), 6.70 (1H, s), 7.16-7.42 (7H, m), 7.59 (2H, d, J=8.4 Hz), 8.29-8.42 (2H, m) |
| 257 | 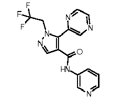 | MS-ESI: [M+H]+ 349 | |
| 258 | 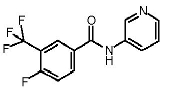 | MS-ESI: [M+H]+ 285 | |
| 259 | 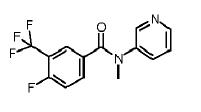 | MS-ESI: [M+H]+ 299 | |
| 260 | 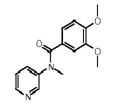 | MS-ESI: [M+H]+ 273 | |
| 261 | 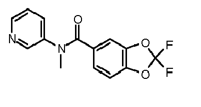 | MS-ESI: [M+H]+ 293 | |
| 262 | 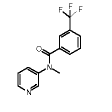 | MS-ESI: [M+H]+ 281 | |
| 263 | 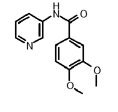 | MS-ESI: [M+H]+ 259 | |
| 264 | 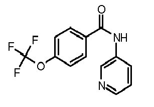 | MS-ESI: [M+H]+ 283 | |
| 265 | 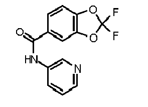 | MS-ESI: [M+H]+ 279 | |
| 266 | 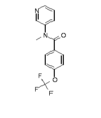 | MS-ESI: [M+H]+ 297 | |
| 267 | 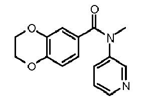 | MS-ESI: [M+H]+ 271 | |
| 268 | 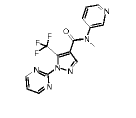 | | (D6-DMSO) δ: 3.4 (s, 3H),7.4(m, 1H), 7.65 (m, 1H), 7.7.5 (m, 1H), 7.85 (m, 1H), 8.45 (m, 2H), 8.95 (m, 2H) +G289 |
| 269 | 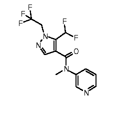 | | (D6-DMSO) δ: 3.4 (s, 3H), 5.1 (q, 2H), 7.1 (s, 1H), 7.36 (t, 1H), 7.4 (m, 1H), 7.75 (m, 1H), 7.95 (m, 2H) |
| 270 | 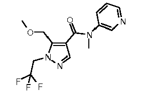 | MS-ESI: [M+H]+ 329 | |
| 271 | 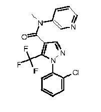 | MS-ESI: [M+H]+ 381 | |
| 272 | 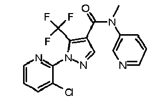 | MS-ESI: [M+H]+ 382 | |
| 273 | 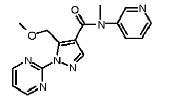 | MS-ESI: [M+H]+ 325 | |
| 274 | 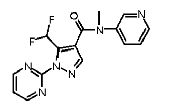 | MS-ESI: [M+H]+ 331 | |
| 275 | 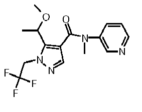 | | (D6-DMSO) δ:1.4 (d, 3H), 3.2 (s, 3H), 3.35 (s, 3H), 4.9-5.1 (m, 3H), 6.9 (s, 1H), 7.4 (m, 1H), 7.7 (m, 1H), 8.4 (m, 2H) |
| 276 | 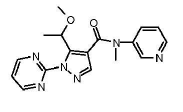 | MS-ESI: [M+H]+ 339 | |
| 277 | 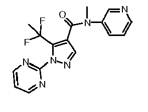 | MS-ESI: [M+H]+ 345 | |
| 278 | 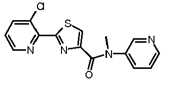 | MS-ESI: [M+H]+ 331 | |
| 279 | 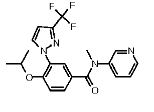 | MS-ESI: [M+H]+ 405 | |
| 280 | 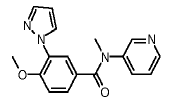 | | (D6-DMSO) δ:3.4 (s, 3H), 3.7 (s, 3H), 6.4 (m, 1H), 7.1 (d, 1H), 7.2 (d, 1H), 7.3 (m, 1H), 7.2 (m, 3H), 8.05 (m, 1H), 8.35 (m, 2H) |
| 281 | 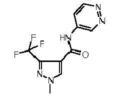 | MS-ESI: [M+H]+ 272 | |
| 282 | 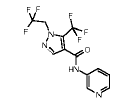 | MS-ESI: [M+H]+ 339 | |
| 283 | 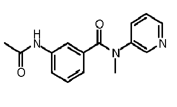 | MS-ESI: [M+H]+ 270 | |
| 284 | 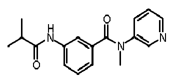 | MS-ESI: [M+H]+ 298 | |
| 285 | 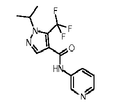 | MS-ESI: [M+H]+ 299 | |
| 286 | 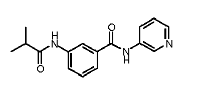 | MS-ESI: [M+H]+ 284 | |
| 287 | 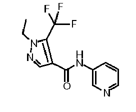 | MS-ESI: [M+H]+ 285 | |
| 288 | 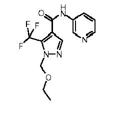 | MS-ESI: [M+H]+ 315 | |
| 289 | 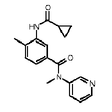 | MS-ESI: [M+H]+ 328 | |
| 290 | 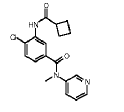 | MS-ESI: [M+H]+ 312 | |
| 291 | 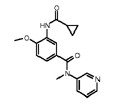 | MS-ESI: [M+H]+ 314 | |
| 292 | 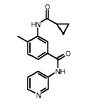 | MS-ESI: [M+H]+ 296 | |
| 293 | 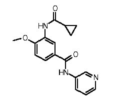 | MS-ESI: [M+H]+ 312 | |
| 294 | 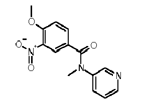 | MS-ESI: [M+H]+ 288 | |
| 295 | 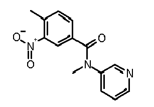 | MS-ESI: [M+H]+ 272 | |
| 296 | 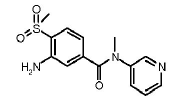 | MS-ESI: [M+H]+ 306 | |
| 297 | 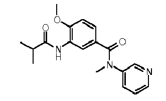 | MS-ESI: [M+H]+ 328 | |
| 298 | 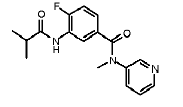 | MS-ESI: [M+H]+ 316 | |
| 299 | 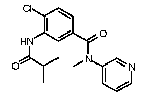 | MS-ESI: [M+H]+ 332 | |
| 300 | 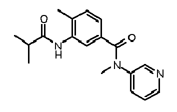 | MS-ESI: [M+H]+ 312 | |
| 301 | 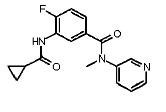 | MS-ESI: [M+H]+ 314 | |
| 302 | 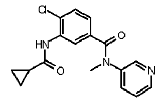 | MS-ESI: [M+H]+ 330 | |
| 303 | 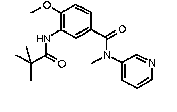 | MS-ESI: [M+H]+ 342 | |
| 304 | 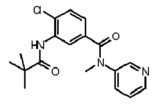 | MS-ESI: [M+H]+ 346 | |
| 305 | 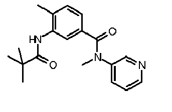 | MS-ESI: [M+H]+ 326 | |
| 306 | 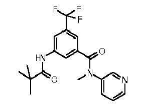 | MS-ESI: [M+H]+ 380 | |
| 307 | 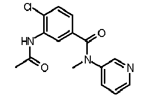 | MS-ESI: [M+H]+ 304 | |
| 308 | 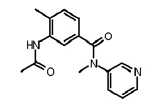 | MS-ESI: [M+H]+ 284 | |
| 309 | 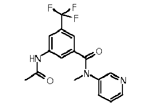 | MS-ESI: [M+H]+ 338 | |
| 310 | 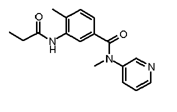 | MS-ESI: [M+H]+ 298 | |
| 311 | 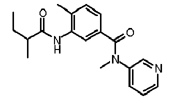 | MS-ESI: [M+H]+ 326 | |
| 312 | 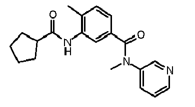 | MS-ESI: [M+H]+ 338 | |
| 313 | 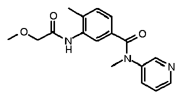 | MS-ESI: [M+H]+ 314 | |
| 314 | 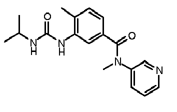 | MS-ESI: [M+H]+ 327 | |
| 315 | 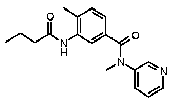 | MS-ESI: [M+H]+ 312 | |
| 316 | 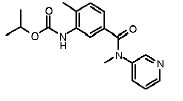 | MS-ESI: [M+H]+ 328 | |
| 317 | 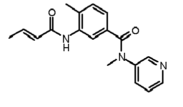 | MS-ESI: [M+H]+ 310 | |
| 318 | 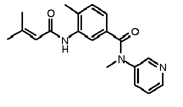 | MS-ESI: [M+H]+ 324 | |
| 319 | 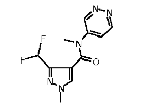 | MS-ESI: [M+H]+ 268 | |
| 320 | 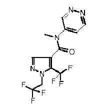 | MS-ESI: [M+H]+ 354 | |
| 321 | 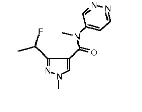 | MS-ESI: [M+H]+ 264 | |
| 322 | 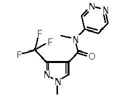 | MS-ESI: [M+H]+ 286 | |
| 323 | 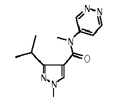 | MS-ESI: [M+H]+ 260 | |
| 324 | 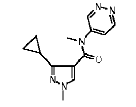 | MS-ESI: [M+H]+ 258 | |
| 325 | 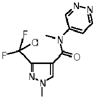 | MS-ESI: [M+H]+ 302 | |
| 326 | 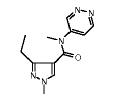 | MS-ESI: [M+H]+ 246 | |
| 327 | 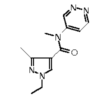 | MS-ESI: [M+H]+ 246 | |
| 328 | 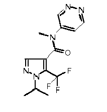 | MS-ESI: [M+H]+ 314 | |
| 329 | 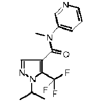 | MS-ESI: [M+H]+ 313 | |
| 330 | 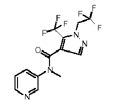 | MS-ESI: [M+H]+ 353 | |
| 331 | 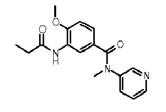 | MS-ESI: [M+H]+ 314 | |
| 332 | 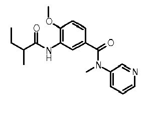 | MS-ESI: [M+H]+ 342 | |
| 333 | 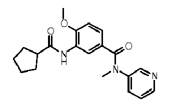 | MS-ESI: [M+H]+ 354 | |
| 334 | 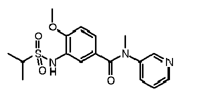 | MS-ESI: [M+H]+ 364 | |
| 335 | 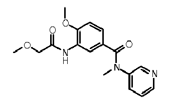 | MS-ESI: [M+H]+ 330 | |
| 336 | 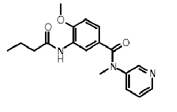 | MS-ESI: [M+H]+ 328 | |
| 337 | 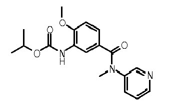 | MS-ESI: [M+H]+ 344 | |
| 338 | 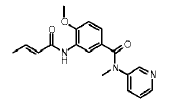 | MS-ESI: [M+H]+ 326 | |

Die Bestimmung der ¹H-NMR-Daten erfolgt mit einem Bruker Avance 400, mit Tetramethylsilan als Referenz (0.0 ppm) und den Lösungsmitteln CD₃CN, CDCl₃, [D₆]-DMSO. Die Charakterisierung der Signalaufspaltung erfolgt mit s = Singulett, br. s = breites Singulett, d = Dublett, t = Triplett, q = Quartett, m = Multiplett, dd = doppeltes Dublett.

Die Bestimmung des M+ mit der LC-MS im sauren Bereich erfolgt bei pH 2,7 Acetonitril (enthält 0,1% Ameisensäure) und Wasser als Eluenten; linearer Gradient von 10% Acetonitril bis 95% Acetonitril Gerät: Agilent 1100 LC-System, Agilent MSD -System, HTS PAL.

Weitere erfindungsgemäße Verbindungen der Formel (I) sind in Tabelle 2 enthalten.

**Tabelle 2:**

| Number (I- ) | 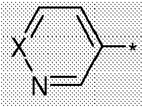 | R¹ | Q |
|---|---|---|---|
| 383 | Pyridin-3-yl | H | 6-Chlorpyridin-3-yl |
| 384 | Pyridin-3-yl | H | 3-(Trifluormethyl)phenyl |
| 385 | Pyridin-3-yl | H | 4-{[Chlor(difluor)acetyl](methyl)amino}phenyl |
| 386 | Pyridin-3-yl | H | 6-{[4-(Trifluormethyl)phenyl]sulfanyl}pyridin-3-yl |
| 387 | Pyridin-3-yl | H | 3-Fluor-4-(methylsulfanyl)phenyl |
| 388 | Pyridin-3-yl | H | 6-[(Methylsulfanyl)methyl]pyridin-3-yl |
| 389 | Pyridin-3-yl | H | 1-Methyl-3-(trifluormethyl)-1H-pyrazol-4-yl |
| 390 | Pyridin-3-yl | H | 4-(Acetylamino)phenyl |
| 391 | Pyridin-3-yl | H | 3-[(4,6-Dimethoxypyrimidin-2-yl)oxy]phenyl |
| 392 | Pyridin-3-yl | H | 4-Methyl-3-nitrophenyl |
| 393 | Pyridin-3-yl | H | 4-{[(4,6-Dimethylpyrimidin-2-yl)sulfanyl]methyl}phenyl |
| 394 | Pyridin-3-yl | H | 4-Nitrophenyl |
| 395 | Pyridin-3-yl | H | 3-[Chlor(difluor)methyl]-1-methyl-1H-pyrazol-4-yl |
| 396 | Pyridin-3-yl | H | 3-(Methylsulfanyl)phenyl |
| 397 | Pyridin-3-yl | H | 2,3-Dihydro-1,4-benzodioxin-6-yl |
| 398 | Pyridin-3-yl | H | 6-(4-Methylphenoxy)pyridin-3-yl |
| 399 | Pyridin-3-yl | H | 6-[(6-Methylpyridin-3-yl)oxy]pyridin-3-yl |
| 400 | Pyridin-3-yl | H | 4-Methoxy-3-nitrophenyl |
| 401 | Pyridin-3-yl | H | 6-[(4,6-Dimethoxy-1,3,5-triazin-2-yl)sulfanyl]pyridin-3-yl |
| 402 | Pyridin-3-yl | H | 6-[(4-Chlorphenyl)sulfanyl]pyridin-3-yl |
| 403 | Pyridin-3-yl | H | 4-[(Cyclopropylcarbonyl)amino]phenyl |
| 404 | Pyridin-3-yl | H | 3-(Acetylamino)phenyl |
| 405 | Pyridin-3-yl | CH₃ | 6-Chlorpyridin-3-yl |
| 406 | Pyridin-3-yl | CH₃ | 4-(Diethylcarbamoyl)phenyl |
| 407 | Pyridin-3-yl | CH₃ | 4-[(4,6-Dimethylpyrimidin-2-yl)sulfanyl]phenyl |
| 408 | Pyridin-3-yl | CH₃ | 4-(Methoxycarbonyl)phenyl |
| 409 | Pyridin-3-yl | CH₃ | 4-[(tert-Butoxyimino)methyl]phenyl |
| 410 | Pyridin-3-yl | Ethyl | 3-[(4,6-Dimethoxypyrimidin-2-yl)oxy]phenyl |
| 411 | Pyridin-3-yl | Ethyl | 6-[(4,6-Dimethoxy-1,3,5-triazin-2-yl)sulfanyl]pyridin-3-yl |
| 412 | Pyridazin-4-yl | CH₃ | 4-[(Methylsulfanyl)methyl]phenyl |
| 413 | Pyridin-3-yl | H | 1-(2,6-Dichlorpyridin-3-yl)-5-(trifluormethyl)-1H-pyrazol-4-yl |
| 414 | Pyridin-3-yl | H | 5-(1-Methoxyethyl)-1-[3-(trifluormethyl)pyridin-2-yl]-1H-pyrazol-4-yl |
| 415 | Pyridin-3-yl | H | 1-Phenyl-1H-pyrazol-4-yl |
| 416 | Pyridin-3-yl | H | 2-[5-Chlor-3-(trifluormethyl)pyridin-2-yl]-1,3-thiazol-4-yl |
| 417 | Pyridin-3-yl | H | 1-[3-(Difluormethyl)pyridin-2-yl]-1H-pyrazol-4-yl |
| 418 | Pyridin-3-yl | H | 5-(Trifluormethyl)-1-[3-(trifluormethyl)pyridin-2-yl]-1H-pyrazol-4-yl |
| 419 | Pyridin-3-yl | H | 1-(2-Bromphenyl)-5-(1-methoxyethyl)-1H-pyrazol-4-yl |
| 420 | Pyridin-3-yl | H | 2-[3-(1-Methoxyethyl)pyridin-2-yl]-1,3-thiazol-4-yl |
| 421 | Pyridin-3-yl | H | 5-(1-Fluorethyl)-1-(pyrimidin-2-yl)-1H-pyrazol-4-yl |
| 422 | Pyridin-3-yl | H | 1-[2-(Trifluormethyl)phenyl]-1H-pyrazol-4-yl |
| 423 | Pyridin-3-yl | H | 1-[3-Chlor-2-(trifluormethyl)phenyl]-5-(methoxymethyl)-1H-pyrazol-4-yl |
| 424 | Pyridin-3-yl | H | 4-[(Trifluoracetyl)amino]phenyl |
| 425 | Pyridin-3-yl | H | 6-(2,5-Dichlorphenoxy)pyridin-3-yl |
| 426 | Pyridin-3-yl | H | 2-(Pyrimidin-2-yl)-1,3-thiazol-5-yl |
| 427 | Pyridin-3-yl | H | 3-(But-2-enoylamino)-4-methoxyphenyl |
| 428 | Pyridin-3-yl | H | 4-Chlor-3-[(cyclopropylcarbonyl)amino]phenyl |
| 429 | Pyridin-3-yl | H | 1-(2,6-Dichlorphenyl)-5-methyl-1H-pyrazol-4-yl |
| 430 | Pyridin-3-yl | H | 1-(2-Chlorpyridin-3-yl)-5-(difluormethyl)-1H-pyrazol-4-yl |
| 431 | Pyridin-3-yl | H | 1-(3-Chlorpyridin-2-yl)-5-(dimethoxymethyl)-1H-pyrazol-4-yl |
| 432 | Pyridin-3-yl | H | 5-(Difluormethyl)-1-(2,2,2-trifluorethyl)-1H-pyrazol-4-yl |
| 433 | Pyridin-3-yl | H | 1-(2-Brom-6-chlorphenyl)-5-(dimethoxymethyl)-1H-pyrazol-4-yl |
| 434 | Pyridin-3-yl | H | 4-Methoxy-3-[3-(pyridin-2-yl)-1H-pyrazol-1-yl]phenyl |
| 435 | Pyridin-3-yl | H | 3-Chlor-4-{[4-(trifluormethyl)-1H-pyrazol-1-yl]methyl}phenyl |
| 436 | Pyridin-3-yl | H | 4-Methyl-3-[(2-methylbutanoyl)amino]phenyl |
| 437 | Pyridin-3-yl | H | 4-{[3-(3-Chlorpyridin-2-yl)-1H-pyrazol-1-yl]methyl}phenyl |
| 438 | Pyridin-3-yl | H | 6-{[4-(Pyridin-2-yl)-1H-pyrazol-1-yl]methyl}pyridin-3-yl |
| 439 | Pyridin-3-yl | H | 4-Methoxy-3-[3-(pyrimidin-2-yl)-1H-pyrazol-1-yl]phenyl |
| 440 | Pyridin-3-yl | H | 4-{[3-(Pyridin-2-yl)-1H-pyrazol-1-yl]methyl}phenyl |
| 441 | Pyridin-3-yl | H | 4-Chlor-3-[(2,2-dimethylpropanoyl)amino]phenyl |
| 442 | Pyridin-3-yl | H | 1-(2-Chlorpyridin-3-yl)-5-(1-methoxyethyl)-1H-pyrazol-4-yl |
| 443 | Pyridin-3-yl | H | 2-(3,5-Dichlorpyridin-2-yl)-1,3-thiazol-4-yl |
| 444 | Pyridin-3-yl | H | 5-Chlor-6-(4-cyanphenoxy)pyridin-3-yl |
| 445 | Pyridin-3-yl | H | 6-(3,4-Dichlorphenoxy)pyridin-3-yl |
| 446 | Pyridin-3-yl | H | 6-[(2-Chlorpyrimidin-4-yl)oxy]pyridin-3-yl |
| 447 | Pyridin-3-yl | H | 1-(3-Chlorpyridin-2-yl)-5-(1-methoxyethyl)-1H-pyrazol-4-yl |
| 448 | Pyridin-3-yl | H | 2-[2-(Trifluormethyl)phenyl]-1,3-thiazol-4-yl |
| 449 | Pyridin-3-yl | H | 2-(2,6-Dichlorpyridin-3-yl)-1,3-thiazol-4-yl |
| 450 | Pyridin-3-yl | H | 1-[3-(Difluormethyl)pyridin-2-yl]-5-methyl-1H-pyrazol-4-yl |
| 451 | Pyridin-3-yl | H | 1-(2,4-Dichlorphenyl)-1H-pyrazol-4-yl |
| 452 | Pyridin-3-yl | H | 2-[4-Chlor-2-(trifluormethyl)phenyl]-1,3-thiazol-5-yl |
| 453 | Pyridin-3-yl | H | 4-[(2-Methylpyrimidin-4-yl)oxy]phenyl |
| 454 | Pyridin-3-yl | H | 6-{[5-(Trifluormethyl)pyridin-2-yl]sulfanyl}pyridin-3-yl |
| 455 | Pyridin-3-yl | H | 4-{[(Propan-2-yloxy)imino]methyl}phenyl |
| 456 | Pyridin-3-yl | H | 4-Methyl-3-{[(propan-2-yloxy)carbonyl]amino}phenyl |
| 457 | Pyridin-3-yl | H | 1-[4-Fluor-2-(trifluormethyl)phenyl]-5-(methoxymethyl)-1H-pyrazol-4-yl |
| 458 | Pyridin-3-yl | H | 5-(1,1-Difluorethyl)-1-(pyrimidin-2-yl)-1H-pyrazol-4-yl |
| 459 | Pyridin-3-yl | H | 1-(2-Chlorphenyl)-5-(1,1-dimethoxyethyl)-1H-pyrazol-4-yl |
| 460 | Pyridin-3-yl | H | 1-(3-Chlorpyridin-2-yl)-5-(1,1difluorethyl)-1H-pyrazol-4-yl |
| 461 | Pyridin-3-yl | H | 1-(2,6-Dichlorpyridin-3-yl)-5-(methoxymethyl)-1H-pyrazol-4-yl |
| 462 | Pyridin-3-yl | H | 6-(Chinolin-6-yloxy)pyridin-3-yl |
| 463 | Pyridin-3-yl | H | 3-(4-Chlor-1H-pyrazol-1-yl)-4-methoxyphenyl |
| 464 | Pyridin-3-yl | H | 2-(2,4-Dichlorphenyl)-1,3-thiazol-4-yl |
| 465 | Pyridin-3-yl | H | 1-(2-Chlorpyridin-3-yl)-5-(1,1-difluorethyl)-1H-pyrazol-4-yl |
| 466 | Pyridin-3-yl | H | 6-{4-[4-(Methylsulfanyl)phenoxy]phenoxy}pyridin-3-yl |
| 467 | Pyridin-3-yl | H | 6-(Chinolin-5-yloxy)pyridin-3-yl |
| 468 | Pyridin-3-yl | H | 1-[4-Chlor-2-(trifluormethyl)phenyl]-5-(difluormethyl)-1H-pyrazol-4-yl |
| 469 | Pyridin-3-yl | H | 1-[2-(1-Methoxyethyl)phenyl]-1H-pyrazol-4-yl |
| 470 | Pyridin-3-yl | H | 4-Methoxy-3-[(2-methylbutanoyl)amino]phenyl |
| 471 | Pyridin-3-yl | H | 6-(2,4-Dichlorphenoxy)pyridin-3-yl |
| 472 | Pyridin-3-yl | H | 4-{[Acetyl(2-methylpropyl)amino]methyl}phenyl |
| 473 | Pyridin-3-yl | H | 2-(2,4-Dichlorphenyl)-1,3-thiazol-5-yl |
| 474 | Pyridin-3-yl | H | 1-(2-Brom-6-chlorphenyl)-5-(difluormethyl)-1H-pyrazol-4-yl |
| 475 | Pyridin-3-yl | H | 4-(Propan-2-yloxy)-3-[3-(trifluormethyl)-1H-pyrazol-1-yl]phenyl |
| 476 | Pyridin-3-yl | H | 6-(3,5-Dimethylphenoxy)pyridin-3-yl |
| 477 | Pyridin-3-yl | H | 1-[4-Chlor-2-(trifluormethyl)phenyl]-5-methyl-1H-pyrazol-4-yl |
| 478 | Pyridin-3-yl | H | 1-(4-Chlorphenyl)-1H-pyrazol-4-yl |
| 479 | Pyridin-3-yl | H | 2-[2-(Difluormethyl)phenyl]-1,3-thiazol-5-yl |
| 480 | Pyridin-3-yl | H | 5-Chlor-6-[4-(trifluormethyl)phenoxy]pyridin-3-yl |
| 481 | Pyridin-3-yl | H | 4-Methyl-3-(propanoylamino)phenyl |
| 482 | Pyridin-3-yl | H | 2-(2-Chlorpyridin-3-yl)-1,3-thiazol-4-yl |
| 483 | Pyridin-3-yl | H | 4-[(2-Methylpropanoyl)amino]phenyl |
| 484 | Pyridin-3-yl | H | 3-[(2,2-Dimethylpropanoyl)amino]-4-methylphenyl |
| 485 | Pyridin-3-yl | H | 4-Methoxy-3-{[(propan-2-yloxy)carbonyl]amino}phenyl |
| 486 | Pyridin-3-yl | H | 1-(2,3-Dichlorphenyl)-5-(methoxymethyl)-1H-pyrazol-4-yl |
| 487 | Pyridin-3-yl | H | 2-[3-(Difluormethyl)pyridin-2-yl]-1,3-thiazol-5-yl |
| 488 | Pyridin-3-yl | H | 3-(Methoxymethyl)-1-methyl-1H-pyrazol-4-yl |
| 489 | Pyridin-3-yl | H | 2-(3,5-Dichlorpyridin-2-yl)-1,3-thiazol-5-yl |
| 490 | Pyridin-3-yl | H | 1-(2,6-Dichlorpyridin-3-yl)-5-(difluormethyl)-1H-pyrazol-4-yl |
| 491 | Pyridin-3-yl | H | 6-[(4,6-Dimethylpyrimidin-2-yl)oxy]pyridin-3-yl |
| 492 | Pyridin-3-yl | H | 5-Chlor-6-(4-nitrophenoxy)pyridin-3-yl |
| 493 | Pyridin-3-yl | H | 2-[2-(Difluormethyl)phenyl]-1,3-thiazol-4-yl |
| 494 | Pyridin-3-yl | H | 6-[(6-Chlorpyridin-2-yl)oxy]pyridin-3-yl |
| 495 | Pyridin-3-yl | H | 3-(Butanoylamino)-4-methoxyphenyl |
| 496 | Pyridin-3-yl | H | 1-(2-Bromphenyl)-5-(trifluormethyl)-1H-pyrazol-4-yl |
| 497 | Pyridin-3-yl | H | 3-{[3-(Trifluormethyl)-1H-pyrazol-1-yl]methyl}phenyl |
| 498 | Pyridin-3-yl | H | 5-(Difluormethyl)-1-(2-methylphenyl)-1H-pyrazol-4-yl |
| 499 | Pyridin-3-yl | H | 2-[2-(1-Methoxyethyl)phenyl]-1,3-thiazol-5-yl |
| 500 | Pyridin-3-yl | H | 1-(2-Chlorphenyl)-5-(1,1-difluorethyl)-1H-pyrazol-4-yl |
| 501 | Pyridin-3-yl | H | 1-(2-Brom-4-fluorphenyl)-1H-pyrazol-4-yl |
| 502 | Pyridin-3-yl | H | 6-[(2-Methoxypyrimidin-4-yl)oxy]pyridin-3-yl |
| 503 | Pyridin-3-yl | H | 1-[2-(1,1-Difluorethyl)phenyl]-5-methyl-1H-pyrazol-4-yl |
| 504 | Pyridin-3-yl | H | 2-(Pyrimidin-2-yl)-1,3-thiazol-4-yl |
| 505 | Pyridin-3-yl | H | 2-{[3-(Trifluormethyl)-1H-pyrazol-1-yl]methyl}pyridin-4-yl |
| 506 | Pyridin-3-yl | H | 1-(2-Chlorpyridin-3-yl)-5-(trifluormethyl)-1H-pyrazol-4-yl |
| 507 | Pyridin-3-yl | H | 1-(2-Chlor-4-fluorphenyl)-1H-pyrazol-4-yl |
| 508 | Pyridin-3-yl | H | 4-Methoxy-3-[4-(pyridin-2-yl)-1H-pyrazol-1-yl]phenyl |
| 509 | Pyridin-3-yl | H | 1-(2-Chlorphenyl)-1H-pyrazol-4-yl |
| 510 | Pyridin-3-yl | H | 6-(2,6-Dichlorphenoxy)pyridin-3-yl |
| 511 | Pyridin-3-yl | H | 1-(2-Chlorphenyl)-5-methyl-1H-pyrazol-4-yl |
| 512 | Pyridin-3-yl | H | 2-[2-(1-Methoxyethyl)phenyl]-1,3-thiazol-4-yl |
| 513 | Pyridin-3-yl | H | 6-{[6-(1H-Pyrazol-1-yl)pyridin-2-yl]oxy}pyridin-3-yl |
| 514 | Pyridin-3-yl | H | 2-[4-Chlor-2-(trifluormethyl)phenyl]-1,3-thiazol-4-yl |
| 515 | Pyridin-3-yl | H | 4-Methoxy-3-(1H-pyrazol-1-yl)phenyl |
| 516 | Pyridin-3-yl | H | 3-(Acetylamino)-4-methylphenyl |
| 517 | Pyridin-3-yl | H | 2-{[4-(Trifluormethyl)-1H-pyrazol-1-yl]methyl}pyridin-4-yl |
| 518 | Pyridin-3-yl | H | 1-(2,4-Difluorphenyl)-1H-pyrazol-4-yl |
| 519 | Pyridin-3-yl | H | 1-(2-Methylphenyl)-5-(trifluormethyl)-1H-pyrazol-4-yl |
| 520 | Pyridin-3-yl | H | 2-[3-(Difluormethyl)pyridin-2-yl]-1,3-thiazol-4-yl |
| 521 | Pyridin-3-yl | H | 6-(3,5-Dichlorphenoxy)pyridin-3-yl |
| 522 | Pyridin-3-yl | H | 6-[(4-Methylphenyl)sulfanyl]pyridin-3-yl |
| 523 | Pyridin-3-yl | H | 1-(2,4-Dichlorphenyl)-5-(methoxymethyl)-1H-pyrazol-4-yl |
| 524 | Pyridin-3-yl | H | 6-{4-[4-(Trifluormethyl)phenoxy]phenoxy}pyridin-3-yl |
| 525 | Pyridin-3-yl | H | 1-(2,6-Dichlorphenyl)-5-(trifluormethyl)-1H-pyrazol-4-yl |
| 526 | Pyridin-3-yl | H | 6-(Naphthalen-1-yloxy)pyridin-3-yl |
| 527 | Pyridin-3-yl | H | 2-(2,6-Dichlorpyridin-3-yl)-1,3-thiazol-5-yl |
| 528 | Pyridin-3-yl | H | 1-[3-Chlor-2-(trifluormethyl)phenyl]-5-(1-methoxyethyl)-1H-pyrazol-4-yl |
| 529 | Pyridin-3-yl | H | 4-Methoxy-3-[(methoxyacetyl)amino]phenyl |
| 530 | Pyridin-3-yl | H | 3-{[4-(Trifluormethyl)-1H-pyrazol-1-yl]methyl}phenyl |
| 531 | Pyridin-3-yl | H | 6-(2-Cyanphenoxy)pyridin-3-yl |
| 532 | Pyridin-3-yl | H | 3-(Dimethoxymethyl)-1-methyl-1H-pyrazol-4-yl |
| 533 | Pyridin-3-yl | H | 1-(3-Brompyridin-2-yl)-5-(methoxymethyl)-1H-pyrazol-4-yl |
| 534 | Pyridin-3-yl | H | 1-(2-Brom-6-chlorphenyl)-5-(trifluormethyl)-1H-pyrazol-4-yl |
| 535 | Pyridin-3-yl | H | 3-{[4-(Trifluormethyl)phenoxy]methyl}phenyl |
| 536 | Pyridin-3-yl | H | 3-(4-Brom-1H-pyrazol-1-yl)-4-methoxyphenyl |
| 537 | Pyridin-3-yl | H | 5-Methyl-1-(2-methylphenyl)-1H-pyrazol-4-yl |
| 538 | Pyridin-3-yl | H | 1-[3-(Difluormethyl)pyridin-2-yl]-5-(methoxymethyl)-1H-pyrazol-4-yl |
| 539 | Pyridin-3-yl | H | 2-[2-(Methoxymethyl)phenyl]-1,3-thiazol-5-yl |
| 540 | Pyridin-3-yl | H | 5-Chlor-6-[(4,5,6-trimethylpyrimidin-2-yl)sulfanyl]pyridin-3-yl |
| 541 | Pyridin-3-yl | H | 6-[3,5-Bis(trifluormethyl)phenoxy]pyridin-3-yl |
| 542 | Pyridin-3-yl | H | 4-{[4-(Pyridin-2-yl)-1H-pyrazol-1-yl]methyl}phenyl |
| 543 | Pyridin-3-yl | H | 1-[3-(1,1-Difluorethyl)pyridin-2-yl]-5-methyl-1H-pyrazol-4-yl |
| 544 | Pyridin-3-yl | H | 1-(2,6-Dichlorphenyl)-5-(difluormethyl)-1H-pyrazol-4-yl |
| 545 | Pyridin-3-yl | H | 1-[4-Fluor-2-(trifluormethyl)phenyl]-5-methyl-1H-pyrazol-4-yl |
| 546 | Pyridin-3-yl | H | 4-Chlor-3-[(cyclobutylcarbonyl)amino]phenyl |
| 547 | Pyridin-3-yl | H | 2-[2-(Methoxymethyl)phenyl]-1,3-thiazol-4-yl |
| 548 | Pyridin-3-yl | H | 4-(1H-Pyrazol-1-ylmethyl)phenyl |
| 549 | Pyridin-3-yl | H | 1-(2-Bromphenyl)-5-methyl-1H-pyrazol-4-yl |
| 550 | Pyridin-3-yl | H | 1-(2,3-Difluorphenyl)-1H-pyrazol-4-yl |
| 551 | Pyridin-3-yl | H | 6-{4-[(Trifluormethyl)sulfanyl]phenoxy}pyridin-3-yl |
| 552 | Pyridin-3-yl | H | 4-{[3-(Pyrimidin-2-yl)-1H-pyrazol-1-yl]methyl}phenyl |
| 553 | Pyridin-3-yl | H | 1-(2-Chlorphenyl)-5-(difluormethyl)-1H-pyrazol-4-yl |
| 554 | Pyridin-3-yl | H | 5-(Difluormethyl)-1-[2-(trifluormethyl)phenyl]-1H-pyrazol-4-yl |
| 555 | Pyridin-3-yl | H | 1-[2-(Dimethoxymethyl)phenyl]-1H-pyrazol-4-yl |
| 556 | Pyridin-3-yl | H | 5-(Methoxymethyl)-1-(pyrimidin-2-yl)-1H-pyrazol-4-yl |
| 557 | Pyridin-3-yl | H | 6-tert-Butoxypyridin-3-yl |
| 558 | Pyridin-3-yl | H | 4-[(Cyclopentylimino)methyl]phenyl |
| 559 | Pyridin-3-yl | H | 1-(3-Chlorphenyl)-1H-pyrazol-4-yl |
| 560 | Pyridin-3-yl | H | 4-Chlor-3-[(2-methylpropanoyl)amino]phenyl |
| 561 | Pyridin-3-yl | H | 1-[2-(Difluormethyl)phenyl]-5-(methoxymethyl)-1H-pyrazol-4-yl |
| 562 | Pyridin-3-yl | H | 4-Methoxy-3-[4-(pyrimidin-2-yl)-1H-pyrazol-1-yl]phenyl |
| 563 | Pyridin-3-yl | H | 1-(2-Chlorphenyl)-5-(methoxymethyl)-1H-pyrazol-4-yl |
| 564 | Pyridin-3-yl | H | 5-Chlor-6-[4-(trifluormethoxy)phenoxy]pyridin-3-yl |
| 565 | Pyridin-3-yl | H | 4-[(tert-Butoxyimino)methyl]phenyl |
| 566 | Pyridin-3-yl | H | 2-(3-Chlorpyridin-2-yl)-1,3-thiazol-5-yl |
| 567 | Pyridin-3-yl | H | 1-(2-Fluorphenyl)-1H-pyrazol-4-yl |
| 568 | Pyridin-3-yl | H | 3-[(2,2-Dimethylpropanoyl)amino]-5-(trifluormethyl)phenyl |
| 569 | Pyridin-3-yl | H | 1-(3-Chlorphenyl)-5-(1,1-dimethoxyethyl)-1H-pyrazol-4-yl |
| 570 | Pyridin-3-yl | H | 1-(2,4-Dichlorphenyl)-5-(1,1-dimethoxyethyl)-1H-pyrazol-4-yl |
| 571 | Pyridin-3-yl | H | 3-[(Cyclopropylcarbonyl)amino]-4-fluorphenyl |
| 572 | Pyridin-3-yl | H | 6-[4-(Dimethylsulfamoyl)phenoxy]pyridin-3-yl |
| 573 | Pyridin-3-yl | H | 1-(2,4-Difluorphenyl)-5-methyl-1H-pyrazol-4-yl |
| 574 | Pyridin-3-yl | H | 3-(Acetylamino)-4-chlorphenyl |
| 575 | Pyridin-3-yl | H | 4-[(4-Brom-1H-pyrazol-1-yl)methyl]phenyl |
| 576 | Pyridin-3-yl | H | 4-Methyl-3-[(propan-2-ylcarbamoyl)amino]phenyl |
| 577 | Pyridin-3-yl | H | 1-(2,2-Diethoxyethyl)-3-(trifluormethyl)-1H-pyrazol-4-yl |
| 578 | Pyridin-3-yl | H | 4-(1H-1,2,4-Triazol-1-ylmethyl)phenyl |
| 579 | Pyridin-3-yl | H | 5-(Methoxymethyl)-1-[2-(trifluormethyl)phenyl]-1H-pyrazol-4-yl |
| 580 | Pyridin-3-yl | H | 5-Chlor-6-[(4,6-dimethylpyrimidin-2-yl)sulfanyl]pyridin-3-yl |
| 581 | Pyridin-3-yl | H | 3-[(2,2-Dimethylpropanoyl)amino]-4-methoxyphenyl |
| 582 | Pyridin-3-yl | H | 2-[2-(Trifluormethyl)pyridin-3-yl]-1,3-thiazol-4-yl |
| 583 | Pyridin-3-yl | H | 5-(Methoxymethyl)-1-(2,2,2-trifluorethyl)-1H-pyrazol-4-yl |
| 584 | Pyridin-3-yl | H | 4-Methoxy-3-(propanoylamino)phenyl |
| 585 | Pyridin-3-yl | H | 4-Methoxy-3-[(2-methylpropanoyl)amino]phenyl |
| 586 | Pyridin-3-yl | H | 6-(Chinolin-7-yloxy)pyridin-3-yl |
| 587 | Pyridin-3-yl | H | 4-{[3-(Trifluormethyl)-1H-pyrazol-1-yl]methyl}phenyl |
| 588 | Pyridin-3-yl | H | 3-Fluor-4-{[4-(trifluormethyl)-1H-pyrazol-1-yl]methyl}phenyl |
| 589 | Pyridin-3-yl | H | 2-[3-(Methoxymethyl)pyridin-2-yl]-1,3-thiazol-5-yl |
| 590 | Pyridin-3-yl | H | 6-({6-[3-(Trifluormethyl)-1H-pyrazol-1-yl]pyridin-2-yl}oxy)pyridin-3-yl |
| 591 | Pyridin-3-yl | H | 3-{[3-(Pyridin-2-yl)-1H-pyrazol-1-yl]methyl}phenyl |
| 592 | Pyridin-3-yl | H | 1-(2-Brom-4-chlorphenyl)-5-methyl-1H-pyrazol-4-yl |
| 593 | Pyridin-3-yl | H | 2-[3-(Trifluormethyl)pyridin-2-yl]-1,3-thiazol-4-yl |
| 594 | Pyridin-3-yl | H | 4-(Benzylcarbamoyl)phenyl |
| 595 | Pyridin-3-yl | H | 4-[(2-Chlorpyrimidin-4-yl)oxy]phenyl |
| 596 | Pyridin-3-yl | H | 1-[3-(Trifluormethyl)pyridin-2-yl]-1H-pyrazol-4-yl |
| 597 | Pyridin-3-yl | H | 5-Chlor-6-(4-chlorphenoxy)pyridin-3-yl |
| 598 | Pyridin-3-yl | H | 3-Amino-4-(methylsulfonyl)phenyl |
| 599 | Pyridin-3-yl | H | 4-Methyl-3-[(2-methylpropanoyl)amino]phenyl |
| 600 | Pyridin-3-yl | H | 1-(2,6-Dibromphenyl)-5-(trifluormethyl)-1H-pyrazol-4-yl |
| 601 | Pyridin-3-yl | H | 3-{[3-(Trifluormethyl)-1H-1,2,4-triazol-1-yl]methyl}phenyl |
| 602 | Pyridin-3-yl | H | 4-[(3,3,3-Trifluorpropanoyl)amino]phenyl |
| 603 | Pyridin-3-yl | H | 1-(2,6-Dichlorphenyl)-5-(dimethoxymethyl)-1H-pyrazol-4-yl |
| 604 | Pyridin-3-yl | H | 1-(4-Chlorphenyl)-5-(1,1-dimethoxyethyl)-1H-pyrazol-4-yl |
| 605 | Pyridin-3-yl | H | 4-({6-[3-(Trifluormethyl)-1H-pyrazol-1-yl]pyridin-2-yl}oxy)phenyl |
| 606 | Pyridin-3-yl | H | 1-[2-(1-Methoxyethyl)phenyl]-5-methyl-1H-pyrazol-4-yl |
| 607 | Pyridin-3-yl | H | 6-(4-Chlor-2-cyanphenoxy)pyridin-3-yl |
| 608 | Pyridin-3-yl | H | 4-{[4-(Trifluormethyl)-1H-pyrazol-1-yl]methyl}phenyl |
| 609 | Pyridin-3-yl | H | 6-Methoxypyridin-3-yl |
| 610 | Pyridin-3-yl | H | 4-[(Ethoxyimino)methyl]phenyl |
| 611 | Pyridin-3-yl | H | 4-{[Acetyl(cyclopentyl)amino]methyl}phenyl |
| 612 | Pyridin-3-yl | H | 3-{[3,5-Bis(trifluormethyl)-1H-pyrazol-1-yl]methyl}phenyl |
| 613 | Pyridin-3-yl | H | 1-(2-Brom-4-chlorphenyl)-1H-pyrazol-4-yl |
| 614 | Pyridin-3-yl | H | 1-(2-Chlorphenyl)-5-(trifluormethyl)-1H-pyrazol-4-yl |
| 615 | Pyridin-3-yl | H | 1-(2,6-Dibromphenyl)-5-(difluormethyl)-1H-pyrazol-4-yl |
| 616 | Pyridin-3-yl | H | 6-[(4-Methoxyphenyl)sulfanyl]pyridin-3-yl |
| 617 | Pyridin-3-yl | H | 1-(2,6-Dichlorphenyl)-5-(1-methoxyethyl)-1H-pyrazol-4-yl |
| 618 | Pyridin-3-yl | H | 4-[(6-Chlorpyridin-2-yl)oxy]phenyl |
| 619 | Pyridin-3-yl | H | 3-Fluor-4-{[3-(trifluormethyl)-1H-pyrazol-1-yl]methyl}phenyl |
| 620 | Pyridin-3-yl | H | 4-{[2-(3,5-Dichlorphenyl)propan-2-yl]carbamoyl}phenyl |
| 621 | Pyridin-3-yl | H | 3-(1H-1,2,4-Triazol-1-ylmethyl)phenyl |
| 622 | Pyridin-3-yl | H | 2-(3-Chlorpyridin-2-yl)-1,3-thiazol-4-yl |
| 623 | Pyridin-3-yl | H | 5-(1-Methoxyethyl)-1-(pyrimidin-2-yl)-1H-pyrazol-4-yl |
| 624 | Pyridin-3-yl | H | 1-(3-Chlorpyridin-2-yl)-5-(1,1-dimethoxyethyl)-1H-pyrazol-4-yl |
| 625 | Pyridin-3-yl | H | 1-(2-Brom-4-fluorphenyl)-5-methyl-1H-pyrazol-4-yl |
| 626 | Pyridin-3-yl | H | 3-(Butanoylamino)-4-methylphenyl |
| 627 | Pyridin-3-yl | H | 5-(Methoxymethyl)-1-[3-(trifluormethyl)pyridin-2-yl]-1H-pyrazol-4-yl |
| 628 | Pyridin-3-yl | H | 1-(2,6-Dichlorpyridin-3-yl)-5-(1-methoxyethyl)-1H-pyrazol-4-yl |
| 629 | Pyridin-3-yl | H | 1-[2-(Difluormethyl)phenyl]-5-methyl-1H-pyrazol-4-yl |
| 630 | Pyridin-3-yl | H | 2-[2-(Trifluormethyl)phenyl]-1,3-thiazol-5-yl |
| 631 | Pyridin-3-yl | H | 2-[2-(Trifluormethyl)pyridin-3-yl]-1,3-thiazol-5-yl |
| 632 | Pyridin-3-yl | H | 2-(2-Chlorpyridin-3-yl)-1,3-thiazol-5-yl |
| 633 | Pyridin-3-yl | H | 1-(2,6-Dichlorpyridin-3-yl)-5-methyl-1H-pyrazol-4-yl |
| 634 | Pyridin-3-yl | H | 1-(3-Chlorpyridin-2-yl)-5-(methoxymethyl)-1H-pyrazol-4-yl |
| 635 | Pyridin-3-yl | H | 3-[(Cyclopentylcarbonyl)amino]-4-methylphenyl |
| 636 | Pyridin-3-yl | H | 6-Ethoxypyridin-3-yl |
| 637 | Pyridin-3-yl | H | 2-(2,6-Dichlorphenyl)-1,3-thiazol-4-yl |
| 638 | Pyridin-3-yl | H | 5-Chlor-6-[(4-methylpyrimidin-2-yl)sulfanyl]pyridin-3-yl |
| 639 | Pyridin-3-yl | H | 1-[4-Chlor-2-(trifluormethyl)phenyl]-5-(1-methoxyethyl)-1H-pyrazol-4-yl |
| 640 | Pyridin-3-yl | H | 6-[3-(Trifluormethoxy)phenoxy]pyridin-3-yl |
| 641 | Pyridin-3-yl | H | 5-(Difluormethyl)-1-(pyrimidin-2-yl)-1H-pyrazol-4-yl |
| 642 | Pyridin-3-yl | H | 1-(3-Brompyridin-2-yl)-5-(1-methoxyethyl)-1H-pyrazol-4-yl |
| 643 | Pyridin-3-yl | H | 4-Methyl-3-[(3-methylbut-2-enoyl)amino]phenyl |
| 644 | Pyridin-3-yl | H | 1-[3-(Methoxymethyl)pyridin-2-yl]-1H-pyrazol-4-yl |
| 645 | Pyridin-3-yl | H | 6-(Naphthalen-2-yloxy)pyridin-3-yl |
| 646 | Pyridin-3-yl | H | 1-(2-Chlorphenyl)-5-(1-methoxyethyl)-1H-pyrazol-4-yl |
| 647 | Pyridin-3-yl | H | 1-[2-(Difluormethyl)phenyl]-5-(1-methoxyethyl)-1H-pyrazol-4-yl |
| 648 | Pyridin-3-yl | H | 3-[(Cyclopentylcarbonyl)amino]-4-methoxyphenyl |
| 649 | Pyridin-3-yl | H | 2-[3-(Trifluormethyl)pyridin-2-yl]-1,3-thiazol-5-yl |
| 650 | Pyridin-3-yl | H | 4-{[3-(Methoxycarbonyl)-1H-1,2,4-triazol-1-yl]methyl}phenyl |
| 651 | Pyridin-3-yl | H | 1-[3-(1,1-Difluorethyl)pyridin-2-yl]-1H-pyrazol-4-yl |
| 652 | Pyridin-3-yl | H | 6-{[3-(Pyridin-2-yl)-1H-pyrazol-1-yl]methyl}pyridin-3-yl |
| 653 | Pyridin-3-yl | H | 1-(3-Chlorpyridin-2-yl)-5-(trifluormethyl)-1H-pyrazol-4-yl |
| 654 | Pyridin-3-yl | H | 2-{[3-(Pyridin-2-yl)-1H-pyrazol-1-yl]methyl}pyridin-4-yl |
| 655 | Pyridin-3-yl | H | 3-[(Methoxyacetyl)amino]-4-methylphenyl |
| 656 | Pyridin-3-yl | H | 1-(3-Brompyridin-2-yl)-5-(trifluormethyl)-1H-pyrazol-4-yl |
| 657 | Pyridin-3-yl | H | 4-[(Methoxyimino)methyl]phenyl |
| 658 | Pyridin-3-yl | H | 1-(Pyrimidin-2-yl)-5-(trifluormethyl)-1H-pyrazol-4-yl |
| 659 | Pyridin-3-yl | H | 2-(2-Chlorphenyl)-1,3-thiazol-4-yl |
| 660 | Pyridin-3-yl | H | 4-Methoxy-3-[(propan-2-ylsulfonyl)amino]phenyl |
| 661 | Pyridin-3-yl | H | 6-(3-Cyanphenoxy)pyridin-3-yl |
| 662 | Pyridin-3-yl | H | 4-Fluor-3-[(2-methylpropanoyl)amino]phenyl |
| 663 | Pyridin-3-yl | H | 1-(2-Bromphenyl)-5-(difluormethyl)-1H-pyrazol-4-yl |
| 664 | Pyridin-3-yl | H | 5-(1-Methoxyethyl)-1-(2,2,2-trifluorethyl)-1H-pyrazol-4-yl |
| 665 | Pyridin-3-yl | H | 1-(2,6-Dichlorphenyl)-5-(1-fluorethyl)-1H-pyrazol-4-yl |
| 666 | Pyridin-3-yl | H | 3-(Acetylamino)-5-(trifluormethyl)phenyl |
| 667 | Pyridin-3-yl | H | 2-[3-(Methoxymethyl)pyridin-2-yl]-1,3-thiazol-4-yl |
| 668 | Pyridin-3-yl | H | 2-[3-(1-Methoxyethyl)pyridin-2-yl]-1,3-thiazol-5-yl |
| 669 | Pyridin-3-yl | H | 1-[3-(Dimethoxymethyl)pyridin-2-yl]-1H-pyrazol-4-yl |
| 670 | Pyridin-3-yl | H | 5-(1-Methoxyethyl)-1-(2-methylphenyl)-1H-pyrazol-4-yl |
| 671 | Pyridin-3-yl | H | 1-(2-Brom-6-chlorphenyl)-5-(1-methoxyethyl)-1H-pyrazol-4-yl |
| 672 | Pyridin-3-yl | H | 5-Chlor-6-[4-(methylsulfonyl)phenoxy]pyridin-3-yl |
| 673 | Pyridin-3-yl | H | 1-[3-(1-Methoxyethyl)pyridin-2-yl]-1H-pyrazol-4-yl |
| 674 | Pyridin-3-yl | H | 6-(4-tert-Butylphenoxy)pyridin-3-yl |
| 675 | Pyridin-3-yl | H | 1-(2,4-Dichlorphenyl)-5-methyl-1H-pyrazol-4-yl |
| 676 | Pyridin-3-yl | H | 3-(But-2-enoylamino)-4-methylphenyl |
| 677 | Pyridin-3-yl | H | 4-{[4-(Pyrimidin-2-yl)-1H-pyrazol-1-yl]methyl}phenyl |
| 678 | Pyridin-3-yl | H | 4-{[6-(1H-Pyrazol-1-yl)pyridin-2-yl]oxy}phenyl |
| 679 | Pyridin-3-yl | H | 4-[(2-Methoxypyrimidin-4-yl)oxy]phenyl |
| 680 | Pyridin-3-yl | H | 1-[4-Chlor-2-(trifluormethyl)phenyl]-5-(methoxymethyl)-1H-pyrazol-4-yl |
| 681 | Pyridin-3-yl | H | 5-(Trifluormethyl)-1-[2-(trifluormethyl)phenyl]-1H-pyrazol-4-yl |
| 682 | Pyridin-3-yl | H | 2-[5-Chlor-3-(trifluormethyl)pyridin-2-yl]-1,3-thiazol-5-yl |
| 683 | Pyridin-3-yl | H | 4-{[4-(3-Chlorpyridin-2-yl)-1H-pyrazol-1-yl]methyl}phenyl |
| 684 | Pyridin-3-yl | H | 4-[(2,2,3,3,4,4,4-Heptafluorbutanoyl)(methyl)amino]phenyl |
| 685 | Pyridin-3-yl | H | 1-(2-Bromphenyl)-5-(methoxymethyl)-1H-pyrazol-4-yl |
| 686 | Pyridin-3-yl | H | 6-[(4-Brom-1H-pyrazol-1-yl)methyl]pyridin-3-yl |
| 687 | Pyridin-3-yl | H | 3-Chlor-4-{[3-(trifluormethyl)-1H-pyrazol-1-yl]methyl}phenyl |
| 688 | Pyridin-3-yl | H | 6-{[4-(Trifluormethyl)-1H-pyrazol-1-yl]methyl}pyridin-3-yl |
| 689 | Pyridin-3-yl | H | 1-(3-Brompyridin-2-yl)-5-(difluormethyl)-1H-pyrazol-4-yl |
| 690 | Pyridin-3-yl | CH₃ | 1-(2,6-Dichlorpyridin-3-yl)-5-(trifluormethyl)-1H-pyrazol-4-yl |
| 691 | Pyridin-3-yl | CH₃ | 5-(1-Methoxyethyl)-1-[3-(trifluormethyl)pyridin-2-yl]-1H-pyrazol-4-yl |
| 692 | Pyridin-3-yl | CH₃ | 1-Phenyl-1H-pyrazol-4-yl |
| 693 | Pyridin-3-yl | CH₃ | 2-[5-Chlor-3-(trifluormethyl)pyridin-2-yl]-1,3-thiazol-4-yl |
| 694 | Pyridin-3-yl | CH₃ | 3-(Trifluormethyl)-1H-pyrazol-4-yl |
| 695 | Pyridin-3-yl | CH₃ | 1-[3-(Difluormethyl)pyridin-2-yl]-1H-pyrazol-4-yl |
| 696 | Pyridin-3-yl | CH₃ | 5-(Trifluormethyl)-1-[3-(trifluormethyl)pyridin-2-yl]-1H-pyrazol-4-yl |
| 697 | Pyridin-3-yl | CH₃ | 1-(2-Bromphenyl)-5-(1-methoxyethyl)-1H-pyrazol-4-yl |
| 698 | Pyridin-3-yl | CH₃ | 1-(1,3-Thiazol-2-ylmethyl)-3-(trifluormethyl)-1H-pyrazol-4-yl |
| 699 | Pyridin-3-yl | CH₃ | 2-[3-(1-Methoxyethyl)pyridin-2-yl]-1,3-thiazol-4-yl |
| 700 | Pyridin-3-yl | CH₃ | 5-(1-Fluorethyl)-1-(pyrimidin-2-yl)-1H-pyrazol-4-yl |
| 701 | Pyridin-3-yl | CH₃ | 1-[2-(Trifluormethyl)phenyl]-1H-pyrazol-4-yl |
| 702 | Pyridin-3-yl | CH₃ | 1-[3-Chlor-2-(trifluormethyl)phenyl]-5-(methoxymethyl)-1H-pyrazol-4-yl |
| 703 | Pyridin-3-yl | CH₃ | 4-[(2-Cyanethyl)(methyl)carbamoyl]phenyl |
| 704 | Pyridin-3-yl | CH₃ | 1-Benzyl-3-(trifluormethyl)-1H-pyrazol-4-yl |
| 705 | Pyridin-3-yl | CH₃ | 2-(Pyrimidin-2-yl)-1,3-thiazol-5-yl |
| 706 | Pyridin-3-yl | CH₃ | 4-(Dimethylcarbamoyl)phenyl |
| 707 | Pyridin-3-yl | CH₃ | 1-(2,6-Dichlorphenyl)-5-methyl-1H-pyrazol-4-yl |
| 708 | Pyridin-3-yl | CH₃ | 1-(2-Chlorpyridin-3-yl)-5-(difluormethyl)-1H-pyrazol-4-yl |
| 709 | Pyridin-3-yl | CH₃ | 1-(3-Chlorpyridin-2-yl)-5-(dimethoxymethyl)-1H-pyrazol-4-yl |
| 710 | Pyridin-3-yl | CH₃ | 1-(2-Brom-6-chlorphenyl)-5-(dimethoxymethyl)-1H-pyrazol-4-yl |
| 711 | Pyridin-3-yl | CH₃ | 4-Methoxy-3-[3-(pyridin-2-yl)-1H-pyrazol-1-yl]phenyl |
| 712 | Pyridin-3-yl | CH₃ | 3-Chlor-4-{[4-(trifluormethyl)-1H-pyrazol-1-yl]methyl}phenyl |
| 713 | Pyridin-3-yl | CH₃ | 4-{[3-(3-Chlorpyridin-2-yl)-1H-pyrazol-1-yl]methyl}phenyl |
| 714 | Pyridin-3-yl | CH₃ | 6-{[4-(Pyridin-2-yl)-1H-pyrazol-1-yl]methyl}pyridin-3-yl |
| 715 | Pyridin-3-yl | CH₃ | 4-Methoxy-3-[3-(pyrimidin-2-yl)-1H-pyrazol-1-yl]phenyl |
| 716 | Pyridin-3-yl | CH₃ | 4-{[3-(Pyridin-2-yl)-1H-pyrazol-1-yl]methyl}phenyl |
| 717 | Pyridin-3-yl | CH₃ | 1-(2-Chlorpyridin-3-yl)-5-(1-methoxyethyl)-1H-pyrazol-4-yl |
| 718 | Pyridin-3-yl | CH₃ | 2-(3,5-Dichlorpyridin-2-yl)-1,3-thiazol-4-yl |
| 719 | Pyridin-3-yl | CH₃ | 6-(Propylamino)pyridin-3-yl |
| 720 | Pyridin-3-yl | CH₃ | 6-[(2-Chlorpyrimidin-4-yl)oxy]pyridin-3-yl |
| 721 | Pyridin-3-yl | CH₃ | 1-(3-Chlorpyridin-2-yl)-5-(1-methoxyethyl)-1H-pyrazol-4-yl |
| 722 | Pyridin-3-yl | CH₃ | 2-[2-(Trifluormethyl)phenyl]-1,3-thiazol-4-yl |
| 723 | Pyridin-3-yl | CH₃ | 2-(2,6-Dichlorpyridin-3-yl)-1,3-thiazol-4-yl |
| 724 | Pyridin-3-yl | CH₃ | 1-Ethyl-5-(trifluormethyl)-1H-pyrazol-4-yl |
| 725 | Pyridin-3-yl | CH₃ | 1-[3-(Difluormethyl)pyridin-2-yl]-5-methyl-1H-pyrazol-4-yl |
| 726 | Pyridin-3-yl | CH₃ | 1-(2,4-Dichlorphenyl)-1H-pyrazol-4-yl |
| 727 | Pyridin-3-yl | CH₃ | 2-[4-Chlor-2-(trifluormethyl)phenyl]-1,3-thiazol-5-yl |
| 728 | Pyridin-3-yl | CH₃ | 4-[(2-Methylpyrimidin-4-yl)oxy]phenyl |
| 729 | Pyridin-3-yl | CH₃ | 6-[(4-Nitrophenyl)sulfanyl]pyridin-3-yl |
| 730 | Pyridin-3-yl | CH₃ | 1-[4-Fluor-2-(trifluormethyl)phenyl]-5-(methoxymethyl)-1H-pyrazol-4-yl |
| 731 | Pyridin-3-yl | CH₃ | 3-{[3,5-Bis(trifluormethyl)-1H-1,2,4-triazol-1-yl]methyl}phenyl |
| 732 | Pyridin-3-yl | CH₃ | 4-{[3,5-Bis(trifluormethyl)-1H-pyrazol-1-yl]methyl}phenyl |
| 733 | Pyridin-3-yl | CH₃ | 1-(2-Chlorphenyl)-5-(1,1-dimethoxyethyl)-1H-pyrazol-4-yl |
| 734 | Pyridin-3-yl | CH₃ | 1-(3-Chlorpyridin-2-yl)-5-(1,1-difluorethyl)-1H-pyrazol-4-yl |
| 735 | Pyridin-3-yl | CH₃ | 1-(2,6-Dichlorpyridin-3-yl)-5-(methoxymethyl)-1H-pyrazol-4-yl |
| 736 | Pyridin-3-yl | CH₃ | 3-(4-Chlor-1H-pyrazol-1-yl)-4-methoxyphenyl |
| 737 | Pyridin-3-yl | CH₃ | 2-(2,4-Dichlorphenyl)-1,3-thiazol-4-yl |
| 738 | Pyridin-3-yl | CH₃ | 1-(2-Chlorpyridin-3-yl)-5-(1,1-difluorethyl)-1H-pyrazol-4-yl |
| 739 | Pyridin-3-yl | CH₃ | 1-[4-Chlor-2-(trifluormethyl)phenyl]-5-(difluormethyl)-1H-pyrazol-4-yl |
| 740 | Pyridin-3-yl | CH₃ | 1-[2-(1-Methoxyethyl)phenyl]-1H-pyrazol-4-yl |
| 741 | Pyridin-3-yl | CH₃ | 2-(2,4-Dichlorphenyl)-1,3-thiazol-5-yl |
| 742 | Pyridin-3-yl | CH₃ | 6-[2-Chlor-4-(methylsulfanyl)phenoxy]pyridin-3-yl |
| 743 | Pyridin-3-yl | CH₃ | 1-(2-Brom-6-chlorphenyl)-5-(difluormethyl)-1H-pyrazol-4-yl |
| 744 | Pyridin-3-yl | CH₃ | 1-[4-Chlor-2-(trifluormethyl)phenyl]-5-methyl-1H-pyrazol-4-yl |
| 745 | Pyridin-3-yl | CH₃ | 1-(4-Chlorphenyl)-1H-pyrazol-4-yl |
| 746 | Pyridin-3-yl | CH₃ | 2-[2-(Difluormethyl)phenyl]-1,3-thiazol-5-yl |
| 747 | Pyridin-3-yl | CH₃ | 1-Ethyl-3-(trifluormethyl)-1H-pyrazol-4-yl |
| 748 | Pyridin-3-yl | CH₃ | 2-(2-Chlorpyridin-3-yl)-1,3-thiazol-4-yl |
| 749 | Pyridin-3-yl | CH₃ | 1-(2,3-Dichlorphenyl)-5-(methoxymethyl)-1H-pyrazol-4-yl |
| 750 | Pyridin-3-yl | CH₃ | 3-(Hydroxymethyl)-1-methyl-1H-pyrazol-4-yl |
| 751 | Pyridin-3-yl | CH₃ | 1-(Propan-2-yl)-3-(trifluormethyl)-1H-pyrazol-4-yl |
| 752 | Pyridin-3-yl | CH₃ | 2-[3-(Difluormethyl)pyridin-2-yl]-1,3-thiazol-5-yl |
| 753 | Pyridin-3-yl | CH₃ | 4-(Pyrrolidin-1-ylcarbonyl)phenyl |
| 754 | Pyridin-3-yl | CH₃ | 2-(3,5-Dichlorpyridin-2-yl)-1,3-thiazol-5-yl |
| 755 | Pyridin-3-yl | CH₃ | 1-(2,6-Dichlorpyridin-3-yl)-5-(difluormethyl)-1H-pyrazol-4-yl |
| 756 | Pyridin-3-yl | CH₃ | 2-[2-(Difluormethyl)phenyl]-1,3-thiazol-4-yl |
| 757 | Pyridin-3-yl | CH₃ | 1-(Difluormethyl)-3-(trifluormethyl)-1H-pyrazol-4-yl |
| 758 | Pyridin-3-yl | CH₃ | 6-[(6-Chlorpyridin-2-yl)oxy]pyridin-3-yl |
| 759 | Pyridin-3-yl | CH₃ | 1-(2-Bromphenyl)-5-(trifluormethyl)-1H-pyrazol-4-yl |
| 760 | Pyridin-3-yl | CH₃ | 5-(Difluormethyl)-1-(2-methylphenyl)-1H-pyrazol-4-yl |
| 761 | Pyridin-3-yl | CH₃ | 2-[2-(1-Methoxyethyl)phenyl]-1,3-thiazol-5-yl |
| 762 | Pyridin-3-yl | CH₃ | 1-(2-Chlorphenyl)-5-(1,1-difluorethyl)-1H-pyrazol-4-yl |
| 763 | Pyridin-3-yl | CH₃ | 1-(2-Brom-4-fluorphenyl)-1H-pyrazol-4-yl |
| 764 | Pyridin-3-yl | CH₃ | 6-[(Cyclopropylmethyl)amino]pyridin-3-yl |
| 765 | Pyridin-3-yl | CH₃ | 6-[(2-Methoxypyrimidin-4-yl)oxy]pyridin-3-yl |
| 766 | Pyridin-3-yl | CH₃ | 1-[2-(1,1-Difluorethyl)phenyl]-5-methyl-1H-pyrazol-4-yl |
| 767 | Pyridin-3-yl | CH₃ | 3-{[3,5-Bis(trifluormethyl)-1H-1,2,4-triazol-1-yl]methyl}-4-chlorphenyl |
| 768 | Pyridin-3-yl | CH₃ | 6-(Morpholin-4-yl)pyridin-3-yl |
| 769 | Pyridin-3-yl | CH₃ | 2-(Pyrimidin-2-yl)-1,3-thiazol-4-yl |
| 770 | Pyridin-3-yl | CH₃ | 2-{[3-(Trifluormethyl)-1H-pyrazol-1-yl]methyl}pyridin-4-yl |
| 771 | Pyridin-3-yl | CH₃ | 1-(2-Chlorpyridin-3-yl)-5-(trifluormethyl)-1H-pyrazol-4-yl |
| 772 | Pyridin-3-yl | CH₃ | 6-(4-Bromphenoxy)pyridin-3-yl |
| 773 | Pyridin-3-yl | CH₃ | 1-(2-Chlor-4-fluorphenyl)-1H-pyrazol-4-yl |
| 774 | Pyridin-3-yl | CH₃ | 4-Methoxy-3-[4-(pyridin-2-yl)-1H-pyrazol-1-yl]phenyl |
| 775 | Pyridin-3-yl | CH₃ | 1-(2-Chlorphenyl)-1H-pyrazol-4-yl |
| 776 | Pyridin-3-yl | CH₃ | 1-(2-Chlorphenyl)-5-methyl-1H-pyrazol-4-yl |
| 777 | Pyridin-3-yl | CH₃ | 2-[2-(1-Methoxyethyl)phenyl]-1,3-thiazol-4-yl |
| 778 | Pyridin-3-yl | CH₃ | 6-{[6-(1H-Pyrazol-1-yl)pyridin-2-yl]oxy}pyridin-3-yl |
| 779 | Pyridin-3-yl | CH₃ | 2-[4-Chlor-2-(trifluormethyl)phenyl]-1,3-thiazol-4-yl |
| 780 | Pyridin-3-yl | CH₃ | 2-{[4-(Trifluormethyl)-1H-pyrazol-1-yl]methyl}pyridin-4-yl |
| 781 | Pyridin-3-yl | CH₃ | 1-(2,4-Difluorphenyl)-1H-pyrazol-4-yl |
| 782 | Pyridin-3-yl | CH₃ | 1-(2-Methylphenyl)-5-(trifluormethyl)-1H-pyrazol-4-yl |
| 783 | Pyridin-3-yl | CH₃ | 2-[3-(Difluormethyl)pyridin-2-yl]-1,3-thiazol-4-yl |
| 784 | Pyridin-3-yl | CH₃ | 1-(Pyrimidin-2-ylmethyl)-3-(trifluormethyl)-1H-pyrazol-4-yl |
| 785 | Pyridin-3-yl | CH₃ | 1-(2,4-Dichlorphenyl)-5-(methoxymethyl)-1H-pyrazol-4-yl |
| 786 | Pyridin-3-yl | CH₃ | 1-(2,6-Dichlorphenyl)-5-(trifluormethyl)-1H-pyrazol-4-yl |
| 787 | Pyridin-3-yl | CH₃ | 2-(2,6-Dichlorpyridin-3-yl)-1,3-thiazol-5-yl |
| 788 | Pyridin-3-yl | CH₃ | 6-{[(4,6-Dimethoxypyrimidin-2-yl)methyl]sulfanyl}pyridin-3-yl |
| 789 | Pyridin-3-yl | CH₃ | 1-[3-Chlor-2-(trifluormethyl)phenyl]-5-(1-methoxyethyl)-1H-pyrazol-4-yl |
| 790 | Pyridin-3-yl | CH₃ | 3-{[4-(Trifluormethyl)-1H-pyrazol-1-yl]methyl}phenyl |
| 791 | Pyridin-3-yl | CH₃ | 1-(Ethoxymethyl)-3-(trifluormethyl)-1H-pyrazol-4-yl |
| 792 | Pyridin-3-yl | CH₃ | 1-(3-Brompyridin-2-yl)-5-(methoxymethyl)-1H-pyrazol-4-yl |
| 793 | Pyridin-3-yl | CH₃ | 1-(2-Brom-6-chlorphenyl)-5-(trifluormethyl)-1H-pyrazol-4-yl |
| 794 | Pyridin-3-yl | CH₃ | 1-Ethenyl-3-(trifluormethyl)-1H-pyrazol-4-yl |
| 795 | Pyridin-3-yl | CH₃ | 3-(4-Brom-1H-pyrazol-1-yl)-4-methoxyphenyl |
| 796 | Pyridin-3-yl | CH₃ | 5-Methyl-1-(2-methylphenyl)-1H-pyrazol-4-yl |
| 797 | Pyridin-3-yl | CH₃ | 1-[3-(Difluormethyl)pyridin-2-yl]-5-(methoxymethyl)-1H-pyrazol-4-yl |
| 798 | Pyridin-3-yl | CH₃ | 2-[2-(Methoxymethyl)phenyl]-1,3-thiazol-5-yl |
| 799 | Pyridin-3-yl | CH₃ | 1-(Ethoxymethyl)-5-(trifluormethyl)-1H-pyrazol-4-yl |
| 800 | Pyridin-3-yl | CH₃ | 4-{[4-(Pyridin-2-yl)-1H-pyrazol-1-yl]methyl}phenyl |
| 801 | Pyridin-3-yl | CH₃ | 1-[3-(1,1-Difluorethyl)pyridin-2-yl]-5-methyl-1H-pyrazol-4-yl |
| 802 | Pyridin-3-yl | CH₃ | 1-(2,6-Dichlorphenyl)-5-(difluormethyl)-1H-pyrazol-4-yl |
| 803 | Pyridin-3-yl | CH₃ | 1-[4-Fluor-2-(trifluormethyl)phenyl]-5-methyl-1H-pyrazol-4-yl |
| 804 | Pyridin-3-yl | CH₃ | 2-[2-(Methoxymethyl)phenyl]-1,3-thiazol-4-yl |
| 805 | Pyridin-3-yl | CH₃ | 1-(2-Bromphenyl)-5-methyl-1H-pyrazol-4-yl |
| 806 | Pyridin-3-yl | CH₃ | 1-(2,3-Difluorphenyl)-1H-pyrazol-4-yl |
| 807 | Pyridin-3-yl | CH₃ | 4-{[3-(Pentafluorethyl)-1H-1,2,4-triazol-1-yl]methyl}phenyl |
| 808 | Pyridin-3-yl | CH₃ | 4-{[3-(Pyrimidin-2-yl)-1H-pyrazol-1-yl]methyl}phenyl |
| 809 | Pyridin-3-yl | CH₃ | 1-(2-Chlorphenyl)-5-(difluormethyl)-1H-pyrazol-4-yl |
| 810 | Pyridin-3-yl | CH₃ | 5-(Difluormethyl)-1-[2-(trifluormethyl)phenyl]-1H-pyrazol-4-yl |
| 811 | Pyridin-3-yl | CH₃ | 1-[2-(Dimethoxymethyl)phenyl]-1H-pyrazol-4-yl |
| 812 | Pyridin-3-yl | CH₃ | 1-(3-Chlorphenyl)-1H-pyrazol-4-yl |
| 813 | Pyridin-3-yl | CH₃ | 4-(Propylsulfanyl)phenyl |
| 814 | Pyridin-3-yl | CH₃ | 1-[2-(Difluormethyl)phenyl]-5-(methoxymethyl)-1H-pyrazol-4-yl |
| 815 | Pyridin-3-yl | CH₃ | 4-Methoxy-3-[4-(pyrimidin-2-yl)-1H-pyrazol-1-yl]phenyl |
| 816 | Pyridin-3-yl | CH₃ | 1-(2-Chlorphenyl)-5-(methoxymethyl)-1H-pyrazol-4-yl |
| 817 | Pyridin-3-yl | CH₃ | 2-(3-Chlorpyridin-2-yl)-1,3-thiazol-5-yl |
| 818 | Pyridin-3-yl | CH₃ | 1-(2-Fluorphenyl)-1H-pyrazol-4-yl |
| 819 | Pyridin-3-yl | CH₃ | 6-{[2-(Morpholin-4-yl)ethyl]amino}pyridin-3-yl |
| 820 | Pyridin-3-yl | CH₃ | 3-Formyl-1-methyl-1H-pyrazol-4-yl |
| 821 | Pyridin-3-yl | CH₃ | 1-(3-Chlorphenyl)-5-(l,l-dimethoxyethyl)-1H-pyrazol-4-yl |
| 822 | Pyridin-3-yl | CH₃ | 6-{[5-(Methoxycarbonyl)pyridin-3-yl]oxy}pyridin-3-yl |
| 823 | Pyridin-3-yl | CH₃ | 3-{[4-(Trifluormethyl)benzyl]oxy}phenyl |
| 824 | Pyridin-3-yl | CH₃ | 1-(2,4-Dichlorphenyl)-5-(1,1-dimethoxyethyl)-1H-pyrazol-4-yl |
| 825 | Pyridin-3-yl | CH₃ | 4-{[4-Brom-3-(trifluormethyl)-1H-pyrazol-1-yl]methyl}phenyl |
| 826 | Pyridin-3-yl | CH₃ | 5-Methyl-1-(2-methylpyridin-3-yl)-1H-pyrazol-4-yl |
| 827 | Pyridin-3-yl | CH₃ | 1-(2,4-Difluorphenyl)-5-methyl-1H-pyrazol-4-yl |
| 828 | Pyridin-3-yl | CH₃ | 4-[(4-Brom-1H-pyrazol-1-yl)methyl]phenyl |
| 829 | Pyridin-3-yl | CH₃ | 6-(1H-Pyrazol-1-yl)pyridin-3-yl |
| 830 | Pyridin-3-yl | CH₃ | 5-(Methoxymethyl)-1-[2-(trifluormethyl)phenyl]-1H-pyrazol-4-yl |
| 831 | Pyridin-3-yl | CH₃ | 5-Methylpyridin-3-yl |
| 832 | Pyridin-3-yl | CH₃ | 2-[2-(Trifluormethyl)pyridin-3-yl]-1,3-thiazol-4-yl |
| 833 | Pyridin-3-yl | CH₃ | 3-Methyl-1H-pyrazol-4-yl |
| 834 | Pyridin-3-yl | CH₃ | 3-Fluor-4-{[4-(trifluormethyl)-1H-pyrazol-1-yl]methyl}phenyl |
| 835 | Pyridin-3-yl | CH₃ | 2-[3-(Methoxymethyl)pyridin-2-yl]-1,3-thiazol-5-yl |
| 836 | Pyridin-3-yl | CH₃ | 6-({6-[3-(Trifluormethyl)-1H-pyrazol-1-yl]pyridin-2-yl}oxy)pyridin-3-yl |
| 837 | Pyridin-3-yl | CH₃ | 3-{[3-(Pyridin-2-yl)-1H-pyrazol-1-yl]methyl}phenyl |
| 838 | Pyridin-3-yl | CH₃ | 1-(2-Brom-4-chlorphenyl)-5-methyl-1H-pyrazol-4-yl |
| 839 | Pyridin-3-yl | CH₃ | 2-[3-(Trifluormethyl)pyridin-2-yl]-1,3-thiazol-4-yl |
| 840 | Pyridin-3-yl | CH₃ | 4-[(2-Chlorpyrimidin-4-yl)oxy]phenyl |
| 841 | Pyridin-3-yl | CH₃ | 1-[3-(Trifluormethyl)pyridin-2-yl]-1H-pyrazol-4-yl |
| 842 | Pyridin-3-yl | CH₃ | 3-Cyan-4-(1H-1,2,4-triazol-1-yl)phenyl |
| 843 | Pyridin-3-yl | CH₃ | 1-(2,6-Dibromphenyl)-5-(trifluormethyl)-1H-pyrazol-4-yl |
| 844 | Pyridin-3-yl | CH₃ | 5-(Pyrazin-2-yl)-1-(2,2,2-trifluorethyl)-1H-pyrazol-4-yl |
| 845 | Pyridin-3-yl | CH₃ | 1-(2,6-Dichlorphenyl)-5-(dimethoxymethyl)-1H-pyrazol-4-yl |
| 846 | Pyridin-3-yl | CH₃ | 1-(4-Chlorphenyl)-5-(1,1-dimethoxyethyl)-1H-pyrazol-4-yl |
| 847 | Pyridin-3-yl | CH₃ | 4-({6-[3-(Trifluormethyl)-1H-pyrazol-1-yl]pyridin-2-yl}oxy)phenyl |
| 848 | Pyridin-3-yl | CH₃ | 1-[2-(1-Methoxyethyl)phenyl]-5-methyl-1H-pyrazol-4-yl |
| 849 | Pyridin-3-yl | CH₃ | 1-Propyl-3-(trifluormethyl)-1H-pyrazol-4-yl |
| 850 | Pyridin-3-yl | CH₃ | 4-{[4-(Trifluormethyl)-1H-pyrazol-1-yl]methyl}phenyl |
| 851 | Pyridin-3-yl | CH₃ | 4-[(Ethoxyimino)methyl]phenyl |
| 852 | Pyridin-3-yl | CH₃ | 1-(2-Brom-4-chlorphenyl)-1H-pyrazol-4-yl |
| 853 | Pyridin-3-yl | CH₃ | 1-(2,6-Dibromphenyl)-5-(difluormethyl)-1H-pyrazol-4-yl |
| 854 | Pyridin-3-yl | CH₃ | 1-(2,6-Dichlorphenyl)-5-(1-methoxyethyl)-1H-pyrazol-4-yl |
| 855 | Pyridin-3-yl | CH₃ | 4-[(6-Chlorpyridin-2-yl)oxy]phenyl |
| 856 | Pyridin-3-yl | CH₃ | 3-Fluor-4-{[3-(trifluormethyl)-1H-pyrazol-1-yl]methyl}phenyl |
| 857 | Pyridin-3-yl | CH₃ | 6-{[3-(Trifluormethyl)-1H-pyrazol-1-yl]methyl}pyridin-3-yl |
| 858 | Pyridin-3-yl | CH₃ | 1-(3-Chlorpyridin-2-yl)-5-(1,1-dimethoxyethyl)-1H-pyrazol-4-yl |
| 859 | Pyridin-3-yl | CH₃ | 1-(2-Brom-4-fluorphenyl)-5-methyl-1H-pyrazol-4-yl |
| 860 | Pyridin-3-yl | CH₃ | 5-(Methoxymethyl)-1-[3-(trifluormethyl)pyridin-2-yl]-1H-pyrazol-4-yl |
| 861 | Pyridin-3-yl | CH₃ | 6-[3-(Trifluormethyl)-1H-pyrazol-1-yl]pyridin-3-yl |
| 862 | Pyridin-3-yl | CH₃ | 1-(2,6-Dichlorpyridin-3-yl)-5-(1-methoxyethyl)-1H-pyrazol-4-yl |
| 863 | Pyridin-3-yl | CH₃ | 1-[2-(Difluormethyl)phenyl]-5-methyl-1H-pyrazol-4-yl |
| 864 | Pyridin-3-yl | CH₃ | 2-[2-(Trifluormethyl)phenyl]-1,3-thiazol-5-yl |
| 865 | Pyridin-3-yl | CH₃ | 2-[2-(Trifluormethyl)pyridin-3-yl]-1,3-thiazol-5-yl |
| 866 | Pyridin-3-yl | CH₃ | 2-(2-Chlorpyridin-3-yl)-1,3-thiazol-5-yl |
| 867 | Pyridin-3-yl | CH₃ | 1-(2,6-Dichlorpyridin-3-yl)-5-methyl-1H-pyrazol-4-yl |
| 868 | Pyridin-3-yl | CH₃ | 1-(3-Chlorpyridin-2-yl)-5-(methoxymethyl)-1H-pyrazol-4-yl |
| 869 | Pyridin-3-yl | CH₃ | 6-(Piperidin-1-yl)pyridin-3-yl |
| 870 | Pyridin-3-yl | CH₃ | 2-(2,6-Dichlorphenyl)-1,3-thiazol-4-yl |
| 871 | Pyridin-3-yl | CH₃ | 1-[4-Chlor-2-(trifluormethyl)phenyl]-5-(1-methoxyethyl)-1H-pyrazol-4-yl |
| 872 | Pyridin-3-yl | CH₃ | 4-[Ethyl(methyl)carbamoyl]phenyl |
| 873 | Pyridin-3-yl | CH₃ | 1-(3-Brompyridin-2-yl)-5-(1-methoxyethyl)-1H-pyrazol-4-yl |
| 874 | Pyridin-3-yl | CH₃ | 1-[3-(Methoxymethyl)pyridin-2-yl]-1H-pyrazol-4-yl |
| 875 | Pyridin-3-yl | CH₃ | 1-(2-Chlorphenyl)-5-(1-methoxyethyl)-1H-pyrazol-4-yl |
| 876 | Pyridin-3-yl | CH₃ | 1-[2-(Difluormethyl)phenyl]-5-(1-methoxyethyl)-1H-pyrazol-4-yl |
| 877 | Pyridin-3-yl | CH₃ | 1-(3-Chlorpyridin-2-yl)-5-methyl-1H-pyrazol-4-yl |
| 878 | Pyridin-3-yl | CH₃ | 6-[4-(Methylsulfanyl)phenoxy]pyridin-3-yl |
| 879 | Pyridin-3-yl | CH₃ | 2-[3-(Trifluormethyl)pyridin-2-yl]-1,3-thiazol-5-yl |
| 880 | Pyridin-3-yl | CH₃ | 1-[3-(1,1-Difluorethyl)pyridin-2-yl]-1H-pyrazol-4-yl |
| 881 | Pyridin-3-yl | CH₃ | 6-{[3-(Pyridin-2-yl)-1H-pyrazol-1-yl]methyl}pyridin-3-yl |
| 882 | Pyridin-3-yl | CH₃ | 2-{[3-(Pyridin-2-yl)-1H-pyrazol-1-yl]methyl}pyridin-4-yl |
| 883 | Pyridin-3-yl | CH₃ | 1-(3-Brompyridin-2-yl)-5-(trifluormethyl)-1H-pyrazol-4-yl |
| 884 | Pyridin-3-yl | CH₃ | 4-[(Methoxyimino)methyl]phenyl |
| 885 | Pyridin-3-yl | CH₃ | 2-(2-Chlorphenyl)-1,3-thiazol-4-yl |
| 886 | Pyridin-3-yl | CH₃ | 1-(2-Bromphenyl)-5-(difluormethyl)-1H-pyrazol-4-yl |
| 887 | Pyridin-3-yl | CH₃ | 1-(2,6-Dichlorphenyl)-5-(1-fluorethyl)-1H-pyrazol-4-yl |
| 888 | Pyridin-3-yl | CH₃ | 6-(Pyrrolidin-1-yl)pyridin-3-yl |
| 889 | Pyridin-3-yl | CH₃ | 2-[3-(Methoxymethyl)pyridin-2-yl]-1,3-thiazol-4-yl |
| 890 | Pyridin-3-yl | CH₃ | 2-[3-(1-Methoxyethyl)pyridin-2-yl]-1,3-thiazol-5-yl |
| 891 | Pyridin-3-yl | CH₃ | 1-[3-(Dimethoxymethyl)pyridin-2-yl]-1H-pyrazol-4-yl |
| 892 | Pyridin-3-yl | CH₃ | 5-(1-Methoxyethyl)-1-(2-methylphenyl)-1H-pyrazol-4-yl |
| 893 | Pyridin-3-yl | CH₃ | 1-Methyl-1H-benzotriazol-5-yl |
| 894 | Pyridin-3-yl | CH₃ | 4-[(Butylsulfanyl)methyl]phenyl |
| 895 | Pyridin-3-yl | CH₃ | 1H-Pyrazol-4-yl |
| 896 | Pyridin-3-yl | CH₃ | 1-(2-Brom-6-chlorphenyl)-5-(1-methoxyethyl)-1H-pyrazol-4-yl |
| 897 | Pyridin-3-yl | CH₃ | 1-[3-(1-Methoxyethyl)pyridin-2-yl]-1H-pyrazol-4-yl |
| 898 | Pyridin-3-yl | CH₃ | 4-[(6-Methylpyridin-3-yl)oxy]phenyl |
| 899 | Pyridin-3-yl | CH₃ | 3-Methoxy-1-methyl-1H-pyrazol-4-yl |
| 900 | Pyridin-3-yl | CH₃ | 1-(2,4-Dichlorphenyl)-5-methyl-1H-pyrazol-4-yl |
| 901 | Pyridin-3-yl | CH₃ | 4-{[4-(Pyrimidin-2-yl)-1H-pyrazol-1-yl]methyl}phenyl |
| 902 | Pyridin-3-yl | CH₃ | 4-{[6-(1H-Pyrazol-1-yl)pyridin-2-yl]oxy}phenyl |
| 903 | Pyridin-3-yl | CH₃ | 4-{[3,5-Bis(trifluormethyl)-1H-1,2,4-triazol-1-yl]methyl}-3-chlorphenyl |
| 904 | Pyridin-3-yl | CH₃ | 4-[(2-Methoxypyrimidin-4-yl)oxy]phenyl |
| 905 | Pyridin-3-yl | CH₃ | 1-[4-Chlor-2-(trifluormethyl)phenyl]-5-(methoxymethyl)-1H-pyrazol-4-yl |
| 906 | Pyridin-3-yl | CH₃ | 5-(Trifluormethyl)-1-[2-(trifluormethyl)phenyl]-1H-pyrazol-4-yl |
| 907 | Pyridin-3-yl | CH₃ | 6-(Dimethylamino)pyridin-3-yl |
| 908 | Pyridin-3-yl | CH₃ | 2-[5-Chlor-3-(trifluormethyl)pyridin-2-yl]-1,3-thiazol-5-yl |
| 909 | Pyridin-3-yl | CH₃ | 4-{[4-(3-Chlorpyridin-2-yl)-1H-pyrazol-1-yl]methyl}phenyl |
| 910 | Pyridin-3-yl | CH₃ | 1-(2-Bromphenyl)-5-(methoxymethyl)-1H-pyrazol-4-yl |
| 911 | Pyridin-3-yl | CH₃ | 6-[(4-Brom-1H-pyrazol-1-yl)methyl]pyridin-3-yl |
| 912 | Pyridin-3-yl | CH₃ | 3-Chlor-4-{[3-(trifluormethyl)-1H-pyrazol-1-yl]methyl}phenyl |
| 913 | Pyridin-3-yl | CH₃ | 6-{[4-(Trifluormethyl)-1H-pyrazol-1-yl]methyl}pyridin-3-yl |
| 914 | Pyridin-3-yl | CH₃ | 1-(3-Brompyridin-2-yl)-5-(difluormethyl)-1H-pyrazol-4-yl |
| 915 | Pyridin-3-yl | Ethyl | 1-(2,6-Dichlorpyridin-3-yl)-5-(trifluormethyl)-1H-pyrazol-4-yl |
| 916 | Pyridin-3-yl | Ethyl | 5-(1-Methoxyethyl)-1-[3-(trifluormethyl)pyridin-2-yl]-1H-pyrazol-4-yl |
| 917 | Pyridin-3-yl | Ethyl | 1-Phenyl-1H-pyrazol-4-yl |
| 918 | Pyridin-3-yl | Ethyl | 2-[5-Chlor-3-(trifluormethyl)pyridin-2-yl]-1,3-thiazol-4-yl |
| 919 | Pyridin-3-yl | Ethyl | 3-(Trifluormethyl)-1H-pyrazol-4-yl |
| 920 | Pyridin-3-yl | Ethyl | 1-[3-(Difluormethyl)pyridin-2-yl]-1H-pyrazol-4-yl |
| 921 | Pyridin-3-yl | Ethyl | 5-(Trifluormethyl)-1-[3-(trifluormethyl)pyridin-2-yl]-1H-pyrazol-4-yl |
| 922 | Pyridin-3-yl | Ethyl | 1-(2-Bromphenyl)-5-(1-methoxyethyl)-1H-pyrazol-4-yl |
| 923 | Pyridin-3-yl | Ethyl | 1-(1,3-Thiazol-2-ylmethyl)-3-(trifluormethyl)-1H-pyrazol-4-yl |
| 924 | Pyridin-3-yl | Ethyl | 3-(Trifluormethyl)phenyl |
| 925 | Pyridin-3-yl | Ethyl | 2-[3-(1-Methoxyethyl)pyridin-2-yl]-1,3-thiazol-4-yl |
| 926 | Pyridin-3-yl | Ethyl | 5-(1-Fluorethyl)-1-(pyrimidin-2-yl)-1H-pyrazol-4-yl |
| 927 | Pyridin-3-yl | Ethyl | 1-[2-(Trifluormethyl)phenyl]-1H-pyrazol-4-yl |
| 928 | Pyridin-3-yl | Ethyl | 1-[3-Chlor-2-(trifluormethyl)phenyl]-5-(methoxymethyl)-1H-pyrazol-4-yl |
| 929 | Pyridin-3-yl | Ethyl | 4-[(2-Cyanethyl)(methyl)carbamoyl]phenyl |
| 930 | Pyridin-3-yl | Ethyl | 4-[(Trifluoracetyl)amino]phenyl |
| 931 | Pyridin-3-yl | Ethyl | 1-Benzyl-3-(trifluormethyl)-1H-pyrazol-4-yl |
| 932 | Pyridin-3-yl | Ethyl | 6-(2,5-Dichlorphenoxy)pyridin-3-yl |
| 933 | Pyridin-3-yl | Ethyl | 2-(Pyrimidin-2-yl)-1,3-thiazol-5-yl |
| 934 | Pyridin-3-yl | Ethyl | 3-(But-2-enoylamino)-4-methoxyphenyl |
| 935 | Pyridin-3-yl | Ethyl | 4-(Dimethylcarbamoyl)phenyl |
| 936 | Pyridin-3-yl | Ethyl | 4-Chlor-3-[(cyclopropylcarbonyl)amino]phenyl |
| 937 | Pyridin-3-yl | Ethyl | 1-(2,6-Dichlorphenyl)-5-methyl-1H-pyrazol-4-yl |
| 938 | Pyridin-3-yl | Ethyl | 1-(2-Chlorpyridin-3-yl)-5-(difluormethyl)-1H-pyrazol-4-yl |
| 939 | Pyridin-3-yl | Ethyl | 1-(3-Chlorpyridin-2-yl)-5-(dimethoxymethyl)-1H-pyrazol-4-yl |
| 940 | Pyridin-3-yl | Ethyl | 5-(Difluormethyl)-1-(2,2,2-trifluorethyl)-1H-pyrazol-4-yl |
| 941 | Pyridin-3-yl | Ethyl | 4-[(Propan-2-ylsulfanyl)methyl]phenyl |
| 942 | Pyridin-3-yl | Ethyl | 1-(2-Brom-6-chlorphenyl)-5-(dimethoxymethyl)-1H-pyrazol-4-yl |
| 943 | Pyridin-3-yl | Ethyl | 4-Methoxy-3-[3-(pyridin-2-yl)-1H-pyrazol-1-yl]phenyl |
| 944 | Pyridin-3-yl | Ethyl | 3-Chlor-4-{[4-(trifluormethyl)-1H-pyrazol-1-yl]methyl}phenyl |
| 945 | Pyridin-3-yl | Ethyl | 4-Methyl-3-[(2-methylbutanoyl)amino]phenyl |
| 946 | Pyridin-3-yl | Ethyl | 4-{[3-(3-Chlorpyridin-2-yl)-1H-pyrazol-1-yl]methyl}phenyl |
| 947 | Pyridin-3-yl | Ethyl | 6-{[4-(Pyridin-2-yl)-1H-pyrazol-1-yl]methyl}pyridin-3-yl |
| 948 | Pyridin-3-yl | Ethyl | 4-Methoxy-3-[3-(pyrimidin-2-yl)-1H-pyrazol-1-yl]phenyl |
| 949 | Pyridin-3-yl | Ethyl | 6-{[4-(Trifluormethyl)phenyl]sulfanyl}pyridin-3-yl |
| 950 | Pyridin-3-yl | Ethyl | 4-{[3-(Pyridin-2-yl)-1H-pyrazol-1-yl]methyl}phenyl |
| 951 | Pyridin-3-yl | Ethyl | 4-Chlor-3-[(2,2-dimethylpropanoyl)amino]phenyl |
| 952 | Pyridin-3-yl | Ethyl | 1-(2-Chlorpyridin-3-yl)-5-(1-methoxyethyl)-1H-pyrazol-4-yl |
| 953 | Pyridin-3-yl | Ethyl | 2-(3,5-Dichlorpyridin-2-yl)-1,3-thiazol-4-yl |
| 954 | Pyridin-3-yl | Ethyl | 4-[(Methylsulfanyl)methyl]phenyl |
| 955 | Pyridin-3-yl | Ethyl | 6-(Propylamino)pyridin-3-yl |
| 956 | Pyridin-3-yl | Ethyl | 5-Chlor-6-(4-cyanphenoxy)pyridin-3-yl |
| 957 | Pyridin-3-yl | Ethyl | 6-(3,4-Dichlorphenoxy)pyridin-3-yl |
| 958 | Pyridin-3-yl | Ethyl | 6-[(2-Chlorpyrimidin-4-yl)oxy]pyridin-3-yl |
| 959 | Pyridin-3-yl | Ethyl | 1-(3-Chlorpyridin-2-yl)-5-(1-methoxyethyl)-1H-pyrazol-4-yl |
| 960 | Pyridin-3-yl | Ethyl | 2-[2-(Trifluormethyl)phenyl]-1,3-thiazol-4-yl |
| 961 | Pyridin-3-yl | Ethyl | 3-(Propan-2-ylcarbamoyl)phenyl |
| 962 | Pyridin-3-yl | Ethyl | 4-(Heptafluorpropyl)phenyl |
| 963 | Pyridin-3-yl | Ethyl | 2-(2,6-Dichlorpyridin-3-yl)-1,3-thiazol-4-yl |
| 964 | Pyridin-3-yl | Ethyl | 1-Ethyl-5-(trifluormethyl)-1H-pyrazol-4-yl |
| 965 | Pyridin-3-yl | Ethyl | 1-[3-(Difluormethyl)pyridin-2-yl]-5-methyl-1H-pyrazol-4-yl |
| 966 | Pyridin-3-yl | Ethyl | 1-(2,4-Dichlorphenyl)-1H-pyrazol-4-yl |
| 967 | Pyridin-3-yl | Ethyl | 2-[4-Chlor-2-(trifluormethyl)phenyl]-1,3-thiazol-5-yl |
| 968 | Pyridin-3-yl | Ethyl | 1-(Propan-2-yl)-5-(trifluormethyl)-1H-pyrazol-4-yl |
| 969 | Pyridin-3-yl | Ethyl | 4-[(2-Methylpyrimidin-4-yl)oxy]phenyl |
| 970 | Pyridin-3-yl | Ethyl | 6-{[5-(Trifluormethyl)pyridin-2-yl]sulfanyl}pyridin-3-yl |
| 971 | Pyridin-3-yl | Ethyl | 6-[(4-Nitrophenyl)sulfanyl]pyridin-3-yl |
| 972 | Pyridin-3-yl | Ethyl | 4-Methyl-3-{[(propan-2-yloxy)carbonyl]amino}phenyl |
| 973 | Pyridin-3-yl | Ethyl | 1-[4-Fluor-2-(trifluormethyl)phenyl]-5-(methoxymethyl)-1H-pyrazol-4-yl |
| 974 | Pyridin-3-yl | Ethyl | 5-(1,1-Difluorethyl)-1-(pyrimidin-2-yl)-1H-pyrazol-4-yl |
| 975 | Pyridin-3-yl | Ethyl | 3-{[3,5-Bis(trifluormethyl)-1H-1,2,4-triazol-1-yl]methyl}phenyl |
| 976 | Pyridin-3-yl | Ethyl | 4-{[3,5-Bis(trifluormethyl)-1H-pyrazol-1-yl]methyl}phenyl |
| 977 | Pyridin-3-yl | Ethyl | 1-(2-Chlorphenyl)-5-(1,1-dimethoxyethyl)-1H-pyrazol-4-yl |
| 978 | Pyridin-3-yl | Ethyl | 1-(3-Chlorpyridin-2-yl)-5-(1,1-difluorethyl)-1H-pyrazol-4-yl |
| 979 | Pyridin-3-yl | Ethyl | 3-(1-Fluorethyl)-1-methyl-1H-pyrazol-4-yl |
| 980 | Pyridin-3-yl | Ethyl | 1-(2,6-Dichlorpyridin-3-yl)-5-(methoxymethyl)-1H-pyrazol-4-yl |
| 981 | Pyridin-3-yl | Ethyl | 6-(Chinolin-6-yloxy)pyridin-3-yl |
| 982 | Pyridin-3-yl | Ethyl | 3-(4-Chlor-1H-pyrazol-1-yl)-4-methoxyphenyl |
| 983 | Pyridin-3-yl | Ethyl | 2-(2,4-Dichlorphenyl)-1,3-thiazol-4-yl |
| 984 | Pyridin-3-yl | Ethyl | 1-(2-Chlorpyridin-3-yl)-5-(1,1-difluorethyl)-1H-pyrazol-4-yl |
| 985 | Pyridin-3-yl | Ethyl | 6-{4-[4-(Methylsulfanyl)phenoxy]phenoxy}pyridin-3-yl |
| 986 | Pyridin-3-yl | Ethyl | 6-(Chinolin-5-yloxy)pyridin-3-yl |
| 987 | Pyridin-3-yl | Ethyl | 1-[4-Chlor-2-(trifluormethyl)phenyl]-5-(difluormethyl)-1H-pyrazol-4-yl |
| 988 | Pyridin-3-yl | Ethyl | 1-[2-(1-Methoxyethyl)phenyl]-1H-pyrazol-4-yl |
| 989 | Pyridin-3-yl | Ethyl | 4-Methoxy-3-[(2-methylbutanoyl)amino]phenyl |
| 990 | Pyridin-3-yl | Ethyl | 6-(2,4-Dichlorphenoxy)pyridin-3-yl |
| 991 | Pyridin-3-yl | Ethyl | 4-{[Acetyl(2-methylpropyl)amino]methyl}phenyl |
| 992 | Pyridin-3-yl | Ethyl | 6-[(Methylsulfanyl)methyl]pyridin-3-yl |
| 993 | Pyridin-3-yl | Ethyl | 2-(2,4-Dichlorphenyl)-1,3-thiazol-5-yl |
| 994 | Pyridin-3-yl | Ethyl | 6-[2-Chlor-4-(methylsulfanyl)phenoxy]pyridin-3-yl |
| 995 | Pyridin-3-yl | Ethyl | 1-(2-Brom-6-chlorphenyl)-5-(difluormethyl)-1H-pyrazol-4-yl |
| 996 | Pyridin-3-yl | Ethyl | 4-(Propan-2-yloxy)-3-[3-(trifluormethyl)-1H-pyrazol-1-yl]phenyl |
| 997 | Pyridin-3-yl | Ethyl | 3-[Ethyl(methyl)carbamoyl]phenyl |
| 998 | Pyridin-3-yl | Ethyl | 1-Methyl-3-(trifluormethyl)-1H-pyrazol-4-yl |
| 999 | Pyridin-3-yl | Ethyl | 6-(3,5-Dimethylphenoxy)pyridin-3-yl |
| 1000 | Pyridin-3-yl | Ethyl | 1-[4-Chlor-2-(trifluormethyl)phenyl]-5-methyl-1H-pyrazol-4-yl |
| 1001 | Pyridin-3-yl | Ethyl | 4-Fluor-3-(trifluormethyl)phenyl |
| 1002 | Pyridin-3-yl | Ethyl | 6-[4-(Methoxycarbonyl)phenoxy]pyridin-3-yl |
| 1003 | Pyridin-3-yl | Ethyl | 3-[(Cyclopropylcarbonyl)amino]-4-methylphenyl |
| 1004 | Pyridin-3-yl | Ethyl | 1-(4-Chlorphenyl)-1H-pyrazol-4-yl |
| 1005 | Pyridin-3-yl | Ethyl | 2-[2-(Difluormethyl)phenyl]-1,3-thiazol-5-yl |
| 1006 | Pyridin-3-yl | Ethyl | 1-Ethyl-3-(trifluormethyl)-1H-pyrazol-4-yl |
| 1007 | Pyridin-3-yl | Ethyl | 5-Chlor-6-[4-(trifluormethyl)phenoxy]pyridin-3-yl |
| 1008 | Pyridin-3-yl | Ethyl | 4-Methyl-3-(propanoylamino)phenyl |
| 1009 | Pyridin-3-yl | Ethyl | 2-(2-Chlorpyridin-3-yl)-1,3-thiazol-4-yl |
| 1010 | Pyridin-3-yl | Ethyl | 4-[(2-Methylpropanoyl)amino]phenyl |
| 1011 | Pyridin-3-yl | Ethyl | 3-[(2,2-Dimethylpropanoyl)amino]-4-methylphenyl |
| 1012 | Pyridin-3-yl | Ethyl | 4-Methoxy-3-{[(propan-2-yloxy)carbonyl]amino}phenyl |
| 1013 | Pyridin-3-yl | Ethyl | 1-(2,3-Dichlorphenyl)-5-(methoxymethyl)-1H-pyrazol-4-yl |
| 1014 | Pyridin-3-yl | Ethyl | 3-(Hydroxymethyl)-1-methyl-1H-pyrazol-4-yl |
| 1015 | Pyridin-3-yl | Ethyl | 1-(Propan-2-yl)-3-(trifluormethyl)-1H-pyrazol-4-yl |
| 1016 | Pyridin-3-yl | Ethyl | 2-[3-(Difluormethyl)pyridin-2-yl]-1,3-thiazol-5-yl |
| 1017 | Pyridin-3-yl | Ethyl | 4-(Pyrrolidin-1-ylcarbonyl)phenyl |
| 1018 | Pyridin-3-yl | Ethyl | 3-(Methoxymethyl)-1-methyl-1H-pyrazol-4-yl |
| 1019 | Pyridin-3-yl | Ethyl | 2-(3,5-Dichlorpyridin-2-yl)-1,3-thiazol-5-yl |
| 1020 | Pyridin-3-yl | Ethyl | 1-(2,6-Dichlorpyridin-3-yl)-5-(difluormethyl)-1H-pyrazol-4-yl |
| 1021 | Pyridin-3-yl | Ethyl | 5-Chlor-6-(4-nitrophenoxy)pyridin-3-yl |
| 1022 | Pyridin-3-yl | Ethyl | 2-[2-(Difluormethyl)phenyl]-1,3-thiazol-4-yl |
| 1023 | Pyridin-3-yl | Ethyl | 1-(Difluormethyl)-3-(trifluormethyl)-1H-pyrazol-4-yl |
| 1024 | Pyridin-3-yl | Ethyl | 6-[(6-Chlorpyridin-2-yl)oxy]pyridin-3-yl |
| 1025 | Pyridin-3-yl | Ethyl | 3-(Butanoylamino)-4-methoxyphenyl |
| 1026 | Pyridin-3-yl | Ethyl | 4-(Acetylamino)phenyl |
| 1027 | Pyridin-3-yl | Ethyl | 1-(2-Bromphenyl)-5-(trifluormethyl)-1H-pyrazol-4-yl |
| 1028 | Pyridin-3-yl | Ethyl | 3-{[3-(Trifluormethyl)-1H-pyrazol-1-yl]methyl}phenyl |
| 1029 | Pyridin-3-yl | Ethyl | 5-(Difluormethyl)-1-(2-methylphenyl)-1H-pyrazol-4-yl |
| 1030 | Pyridin-3-yl | Ethyl | 2-[2-(1-Methoxyethyl)phenyl]-1,3-thiazol-5-yl |
| 1031 | Pyridin-3-yl | Ethyl | 1-(2-Chlorphenyl)-5-(1,1-difluorethyl)-1H-pyrazol-4-yl |
| 1032 | Pyridin-3-yl | Ethyl | 1-(2-Brom-4-fluorphenyl)-1H-pyrazol-4-yl |
| 1033 | Pyridin-3-yl | Ethyl | 6-[(Cyclopropylmethyl)amino]pyridin-3-yl |
| 1034 | Pyridin-3-yl | Ethyl | 6-[(2-Methoxypyrimidin-4-yl)oxy]pyridin-3-yl |
| 1035 | Pyridin-3-yl | Ethyl | 1-[2-(1,1-Difluorethyl)phenyl]-5-methyl-1H-pyrazol-4-yl |
| 1036 | Pyridin-3-yl | Ethyl | 3-{[3,5-Bis(trifluormethyl)-1H-1,2,4-triazol-1-yl]methyl}-4-chlorphenyl |
| 1037 | Pyridin-3-yl | Ethyl | 6-(Morpholin-4-yl)pyridin-3-yl |
| 1038 | Pyridin-3-yl | Ethyl | 2-(Pyrimidin-2-yl)-1,3-thiazol-4-yl |
| 1039 | Pyridin-3-yl | Ethyl | 2-{[3-(Trifluormethyl)-1H-pyrazol-1-yl]methyl}pyridin-4-yl |
| 1040 | Pyridin-3-yl | Ethyl | 1-(2-Chlorpyridin-3-yl)-5-(trifluormethyl)-1H-pyrazol-4-yl |
| 1041 | Pyridin-3-yl | Ethyl | 6-(4-Bromphenoxy)pyridin-3-yl |
| 1042 | Pyridin-3-yl | Ethyl | 4-[(Propylsulfanyl)methyl]phenyl |
| 1043 | Pyridin-3-yl | Ethyl | 1-(2-Chlor-4-fluorphenyl)-1H-pyrazol-4-yl |
| 1044 | Pyridin-3-yl | Ethyl | 4-Methoxy-3-[4-(pyridin-2-yl)-1H-pyrazol-1-yl]phenyl |
| 1045 | Pyridin-3-yl | Ethyl | 1-(2-Chlorphenyl)-1H-pyrazol-4-yl |
| 1046 | Pyridin-3-yl | Ethyl | 6-(2,6-Dichlorphenoxy)pyridin-3-yl |
| 1047 | Pyridin-3-yl | Ethyl | 1-(2-Chlorphenyl)-5-methyl-1H-pyrazol-4-yl |
| 1048 | Pyridin-3-yl | Ethyl | 3-[Ethyl(methyl)carbamoyl]-4-methoxyphenyl |
| 1049 | Pyridin-3-yl | Ethyl | 4-[(Ethylsulfanyl)methyl]phenyl |
| 1050 | Pyridin-3-yl | Ethyl | 2-[2-(1-Methoxyethyl)phenyl]-1,3-thiazol-4-yl |
| 1051 | Pyridin-3-yl | Ethyl | 6-{[6-(1H-Pyrazol-1-yl)pyridin-2-yl]oxy}pyridin-3-yl |
| 1052 | Pyridin-3-yl | Ethyl | 2-[4-Chlor-2-(trifluormethyl)phenyl]-1,3-thiazol-4-yl |
| 1053 | Pyridin-3-yl | Ethyl | 4-Methoxy-3-(1H-pyrazol-1-yl)phenyl |
| 1054 | Pyridin-3-yl | Ethyl | 3-(Acetylamino)-4-methylphenyl |
| 1055 | Pyridin-3-yl | Ethyl | 2-{[4-(Trifluormethyl)-1H-pyrazol-1-yl]methyl}pyridin-4-yl |
| 1056 | Pyridin-3-yl | Ethyl | 1-(2,4-Difluorphenyl)-1H-pyrazol-4-yl |
| 1057 | Pyridin-3-yl | Ethyl | 1-(2-Methylphenyl)-5-(trifluormethyl)-1H-pyrazol-4-yl |
| 1058 | Pyridin-3-yl | Ethyl | 2-[3-(Difluormethyl)pyridin-2-yl]-1,3-thiazol-4-yl |
| 1059 | Pyridin-3-yl | Ethyl | 6-(3,5-Dichlorphenoxy)pyridin-3-yl |
| 1060 | Pyridin-3-yl | Ethyl | 1-(Pyrimidin-2-ylmethyl)-3-(trifluormethyl)-1H-pyrazol-4-yl |
| 1061 | Pyridin-3-yl | Ethyl | 6-Methylpyridin-3-yl |
| 1062 | Pyridin-3-yl | Ethyl | 6-[(4-Methylphenyl)sulfanyl]pyridin-3-yl |
| 1063 | Pyridin-3-yl | Ethyl | 1-(2,4-Dichlorphenyl)-5-(methoxymethyl)-1H-pyrazol-4-yl |
| 1064 | Pyridin-3-yl | Ethyl | 6-{4-[4-(Trifluormethyl)phenoxy]phenoxy}pyridin-3-yl |
| 1065 | Pyridin-3-yl | Ethyl | 1-(2,6-Dichlorphenyl)-5-(trifluormethyl)-1H-pyrazol-4-yl |
| 1066 | Pyridin-3-yl | Ethyl | 6-(Naphthalen-1-yloxy)pyridin-3-yl |
| 1067 | Pyridin-3-yl | Ethyl | 3-Ethyl-1-methyl-1H-pyrazol-4-yl |
| 1068 | Pyridin-3-yl | Ethyl | 2-(2,6-Dichlorpyridin-3-yl)-1,3-thiazol-5-yl |
| 1069 | Pyridin-3-yl | Ethyl | 6-{[(4,6-Dimethoxypyrimidin-2-yl)methyl]sulfanyl}pyridin-3-yl |
| 1070 | Pyridin-3-yl | Ethyl | 1-[3-Chlor-2-(trifluormethyl)phenyl]-5-(1-methoxyethyl)-1H-pyrazol-4-yl |
| 1071 | Pyridin-3-yl | Ethyl | 3,4-Dimethoxyphenyl |
| 1072 | Pyridin-3-yl | Ethyl | 4-Methoxy-3-[(methoxyacetyl)amino]phenyl |
| 1073 | Pyridin-3-yl | Ethyl | 3-{[4-(Trifluormethyl)-1H-pyrazol-1-yl]methyl}phenyl |
| 1074 | Pyridin-3-yl | Ethyl | 6-(2-Cyanphenoxy)pyridin-3-yl |
| 1075 | Pyridin-3-yl | Ethyl | 3-[(Methylsulfanyl)methyl]phenyl |
| 1076 | Pyridin-3-yl | Ethyl | 3-(Dimethoxymethyl)-1-methyl-1H-pyrazol-4-yl |
| 1077 | Pyridin-3-yl | Ethyl | 1-(Ethoxymethyl)-3-(trifluormethyl)-1H-pyrazol-4-yl |
| 1078 | Pyridin-3-yl | Ethyl | 1-(3-Brompyridin-2-yl)-5-(methoxymethyl)-1H-pyrazol-4-yl |
| 1079 | Pyridin-3-yl | Ethyl | 6-(Methylsulfanyl)pyridin-3-yl |
| 1080 | Pyridin-3-yl | Ethyl | 1-(2-Brom-6-chlorphenyl)-5-(trifluormethyl)-1H-pyrazol-4-yl |
| 1081 | Pyridin-3-yl | Ethyl | 3-{[4-(Trifluormethyl)phenoxy]methyl}phenyl |
| 1082 | Pyridin-3-yl | Ethyl | 6-(Heptafluorpropyl)pyridin-3-yl |
| 1083 | Pyridin-3-yl | Ethyl | 1-Ethenyl-3-(trifluormethyl)-1H-pyrazol-4-yl |
| 1084 | Pyridin-3-yl | Ethyl | 4-Methyl-3-nitrophenyl |
| 1085 | Pyridin-3-yl | Ethyl | 3-(4-Brom-1H-pyrazol-1-yl)-4-methoxyphenyl |
| 1086 | Pyridin-3-yl | Ethyl | 5-Methyl-1-(2-methylphenyl)-1H-pyrazol-4-yl |
| 1087 | Pyridin-3-yl | Ethyl | 1-[3-(Difluormethyl)pyridin-2-yl]-5-(methoxymethyl)-1H-pyrazol-4-yl |
| 1088 | Pyridin-3-yl | Ethyl | 6-{[2-(Trifluormethyl)pyrimidin-5-yl]oxy}pyridin-3-yl |
| 1089 | Pyridin-3-yl | Ethyl | 2-[2-(Methoxymethyl)phenyl]-1,3-thiazol-5-yl |
| 1090 | Pyridin-3-yl | Ethyl | 1-(Ethoxymethyl)-5-(trifluormethyl)-1H-pyrazol-4-yl |
| 1091 | Pyridin-3-yl | Ethyl | 5-Chlor-6-[(4,5,6-trimethylpyrimidin-2-yl)sulfanyl]pyridin-3-yl |
| 1092 | Pyridin-3-yl | Ethyl | 6-[3,5-Bis(trifluormethyl)phenoxy]pyridin-3-yl |
| 1093 | Pyridin-3-yl | Ethyl | 4-{[4-(Pyridin-2-yl)-1H-pyrazol-1-yl]methyl}phenyl |
| 1094 | Pyridin-3-yl | Ethyl | 1-[3-(1,1-Difluorethyl)pyridin-2-yl]-5-methyl-1H-pyrazol-4-yl |
| 1095 | Pyridin-3-yl | Ethyl | 1-(2,6-Dichlorphenyl)-5-(difluormethyl)-1H-pyrazol-4-yl |
| 1096 | Pyridin-3-yl | Ethyl | 1-[4-Fluor-2-(trifluormethyl)phenyl]-5-methyl-1H-pyrazol-4-yl |
| 1097 | Pyridin-3-yl | Ethyl | 4-Chlor-3-[(cyclobutylcarbonyl)amino]phenyl |
| 1098 | Pyridin-3-yl | Ethyl | 2-[2-(Methoxymethyl)phenyl]-1,3-thiazol-4-yl |
| 1099 | Pyridin-3-yl | Ethyl | 4-(1H-Pyrazol-1-ylmethyl)phenyl |
| 1100 | Pyridin-3-yl | Ethyl | 1-(2-Bromphenyl)-5-methyl-1H-pyrazol-4-yl |
| 1101 | Pyridin-3-yl | Ethyl | 1-(2,3-Difluorphenyl)-1H-pyrazol-4-yl |
| 1102 | Pyridin-3-yl | Ethyl | 6-{4-[(Trifluormethyl)sulfanyl]phenoxy}pyridin-3-yl |
| 1103 | Pyridin-3-yl | Ethyl | 4-{[3-(Pentafluorethyl)-1H-1,2,4-triazol-1-yl]methyl}phenyl |
| 1104 | Pyridin-3-yl | Ethyl | 4-{[3-(Pyrimidin-2-yl)-1H-pyrazol-1-yl]methyl}phenyl |
| 1105 | Pyridin-3-yl | Ethyl | 1-(2-Chlorphenyl)-5-(difluormethyl)-1H-pyrazol-4-yl |
| 1106 | Pyridin-3-yl | Ethyl | 5-(Difluormethyl)-1-[2-(trifluormethyl)phenyl]-1H-pyrazol-4-yl |
| 1107 | Pyridin-3-yl | Ethyl | 6-(4-Methoxyphenoxy)pyridin-3-yl |
| 1108 | Pyridin-3-yl | Ethyl | 1-[2-(Dimethoxymethyl)phenyl]-1H-pyrazol-4-yl |
| 1109 | Pyridin-3-yl | Ethyl | 5-(Methoxymethyl)-1-(pyrimidin-2-yl)-1H-pyrazol-4-yl |
| 1110 | Pyridin-3-yl | Ethyl | 6-tert-Butoxypyridin-3-yl |
| 1111 | Pyridin-3-yl | Ethyl | 4-[(Cyclopentylimino)methyl]phenyl |
| 1112 | Pyridin-3-yl | Ethyl | 1-(3-Chlorphenyl)-1H-pyrazol-4-yl |
| 1113 | Pyridin-3-yl | Ethyl | 3-[(2-Methylpropanoyl)amino]phenyl |
| 1114 | Pyridin-3-yl | Ethyl | 6-{[3,5-Bis(trifluormethyl)-1H-pyrazol-1-yl]methyl}pyridin-3-yl |
| 1115 | Pyridin-3-yl | Ethyl | 4-Chlor-3-[(2-methylpropanoyl)amino]phenyl |
| 1116 | Pyridin-3-yl | Ethyl | 4-(Propylsulfanyl)phenyl |
| 1117 | Pyridin-3-yl | Ethyl | 1-[2-(Difluormethyl)phenyl]-5-(methoxymethyl)-1H-pyrazol-4-yl |
| 1118 | Pyridin-3-yl | Ethyl | 4-Methoxy-3-[4-(pyrimidin-2-yl)-1H-pyrazol-1-yl]phenyl |
| 1119 | Pyridin-3-yl | Ethyl | 4-{[(4,6-Dimethylpyrimidin-2-yl)sulfanyl]methyl}phenyl |
| 1120 | Pyridin-3-yl | Ethyl | 1-(2-Chlorphenyl)-5-(methoxymethyl)-1H-pyrazol-4-yl |
| 1121 | Pyridin-3-yl | Ethyl | 6-{[3,5-Bis(trifluormethyl)-1H-1,2,4-triazol-1-yl]methyl}pyridin-3-yl |
| 1122 | Pyridin-3-yl | Ethyl | 5-Chlor-6-[4-(trifluormethoxy)phenoxy]pyridin-3-yl |
| 1123 | Pyridin-3-yl | Ethyl | 2-(3-Chlorpyridin-2-yl)-1,3-thiazol-5-yl |
| 1124 | Pyridin-3-yl | Ethyl | 1-(2-Fluorphenyl)-1H-pyrazol-4-yl |
| 1125 | Pyridin-3-yl | Ethyl | 4-(Trifluormethoxy)phenyl |
| 1126 | Pyridin-3-yl | Ethyl | 6-(2-Chlorphenoxy)pyridin-3-yl |
| 1127 | Pyridin-3-yl | Ethyl | 3-[(2,2-Dimethylpropanoyl)amino]-5-(trifluormethyl)phenyl |
| 1128 | Pyridin-3-yl | Ethyl | 6-{[2-(Morpholin-4-yl)ethyl]amino}pyridin-3-yl |
| 1129 | Pyridin-3-yl | Ethyl | 3-Formyl-1-methyl-1H-pyrazol-4-yl |
| 1130 | Pyridin-3-yl | Ethyl | 1-Methyl-3-(propan-2-yl)-1H-pyrazol-4-yl |
| 1131 | Pyridin-3-yl | Ethyl | 1-(3-Chlorphenyl)-5-(1,1-dimethoxyethyl)-1H-pyrazol-4-yl |
| 1132 | Pyridin-3-yl | Ethyl | 4-Nitrophenyl |
| 1133 | Pyridin-3-yl | Ethyl | 6-{[5-(Methoxycarbonyl)pyridin-3-yl]oxy}pyridin-3-yl |
| 1134 | Pyridin-3-yl | Ethyl | 3-{[4-(Trifluormethyl)benzyl]oxy}phenyl |
| 1135 | Pyridin-3-yl | Ethyl | 1-(2,4-Dichlorphenyl)-5-(1,1-dimethoxyethyl)-1H-pyrazol-4-yl |
| 1136 | Pyridin-3-yl | Ethyl | 4-{[4-Brom-3-(trifluormethyl)-1H-pyrazol-1-yl]methyl}phenyl |
| 1137 | Pyridin-3-yl | Ethyl | 3-[Chlor(difluor)methyl]-1-methyl-1H-pyrazol-4-yl |
| 1138 | Pyridin-3-yl | Ethyl | 3-[(Cyclopropylcarbonyl)amino]-4-fluorphenyl |
| 1139 | Pyridin-3-yl | Ethyl | 6-[4-(Dimethylsulfamoyl)phenoxy]pyridin-3-yl |
| 1140 | Pyridin-3-yl | Ethyl | 5-Methyl-1-(2-methylpyridin-3-yl)-1H-pyrazol-4-yl |
| 1141 | Pyridin-3-yl | Ethyl | 1-(2,4-Difluorphenyl)-5-methyl-1H-pyrazol-4-yl |
| 1142 | Pyridin-3-yl | Ethyl | 3-(Methylsulfanyl)phenyl |
| 1143 | Pyridin-3-yl | Ethyl | 3-(Acetylamino)-4-chlorphenyl |
| 1144 | Pyridin-3-yl | Ethyl | 4-[(4-Brom-1H-pyrazol-1-yl)methyl]phenyl |
| 1145 | Pyridin-3-yl | Ethyl | 4-Methyl-3-[(propan-2-ylcarbamoyl)amino]phenyl |
| 1146 | Pyridin-3-yl | Ethyl | 1-(2,2-Diethoxyethyl)-3-(trifluormethyl)-1H-pyrazol-4-yl |
| 1147 | Pyridin-3-yl | Ethyl | 6-(1H-Pyrazol-1-yl)pyridin-3-yl |
| 1148 | Pyridin-3-yl | Ethyl | 4-(1H-1,2,4-Triazol-1-ylmethyl)phenyl |
| 1149 | Pyridin-3-yl | Ethyl | 5-(Methoxymethyl)-1-[2-(trifluormethyl)phenyl]-1H-pyrazol-4-yl |
| 1150 | Pyridin-3-yl | Ethyl | 5-Methylpyridin-3-yl |
| 1151 | Pyridin-3-yl | Ethyl | 5-Chlor-6-[(4,6-dimethylpyrimidin-2-yl)sulfanyl]pyridin-3-yl |
| 1152 | Pyridin-3-yl | Ethyl | 3-[(2,2-Dimethylpropanoyl)amino]-4-methoxyphenyl |
| 1153 | Pyridin-3-yl | Ethyl | 2-[2-(Trifluormethyl)pyridin-3-yl]-1,3-thiazol-4-yl |
| 1154 | Pyridin-3-yl | Ethyl | 3-Cyclopropyl-1-methyl-1H-pyrazol-4-yl |
| 1155 | Pyridin-3-yl | Ethyl | 3-Methyl-1H-pyrazol-4-yl |
| 1156 | Pyridin-3-yl | Ethyl | 5-(Methoxymethyl)-1-(2,2,2-trifluorethyl)-1H-pyrazol-4-yl |
| 1157 | Pyridin-3-yl | Ethyl | 4-Methoxy-3-(propanoylamino)phenyl |
| 1158 | Pyridin-3-yl | Ethyl | 4-Methoxy-3-[(2-methylpropanoyl)amino]phenyl |
| 1159 | Pyridin-3-yl | Ethyl | 6-(Chinolin-7-yloxy)pyridin-3-yl |
| 1160 | Pyridin-3-yl | Ethyl | 4-{[3-(Trifluormethyl)-1H-pyrazol-1-yl]methyl}phenyl |
| 1161 | Pyridin-3-yl | Ethyl | 4-{[3,5-Bis(trifluormethyl)-1H-1,2,4-triazol-1-yl]methyl}phenyl |
| 1162 | Pyridin-3-yl | Ethyl | 3-Fluor-4-{[4-(trifluormethyl)-1H-pyrazol-1-yl]methyl}phenyl |
| 1163 | Pyridin-3-yl | Ethyl | 2,3-Dihydro-1,4-benzodioxin-6-yl |
| 1164 | Pyridin-3-yl | Ethyl | 2-[3-(Methoxymethyl)pyridin-2-yl]-1,3-thiazol-5-yl |
| 1165 | Pyridin-3-yl | Ethyl | 6-({6-[3-(Trifluormethyl)-1H-pyrazol-1-yl]pyridin-2-yl}oxy)pyridin-3-yl |
| 1166 | Pyridin-3-yl | Ethyl | 3-{[3-(Pyridin-2-yl)-1H-pyrazol-1-yl]methyl}phenyl |
| 1167 | Pyridin-3-yl | Ethyl | 1-(2-Brom-4-chlorphenyl)-5-methyl-1H-pyrazol-4-yl |
| 1168 | Pyridin-3-yl | Ethyl | 2,2-Difluor-1,3-benzodioxol-5-yl |
| 1169 | Pyridin-3-yl | Ethyl | 2-[3-(Trifluormethyl)pyridin-2-yl]-1,3-thiazol-4-yl |
| 1170 | Pyridin-3-yl | Ethyl | 4-(Benzylcarbamoyl)phenyl |
| 1171 | Pyridin-3-yl | Ethyl | 4-[(2-Chlorpyrimidin-4-yl)oxy]phenyl |
| 1172 | Pyridin-3-yl | Ethyl | 1-[3-(Trifluormethyl)pyridin-2-yl]-1H-pyrazol-4-yl |
| 1173 | Pyridin-3-yl | Ethyl | 5-Chlor-6-(4-chlorphenoxy)pyridin-3-yl |
| 1174 | Pyridin-3-yl | Ethyl | 3-Cyan-4-(1H-1,2,4-triazol-1-yl)phenyl |
| 1175 | Pyridin-3-yl | Ethyl | 3-Amino-4-(methylsulfonyl)phenyl |
| 1176 | Pyridin-3-yl | Ethyl | 4-Methyl-3-[(2-methylpropanoyl)amino]phenyl |
| 1177 | Pyridin-3-yl | Ethyl | 1-(2,6-Dibromphenyl)-5-(trifluormethyl)-1H-pyrazol-4-yl |
| 1178 | Pyridin-3-yl | Ethyl | 3-{[3-(Trifluormethyl)-1H-1,2,4-triazol-1-yl]methyl}phenyl |
| 1179 | Pyridin-3-yl | Ethyl | 4-[(3,3,3-Trifluorpropanoyl)amino]phenyl |
| 1180 | Pyridin-3-yl | Ethyl | 5-(Pyrazin-2-yl)-1-(2,2,2-trifluorethyl)-1H-pyrazol-4-yl |
| 1181 | Pyridin-3-yl | Ethyl | 1-(2,6-Dichlorphenyl)-5-(dimethoxymethyl)-1H-pyrazol-4-yl |
| 1182 | Pyridin-3-yl | Ethyl | 1-(4-Chlorphenyl)-5-(1,1-dimethoxyethyl)-1H-pyrazol-4-yl |
| 1183 | Pyridin-3-yl | Ethyl | 4-({6-[3-(Trifluormethyl)-1H-pyrazol-1-yl]pyridin-2-yl}oxy)phenyl |
| 1184 | Pyridin-3-yl | Ethyl | 4-Methoxy-3-(propan-2-ylcarbamoyl)phenyl |
| 1185 | Pyridin-3-yl | Ethyl | 1-[2-(1-Methoxyethyl)phenyl]-5-methyl-1H-pyrazol-4-yl |
| 1186 | Pyridin-3-yl | Ethyl | 1-Ethyl-3-methyl-1H-pyrazol-4-yl |
| 1187 | Pyridin-3-yl | Ethyl | 6-(4-Methylphenoxy)pyridin-3-yl |
| 1188 | Pyridin-3-yl | Ethyl | 6-(4-Chlor-2-cyanphenoxy)pyridin-3-yl |
| 1189 | Pyridin-3-yl | Ethyl | 1-Propyl-3-(trifluormethyl)-1H-pyrazol-4-yl |
| 1190 | Pyridin-3-yl | Ethyl | 6-(2-Methylphenoxy)pyridin-3-yl |
| 1191 | Pyridin-3-yl | Ethyl | 4-{[4-(Trifluormethyl)-1H-pyrazol-1-yl]methyl}phenyl |
| 1192 | Pyridin-3-yl | Ethyl | 6-Methoxypyridin-3-yl |
| 1193 | Pyridin-3-yl | Ethyl | 4-{[Acetyl(cyclopentyl)amino]methyl}phenyl |
| 1194 | Pyridin-3-yl | Ethyl | 3-{[3,5-Bis(trifluormethyl)-1H-pyrazol-1-yl]methyl}phenyl |
| 1195 | Pyridin-3-yl | Ethyl | 1-(2-Brom-4-chlorphenyl)-1H-pyrazol-4-yl |
| 1196 | Pyridin-3-yl | Ethyl | 1-(2-Chlorphenyl)-5-(trifluormethyl)-1H-pyrazol-4-yl |
| 1197 | Pyridin-3-yl | Ethyl | 4-Methoxy-3-nitrophenyl |
| 1198 | Pyridin-3-yl | Ethyl | 1-(2,6-Dibromphenyl)-5-(difluormethyl)-1H-pyrazol-4-yl |
| 1199 | Pyridin-3-yl | Ethyl | 6-[(4-Methoxyphenyl)sulfanyl]pyridin-3-yl |
| 1200 | Pyridin-3-yl | Ethyl | 1-(2,6-Dichlorphenyl)-5-(1-methoxyethyl)-1H-pyrazol-4-yl |
| 1201 | Pyridin-3-yl | Ethyl | 4-[(6-Chlorpyridin-2-yl)oxy]phenyl |
| 1202 | Pyridin-3-yl | Ethyl | 3-Fluor-4-{[3-(trifluormethyl)-1H-pyrazol-1-yl]methyl}phenyl |
| 1203 | Pyridin-3-yl | Ethyl | 4-{[(Difluormethyl)sulfanyl]methyl}phenyl |
| 1204 | Pyridin-3-yl | Ethyl | 6-{[3-(Trifluormethyl)-1H-pyrazol-1-yl]methyl}pyridin-3-yl |
| 1205 | Pyridin-3-yl | Ethyl | 4-{[2-(3,5-Dichlorphenyl)propan-2-yl]carbamoyl}phenyl |
| 1206 | Pyridin-3-yl | Ethyl | 3-(1H-1,2,4-Triazol-1-ylmethyl)phenyl |
| 1207 | Pyridin-3-yl | Ethyl | 2-(3-Chlorpyridin-2-yl)-1,3-thiazol-4-yl |
| 1208 | Pyridin-3-yl | Ethyl | 5-(1-Methoxyethyl)-1-(pyrimidin-2-yl)-1H-pyrazol-4-yl |
| 1209 | Pyridin-3-yl | Ethyl | 1-(3-Chlorpyridin-2-yl)-5-(1,1-dimethoxyethyl)-1H-pyrazol-4-yl |
| 1210 | Pyridin-3-yl | Ethyl | 1-(2-Brom-4-fluorphenyl)-5-methyl-1H-pyrazol-4-yl |
| 1211 | Pyridin-3-yl | Ethyl | 3-(Butanoylamino)-4-methylphenyl |
| 1212 | Pyridin-3-yl | Ethyl | 5-(Methoxymethyl)-1-[3-(trifluormethyl)pyridin-2-yl]-1H-pyrazol-4-yl |
| 1213 | Pyridin-3-yl | Ethyl | 6-[3-(Trifluormethyl)-1H-pyrazol-1-yl]pyridin-3-yl |
| 1214 | Pyridin-3-yl | Ethyl | 6-(3-Chlorphenoxy)pyridin-3-yl |
| 1215 | Pyridin-3-yl | Ethyl | 1-(2,6-Dichlorpyridin-3-yl)-5-(1-methoxyethyl)-1H-pyrazol-4-yl |
| 1216 | Pyridin-3-yl | Ethyl | 1-[2-(Difluormethyl)phenyl]-5-methyl-1H-pyrazol-4-yl |
| 1217 | Pyridin-3-yl | Ethyl | 2-[2-(Trifluormethyl)phenyl]-1,3-thiazol-5-yl |
| 1218 | Pyridin-3-yl | Ethyl | 2-[2-(Trifluormethyl)pyridin-3-yl]-1,3-thiazol-5-yl |
| 1219 | Pyridin-3-yl | Ethyl | 2-(2-Chlorpyridin-3-yl)-1,3-thiazol-5-yl |
| 1220 | Pyridin-3-yl | Ethyl | 1-(2,6-Dichlorpyridin-3-yl)-5-methyl-1H-pyrazol-4-yl |
| 1221 | Pyridin-3-yl | Ethyl | 1-(3-Chlorpyridin-2-yl)-5-(methoxymethyl)-1H-pyrazol-4-yl |
| 1222 | Pyridin-3-yl | Ethyl | 6-[(4-Chlorphenyl)sulfanyl]pyridin-3-yl |
| 1223 | Pyridin-3-yl | Ethyl | 6-(Piperidin-1-yl)pyridin-3-yl |
| 1224 | Pyridin-3-yl | Ethyl | 3-[(Cyclopentylcarbonyl)amino]-4-methylphenyl |
| 1225 | Pyridin-3-yl | Ethyl | 6-Ethoxypyridin-3-yl |
| 1226 | Pyridin-3-yl | Ethyl | 2-(2,6-Dichlorphenyl)-1,3-thiazol-4-yl |
| 1227 | Pyridin-3-yl | Ethyl | 5-Chlor-6-[(4-methylpyrimidin-2-yl)sulfanyl]pyridin-3-yl |
| 1228 | Pyridin-3-yl | Ethyl | 1-[4-Chlor-2-(trifluormethyl)phenyl]-5-(1-methoxyethyl)-1H-pyrazol-4-yl |
| 1229 | Pyridin-3-yl | Ethyl | 6-(3-Methylphenoxy)pyridin-3-yl |
| 1230 | Pyridin-3-yl | Ethyl | 4-[Ethyl(methyl)carbamoyl]phenyl |
| 1231 | Pyridin-3-yl | Ethyl | 6-[3-(Trifluormethoxy)phenoxy]pyridin-3-yl |
| 1232 | Pyridin-3-yl | Ethyl | 5-(Difluormethyl)-1-(pyrimidin-2-yl)-1H-pyrazol-4-yl |
| 1233 | Pyridin-3-yl | Ethyl | 4-[(Cyclopropylcarbonyl)amino]phenyl |
| 1234 | Pyridin-3-yl | Ethyl | 1-(3-Brompyridin-2-yl)-5-(1-methoxyethyl)-1H-pyrazol-4-yl |
| 1235 | Pyridin-3-yl | Ethyl | 3-[(Cyclopropylcarbonyl)amino]-4-methoxyphenyl |
| 1236 | Pyridin-3-yl | Ethyl | 4-Methyl-3-[(3-methylbut-2-enoyl)amino]phenyl |
| 1237 | Pyridin-3-yl | Ethyl | 1-[3-(Methoxymethyl)pyridin-2-yl]-1H-pyrazol-4-yl |
| 1238 | Pyridin-3-yl | Ethyl | 6-(Naphthalen-2-yloxy)pyridin-3-yl |
| 1239 | Pyridin-3-yl | Ethyl | 1-(2-Chlorphenyl)-5-(1-methoxyethyl)-1H-pyrazol-4-yl |
| 1240 | Pyridin-3-yl | Ethyl | 1-[2-(Difluormethyl)phenyl]-5-(1-methoxyethyl)-1H-pyrazol-4-yl |
| 1241 | Pyridin-3-yl | Ethyl | 1-(3-Chlorpyridin-2-yl)-5-methyl-1H-pyrazol-4-yl |
| 1242 | Pyridin-3-yl | Ethyl | 4-{[3-(Trifluormethyl)-1H-1,2,4-triazol-1-yl]methyl}phenyl |
| 1243 | Pyridin-3-yl | Ethyl | 3-[(Cyclopentylcarbonyl)amino]-4-methoxyphenyl |
| 1244 | Pyridin-3-yl | Ethyl | 1-(2,2,2-Trifluorethyl)-5-(trifluormethyl)-1H-pyrazol-4-yl |
| 1245 | Pyridin-3-yl | Ethyl | 6-[4-(Methylsulfanyl)phenoxy]pyridin-3-yl |
| 1246 | Pyridin-3-yl | Ethyl | 2-[3-(Trifluormethyl)pyridin-2-yl]-1,3-thiazol-5-yl |
| 1247 | Pyridin-3-yl | Ethyl | 4-{[3-(Methoxycarbonyl)-1H-1,2,4-triazol-1-yl]methyl}phenyl |
| 1248 | Pyridin-3-yl | Ethyl | 1-[3-(1,1-Difluorethyl)pyridin-2-yl]-1H-pyrazol-4-yl |
| 1249 | Pyridin-3-yl | Ethyl | 6-{[3-(Pyridin-2-yl)-1H-pyrazol-1-yl]methyl}pyridin-3-yl |
| 1250 | Pyridin-3-yl | Ethyl | 3-(Acetylamino)phenyl |
| 1251 | Pyridin-3-yl | Ethyl | 1-(3-Chlorpyridin-2-yl)-5-(trifluormethyl)-1H-pyrazol-4-yl |
| 1252 | Pyridin-3-yl | Ethyl | 2-{[3-(Pyridin-2-yl)-1H-pyrazol-1-yl]methyl}pyridin-4-yl |
| 1253 | Pyridin-3-yl | Ethyl | 3-[(Methoxyacetyl)amino]-4-methylphenyl |
| 1254 | Pyridin-3-yl | Ethyl | 1-(3-Brompyridin-2-yl)-5-(trifluormethyl)-1H-pyrazol-4-yl |
| 1255 | Pyridin-3-yl | Ethyl | 1-(Pyrimidin-2-yl)-5-(trifluormethyl)-1H-pyrazol-4-yl |
| 1256 | Pyridin-3-yl | Ethyl | 2-(2-Chlorphenyl)-1,3-thiazol-4-yl |
| 1257 | Pyridin-3-yl | Ethyl | 4-Methoxy-3-[(propan-2-ylsulfonyl)amino]phenyl |
| 1258 | Pyridin-3-yl | Ethyl | 6-(3-Cyanphenoxy)pyridin-3-yl |
| 1259 | Pyridin-3-yl | Ethyl | 4-Fluor-3-[(2-methylpropanoyl)amino]phenyl |
| 1260 | Pyridin-3-yl | Ethyl | 1-(2-Bromphenyl)-5-(difluormethyl)-1H-pyrazol-4-yl |
| 1261 | Pyridin-3-yl | Ethyl | 5-(1-Methoxyethyl)-1-(2,2,2-trifluorethyl)-1H-pyrazol-4-yl |
| 1262 | Pyridin-3-yl | Ethyl | 1-(2,6-Dichlorphenyl)-5-(1-fluorethyl)-1H-pyrazol-4-yl |
| 1263 | Pyridin-3-yl | Ethyl | 6-(Pyrrolidin-1-yl)pyridin-3-yl |
| 1264 | Pyridin-3-yl | Ethyl | 3-(Acetylamino)-5-(trifluormethyl)phenyl |
| 1265 | Pyridin-3-yl | Ethyl | 2-[3-(Methoxymethyl)pyridin-2-yl]-1,3-thiazol-4-yl |
| 1266 | Pyridin-3-yl | Ethyl | 2-[3-(1-Methoxyethyl)pyridin-2-yl]-1,3-thiazol-5-yl |
| 1267 | Pyridin-3-yl | Ethyl | 1-[3-(Dimethoxymethyl)pyridin-2-yl]-1H-pyrazol-4-yl |
| 1268 | Pyridin-3-yl | Ethyl | 5-(1-Methoxyethyl)-1-(2-methylphenyl)-1H-pyrazol-4-yl |
| 1269 | Pyridin-3-yl | Ethyl | 1-Methyl-1H-benzotriazol-5-yl |
| 1270 | Pyridin-3-yl | Ethyl | 4-[(Butylsulfanyl)methyl]phenyl |
| 1271 | Pyridin-3-yl | Ethyl | 1H-Pyrazol-4-yl |
| 1272 | Pyridin-3-yl | Ethyl | 1-(2-Brom-6-chlorphenyl)-5-(1-methoxyethyl)-1H-pyrazol-4-yl |
| 1273 | Pyridin-3-yl | Ethyl | 5-Chlor-6-[4-(methylsulfonyl)phenoxy]pyridin-3-yl |
| 1274 | Pyridin-3-yl | Ethyl | 1-[3-(1-Methoxyethyl)pyridin-2-yl]-1H-pyrazol-4-yl |
| 1275 | Pyridin-3-yl | Ethyl | 4-[(6-Methylpyridin-3-yl)oxy]phenyl |
| 1276 | Pyridin-3-yl | Ethyl | 3-Methoxy-1-methyl-1H-pyrazol-4-yl |
| 1277 | Pyridin-3-yl | Ethyl | 6-(4-tert-Butylphenoxy)pyridin-3-yl |
| 1278 | Pyridin-3-yl | Ethyl | 1-(2,4-Dichlorphenyl)-5-methyl-1H-pyrazol-4-yl |
| 1279 | Pyridin-3-yl | Ethyl | 3-(But-2-enoylamino)-4-methylphenyl |
| 1280 | Pyridin-3-yl | Ethyl | 4-{[4-(Pyrimidin-2-yl)-1H-pyrazol-1-yl]methyl}phenyl |
| 1281 | Pyridin-3-yl | Ethyl | 4-{[6-(1H-Pyrazol-1-yl)pyridin-2-yl]oxy}phenyl |
| 1282 | Pyridin-3-yl | Ethyl | 4-{[3,5-Bis(trifluormethyl)-1H-1,2,4-triazol-1-yl]methyl}-3-chlorphenyl |
| 1283 | Pyridin-3-yl | Ethyl | 4-[(2-Methoxypyrimidin-4-yl)oxy]phenyl |
| 1284 | Pyridin-3-yl | Ethyl | 1-[4-Chlor-2-(trifluormethyl)phenyl]-5-(methoxymethyl)-1H-pyrazol-4-yl |
| 1285 | Pyridin-3-yl | Ethyl | 5-(Trifluormethyl)-1-[2-(trifluormethyl)phenyl]-1H-pyrazol-4-yl |
| 1286 | Pyridin-3-yl | Ethyl | 6-(Dimethylamino)pyridin-3-yl |
| 1287 | Pyridin-3-yl | Ethyl | 2-[5-Chlor-3-(trifluormethyl)pyridin-2-yl]-1,3-thiazol-5-yl |
| 1288 | Pyridin-3-yl | Ethyl | 4-{[4-(3-Chlorpyridin-2-yl)-1H-pyrazol-1-yl]methyl}phenyl |
| 1289 | Pyridin-3-yl | Ethyl | 4-[(Trifluormethyl)sulfanyl]phenyl |
| 1290 | Pyridin-3-yl | Ethyl | 1-(2-Bromphenyl)-5-(methoxymethyl)-1H-pyrazol-4-yl |
| 1291 | Pyridin-3-yl | Ethyl | 6-[(4-Brom-1H-pyrazol-1-yl)methyl]pyridin-3-yl |
| 1292 | Pyridin-3-yl | Ethyl | 3-Chlor-4-{[3-(trifluormethyl)-1H-pyrazol-1-yl]methyl}phenyl |
| 1293 | Pyridin-3-yl | Ethyl | 6-{[4-(Trifluormethyl)-1H-pyrazol-1-yl]methyl}pyridin-3-yl |
| 1294 | Pyridin-3-yl | Ethyl | 1-(3-Brompyridin-2-yl)-5-(difluormethyl)-1H-pyrazol-4-yl |
| 1295 | Pyridazin-4-yl | Ethyl | 1-(2,6-Dichlorpyridin-3-yl)-5-(trifluormethyl)-1H-pyrazol-4-yl |
| 1296 | Pyridazin-4-yl | Ethyl | 5-(1-Methoxyethyl)-1-[3-(trifluormethyl)pyridin-2-yl]-1H-pyrazol-4-yl |
| 1297 | Pyridazin-4-yl | Ethyl | 1-Phenyl-1H-pyrazol-4-yl |
| 1298 | Pyridazin-4-yl | Ethyl | 6-Chlorpyridin-3-yl |
| 1299 | Pyridazin-4-yl | Ethyl | 2-[5-Chlor-3-(trifluormethyl)pyridin-2-yl]-1,3-thiazol-4-yl |
| 1300 | Pyridazin-4-yl | Ethyl | 3-(Trifluormethyl)-1H-pyrazol-4-yl |
| 1301 | Pyridazin-4-yl | Ethyl | 1-[3-(Difluormethyl)pyridin-2-yl]-1H-pyrazol-4-yl |
| 1302 | Pyridazin-4-yl | Ethyl | 5-(Trifluormethyl)-1-[3-(trifluormethyl)pyridin-2-yl]-1H-pyrazol-4-yl |
| 1303 | Pyridazin-4-yl | Ethyl | 1-(2-Bromphenyl)-5-(1-methoxyethyl)-1H-pyrazol-4-yl |
| 1304 | Pyridazin-4-yl | Ethyl | 1-(1,3-Thiazol-2-ylmethyl)-3-(trifluormethyl)-1H-pyrazol-4-yl |
| 1305 | Pyridazin-4-yl | Ethyl | 3-(Trifluormethyl)phenyl |
| 1306 | Pyridazin-4-yl | Ethyl | 2-[3-(1-Methoxyethyl)pyridin-2-yl]-1,3-thiazol-4-yl |
| 1307 | Pyridazin-4-yl | Ethyl | 5-(1-Fluorethyl)-1-(pyrimidin-2-yl)-1H-pyrazol-4-yl |
| 1308 | Pyridazin-4-yl | Ethyl | 6-(4-Cyanphenoxy)pyridin-3-yl |
| 1309 | Pyridazin-4-yl | Ethyl | 1-[2-(Trifluormethyl)phenyl]-1H-pyrazol-4-yl |
| 1310 | Pyridazin-4-yl | Ethyl | 1-[3-Chlor-2-(trifluormethyl)phenyl]-5-(methoxymethyl)-1H-pyrazol-4-yl |
| 1311 | Pyridazin-4-yl | Ethyl | 4-[(2-Cyanethyl)(methyl)carbamoyl]phenyl |
| 1312 | Pyridazin-4-yl | Ethyl | 4-[(Trifluoracetyl)amino]phenyl |
| 1313 | Pyridazin-4-yl | Ethyl | 1-Benzyl-3-(trifluormethyl)-1H-pyrazol-4-yl |
| 1314 | Pyridazin-4-yl | Ethyl | 6-(2,5-Dichlorphenoxy)pyridin-3-yl |
| 1315 | Pyridazin-4-yl | Ethyl | 2-(Pyrimidin-2-yl)-1,3-thiazol-5-yl |
| 1316 | Pyridazin-4-yl | Ethyl | 3-(But-2-enoylamino)-4-methoxyphenyl |
| 1317 | Pyridazin-4-yl | Ethyl | 4-(Dimethylcarbamoyl)phenyl |
| 1318 | Pyridazin-4-yl | Ethyl | 4-Chlor-3-[(cyclopropylcarbonyl)amino]phenyl |
| 1319 | Pyridazin-4-yl | Ethyl | 1-(2,6-Dichlorphenyl)-5-methyl-1H-pyrazol-4-yl |
| 1320 | Pyridazin-4-yl | Ethyl | 1-(2-Chlorpyridin-3-yl)-5-(difluormethyl)-1H-pyrazol-4-yl |
| 1321 | Pyridazin-4-yl | Ethyl | 1-(3-Chlorpyridin-2-yl)-5-(dimethoxymethyl)-1H-pyrazol-4-yl |
| 1322 | Pyridazin-4-yl | Ethyl | 5-(Difluormethyl)-1-(2,2,2-trifluorethyl)-1H-pyrazol-4-yl |
| 1323 | Pyridazin-4-yl | Ethyl | 4-[(Propan-2-ylsulfanyl)methyl]phenyl |
| 1324 | Pyridazin-4-yl | Ethyl | 1-(2-Brom-6-chlorphenyl)-5-(dimethoxymethyl)-1H-pyrazol-4-yl |
| 1325 | Pyridazin-4-yl | Ethyl | 4-Methoxy-3-[3-(pyridin-2-yl)-1H-pyrazol-1-yl]phenyl |
| 1326 | Pyridazin-4-yl | Ethyl | 3-Chlor-4-{[4-(trifluormethyl)-1H-pyrazol-1-yl]methyl}phenyl |
| 1327 | Pyridazin-4-yl | Ethyl | 3-[(4,6-Dimethoxypyrimidin-2-yl)methoxy]phenyl |
| 1328 | Pyridazin-4-yl | Ethyl | 4-Methyl-3-[(2-methylbutanoyl)amino]phenyl |
| 1329 | Pyridazin-4-yl | Ethyl | 4-{[Chlor(difluor)acetyl](methyl)amino}phenyl |
| 1330 | Pyridazin-4-yl | Ethyl | 4-{[3-(3-Chlorpyridin-2-yl)-1H-pyrazol-1-yl]methyl}phenyl |
| 1331 | Pyridazin-4-yl | Ethyl | 6-{[4-(Pyridin-2-yl)-1H-pyrazol-1-yl]methyl}pyridin-3-yl |
| 1332 | Pyridazin-4-yl | Ethyl | 4-Methoxy-3-[3-(pyrimidin-2-yl)-1H-pyrazol-1-yl]phenyl |
| 1333 | Pyridazin-4-yl | Ethyl | 6-{[4-(Trifluormethyl)phenyl]sulfanyl}pyridin-3-yl |
| 1334 | Pyridazin-4-yl | Ethyl | 4-{[3-(Pyridin-2-yl)-1H-pyrazol-1-yl]methyl}phenyl |
| 1335 | Pyridazin-4-yl | Ethyl | 4-Chlor-3-[(2,2-dimethylpropanoyl)amino]phenyl |
| 1336 | Pyridazin-4-yl | Ethyl | 1-(2-Chlorpyridin-3-yl)-5-(1-methoxyethyl)-1H-pyrazol-4-yl |
| 1337 | Pyridazin-4-yl | Ethyl | 2-(3,5-Dichlorpyridin-2-yl)-1,3-thiazol-4-yl |
| 1338 | Pyridazin-4-yl | Ethyl | 4-[(Methylsulfanyl)methyl]phenyl |
| 1339 | Pyridazin-4-yl | Ethyl | 6-(Propylamino)pyridin-3-yl |
| 1340 | Pyridazin-4-yl | Ethyl | 5-Chlor-6-(4-cyanphenoxy)pyridin-3-yl |
| 1341 | Pyridazin-4-yl | Ethyl | 6-(3,4-Dichlorphenoxy)pyridin-3-yl |
| 1342 | Pyridazin-4-yl | Ethyl | 6-[(2-Chlorpyrimidin-4-yl)oxy]pyridin-3-yl |
| 1343 | Pyridazin-4-yl | Ethyl | 1-(3-Chlorpyridin-2-yl)-5-(1-methoxyethyl)-1H-pyrazol-4-yl |
| 1344 | Pyridazin-4-yl | Ethyl | 2-[2-(Trifluormethyl)phenyl]-1,3-thiazol-4-yl |
| 1345 | Pyridazin-4-yl | Ethyl | 3-(Propan-2-ylcarbamoyl)phenyl |
| 1346 | Pyridazin-4-yl | Ethyl | 4-(Heptafluorpropyl)phenyl |
| 1347 | Pyridazin-4-yl | Ethyl | 2-(2,6-Dichlorpyridin-3-yl)-1,3-thiazol-4-yl |
| 1348 | Pyridazin-4-yl | Ethyl | 1-Ethyl-5-(trifluormethyl)-1H-pyrazol-4-yl |
| 1349 | Pyridazin-4-yl | Ethyl | 1-[3-(Difluormethyl)pyridin-2-yl]-5-methyl-1H-pyrazol-4-yl |
| 1350 | Pyridazin-4-yl | Ethyl | 1-(2,4-Dichlorphenyl)-1H-pyrazol-4-yl |
| 1351 | Pyridazin-4-yl | Ethyl | 3-Fluor-4-(methylsulfanyl)phenyl |
| 1352 | Pyridazin-4-yl | Ethyl | 2-[4-Chlor-2-(trifluormethyl)phenyl]-1,3-thiazol-5-yl |
| 1353 | Pyridazin-4-yl | Ethyl | 1-(Propan-2-yl)-5-(trifluormethyl)-1H-pyrazol-4-yl |
| 1354 | Pyridazin-4-yl | Ethyl | 4-[(2-Methylpyrimidin-4-yl)oxy]phenyl |
| 1355 | Pyridazin-4-yl | Ethyl | 6-{[5-(Trifluormethyl)pyridin-2-yl]sulfanyl}pyridin-3-yl |
| 1356 | Pyridazin-4-yl | Ethyl | 4-{[(Propan-2-yloxy)imino]methyl}phenyl |
| 1357 | Pyridazin-4-yl | Ethyl | 4-(Diethylcarbamoyl)phenyl |
| 1358 | Pyridazin-4-yl | Ethyl | 6-[(4-Nitrophenyl)sulfanyl]pyridin-3-yl |
| 1359 | Pyridazin-4-yl | Ethyl | 4-Methyl-3-{[(propan-2-yloxy)carbonyl]amino}phenyl |
| 1360 | Pyridazin-4-yl | Ethyl | 1-[4-Fluor-2-(trifluormethyl)phenyl]-5-(methoxymethyl)-1H-pyrazol-4-yl |
| 1361 | Pyridazin-4-yl | Ethyl | 5-(1,1-Difluorethyl)-1-(pyrimidin-2-yl)-1H-pyrazol-4-yl |
| 1362 | Pyridazin-4-yl | Ethyl | 3-{[3,5-Bis(trifluormethyl)-1H-1,2,4-triazol-1-yl]methyl}phenyl |
| 1363 | Pyridazin-4-yl | Ethyl | 4-{[3,5-Bis(trifluormethyl)-1H-pyrazol-1-yl]methyl}phenyl |
| 1364 | Pyridazin-4-yl | Ethyl | 1-(2-Chlorphenyl)-5-(1,1-dimethoxyethyl)-1H-pyrazol-4-yl |
| 1365 | Pyridazin-4-yl | Ethyl | 1-(3-Chlorpyridin-2-yl)-5-(1,1-difluorethyl)-1H-pyrazol-4-yl |
| 1366 | Pyridazin-4-yl | Ethyl | 3-(1-Fluorethyl)-1-methyl-1H-pyrazol-4-yl |
| 1367 | Pyridazin-4-yl | Ethyl | 1-(2,6-Dichlorpyridin-3-yl)-5-(methoxymethyl)-1H-pyrazol-4-yl |
| 1368 | Pyridazin-4-yl | Ethyl | 6-(Chinolin-6-yloxy)pyridin-3-yl |
| 1369 | Pyridazin-4-yl | Ethyl | 3-(4-Chlor-1H-pyrazol-1-yl)-4-methoxyphenyl |
| 1370 | Pyridazin-4-yl | Ethyl | 2-(2,4-Dichlorphenyl)-1,3-thiazol-4-yl |
| 1371 | Pyridazin-4-yl | Ethyl | 1-(2-Chlorpyridin-3-yl)-5-(1,1-difluorethyl)-1H-pyrazol-4-yl |
| 1372 | Pyridazin-4-yl | Ethyl | 6-{4-[4-(Methylsulfanyl)phenoxy]phenoxy}pyridin-3-yl |
| 1373 | Pyridazin-4-yl | Ethyl | 6-(Chinolin-5-yloxy)pyridin-3-yl |
| 1374 | Pyridazin-4-yl | Ethyl | 1-[4-Chlor-2-(trifluormethyl)phenyl]-5-(difluormethyl)-1H-pyrazol-4-yl |
| 1375 | Pyridazin-4-yl | Ethyl | 1-[2-(1-Methoxyethyl)phenyl]-1H-pyrazol-4-yl |
| 1376 | Pyridazin-4-yl | Ethyl | 4-Methoxy-3-[(2-methylbutanoyl)amino]phenyl |
| 1377 | Pyridazin-4-yl | Ethyl | 6-(2,4-Dichlorphenoxy)pyridin-3-yl |
| 1378 | Pyridazin-4-yl | Ethyl | 4-{[Acetyl(2-methylpropyl)amino]methyl}phenyl |
| 1379 | Pyridazin-4-yl | Ethyl | 6-[(Methylsulfanyl)methyl]pyridin-3-yl |
| 1380 | Pyridazin-4-yl | Ethyl | 2-(2,4-Dichlorphenyl)-1,3-thiazol-5-yl |
| 1381 | Pyridazin-4-yl | Ethyl | 6-[(4,5,6-Trimethylpyrimidin-2-yl)sulfanyl]pyridin-3-yl |
| 1382 | Pyridazin-4-yl | Ethyl | 4-[(4,6-Dimethylpyrimidin-2-yl)sulfanyl]phenyl |
| 1383 | Pyridazin-4-yl | Ethyl | 6-[2-Chlor-4-(methylsulfanyl)phenoxy]pyridin-3-yl |
| 1384 | Pyridazin-4-yl | Ethyl | 1-(2-Brom-6-chlorphenyl)-5-(difluormethyl)-1H-pyrazol-4-yl |
| 1385 | Pyridazin-4-yl | Ethyl | 4-(Propan-2-yloxy)-3-[3-(trifluormethyl)-1H-pyrazol-1-yl]phenyl |
| 1386 | Pyridazin-4-yl | Ethyl | 3-[Ethyl(methyl)carbamoyl]phenyl |
| 1387 | Pyridazin-4-yl | Ethyl | 1-Methyl-3-(trifluormethyl)-1H-pyrazol-4-yl |
| 1388 | Pyridazin-4-yl | Ethyl | 6-(3,5-Dimethylphenoxy)pyridin-3-yl |
| 1389 | Pyridazin-4-yl | Ethyl | 1-[4-Chlor-2-(trifluormethyl)phenyl]-5-methyl-1H-pyrazol-4-yl |
| 1390 | Pyridazin-4-yl | Ethyl | 4-Fluor-3-(trifluormethyl)phenyl |
| 1391 | Pyridazin-4-yl | Ethyl | 6-[4-(Methoxycarbonyl)phenoxy]pyridin-3-yl |
| 1392 | Pyridazin-4-yl | Ethyl | 3-[(Cyclopropylcarbonyl)amino]-4-methylphenyl |
| 1393 | Pyridazin-4-yl | Ethyl | 1-(4-Chlorphenyl)-1H-pyrazol-4-yl |
| 1394 | Pyridazin-4-yl | Ethyl | 2-[2-(Difluormethyl)phenyl]-1,3-thiazol-5-yl |
| 1395 | Pyridazin-4-yl | Ethyl | 1-Ethyl-3-(trifluormethyl)-1H-pyrazol-4-yl |
| 1396 | Pyridazin-4-yl | Ethyl | 5-Chlor-6-[4-(trifluormethyl)phenoxy]pyridin-3-yl |
| 1397 | Pyridazin-4-yl | Ethyl | 4-Methyl-3-(propanoylamino)phenyl |
| 1398 | Pyridazin-4-yl | Ethyl | 2-(2-Chlorpyridin-3-yl)-1,3-thiazol-4-yl |
| 1399 | Pyridazin-4-yl | Ethyl | 4-[(2-Methylpropanoyl)amino]phenyl |
| 1400 | Pyridazin-4-yl | Ethyl | 3-[(2,2-Dimethylpropanoyl)amino]-4-methylphenyl |
| 1401 | Pyridazin-4-yl | Ethyl | 4-Methoxy-3-{[(propan-2-yloxy)carbonyl]amino}phenyl |
| 1402 | Pyridazin-4-yl | Ethyl | 1-(2,3-Dichlorphenyl)-5-(methoxymethyl)-1H-pyrazol-4-yl |
| 1403 | Pyridazin-4-yl | Ethyl | 3-(Hydroxymethyl)-1-methyl-1H-pyrazol-4-yl |
| 1404 | Pyridazin-4-yl | Ethyl | 1-(Propan-2-yl)-3-(trifluormethyl)-1H-pyrazol-4-yl |
| 1405 | Pyridazin-4-yl | Ethyl | 2-[3-(Difluormethyl)pyridin-2-yl]-1,3-thiazol-5-yl |
| 1406 | Pyridazin-4-yl | Ethyl | 4-(Pyrrolidin-1-ylcarbonyl)phenyl |
| 1407 | Pyridazin-4-yl | Ethyl | 3-(Methoxymethyl)-1-methyl-1H-pyrazol-4-yl |
| 1408 | Pyridazin-4-yl | Ethyl | 2-(3,5-Dichlorpyridin-2-yl)-1,3-thiazol-5-yl |
| 1409 | Pyridazin-4-yl | Ethyl | 1-(2,6-Dichlorpyridin-3-yl)-5-(difluormethyl)-1H-pyrazol-4-yl |
| 1410 | Pyridazin-4-yl | Ethyl | 6-[(4,6-Dimethylpyrimidin-2-yl)oxy]pyridin-3-yl |
| 1411 | Pyridazin-4-yl | Ethyl | 4-[(4,6-Dimethoxy-1,3,5-triazin-2-yl)sulfanyl]phenyl |
| 1412 | Pyridazin-4-yl | Ethyl | 4-(Methylsulfanyl)phenyl |
| 1413 | Pyridazin-4-yl | Ethyl | 4-(Ethylsulfanyl)phenyl |
| 1414 | Pyridazin-4-yl | Ethyl | 6-[4-(Trifluormethoxy)phenoxy]pyridin-3-yl |
| 1415 | Pyridazin-4-yl | Ethyl | 5-Chlor-6-(4-nitrophenoxy)pyridin-3-yl |
| 1416 | Pyridazin-4-yl | Ethyl | 2-[2-(Difluormethyl)phenyl]-1,3-thiazol-4-yl |
| 1417 | Pyridazin-4-yl | Ethyl | 3-(Difluormethyl)-1-methyl-1H-pyrazol-4-yl |
| 1418 | Pyridazin-4-yl | Ethyl | 1-(Difluormethyl)-3-(trifluormethyl)-1H-pyrazol-4-yl |
| 1419 | Pyridazin-4-yl | Ethyl | 6-[(6-Chlorpyridin-2-yl)oxy]pyridin-3-yl |
| 1420 | Pyridazin-4-yl | Ethyl | 3-(Butanoylamino)-4-methoxyphenyl |
| 1421 | Pyridazin-4-yl | Ethyl | 4-(Acetylamino)phenyl |
| 1422 | Pyridazin-4-yl | Ethyl | 1-(2-Bromphenyl)-5-(trifluormethyl)-1H-pyrazol-4-yl |
| 1423 | Pyridazin-4-yl | Ethyl | 6-(4-Chlorphenoxy)pyridin-3-yl |
| 1424 | Pyridazin-4-yl | Ethyl | 3-{[3-(Trifluormethyl)-1H-pyrazol-1-yl]methyl}phenyl |
| 1425 | Pyridazin-4-yl | Ethyl | 5-(Difluormethyl)-1-(2-methylphenyl)-1H-pyrazol-4-yl |
| 1426 | Pyridazin-4-yl | Ethyl | 2-[2-(1-Methoxyethyl)phenyl]-1,3-thiazol-5-yl |
| 1427 | Pyridazin-4-yl | Ethyl | 1-(2-Chlorphenyl)-5-(1,1-difluorethyl)-1H-pyrazol-4-yl |
| 1428 | Pyridazin-4-yl | Ethyl | 1-(2-Brom-4-fluorphenyl)-1H-pyrazol-4-yl |
| 1429 | Pyridazin-4-yl | Ethyl | 6-[(Cyclopropylmethyl)amino]pyridin-3-yl |
| 1430 | Pyridazin-4-yl | Ethyl | 6-[(2-Methoxypyrimidin-4-yl)oxy]pyridin-3-yl |
| 1431 | Pyridazin-4-yl | Ethyl | 4-(Methoxycarbonyl)phenyl |
| 1432 | Pyridazin-4-yl | Ethyl | 1-[2-(1,1-Difluorethyl)phenyl]-5-methyl-1H-pyrazol-4-yl |
| 1433 | Pyridazin-4-yl | Ethyl | 3-{[3,5-Bis(trifluormethyl)-1H-1,2,4-triazol-1-yl]methyl}-4 chlorphenyl |
| 1434 | Pyridazin-4-yl | Ethyl | 6-(Morpholin-4-yl)pyridin-3-yl |
| 1435 | Pyridazin-4-yl | Ethyl | 2-(Pyrimidin-2-yl)-1,3-thiazol-4-yl |
| 1436 | Pyridazin-4-yl | Ethyl | 4-[(4,6-Dimethoxypyrimidin-2-yl)oxy]phenyl |
| 1437 | Pyridazin-4-yl | Ethyl | 2-{[3-(Trifluormethyl)-1H-pyrazol-1-yl]methyl}pyridin-4-yl |
| 1438 | Pyridazin-4-yl | Ethyl | 1-(2-Chlorpyridin-3-yl)-5-(trifluormethyl)-1H-pyrazol-4-yl |
| 1439 | Pyridazin-4-yl | Ethyl | 6-(4-Bromphenoxy)pyridin-3-yl |
| 1440 | Pyridazin-4-yl | Ethyl | 4-[(Propylsulfanyl)methyl]phenyl |
| 1441 | Pyridazin-4-yl | Ethyl | 6-(Pyrimidin-2-ylsulfanyl)pyridin-3-yl |
| 1442 | Pyridazin-4-yl | Ethyl | 1-(2-Chlor-4-fluorphenyl)-1H-pyrazol-4-yl |
| 1443 | Pyridazin-4-yl | Ethyl | 4-Methoxy-3-[4-(pyridin-2-yl)-1H-pyrazol-1-yl]phenyl |
| 1444 | Pyridazin-4-yl | Ethyl | 1-(2-Chlorphenyl)-1H-pyrazol-4-yl |
| 1445 | Pyridazin-4-yl | Ethyl | 6-(2,6-Dichlorphenoxy)pyridin-3-yl |
| 1446 | Pyridazin-4-yl | Ethyl | 3-[(4,6-Dimethoxypyrimidin-2-yl)oxy]phenyl |
| 1447 | Pyridazin-4-yl | Ethyl | 6-(4-Nitrophenoxy)pyridin-3-yl |
| 1448 | Pyridazin-4-yl | Ethyl | 1-(2-Chlorphenyl)-5-methyl-1H-pyrazol-4-yl |
| 1449 | Pyridazin-4-yl | Ethyl | 3-[Ethyl(methyl)carbamoyl]-4-methoxyphenyl |
| 1450 | Pyridazin-4-yl | Ethyl | 4-[(Ethylsulfanyl)methyl]phenyl |
| 1451 | Pyridazin-4-yl | Ethyl | 2-[2-(1-Methoxyethyl)phenyl]-1,3-thiazol-4-yl |
| 1452 | Pyridazin-4-yl | Ethyl | 6-{[6-(1H-Pyrazol-1-yl)pyridin-2-yl]oxy}pyridin-3-yl |
| 1453 | Pyridazin-4-yl | Ethyl | 2-[4-Chlor-2-(trifluormethyl)phenyl]-1,3-thiazol-4-yl |
| 1454 | Pyridazin-4-yl | Ethyl | 4-Methoxy-3-(1H-pyrazol-1-yl)phenyl |
| 1455 | Pyridazin-4-yl | Ethyl | 3-(Acetylamino)-4-methylphenyl |
| 1456 | Pyridazin-4-yl | Ethyl | 2-{[4-(Trifluormethyl)-1H-pyrazol-1-yl]methyl}pyridin-4-yl |
| 1457 | Pyridazin-4-yl | Ethyl | 1-(2,4-Difluorphenyl)-1H-pyrazol-4-yl |
| 1458 | Pyridazin-4-yl | Ethyl | 1-(2-Methylphenyl)-5-(trifluormethyl)-1H-pyrazol-4-yl |
| 1459 | Pyridazin-4-yl | Ethyl | 2-[3-(Difluormethyl)pyridin-2-yl]-1,3-thiazol-4-yl |
| 1460 | Pyridazin-4-yl | Ethyl | 6-(3,5-Dichlorphenoxy)pyridin-3-yl |
| 1461 | Pyridazin-4-yl | Ethyl | 1-(Pyrimidin-2-ylmethyl)-3-(trifluormethyl)-1H-pyrazol-4-yl |
| 1462 | Pyridazin-4-yl | Ethyl | 6-Methylpyridin-3-yl |
| 1463 | Pyridazin-4-yl | Ethyl | 6-[(4-Methylphenyl)sulfanyl]pyridin-3-yl |
| 1464 | Pyridazin-4-yl | Ethyl | 1-(2,4-Dichlorphenyl)-5-(methoxymethyl)-1H-pyrazol-4-yl |
| 1465 | Pyridazin-4-yl | Ethyl | 6-{4-[4-(Trifluormethyl)phenoxy]phenoxy}pyridin-3-yl |
| 1466 | Pyridazin-4-yl | Ethyl | 1-(2,6-Dichlorphenyl)-5-(trifluormethyl)-1H-pyrazol-4-yl |
| 1467 | Pyridazin-4-yl | Ethyl | 4-[(4,6-Dimethylpyrimidin-2-yl)oxy]phenyl |
| 1468 | Pyridazin-4-yl | Ethyl | 6-(Naphthalen-1-yloxy)pyridin-3-yl |
| 1469 | Pyridazin-4-yl | Ethyl | 3-Ethyl-1-methyl-1H-pyrazol-4-yl |
| 1470 | Pyridazin-4-yl | Ethyl | 2-(2,6-Dichlorpyridin-3-yl)-1,3-thiazol-5-yl |
| 1471 | Pyridazin-4-yl | Ethyl | 6-{[(4,6-Dimethoxypyrimidin-2-yl)methyl]sulfanyl}pyridin-3-yl |
| 1472 | Pyridazin-4-yl | Ethyl | 1-[3-Chlor-2-(trifluormethyl)phenyl]-5-(1-methoxyethyl)-1H-pyrazol-4-yl |
| 1473 | Pyridazin-4-yl | Ethyl | 3,4-Dimethoxyphenyl |
| 1474 | Pyridazin-4-yl | Ethyl | 4-Methoxy-3-[(methoxyacetyl)amino]phenyl |
| 1475 | Pyridazin-4-yl | Ethyl | 3-{[4-(Trifluormethyl)-1H-pyrazol-1-yl]methyl}phenyl |
| 1476 | Pyridazin-4-yl | Ethyl | 6-(2-Cyanphenoxy)pyridin-3-yl |
| 1477 | Pyridazin-4-yl | Ethyl | 3-[(Methylsulfanyl)methyl]phenyl |
| 1478 | Pyridazin-4-yl | Ethyl | 3-(Dimethoxymethyl)-1-methyl-1H-pyrazol-4-yl |
| 1479 | Pyridazin-4-yl | Ethyl | 1-(Ethoxymethyl)-3-(trifluormethyl)-1H-pyrazol-4-yl |
| 1480 | Pyridazin-4-yl | Ethyl | 1-(3-Brompyridin-2-yl)-5-(methoxymethyl)-1H-pyrazol-4-yl |
| 1481 | Pyridazin-4-yl | Ethyl | 6-(Methylsulfanyl)pyridin-3-yl |
| 1482 | Pyridazin-4-yl | Ethyl | 1-(2-Brom-6-chlorphenyl)-5-(trifluormethyl)-1H-pyrazol-4-yl |
| 1483 | Pyridazin-4-yl | Ethyl | 3-{[4-(Trifluormethyl)phenoxy]methyl}phenyl |
| 1484 | Pyridazin-4-yl | Ethyl | 6-(Heptafluorpropyl)pyridin-3-yl |
| 1485 | Pyridazin-4-yl | Ethyl | 1-Ethenyl-3-(trifluormethyl)-1H-pyrazol-4-yl |
| 1486 | Pyridazin-4-yl | Ethyl | 4-Methyl-3-nitrophenyl |
| 1487 | Pyridazin-4-yl | Ethyl | 3-(4-Brom-1H-pyrazol-1-yl)-4-methoxyphenyl |
| 1488 | Pyridazin-4-yl | Ethyl | 5-Methyl-1-(2-methylphenyl)-1H-pyrazol-4-yl |
| 1489 | Pyridazin-4-yl | Ethyl | 1-[3-(Difluormethyl)pyridin-2-yl]-5-(methoxymethyl)-1H-pyrazol-4-yl |
| 1490 | Pyridazin-4-yl | Ethyl | 6-{[2-(Trifluormethyl)pyrimidin-5-yl]oxy}pyridin-3-yl |
| 1491 | Pyridazin-4-yl | Ethyl | 2-[2-(Methoxymethyl)phenyl]-1,3-thiazol-5-yl |
| 1492 | Pyridazin-4-yl | Ethyl | 1-(Ethoxymethyl)-5-(trifluormethyl)-1H-pyrazol-4-yl |
| 1493 | Pyridazin-4-yl | Ethyl | 5-Chlor-6-[(4,5,6-trimethylpyrimidin-2-yl)sulfanyl]pyridin-3-yl |
| 1494 | Pyridazin-4-yl | Ethyl | 6-[3,5-Bis(trifluormethyl)phenoxy]pyridin-3-yl |
| 1495 | Pyridazin-4-yl | Ethyl | 4-{[4-(Pyridin-2-yl)-1H-pyrazol-1-yl]methyl}phenyl |
| 1496 | Pyridazin-4-yl | Ethyl | 1-[3-(1,1-Difluorethyl)pyridin-2-yl]-5-methyl-1H-pyrazol-4-yl |
| 1497 | Pyridazin-4-yl | Ethyl | 1-(2,6-Dichlorphenyl)-5-(difluormethyl)-1H-pyrazol-4-yl |
| 1498 | Pyridazin-4-yl | Ethyl | 1-[4-Fluor-2-(trifluormethyl)phenyl]-5-methyl-1H-pyrazol-4-yl |
| 1499 | Pyridazin-4-yl | Ethyl | 4-Chlor-3-[(cyclobutylcarbonyl)amino]phenyl |
| 1500 | Pyridazin-4-yl | Ethyl | 2-[2-(Methoxymethyl)phenyl]-1,3-thiazol-4-yl |
| 1501 | Pyridazin-4-yl | Ethyl | 4-(1H-Pyrazol-1-ylmethyl)phenyl |
| 1502 | Pyridazin-4-yl | Ethyl | 1-(2-Bromphenyl)-5-methyl-1H-pyrazol-4-yl |
| 1503 | Pyridazin-4-yl | Ethyl | 1-(2,3-Difluorphenyl)-1H-pyrazol-4-yl |
| 1504 | Pyridazin-4-yl | Ethyl | 6-{4-[(Trifluormethyl)sulfanyl]phenoxy}pyridin-3-yl |
| 1505 | Pyridazin-4-yl | Ethyl | 4-{[3-(Pentafluorethyl)-1H-1,2,4-triazol-1-yl]methyl}phenyl |
| 1506 | Pyridazin-4-yl | Ethyl | 4-{[3-(Pyrimidin-2-yl)-1H-pyrazol-1-yl]methyl}phenyl |
| 1507 | Pyridazin-4-yl | Ethyl | 1-(2-Chlorphenyl)-5-(difluormethyl)-1H-pyrazol-4-yl |
| 1508 | Pyridazin-4-yl | Ethyl | 5-(Difluormethyl)-1-[2-(trifluormethyl)phenyl]-1H-pyrazol-4-yl |
| 1509 | Pyridazin-4-yl | Ethyl | 6-(4-Methoxyphenoxy)pyridin-3-yl |
| 1510 | Pyridazin-4-yl | Ethyl | 1-[2-(Dimethoxymethyl)phenyl]-1H-pyrazol-4-yl |
| 1511 | Pyridazin-4-yl | Ethyl | 5-(Methoxymethyl)-1-(pyrimidin-2-yl)-1H-pyrazol-4-yl |
| 1512 | Pyridazin-4-yl | Ethyl | 6-tert-Butoxypyridin-3-yl |
| 1513 | Pyridazin-4-yl | Ethyl | 4-[(Cyclopentylimino)methyl]phenyl |
| 1514 | Pyridazin-4-yl | Ethyl | 1-(3-Chlorphenyl)-1H-pyrazol-4-yl |
| 1515 | Pyridazin-4-yl | Ethyl | 3-[(2-Methylpropanoyl)amino]phenyl |
| 1516 | Pyridazin-4-yl | Ethyl | 6-{[3,5-Bis(trifluormethyl)-1H-pyrazol-1-yl]methyl}pyridin-3-yl |
| 1517 | Pyridazin-4-yl | Ethyl | 4-Chlor-3-[(2-methylpropanoyl)amino]phenyl |
| 1518 | Pyridazin-4-yl | Ethyl | 4-(Propylsulfanyl)phenyl |
| 1519 | Pyridazin-4-yl | Ethyl | 1-[2-(Difluormethyl)phenyl]-5-(methoxymethyl)-1H-pyrazol-4-yl |
| 1520 | Pyridazin-4-yl | Ethyl | 4-Methoxy-3-[4-(pyrimidin-2-yl)-1H-pyrazol-1-yl]phenyl |
| 1521 | Pyridazin-4-yl | Ethyl | 4-{[(4,6-Dimethylpyrimidin-2-yl)sulfanyl]methyl}phenyl |
| 1522 | Pyridazin-4-yl | Ethyl | 1-(2-Chlorphenyl)-5-(methoxymethyl)-1H-pyrazol-4-yl |
| 1523 | Pyridazin-4-yl | Ethyl | 6-{[3,5-Bis(trifluormethyl)-1H-1,2,4-triazol-1-yl]methyl}pyridin-3-yl |
| 1524 | Pyridazin-4-yl | Ethyl | 5-Chlor-6-[4-(trifluormethoxy)phenoxy]pyridin-3-yl |
| 1525 | Pyridazin-4-yl | Ethyl | 4-[(tert-Butoxyimino)methyl]phenyl |
| 1526 | Pyridazin-4-yl | Ethyl | 2-(3-Chlorpyridin-2-yl)-1,3-thiazol-5-yl |
| 1527 | Pyridazin-4-yl | Ethyl | 1-(2-Fluorphenyl)-1H-pyrazol-4-yl |
| 1528 | Pyridazin-4-yl | Ethyl | 4-(Trifluormethoxy)phenyl |
| 1529 | Pyridazin-4-yl | Ethyl | 6-(2-Chlorphenoxy)pyridin-3-yl |
| 1530 | Pyridazin-4-yl | Ethyl | 3-[(2,2-Dimethylpropanoyl)amino]-5-(trifluormethyl)phenyl |
| 1531 | Pyridazin-4-yl | Ethyl | 6-[(4-Methylpyrimidin-2-yl)sulfanyl]pyridin-3-yl |
| 1532 | Pyridazin-4-yl | Ethyl | 6-{[2-(Morpholin-4-yl)ethyl]amino}pyridin-3-yl |
| 1533 | Pyridazin-4-yl | Ethyl | 3-Formyl-1-methyl-1H-pyrazol-4-yl |
| 1534 | Pyridazin-4-yl | Ethyl | 1-Methyl-3-(propan-2-yl)-1H-pyrazol-4-yl |
| 1535 | Pyridazin-4-yl | Ethyl | 1-(3-Chlorphenyl)-5-(1,1-dimethoxyethyl)-1H-pyrazol-4-yl |
| 1536 | Pyridazin-4-yl | Ethyl | 4-Nitrophenyl |
| 1537 | Pyridazin-4-yl | Ethyl | 6-{[5-(Methoxycarbonyl)pyridin-3-yl]oxy}pyridin-3-yl |
| 1538 | Pyridazin-4-yl | Ethyl | 3-{[4-(Trifluormethyl)benzyl]oxy}phenyl |
| 1539 | Pyridazin-4-yl | Ethyl | 1-(2,4-Dichlorphenyl)-5-(1,1-dimethoxyethyl)-1H-pyrazol-4-yl |
| 1540 | Pyridazin-4-yl | Ethyl | 4-{[4-Brom-3-(trifluormethyl)-1H-pyrazol-1-yl]methyl}phenyl |
| 1541 | Pyridazin-4-yl | Ethyl | 3-[Chlor(difluor)methyl]-1-methyl-1H-pyrazol-4-yl |
| 1542 | Pyridazin-4-yl | Ethyl | 3-[(Cyclopropylcarbonyl)amino]-4-fluorphenyl |
| 1543 | Pyridazin-4-yl | Ethyl | 6-[4-(Dimethylsulfamoyl)phenoxy]pyridin-3-yl |
| 1544 | Pyridazin-4-yl | Ethyl | 5-Methyl-1-(2-methylpyridin-3-yl)-1H-pyrazol-4-yl |
| 1545 | Pyridazin-4-yl | Ethyl | 1-(2,4-Difluorphenyl)-5-methyl-1H-pyrazol-4-yl |
| 1546 | Pyridazin-4-yl | Ethyl | 3-(Methylsulfanyl)phenyl |
| 1547 | Pyridazin-4-yl | Ethyl | 3-(Acetylamino)-4-chlorphenyl |
| 1548 | Pyridazin-4-yl | Ethyl | 4-[(4-Brom-1H-pyrazol-1-yl)methyl]phenyl |
| 1549 | Pyridazin-4-yl | Ethyl | 4-Methyl-3-[(propan-2-ylcarbamoyl)amino]phenyl |
| 1550 | Pyridazin-4-yl | Ethyl | 1-(2,2-Diethoxyethyl)-3-(trifluormethyl)-1H-pyrazol-4-yl |
| 1551 | Pyridazin-4-yl | Ethyl | 6-[(4,6-Dimethoxypyrimidin-2-yl)oxy]pyridin-3-yl |
| 1552 | Pyridazin-4-yl | Ethyl | 6-(1H-Pyrazol-1-yl)pyridin-3-yl |
| 1553 | Pyridazin-4-yl | Ethyl | 4-(1H-1,2,4-Triazol-1-ylmethyl)phenyl |
| 1554 | Pyridazin-4-yl | Ethyl | 5-(Methoxymethyl)-1-[2-(trifluormethyl)phenyl]-1H-pyrazol-4-yl |
| 1555 | Pyridazin-4-yl | Ethyl | 5-Methylpyridin-3-yl |
| 1556 | Pyridazin-4-yl | Ethyl | 5-Chlor-6-[(4,6-dimethylpyrimidin-2-yl)sulfanyl]pyridin-3-yl |
| 1557 | Pyridazin-4-yl | Ethyl | 3-[(2,2-Dimethylpropanoyl)amino]-4-methoxyphenyl |
| 1558 | Pyridazin-4-yl | Ethyl | 2-[2-(Trifluormethyl)pyridin-3-yl]-1,3-thiazol-4-yl |
| 1559 | Pyridazin-4-yl | Ethyl | 3-Cyclopropyl-1-methyl-1H-pyrazol-4-yl |
| 1560 | Pyridazin-4-yl | Ethyl | 3-Methyl-1H-pyrazol-4-yl |
| 1561 | Pyridazin-4-yl | Ethyl | 5-(Methoxymethyl)-1-(2,2,2-trifluorethyl)-1H-pyrazol-4-yl |
| 1562 | Pyridazin-4-yl | Ethyl | 4-Methoxy-3-(propanoylamino)phenyl |
| 1563 | Pyridazin-4-yl | Ethyl | 4-Methoxy-3-[(2-methylpropanoyl)amino]phenyl |
| 1564 | Pyridazin-4-yl | Ethyl | 6-(Chinolin-7-yloxy)pyridin-3-yl |
| 1565 | Pyridazin-4-yl | Ethyl | 4-{[3-(Trifluormethyl)-1H-pyrazol-1-yl]methyl}phenyl |
| 1566 | Pyridazin-4-yl | Ethyl | 3-[(4,6-Dimethylpyrimidin-2-yl)oxy]phenyl |
| 1567 | Pyridazin-4-yl | Ethyl | 4-{[3,5-Bis(trifluormethyl)-1H-1,2,4-triazol-1-yl]methyl}phenyl |
| 1568 | Pyridazin-4-yl | Ethyl | 3-Fluor-4-{[4-(trifluormethyl)-1H-pyrazol-1-yl]methyl}phenyl |
| 1569 | Pyridazin-4-yl | Ethyl | 2,3-Dihydro-1,4-benzodioxin-6-yl |
| 1570 | Pyridazin-4-yl | Ethyl | 2-[3-(Methoxymethyl)pyridin-2-yl]-1,3-thiazol-5-yl |
| 1571 | Pyridazin-4-yl | Ethyl | 6-({6-[3-(Trifluormethyl)-1H-pyrazol-1-yl]pyridin-2-yl}oxy)pyridin-3-yl |
| 1572 | Pyridazin-4-yl | Ethyl | 3-{[3-(Pyridin-2-yl)-1H-pyrazol-1-yl]methyl}phenyl |
| 1573 | Pyridazin-4-yl | Ethyl | 1-(2-Brom-4-chlorphenyl)-5-methyl-1H-pyrazol-4-yl |
| 1574 | Pyridazin-4-yl | Ethyl | 2,2-Difluor-1,3-benzodioxol-5-yl |
| 1575 | Pyridazin-4-yl | Ethyl | 2-[3-(Trifluormethyl)pyridin-2-yl]-1,3-thiazol-4-yl |
| 1576 | Pyridazin-4-yl | Ethyl | 4-(Benzylcarbamoyl)phenyl |
| 1577 | Pyridazin-4-yl | Ethyl | 4-[(2-Chlorpyrimidin-4-yl)oxy]phenyl |
| 1578 | Pyridazin-4-yl | Ethyl | 1-[3-(Trifluormethyl)pyridin-2-yl]-1H-pyrazol-4-yl |
| 1579 | Pyridazin-4-yl | Ethyl | 5-Chlor-6-(4-chlorphenoxy)pyridin-3-yl |
| 1580 | Pyridazin-4-yl | Ethyl | 3-Cyan-4-(1H-1,2,4-triazol-1-yl)phenyl |
| 1581 | Pyridazin-4-yl | Ethyl | 3-Amino-4-(methylsulfonyl)phenyl |
| 1582 | Pyridazin-4-yl | Ethyl | 4-Methyl-3-[(2-methylpropanoyl)amino]phenyl |
| 1583 | Pyridazin-4-yl | Ethyl | 1-(2,6-Dibromphenyl)-5-(trifluormethyl)-1H-pyrazol-4-yl |
| 1584 | Pyridazin-4-yl | Ethyl | 3-{[3-(Trifluormethyl)-1H-1,2,4-triazol-1-yl]methyl}phenyl |
| 1585 | Pyridazin-4-yl | Ethyl | 4-[(3,3,3-Trifluorpropanoyl)amino]phenyl |
| 1586 | Pyridazin-4-yl | Ethyl | 5-(Pyrazin-2-yl)-1-(2,2,2-trifluorethyl)-1H-pyrazol-4-yl |
| 1587 | Pyridazin-4-yl | Ethyl | 1-(2,6-Dichlorphenyl)-5-(dimethoxymethyl)-1H-pyrazol-4-yl |
| 1588 | Pyridazin-4-yl | Ethyl | 1-(4-Chlorphenyl)-5-(l,l-dimethoxyethyl)-1H-pyrazol-4-yl |
| 1589 | Pyridazin-4-yl | Ethyl | 6-[3-(Trifluormethyl)phenoxy]pyridin-3-yl |
| 1590 | Pyridazin-4-yl | Ethyl | 4-({6-[3-(Trifluormethyl)-1H-pyrazol-1-yl]pyridin-2-yl}oxy)phenyl |
| 1591 | Pyridazin-4-yl | Ethyl | 4-Methoxy-3-(propan-2-ylcarbamoyl)phenyl |
| 1592 | Pyridazin-4-yl | Ethyl | 1-[2-(1-Methoxyethyl)phenyl]-5-methyl-1H-pyrazol-4-yl |
| 1593 | Pyridazin-4-yl | Ethyl | 1-Ethyl-3-methyl-1H-pyrazol-4-yl |
| 1594 | Pyridazin-4-yl | Ethyl | 4-[(4,6-Dimethoxypyrimidin-2-yl)sulfanyl]phenyl |
| 1595 | Pyridazin-4-yl | Ethyl | 6-(4-Methylphenoxy)pyridin-3-yl |
| 1596 | Pyridazin-4-yl | Ethyl | 6-(4-Chlor-2-cyanphenoxy)pyridin-3-yl |
| 1597 | Pyridazin-4-yl | Ethyl | 1-Propyl-3-(trifluormethyl)-1H-pyrazol-4-yl |
| 1598 | Pyridazin-4-yl | Ethyl | 6-(2-Methylphenoxy)pyridin-3-yl |
| 1599 | Pyridazin-4-yl | Ethyl | 4-{[4-(Trifluormethyl)-1H-pyrazol-1-yl]methyl}phenyl |
| 1600 | Pyridazin-4-yl | Ethyl | 6-[(6-Methylpyridin-3-yl)oxy]pyridin-3-yl |
| 1601 | Pyridazin-4-yl | Ethyl | 6-Methoxypyridin-3-yl |
| 1602 | Pyridazin-4-yl | Ethyl | 4-[(Ethoxyimino)methyl]phenyl |
| 1603 | Pyridazin-4-yl | Ethyl | 4-{[Acetyl(cyclopentyl)amino]methyl}phenyl |
| 1604 | Pyridazin-4-yl | Ethyl | 3-{[3,5-Bis(trifluormethyl)-1H-pyrazol-1-yl]methyl}phenyl |
| 1605 | Pyridazin-4-yl | Ethyl | 1-(2-Brom-4-chlorphenyl)-1H-pyrazol-4-yl |
| 1606 | Pyridazin-4-yl | Ethyl | 6-[4-(1H-1,2,4-Triazol-1-yl)phenoxy]pyridin-3-yl |
| 1607 | Pyridazin-4-yl | Ethyl | 6-[4-(Trifluormethyl)phenoxy]pyridin-3-yl |
| 1608 | Pyridazin-4-yl | Ethyl | 1-(2-Chlorphenyl)-5-(trifluormethyl)-1H-pyrazol-4-yl |
| 1609 | Pyridazin-4-yl | Ethyl | 4-Methoxy-3-nitrophenyl |
| 1610 | Pyridazin-4-yl | Ethyl | 1-(2,6-Dibromphenyl)-5-(difluormethyl)-1H-pyrazol-4-yl |
| 1611 | Pyridazin-4-yl | Ethyl | 6-[(4-Methoxyphenyl)sulfanyl]pyridin-3-yl |
| 1612 | Pyridazin-4-yl | Ethyl | 1-(2,6-Dichlorphenyl)-5-(1-methoxyethyl)-1H-pyrazol-4-yl |
| 1613 | Pyridazin-4-yl | Ethyl | 4-[(6-Chlorpyridin-2-yl)oxy]phenyl |
| 1614 | Pyridazin-4-yl | Ethyl | 3-Fluor-4-{[3-(trifluormethyl)-1H-pyrazol-1-yl]methyl}phenyl |
| 1615 | Pyridazin-4-yl | Ethyl | 4-{[(Difluormethyl)sulfanyl]methyl}phenyl |
| 1616 | Pyridazin-4-yl | Ethyl | 6-{[3-(Trifluormethyl)-1H-pyrazol-1-yl]methyl}pyridin-3-yl |
| 1617 | Pyridazin-4-yl | Ethyl | 4-{[2-(3,5-Dichlorphenyl)propan-2-yl]carbamoyl}phenyl |
| 1618 | Pyridazin-4-yl | Ethyl | 3-(1H-1,2,4-Triazol-1-ylmethyl)phenyl |
| 1619 | Pyridazin-4-yl | Ethyl | 2-(3-Chlorpyridin-2-yl)-1,3-thiazol-4-yl |
| 1620 | Pyridazin-4-yl | Ethyl | 5-(1-Methoxyethyl)-1-(pyrimidin-2-yl)-1H-pyrazol-4-yl |
| 1621 | Pyridazin-4-yl | Ethyl | 1-(3-Chlorpyridin-2-yl)-5-(1,1-dimethoxyethyl)-1H-pyrazol-4-yl |
| 1622 | Pyridazin-4-yl | Ethyl | 1-(2-Brom-4-fluorphenyl)-5-methyl-1H-pyrazol-4-yl |
| 1623 | Pyridazin-4-yl | Ethyl | 3-(Butanoylamino)-4-methylphenyl |
| 1624 | Pyridazin-4-yl | Ethyl | 5-(Methoxymethyl)-1-[3-(trifluormethyl)pyridin-2-yl]-1H-pyrazol-4-yl |
| 1625 | Pyridazin-4-yl | Ethyl | 6-[3-(Trifluormethyl)-1H-pyrazol-1-yl]pyridin-3-yl |
| 1626 | Pyridazin-4-yl | Ethyl | 6-(3-Chlorphenoxy)pyridin-3-yl |
| 1627 | Pyridazin-4-yl | Ethyl | 1-(2,6-Dichlorpyridin-3-yl)-5-(1-methoxyethyl)-1H-pyrazol-4-yl |
| 1628 | Pyridazin-4-yl | Ethyl | 6-[(4,6-Dimethylpyrimidin-2-yl)sulfanyl]pyridin-3-yl |
| 1629 | Pyridazin-4-yl | Ethyl | 1-[2-(Difluormethyl)phenyl]-5-methyl-1H-pyrazol-4-yl |
| 1630 | Pyridazin-4-yl | Ethyl | 2-[2-(Trifluormethyl)phenyl]-1,3-thiazol-5-yl |
| 1631 | Pyridazin-4-yl | Ethyl | 2-[2-(Trifluormethyl)pyridin-3-yl]-1,3-thiazol-5-yl |
| 1632 | Pyridazin-4-yl | Ethyl | 6-[(4,6-Dimethoxy-1,3,5-triazin-2-yl)sulfanyl]pyridin-3-yl |
| 1633 | Pyridazin-4-yl | Ethyl | 2-(2-Chlorpyridin-3-yl)-1,3-thiazol-5-yl |
| 1634 | Pyridazin-4-yl | Ethyl | 1-(2,6-Dichlorpyridin-3-yl)-5-methyl-1H-pyrazol-4-yl |
| 1635 | Pyridazin-4-yl | Ethyl | 1-(3-Chlorpyridin-2-yl)-5-(methoxymethyl)-1H-pyrazol-4-yl |
| 1636 | Pyridazin-4-yl | Ethyl | 6-[(4-Chlorphenyl)sulfanyl]pyridin-3-yl |
| 1637 | Pyridazin-4-yl | Ethyl | 6-(Piperidin-1-yl)pyridin-3-yl |
| 1638 | Pyridazin-4-yl | Ethyl | 3-[(Cyclopentylcarbonyl)amino]-4-methylphenyl |
| 1639 | Pyridazin-4-yl | Ethyl | 6-Ethoxypyridin-3-yl |
| 1640 | Pyridazin-4-yl | Ethyl | 2-(2,6-Dichlorphenyl)-1,3-thiazol-4-yl |
| 1641 | Pyridazin-4-yl | Ethyl | 5-Chlor-6-[(4-methylpyrimidin-2-yl)sulfanyl]pyridin-3-yl |
| 1642 | Pyridazin-4-yl | Ethyl | 1-[4-Chlor-2-(trifluormethyl)phenyl]-5-(1-methoxyethyl)-1H-pyrazol-4-yl |
| 1643 | Pyridazin-4-yl | Ethyl | 6-(3-Methylphenoxy)pyridin-3-yl |
| 1644 | Pyridazin-4-yl | Ethyl | 4-[Ethyl(methyl)carbamoyl]phenyl |
| 1645 | Pyridazin-4-yl | Ethyl | 6-[3-(Trifluormethoxy)phenoxy]pyridin-3-yl |
| 1646 | Pyridazin-4-yl | Ethyl | 5-(Difluormethyl)-1-(pyrimidin-2-yl)-1H-pyrazol-4-yl |
| 1647 | Pyridazin-4-yl | Ethyl | 4-[(Cyclopropylcarbonyl)amino]phenyl |
| 1648 | Pyridazin-4-yl | Ethyl | 1-(3-Brompyridin-2-yl)-5-(1-methoxyethyl)-1H-pyrazol-4-yl |
| 1649 | Pyridazin-4-yl | Ethyl | 3-[(Cyclopropylcarbonyl)amino]-4-methoxyphenyl |
| 1650 | Pyridazin-4-yl | Ethyl | 4-Methyl-3-[(3-methylbut-2-enoyl)amino]phenyl |
| 1651 | Pyridazin-4-yl | Ethyl | 1-[3-(Methoxymethyl)pyridin-2-yl]-1H-pyrazol-4-yl |
| 1652 | Pyridazin-4-yl | Ethyl | 4-[Acetyl(methyl)amino]phenyl |
| 1653 | Pyridazin-4-yl | Ethyl | 6-(Naphthalen-2-yloxy)pyridin-3-yl |
| 1654 | Pyridazin-4-yl | Ethyl | 1-(2-Chlorphenyl)-5-(1-methoxyethyl)-1H-pyrazol-4-yl |
| 1655 | Pyridazin-4-yl | Ethyl | 1-[2-(Difluormethyl)phenyl]-5-(1-methoxyethyl)-1H-pyrazol-4-yl |
| 1656 | Pyridazin-4-yl | Ethyl | 1-(3-Chlorpyridin-2-yl)-5-methyl-1H-pyrazol-4-yl |
| 1657 | Pyridazin-4-yl | Ethyl | 4-{[3-(Trifluormethyl)-1H-1,2,4-triazol-1-yl]methyl}phenyl |
| 1658 | Pyridazin-4-yl | Ethyl | 3-[(Cyclopentylcarbonyl)amino]-4-methoxyphenyl |
| 1659 | Pyridazin-4-yl | Ethyl | 1-(2,2,2-Trifluorethyl)-5-(trifluormethyl)-1H-pyrazol-4-yl |
| 1660 | Pyridazin-4-yl | Ethyl | 6-[4-(Methylsulfanyl)phenoxy]pyridin-3-yl |
| 1661 | Pyridazin-4-yl | Ethyl | 2-[3-(Trifluormethyl)pyridin-2-yl]-1,3-thiazol-5-yl |
| 1662 | Pyridazin-4-yl | Ethyl | 4-{[3-(Methoxycarbonyl)-1H-1,2,4-triazol-1-yl]methyl}phenyl |
| 1663 | Pyridazin-4-yl | Ethyl | 1-[3-(1,1-Difluorethyl)pyridin-2-yl]-1H-pyrazol-4-yl |
| 1664 | Pyridazin-4-yl | Ethyl | 6-{[3-(Pyridin-2-yl)-1H-pyrazol-1-yl]methyl}pyridin-3-yl |
| 1665 | Pyridazin-4-yl | Ethyl | 3-(Acetylamino)phenyl |
| 1666 | Pyridazin-4-yl | Ethyl | 1-(3-Chlorpyridin-2-yl)-5-(trifluormethyl)-1H-pyrazol-4-yl |
| 1667 | Pyridazin-4-yl | Ethyl | 2-{[3-(Pyridin-2-yl)-1H-pyrazol-1-yl]methyl}pyridin-4-yl |
| 1668 | Pyridazin-4-yl | Ethyl | 3-[(Methoxyacetyl)amino]-4-methylphenyl |
| 1669 | Pyridazin-4-yl | Ethyl | 1-(3-Brompyridin-2-yl)-5-(trifluormethyl)-1H-pyrazol-4-yl |
| 1670 | Pyridazin-4-yl | Ethyl | 4-[(Methoxyimino)methyl]phenyl |
| 1671 | Pyridazin-4-yl | Ethyl | 1-(Pyrimidin-2-yl)-5-(trifluormethyl)-1H-pyrazol-4-yl |
| 1672 | Pyridazin-4-yl | Ethyl | 2-(2-Chlorphenyl)-1,3-thiazol-4-yl |
| 1673 | Pyridazin-4-yl | Ethyl | 4-Methoxy-3-[(propan-2-ylsulfonyl)amino]phenyl |
| 1674 | Pyridazin-4-yl | Ethyl | 6-(3-Cyanphenoxy)pyridin-3-yl |
| 1675 | Pyridazin-4-yl | Ethyl | 4-Fluor-3-[(2-methylpropanoyl)amino]phenyl |
| 1676 | Pyridazin-4-yl | Ethyl | 1-(2-Bromphenyl)-5-(difluormethyl)-1H-pyrazol-4-yl |
| 1677 | Pyridazin-4-yl | Ethyl | 1,3-Dimethyl-1H-pyrazol-4-yl |
| 1678 | Pyridazin-4-yl | Ethyl | 5-(1-Methoxyethyl)-1-(2,2,2-trifluorethyl)-1H-pyrazol-4-yl |
| 1679 | Pyridazin-4-yl | Ethyl | 1-(2,6-Dichlorphenyl)-5-(1-fluorethyl)-1H-pyrazol-4-yl |
| 1680 | Pyridazin-4-yl | Ethyl | 6-(Pyrrolidin-1-yl)pyridin-3-yl |
| 1681 | Pyridazin-4-yl | Ethyl | 3-(Acetylamino)-5-(trifluormethyl)phenyl |
| 1682 | Pyridazin-4-yl | Ethyl | 2-[3-(Methoxymethyl)pyridin-2-yl]-1,3-thiazol-4-yl |
| 1683 | Pyridazin-4-yl | Ethyl | 2-[3-(1-Methoxyethyl)pyridin-2-yl]-1,3-thiazol-5-yl |
| 1684 | Pyridazin-4-yl | Ethyl | 1-[3-(Dimethoxymethyl)pyridin-2-yl]-1H-pyrazol-4-yl |
| 1685 | Pyridazin-4-yl | Ethyl | 5-(1-Methoxyethyl)-1-(2-methylphenyl)-1H-pyrazol-4-yl |
| 1686 | Pyridazin-4-yl | Ethyl | 1-Methyl-1H-benzotriazol-5-yl |
| 1687 | Pyridazin-4-yl | Ethyl | 4-[(Butylsulfanyl)methyl]phenyl |
| 1688 | Pyridazin-4-yl | Ethyl | 1H-Pyrazol-4-yl |
| 1689 | Pyridazin-4-yl | Ethyl | 1-(2-Brom-6-chlorphenyl)-5-(1-methoxyethyl)-1H-pyrazol-4-yl |
| 1690 | Pyridazin-4-yl | Ethyl | 5-Chlor-6-[4-(methylsulfonyl)phenoxy]pyridin-3-yl |
| 1691 | Pyridazin-4-yl | Ethyl | 1-[3-(1-Methoxyethyl)pyridin-2-yl]-1H-pyrazol-4-yl |
| 1692 | Pyridazin-4-yl | Ethyl | 6-[(4,6-Dimethoxypyrimidin-2-yl)sulfanyl]pyridin-3-yl |
| 1693 | Pyridazin-4-yl | Ethyl | 4-[(6-Methylpyridin-3-yl)oxy]phenyl |
| 1694 | Pyridazin-4-yl | Ethyl | 3-Methoxy-1-methyl-1H-pyrazol-4-yl |
| 1695 | Pyridazin-4-yl | Ethyl | 6-(4-tert-Butylphenoxy)pyridin-3-yl |
| 1696 | Pyridazin-4-yl | Ethyl | 1-(2,4-Dichlorphenyl)-5-methyl-1H-pyrazol-4-yl |
| 1697 | Pyridazin-4-yl | Ethyl | 3-(But-2-enoylamino)-4-methylphenyl |
| 1698 | Pyridazin-4-yl | Ethyl | 4-{[4-(Pyrimidin-2-yl)-1H-pyrazol-1-yl]methyl}phenyl |
| 1699 | Pyridazin-4-yl | Ethyl | 4-{[6-(1H-Pyrazol-1-yl)pyridin-2-yl]oxy}phenyl |
| 1700 | Pyridazin-4-yl | Ethyl | 4-{[3,5-Bis(trifluormethyl)-1H-1,2,4-triazol-1-yl]methyl}-3-chlorphenyl |
| 1701 | Pyridazin-4-yl | Ethyl | 4-[(2-Methoxypyrimidin-4-yl)oxy]phenyl |
| 1702 | Pyridazin-4-yl | Ethyl | 1-[4-Chlor-2-(trifluormethyl)phenyl]-5-(methoxymethyl)-1H-pyrazol-4-yl |
| 1703 | Pyridazin-4-yl | Ethyl | 5-(Trifluormethyl)-1-[2-(trifluormethyl)phenyl]-1H-pyrazol-4-yl |
| 1704 | Pyridazin-4-yl | Ethyl | 6-(Dimethylamino)pyridin-3-yl |
| 1705 | Pyridazin-4-yl | Ethyl | 2-[5-Chlor-3-(trifluormethyl)pyridin-2-yl]-1,3-thiazol-5-yl |
| 1706 | Pyridazin-4-yl | Ethyl | 4-{[4-(3-Chlorpyridin-2-yl)-1H-pyrazol-1-yl]methyl}phenyl |
| 1707 | Pyridazin-4-yl | Ethyl | 4-[(2,2,3,3,4,4,4-Heptafluorbutanoyl)(methyl)amino]phenyl |
| 1708 | Pyridazin-4-yl | Ethyl | 4-[(Trifluormethyl)sulfanyl]phenyl |
| 1709 | Pyridazin-4-yl | Ethyl | 6-[4-(Methylsulfonyl)phenoxy]pyridin-3-yl |
| 1710 | Pyridazin-4-yl | Ethyl | 1-(2-Bromphenyl)-5-(methoxymethyl)-1H-pyrazol-4-yl |
| 1711 | Pyridazin-4-yl | Ethyl | 6-[(4-Brom-1H-pyrazol-1-yl)methyl]pyridin-3-yl |
| 1712 | Pyridazin-4-yl | Ethyl | 3-Chlor-4-{[3-(trifluormethyl)-1H-pyrazol-1-yl]methyl}phenyl |
| 1713 | Pyridazin-4-yl | Ethyl | 6-{[4-(Trifluormethyl)-1H-pyrazol-1-yl]methyl}pyridin-3-yl |
| 1714 | Pyridazin-4-yl | Ethyl | 1-(3-Brompyridin-2-yl)-5-(difluormethyl)-1H-pyrazol-4-yl |
| 1715 | Pyridazin-4-yl | H | 1-(2,6-Dichlorpyridin-3-yl)-5-(trifluormethyl)-1H-pyrazol-4-yl |
| 1716 | Pyridazin-4-yl | H | 5-(1-Methoxyethyl)-1-[3-(trifluormethyl)pyridin-2-yl]-1H-pyrazol-4-yl |
| 1717 | Pyridazin-4-yl | H | 1-Phenyl-1H-pyrazol-4-yl |
| 1718 | Pyridazin-4-yl | H | 6-Chlorpyridin-3-yl |
| 1719 | Pyridazin-4-yl | H | 2-[5-Chlor-3-(trifluormethyl)pyridin-2-yl]-1,3-thiazol-4-yl |
| 1720 | Pyridazin-4-yl | H | 3-(Trifluormethyl)-1H-pyrazol-4-yl |
| 1721 | Pyridazin-4-yl | H | 1-[3-(Difluormethyl)pyridin-2-yl]-1H-pyrazol-4-yl |
| 1722 | Pyridazin-4-yl | H | 5-(Trifluormethyl)-1-[3-(trifluormethyl)pyridin-2-yl]-1H-pyrazol-4-yl |
| 1723 | Pyridazin-4-yl | H | 1-(2-Bromphenyl)-5-(1-methoxyethyl)-1H-pyrazol-4-yl |
| 1724 | Pyridazin-4-yl | H | 1-(1,3-Thiazol-2-ylmethyl)-3-(trifluormethyl)-1H-pyrazol-4-yl |
| 1725 | Pyridazin-4-yl | H | 3-(Trifluormethyl)phenyl |
| 1726 | Pyridazin-4-yl | H | 2-[3-(1-Methoxyethyl)pyridin-2-yl]-1,3-thiazol-4-yl |
| 1727 | Pyridazin-4-yl | H | 5-(1-Fluorethyl)-1-(pyrimidin-2-yl)-1H-pyrazol-4-yl |
| 1728 | Pyridazin-4-yl | H | 6-(4-Cyanphenoxy)pyridin-3-yl |
| 1729 | Pyridazin-4-yl | H | 1-[2-(Trifluormethyl)phenyl]-1H-pyrazol-4-yl |
| 1730 | Pyridazin-4-yl | H | 1-[3-Chlor-2-(trifluormethyl)phenyl]-5-(methoxymethyl)-1H-pyrazol-4-yl |
| 1731 | Pyridazin-4-yl | H | 4-[(2-Cyanethyl)(methyl)carbamoyl]phenyl |
| 1732 | Pyridazin-4-yl | H | 4-[(Trifluoracetyl)amino]phenyl |
| 1733 | Pyridazin-4-yl | H | 1-Benzyl-3-(trifluormethyl)-1H-pyrazol-4-yl |
| 1734 | Pyridazin-4-yl | H | 6-(2,5-Dichlorphenoxy)pyridin-3-yl |
| 1735 | Pyridazin-4-yl | H | 2-(Pyrimidin-2-yl)-1,3-thiazol-5-yl |
| 1736 | Pyridazin-4-yl | H | 3-(But-2-enoylamino)-4-methoxyphenyl |
| 1737 | Pyridazin-4-yl | H | 4-(Dimethylcarbamoyl)phenyl |
| 1738 | Pyridazin-4-yl | H | 4-Chlor-3-[(cyclopropylcarbonyl)amino]phenyl |
| 1739 | Pyridazin-4-yl | H | 1-(2,6-Dichlorphenyl)-5-methyl-1H-pyrazol-4-yl |
| 1740 | Pyridazin-4-yl | H | 1-(2-Chlorpyridin-3-yl)-5-(difluormethyl)-1H-pyrazol-4-yl |
| 1741 | Pyridazin-4-yl | H | 1-(3-Chlorpyridin-2-yl)-5-(dimethoxymethyl)-1H-pyrazol-4-yl |
| 1742 | Pyridazin-4-yl | H | 5-(Difluormethyl)-1-(2,2,2-trifluorethyl)-1H-pyrazol-4-yl |
| 1743 | Pyridazin-4-yl | H | 4-[(Propan-2-ylsulfanyl)methyl]phenyl |
| 1744 | Pyridazin-4-yl | H | 1-(2-Brom-6-chlorphenyl)-5-(dimethoxymethyl)-1H-pyrazol-4-yl |
| 1745 | Pyridazin-4-yl | H | 4-Methoxy-3-[3-(pyridin-2-yl)-1H-pyrazol-1-yl]phenyl |
| 1746 | Pyridazin-4-yl | H | 3-Chlor-4-{[4-(trifluormethyl)-1H-pyrazol-1-yl]methyl}phenyl |
| 1747 | Pyridazin-4-yl | H | 3-[(4,6-Dimethoxypyrimidin-2-yl)methoxy]phenyl |
| 1748 | Pyridazin-4-yl | H | 4-Methyl-3-[(2-methylbutanoyl)amino]phenyl |
| 1749 | Pyridazin-4-yl | H | 4-{[Chlor(difluor)acetyl](methyl)amino}phenyl |
| 1750 | Pyridazin-4-yl | H | 4-{[3-(3-Chlorpyridin-2-yl)-1H-pyrazol-1-yl]methyl}phenyl |
| 1751 | Pyridazin-4-yl | H | 6-{[4-(Pyridin-2-yl)-1H-pyrazol-1-yl]methyl}pyridin-3-yl |
| 1752 | Pyridazin-4-yl | H | 4-Methoxy-3-[3-(pyrimidin-2-yl)-1H-pyrazol-1-yl]phenyl |
| 1753 | Pyridazin-4-yl | H | 6-{[4-(Trifluormethyl)phenyl]sulfanyl}pyridin-3-yl |
| 1754 | Pyridazin-4-yl | H | 4-{[3-(Pyridin-2-yl)-1H-pyrazol-1-yl]methyl}phenyl |
| 1755 | Pyridazin-4-yl | H | 4-Chlor-3-[(2,2-dimethylpropanoyl)amino]phenyl |
| 1756 | Pyridazin-4-yl | H | 1-(2-Chlorpyridin-3-yl)-5-(1-methoxyethyl)-1H-pyrazol-4-yl |
| 1757 | Pyridazin-4-yl | H | 2-(3,5-Dichlorpyridin-2-yl)-1,3-thiazol-4-yl |
| 1758 | Pyridazin-4-yl | H | 4-[(Methylsulfanyl)methyl]phenyl |
| 1759 | Pyridazin-4-yl | H | 6-(Propylamino)pyridin-3-yl |
| 1760 | Pyridazin-4-yl | H | 5-Chlor-6-(4-cyanphenoxy)pyridin-3-yl |
| 1761 | Pyridazin-4-yl | H | 6-(3,4-Dichlorphenoxy)pyridin-3-yl |
| 1762 | Pyridazin-4-yl | H | 6-[(2-Chlorpyrimidin-4-yl)oxy]pyridin-3-yl |
| 1763 | Pyridazin-4-yl | H | 1-(3-Chlorpyridin-2-yl)-5-(1-methoxyethyl)-1H-pyrazol-4-yl |
| 1764 | Pyridazin-4-yl | H | 2-[2-(Trifluormethyl)phenyl]-1,3-thiazol-4-yl |
| 1765 | Pyridazin-4-yl | H | 3-(Propan-2-ylcarbamoyl)phenyl |
| 1766 | Pyridazin-4-yl | H | 4-(Heptafluorpropyl)phenyl |
| 1767 | Pyridazin-4-yl | H | 2-(2,6-Dichlorpyridin-3-yl)-1,3-thiazol-4-yl |
| 1768 | Pyridazin-4-yl | H | 1-Ethyl-5-(trifluormethyl)-1H-pyrazol-4-yl |
| 1769 | Pyridazin-4-yl | H | 1-[3-(Difluormethyl)pyridin-2-yl]-5-methyl-1H-pyrazol-4-yl |
| 1770 | Pyridazin-4-yl | H | 1-(2,4-Dichlorphenyl)-1H-pyrazol-4-yl |
| 1771 | Pyridazin-4-yl | H | 3-Fluor-4-(methylsulfanyl)phenyl |
| 1772 | Pyridazin-4-yl | H | 2-[4-Chlor-2-(trifluormethyl)phenyl]-1,3-thiazol-5-yl |
| 1773 | Pyridazin-4-yl | H | 1-(Propan-2-yl)-5-(trifluormethyl)-1H-pyrazol-4-yl |
| 1774 | Pyridazin-4-yl | H | 4-[(2-Methylpyrimidin-4-yl)oxy]phenyl |
| 1775 | Pyridazin-4-yl | H | 6-{[5-(Trifluormethyl)pyridin-2-yl]sulfanyl}pyridin-3-yl |
| 1776 | Pyridazin-4-yl | H | 4-{[(Propan-2-yloxy)imino]methyl}phenyl |
| 1777 | Pyridazin-4-yl | H | 4-(Diethylcarbamoyl)phenyl |
| 1778 | Pyridazin-4-yl | H | 6-[(4-Nitrophenyl)sulfanyl]pyridin-3-yl |
| 1779 | Pyridazin-4-yl | H | 4-Methyl-3-{[(propan-2-yloxy)carbonyl]amino}phenyl |
| 1780 | Pyridazin-4-yl | H | 1-[4-Fluor-2-(trifluormethyl)phenyl]-5-(methoxymethyl)-1H-pyrazol-4-yl |
| 1781 | Pyridazin-4-yl | H | 5-(1,1-Difluorethyl)-1-(pyrimidin-2-yl)-1H-pyrazol-4-yl |
| 1782 | Pyridazin-4-yl | H | 3-{[3,5-Bis(trifluormethyl)-1H-1,2,4-triazol-1-yl]methyl}phenyl |
| 1783 | Pyridazin-4-yl | H | 4-{[3,5-Bis(trifluormethyl)-1H-pyrazol-1-yl]methyl}phenyl |
| 1784 | Pyridazin-4-yl | H | 1-(2-Chlorphenyl)-5-(1,1-dimethoxyethyl)-1H-pyrazol-4-yl |
| 1785 | Pyridazin-4-yl | H | 1-(3-Chlorpyridin-2-yl)-5-(1,1-difluorethyl)-1H-pyrazol-4-yl |
| 1786 | Pyridazin-4-yl | H | 3-(1-Fluorethyl)-1-methyl-1H-pyrazol-4-yl |
| 1787 | Pyridazin-4-yl | H | 1-(2,6-Dichlorpyridin-3-yl)-5-(methoxymethyl)-1H-pyrazol-4-yl |
| 1788 | Pyridazin-4-yl | H | 6-(Chinolin-6-yloxy)pyridin-3-yl |
| 1789 | Pyridazin-4-yl | H | 3-(4-Chlor-1H-pyrazol-1-yl)-4-methoxyphenyl |
| 1790 | Pyridazin-4-yl | H | 2-(2,4-Dichlorphenyl)-1,3-thiazol-4-yl |
| 1791 | Pyridazin-4-yl | H | 1-(2-Chlorpyridin-3-yl)-5-(1,1-difluorethyl)-1H-pyrazol-4-yl |
| 1792 | Pyridazin-4-yl | H | 6-{4-[4-(Methylsulfanyl)phenoxy]phenoxy}pyridin-3-yl |
| 1793 | Pyridazin-4-yl | H | 6-(Chinolin-5-yloxy)pyridin-3-yl |
| 1794 | Pyridazin-4-yl | H | 1-[4-Chlor-2-(trifluormethyl)phenyl]-5-(difluormethyl)-1H-pyrazol-4-yl |
| 1795 | Pyridazin-4-yl | H | 1-[2-(1-Methoxyethyl)phenyl]-1H-pyrazol-4-yl |
| 1796 | Pyridazin-4-yl | H | 4-Methoxy-3-[(2-methylbutanoyl)amino]phenyl |
| 1797 | Pyridazin-4-yl | H | 6-(2,4-Dichlorphenoxy)pyridin-3-yl |
| 1798 | Pyridazin-4-yl | H | 4-{[Acetyl(2-methylpropyl)amino]methyl}phenyl |
| 1799 | Pyridazin-4-yl | H | 6-[(Methylsulfanyl)methyl]pyridin-3-yl |
| 1800 | Pyridazin-4-yl | H | 2-(2,4-Dichlorphenyl)-1,3-thiazol-5-yl |
| 1801 | Pyridazin-4-yl | H | 6-[(4,5,6-Trimethylpyrimidin-2-yl)sulfanyl]pyridin-3-yl |
| 1802 | Pyridazin-4-yl | H | 4-[(4,6-Dimethylpyrimidin-2-yl)sulfanyl]phenyl |
| 1803 | Pyridazin-4-yl | H | 6-[2-Chlor-4-(methylsulfanyl)phenoxy]pyridin-3-yl |
| 1804 | Pyridazin-4-yl | H | 1-(2-Brom-6-chlorphenyl)-5-(difluormethyl)-1H-pyrazol-4-yl |
| 1805 | Pyridazin-4-yl | H | 4-(Propan-2-yloxy)-3-[3-(trifluormethyl)-1H-pyrazol-1-yl]phenyl |
| 1806 | Pyridazin-4-yl | H | 3-[Ethyl(methyl)carbamoyl]phenyl |
| 1807 | Pyridazin-4-yl | H | 6-(3,5-Dimethylphenoxy)pyridin-3-yl |
| 1808 | Pyridazin-4-yl | H | 1-[4-Chlor-2-(trifluormethyl)phenyl]-5-methyl-1H-pyrazol-4-yl |
| 1809 | Pyridazin-4-yl | H | 4-Fluor-3-(trifluormethyl)phenyl |
| 1810 | Pyridazin-4-yl | H | 6-[4-(Methoxycarbonyl)phenoxy]pyridin-3-yl |
| 1811 | Pyridazin-4-yl | H | 3-[(Cyclopropylcarbonyl)amino]-4-methylphenyl |
| 1812 | Pyridazin-4-yl | H | 1-(4-Chlorphenyl)-1H-pyrazol-4-yl |
| 1813 | Pyridazin-4-yl | H | 2-[2-(Difluormethyl)phenyl]-1,3-thiazol-5-yl |
| 1814 | Pyridazin-4-yl | H | 1-Ethyl-3-(trifluormethyl)-1H-pyrazol-4-yl |
| 1815 | Pyridazin-4-yl | H | 5-Chlor-6-[4-(trifluormethyl)phenoxy]pyridin-3-yl |
| 1816 | Pyridazin-4-yl | H | 4-Methyl-3-(propanoylamino)phenyl |
| 1817 | Pyridazin-4-yl | H | 2-(2-Chlorpyridin-3-yl)-1,3-thiazol-4-yl |
| 1818 | Pyridazin-4-yl | H | 4-[(2-Methylpropanoyl)amino]phenyl |
| 1819 | Pyridazin-4-yl | H | 3-[(2,2-Dimethylpropanoyl)amino]-4-methylphenyl |
| 1820 | Pyridazin-4-yl | H | 4-Methoxy-3-{[(propan-2-yloxy)carbonyl]amino}phenyl |
| 1821 | Pyridazin-4-yl | H | 1-(2,3-Dichlorphenyl)-5-(methoxymethyl)-1H-pyrazol-4-yl |
| 1822 | Pyridazin-4-yl | H | 3-(Hydroxymethyl)-1-methyl-1H-pyrazol-4-yl |
| 1823 | Pyridazin-4-yl | H | 1-(Propan-2-yl)-3-(trifluormethyl)-1H-pyrazol-4-yl |
| 1824 | Pyridazin-4-yl | H | 2-[3-(Difluormethyl)pyridin-2-yl]-1,3-thiazol-5-yl |
| 1825 | Pyridazin-4-yl | H | 4-(Pyrrolidin-1-ylcarbonyl)phenyl |
| 1826 | Pyridazin-4-yl | H | 3-(Methoxymethyl)-1-methyl-1H-pyrazol-4-yl |
| 1827 | Pyridazin-4-yl | H | 2-(3,5-Dichlorpyridin-2-yl)-1,3-thiazol-5-yl |
| 1828 | Pyridazin-4-yl | H | 1-(2,6-Dichlorpyridin-3-yl)-5-(difluormethyl)-1H-pyrazol-4-yl |
| 1829 | Pyridazin-4-yl | H | 6-[(4,6-Dimethylpyrimidin-2-yl)oxy]pyridin-3-yl |
| 1830 | Pyridazin-4-yl | H | 4-[(4,6-Dimethoxy-1,3,5-triazin-2-yl)sulfanyl]phenyl |
| 1831 | Pyridazin-4-yl | H | 4-(Methylsulfanyl)phenyl |
| 1832 | Pyridazin-4-yl | H | 4-(Ethylsulfanyl)phenyl |
| 1833 | Pyridazin-4-yl | H | 6-[4-(Trifluormethoxy)phenoxy]pyridin-3-yl |
| 1834 | Pyridazin-4-yl | H | 5-Chlor-6-(4-nitrophenoxy)pyridin-3-yl |
| 1835 | Pyridazin-4-yl | H | 2-[2-(Difluormethyl)phenyl]-1,3-thiazol-4-yl |
| 1836 | Pyridazin-4-yl | H | 3-(Difluormethyl)-1-methyl-1H-pyrazol-4-yl |
| 1837 | Pyridazin-4-yl | H | 1-(Difluormethyl)-3-(trifluormethyl)-1H-pyrazol-4-yl |
| 1838 | Pyridazin-4-yl | H | 6-[(6-Chlorpyridin-2-yl)oxy]pyridin-3-yl |
| 1839 | Pyridazin-4-yl | H | 3-(Butanoylamino)-4-methoxyphenyl |
| 1840 | Pyridazin-4-yl | H | 4-(Acetylamino)phenyl |
| 1841 | Pyridazin-4-yl | H | 1-(2-Bromphenyl)-5-(trifluormethyl)-1H-pyrazol-4-yl |
| 1842 | Pyridazin-4-yl | H | 6-(4-Chlorphenoxy)pyridin-3-yl |
| 1843 | Pyridazin-4-yl | H | 3-{[3-(Trifluormethyl)-1H-pyrazol-1-yl]methyl}phenyl |
| 1844 | Pyridazin-4-yl | H | 5-(Difluormethyl)-1-(2-methylphenyl)-1H-pyrazol-4-yl |
| 1845 | Pyridazin-4-yl | H | 2-[2-(1-Methoxyethyl)phenyl]-1,3-thiazol-5-yl |
| 1846 | Pyridazin-4-yl | H | 1-(2-Chlorphenyl)-5-(1,1-difluorethyl)-1H-pyrazol-4-yl |
| 1847 | Pyridazin-4-yl | H | 1-(2-Brom-4-fluorphenyl)-1H-pyrazol-4-yl |
| 1848 | Pyridazin-4-yl | H | 6-[(Cyclopropylmethyl)amino]pyridin-3-yl |
| 1849 | Pyridazin-4-yl | H | 6-[(2-Methoxypyrimidin-4-yl)oxy]pyridin-3-yl |
| 1850 | Pyridazin-4-yl | H | 4-(Methoxycarbonyl)phenyl |
| 1851 | Pyridazin-4-yl | H | 1-[2-(1,1-Difluorethyl)phenyl]-5-methyl-1H-pyrazol-4-yl |
| 1852 | Pyridazin-4-yl | H | 3-{[3,5-Bis(trifluormethyl)-1H-1,2,4-triazol-1-yl]methyl}-4-chlorphenyl |
| 1853 | Pyridazin-4-yl | H | 6-(Morpholin-4-yl)pyridin-3-yl |
| 1854 | Pyridazin-4-yl | H | 2-(Pyrimidin-2-yl)-1,3-thiazol-4-yl |
| 1855 | Pyridazin-4-yl | H | 4-[(4,6-Dimethoxypyrimidin-2-yl)oxy]phenyl |
| 1856 | Pyridazin-4-yl | H | 2-{[3-(Trifluormethyl)-1H-pyrazol-1-yl]methyl}pyridin-4-yl |
| 1857 | Pyridazin-4-yl | H | 1-(2-Chlorpyridin-3-yl)-5-(trifluormethyl)-1H-pyrazol-4-yl |
| 1858 | Pyridazin-4-yl | H | 6-(4-Bromphenoxy)pyridin-3-yl |
| 1859 | Pyridazin-4-yl | H | 4-[(Propylsulfanyl)methyl]phenyl |
| 1860 | Pyridazin-4-yl | H | 6-(Pyrimidin-2-ylsulfanyl)pyridin-3-yl |
| 1861 | Pyridazin-4-yl | H | 1-(2-Chlor-4-fluorphenyl)-1H-pyrazol-4-yl |
| 1862 | Pyridazin-4-yl | H | 4-Methoxy-3-[4-(pyridin-2-yl)-1H-pyrazol-1-yl]phenyl |
| 1863 | Pyridazin-4-yl | H | 1-(2-Chlorphenyl)-1H-pyrazol-4-yl |
| 1864 | Pyridazin-4-yl | H | 6-(2,6-Dichlorphenoxy)pyridin-3-yl |
| 1865 | Pyridazin-4-yl | H | 3-[(4,6-Dimethoxypyrimidin-2-yl)oxy]phenyl |
| 1866 | Pyridazin-4-yl | H | 6-(4-Nitrophenoxy)pyridin-3-yl |
| 1867 | Pyridazin-4-yl | H | 1-(2-Chlorphenyl)-5-methyl-1H-pyrazol-4-yl |
| 1868 | Pyridazin-4-yl | H | 3-[Ethyl(methyl)carbamoyl]-4-methoxyphenyl |
| 1869 | Pyridazin-4-yl | H | 4-[(Ethylsulfanyl)methyl]phenyl |
| 1870 | Pyridazin-4-yl | H | 2-[2-(1-Methoxyethyl)phenyl]-1,3-thiazol-4-yl |
| 1871 | Pyridazin-4-yl | H | 6-{[6-(1H-Pyrazol-1-yl)pyridin-2-yl]oxy}pyridin-3-yl |
| 1872 | Pyridazin-4-yl | H | 2-[4-Chlor-2-(trifluormethyl)phenyl]-1,3-thiazol-4-yl |
| 1873 | Pyridazin-4-yl | H | 4-Methoxy-3-(1H-pyrazol-1-yl)phenyl |
| 1874 | Pyridazin-4-yl | H | 3-(Acetylamino)-4-methylphenyl |
| 1875 | Pyridazin-4-yl | H | 2-{[4-(Trifluormethyl)-1H-pyrazol-1-yl]methyl}pyridin-4-yl |
| 1876 | Pyridazin-4-yl | H | 1-(2,4-Difluorphenyl)-1H-pyrazol-4-yl |
| 1877 | Pyridazin-4-yl | H | 1-(2-Methylphenyl)-5-(trifluormethyl)-1H-pyrazol-4-yl |
| 1878 | Pyridazin-4-yl | H | 2-[3-(Difluormethyl)pyridin-2-yl]-1,3-thiazol-4-yl |
| 1879 | Pyridazin-4-yl | H | 6-(3,5-Dichlorphenoxy)pyridin-3-yl |
| 1880 | Pyridazin-4-yl | H | 1-(Pyrimidin-2-ylmethyl)-3-(trifluormethyl)-1H-pyrazol-4-yl |
| 1881 | Pyridazin-4-yl | H | 6-Methylpyridin-3-yl |
| 1882 | Pyridazin-4-yl | H | 6-[(4-Methylphenyl)sulfanyl]pyridin-3-yl |
| 1883 | Pyridazin-4-yl | H | 1-(2,4-Dichlorphenyl)-5-(methoxymethyl)-1H-pyrazol-4-yl |
| 1884 | Pyridazin-4-yl | H | 6-{4-[4-(Trifluormethyl)phenoxy]phenoxy}pyridin-3-yl |
| 1885 | Pyridazin-4-yl | H | 1-(2,6-Dichlorphenyl)-5-(trifluormethyl)-1H-pyrazol-4-yl |
| 1886 | Pyridazin-4-yl | H | 4-[(4,6-Dimethylpyrimidin-2-yl)oxy]phenyl |
| 1887 | Pyridazin-4-yl | H | 6-(Naphthalen-1-yloxy)pyridin-3-yl |
| 1888 | Pyridazin-4-yl | H | 3-Ethyl-1-methyl-1H-pyrazol-4-yl |
| 1889 | Pyridazin-4-yl | H | 2-(2,6-Dichlorpyridin-3-yl)-1,3-thiazol-5-yl |
| 1890 | Pyridazin-4-yl | H | 6-{[(4,6-Dimethoxypyrimidin-2-yl)methyl]sulfanyl}pyridin-3-yl |
| 1891 | Pyridazin-4-yl | H | 1-[3-Chlor-2-(trifluormethyl)phenyl]-5-(1-methoxyethyl)-1H-pyrazol-4-yl |
| 1892 | Pyridazin-4-yl | H | 3,4-Dimethoxyphenyl |
| 1893 | Pyridazin-4-yl | H | 4-Methoxy-3-[(methoxyacetyl)amino]phenyl |
| 1894 | Pyridazin-4-yl | H | 3-{[4-(Trifluormethyl)-1H-pyrazol-1-yl]methyl}phenyl |
| 1895 | Pyridazin-4-yl | H | 6-(2-Cyanphenoxy)pyridin-3-yl |
| 1896 | Pyridazin-4-yl | H | 3-[(Methylsulfanyl)methyl]phenyl |
| 1897 | Pyridazin-4-yl | H | 3-(Dimethoxymethyl)-1-methyl-1H-pyrazol-4-yl |
| 1898 | Pyridazin-4-yl | H | 1-(Ethoxymethyl)-3-(trifluormethyl)-1H-pyrazol-4-yl |
| 1899 | Pyridazin-4-yl | H | 1-(3-Brompyridin-2-yl)-5-(methoxymethyl)-1H-pyrazol-4-yl |
| 1900 | Pyridazin-4-yl | H | 6-(Methylsulfanyl)pyridin-3-yl |
| 1901 | Pyridazin-4-yl | H | 1-(2-Brom-6-chlorphenyl)-5-(trifluormethyl)-1H-pyrazol-4-yl |
| 1902 | Pyridazin-4-yl | H | 3-{[4-(Trifluormethyl)phenoxy]methyl}phenyl |
| 1903 | Pyridazin-4-yl | H | 6-(Heptafluorpropyl)pyridin-3-yl |
| 1904 | Pyridazin-4-yl | H | 1-Ethenyl-3-(trifluormethyl)-1H-pyrazol-4-yl |
| 1905 | Pyridazin-4-yl | H | 4-Methyl-3-nitrophenyl |
| 1906 | Pyridazin-4-yl | H | 3-(4-Brom-1H-pyrazol-1-yl)-4-methoxyphenyl |
| 1907 | Pyridazin-4-yl | H | 5-Methyl-1-(2-methylphenyl)-1H-pyrazol-4-yl |
| 1908 | Pyridazin-4-yl | H | 1-[3-(Difluormethyl)pyridin-2-yl]-5-(methoxymethyl)-1H-pyrazol-4-yl |
| 1909 | Pyridazin-4-yl | H | 6-{[2-(Trifluormethyl)pyrimidin-5-yl]oxy}pyridin-3-yl |
| 1910 | Pyridazin-4-yl | H | 2-[2-(Methoxymethyl)phenyl]-1,3-thiazol-5-yl |
| 1911 | Pyridazin-4-yl | H | 1-(Ethoxymethyl)-5-(trifluormethyl)-1H-pyrazol-4-yl |
| 1912 | Pyridazin-4-yl | H | 5-Chlor-6-[(4,5,6-trimethylpyrimidin-2-yl)sulfanyl]pyridin-3-yl |
| 1913 | Pyridazin-4-yl | H | 6-[3,5-Bis(trifluormethyl)phenoxy]pyridin-3-yl |
| 1914 | Pyridazin-4-yl | H | 4-{[4-(Pyridin-2-yl)-1H-pyrazol-1-yl]methyl}phenyl |
| 1915 | Pyridazin-4-yl | H | 1-[3-(1,1-Difluorethyl)pyridin-2-yl]-5-methyl-1H-pyrazol-4-yl |
| 1916 | Pyridazin-4-yl | H | 1-(2,6-Dichlorphenyl)-5-(difluormethyl)-1H-pyrazol-4-yl |
| 1917 | Pyridazin-4-yl | H | 1-[4-Fluor-2-(trifluormethyl)phenyl]-5-methyl-1H-pyrazol-4-yl |
| 1918 | Pyridazin-4-yl | H | 4-Chlor-3-[(cyclobutylcarbonyl)amino]phenyl |
| 1919 | Pyridazin-4-yl | H | 2-[2-(Methoxymethyl)phenyl]-1,3-thiazol-4-yl |
| 1920 | Pyridazin-4-yl | H | 4-(1H-Pyrazol-1-ylmethyl)phenyl |
| 1921 | Pyridazin-4-yl | H | 1-(2-Bromphenyl)-5-methyl-1H-pyrazol-4-yl |
| 1922 | Pyridazin-4-yl | H | 1-(2,3-Difluorphenyl)-1H-pyrazol-4-yl |
| 1923 | Pyridazin-4-yl | H | 6-{4-[(Trifluormethyl)sulfanyl]phenoxy}pyridin-3-yl |
| 1924 | Pyridazin-4-yl | H | 4-{[3-(Pentafluorethyl)-1H-1,2,4-triazol-1-yl]methyl}phenyl |
| 1925 | Pyridazin-4-yl | H | 4-{[3-(Pyrimidin-2-yl)-1H-pyrazol-1-yl]methyl}phenyl |
| 1926 | Pyridazin-4-yl | H | 1-(2-Chlorphenyl)-5-(difluormethyl)-1H-pyrazol-4-yl |
| 1927 | Pyridazin-4-yl | H | 5-(Difluormethyl)-1-[2-(trifluormethyl)phenyl]-1H-pyrazol-4-yl |
| 1928 | Pyridazin-4-yl | H | 6-(4-Methoxyphenoxy)pyridin-3-yl |
| 1929 | Pyridazin-4-yl | H | 1-[2-(Dimethoxymethyl)phenyl]-1H-pyrazol-4-yl |
| 1930 | Pyridazin-4-yl | H | 5-(Methoxymethyl)-1-(pyrimidin-2-yl)-1H-pyrazol-4-yl |
| 1931 | Pyridazin-4-yl | H | 6-tert-Butoxypyridin-3-yl |
| 1932 | Pyridazin-4-yl | H | 4-[(Cyclopentylimino)methyl]phenyl |
| 1933 | Pyridazin-4-yl | H | 1-(3-Chlorphenyl)-1H-pyrazol-4-yl |
| 1934 | Pyridazin-4-yl | H | 3-[(2-Methylpropanoyl)amino]phenyl |
| 1935 | Pyridazin-4-yl | H | 6-{[3,5-Bis(trifluormethyl)-1H-pyrazol-1-yl]methyl}pyridin-3-yl |
| 1936 | Pyridazin-4-yl | H | 4-Chlor-3-[(2-methylpropanoyl)amino]phenyl |
| 1937 | Pyridazin-4-yl | H | 4-(Propylsulfanyl)phenyl |
| 1938 | Pyridazin-4-yl | H | 1-[2-(Difluormethyl)phenyl]-5-(methoxymethyl)-1H-pyrazol-4-yl |
| 1939 | Pyridazin-4-yl | H | 4-Methoxy-3-[4-(pyrimidin-2-yl)-1H-pyrazol-1-yl]phenyl |
| 1940 | Pyridazin-4-yl | H | 4-{[(4,6-Dimethylpyrimidin-2-yl)sulfanyl]methyl}phenyl |
| 1941 | Pyridazin-4-yl | H | 1-(2-Chlorphenyl)-5-(methoxymethyl)-1H-pyrazol-4-yl |
| 1942 | Pyridazin-4-yl | H | 6-{[3,5-Bis(trifluormethyl)-1H-1,2,4-triazol-1-yl]methyl}pyridin-3-yl |
| 1943 | Pyridazin-4-yl | H | 5-Chlor-6-[4-(trifluormethoxy)phenoxy]pyridin-3-yl |
| 1944 | Pyridazin-4-yl | H | 4-[(tert-Butoxyimino)methyl]phenyl |
| 1945 | Pyridazin-4-yl | H | 2-(3-Chlorpyridin-2-yl)-1,3-thiazol-5-yl |
| 1946 | Pyridazin-4-yl | H | 1-(2-Fluorphenyl)-1H-pyrazol-4-yl |
| 1947 | Pyridazin-4-yl | H | 4-(Trifluormethoxy)phenyl |
| 1948 | Pyridazin-4-yl | H | 6-(2-Chlorphenoxy)pyridin-3-yl |
| 1949 | Pyridazin-4-yl | H | 3-[(2,2-Dimethylpropanoyl)amino]-5-(trifluormethyl)phenyl |
| 1950 | Pyridazin-4-yl | H | 6-[(4-Methylpyrimidin-2-yl)sulfanyl]pyridin-3-yl |
| 1951 | Pyridazin-4-yl | H | 6-{[2-(Morpholin-4-yl)ethyl]amino}pyridin-3-yl |
| 1952 | Pyridazin-4-yl | H | 3-Formyl-1-methyl-1H-pyrazol-4-yl |
| 1953 | Pyridazin-4-yl | H | 1-Methyl-3-(propan-2-yl)-1H-pyrazol-4-yl |
| 1954 | Pyridazin-4-yl | H | 1-(3-Chlorphenyl)-5-(1,1-dimethoxyethyl)-1H-pyrazol-4-yl |
| 1955 | Pyridazin-4-yl | H | 4-Nitrophenyl |
| 1956 | Pyridazin-4-yl | H | 6-{[5-(Methoxycarbonyl)pyridin-3-yl]oxy}pyridin-3-yl |
| 1957 | Pyridazin-4-yl | H | 3-{[4-(Trifluormethyl)benzyl]oxy}phenyl |
| 1958 | Pyridazin-4-yl | H | 1-(2,4-Dichlorphenyl)-5-(l,l-dimethoxyethyl)-1H-pyrazol-4-yl |
| 1959 | Pyridazin-4-yl | H | 4-{[4-Brom-3-(trifluormethyl)-1H-pyrazol-1-yl]methyl}phenyl |
| 1960 | Pyridazin-4-yl | H | 3-[Chlor(difluor)methyl]-1-methyl-1H-pyrazol-4-yl |
| 1961 | Pyridazin-4-yl | H | 3-[(Cyclopropylcarbonyl)amino]-4-fluorphenyl |
| 1962 | Pyridazin-4-yl | H | 6-[4-(Dimethylsulfamoyl)phenoxy]pyridin-3-yl |
| 1963 | Pyridazin-4-yl | H | 5-Methyl-1-(2-methylpyridin-3-yl)-1H-pyrazol-4-yl |
| 1964 | Pyridazin-4-yl | H | 1-(2,4-Difluorphenyl)-5-methyl-1H-pyrazol-4-yl |
| 1965 | Pyridazin-4-yl | H | 3-(Methylsulfanyl)phenyl |
| 1966 | Pyridazin-4-yl | H | 3-(Acetylamino)-4-chlorphenyl |
| 1967 | Pyridazin-4-yl | H | 4-[(4-Brom-1H-pyrazol-1-yl)methyl]phenyl |
| 1968 | Pyridazin-4-yl | H | 4-Methyl-3-[(propan-2-ylcarbamoyl)amino]phenyl |
| 1969 | Pyridazin-4-yl | H | 1-(2,2-Diethoxyethyl)-3-(trifluormethyl)-1H-pyrazol-4-yl |
| 1970 | Pyridazin-4-yl | H | 6-[(4,6-Dimethoxypyrimidin-2-yl)oxy]pyridin-3-yl |
| 1971 | Pyridazin-4-yl | H | 6-(1H-Pyrazol-1-yl)pyridin-3-yl |
| 1972 | Pyridazin-4-yl | H | 4-(1H-1,2,4-Triazol-1-ylmethyl)phenyl |
| 1973 | Pyridazin-4-yl | H | 5-(Methoxymethyl)-1-[2-(trifluormethyl)phenyl]-1H-pyrazol-4-yl |
| 1974 | Pyridazin-4-yl | H | 5-Methylpyridin-3-yl |
| 1975 | Pyridazin-4-yl | H | 5-Chlor-6-[(4,6-dimethylpyrimidin-2-yl)sulfanyl]pyridin-3-yl |
| 1976 | Pyridazin-4-yl | H | 3-[(2,2-Dimethylpropanoyl)amino]-4-methoxyphenyl |
| 1977 | Pyridazin-4-yl | H | 2-[2-(Trifluormethyl)pyridin-3-yl]-1,3-thiazol-4-yl |
| 1978 | Pyridazin-4-yl | H | 3-Cyclopropyl-1-methyl-1H-pyrazol-4-yl |
| 1979 | Pyridazin-4-yl | H | 3-Methyl-1H-pyrazol-4-yl |
| 1980 | Pyridazin-4-yl | H | 5-(Methoxymethyl)-1-(2,2,2-trifluorethyl)-1H-pyrazol-4-yl |
| 1981 | Pyridazin-4-yl | H | 4-Methoxy-3-(propanoylamino)phenyl |
| 1982 | Pyridazin-4-yl | H | 4-Methoxy-3-[(2-methylpropanoyl)amino]phenyl |
| 1983 | Pyridazin-4-yl | H | 6-(Chinolin-7-yloxy)pyridin-3-yl |
| 1984 | Pyridazin-4-yl | H | 4-{[3-(Trifluormethyl)-1H-pyrazol-1-yl]methyl}phenyl |
| 1985 | Pyridazin-4-yl | H | 3-[(4,6-Dimethylpyrimidin-2-yl)oxy]phenyl |
| 1986 | Pyridazin-4-yl | H | 4-{[3,5-Bis(trifluormethyl)-1H-l,2,4-triazol-l-yl]methyl}phenyl |
| 1987 | Pyridazin-4-yl | H | 3-Fluor-4-{[4-(trifluormethyl)-1H-pyrazol-1-yl]methyl}phenyl |
| 1988 | Pyridazin-4-yl | H | 2,3-Dihydro-1,4-benzodioxin-6-yl |
| 1989 | Pyridazin-4-yl | H | 2-[3-(Methoxymethyl)pyridin-2-yl]-1,3-thiazol-5-yl |
| 1990 | Pyridazin-4-yl | H | 6-({6-[3-(Trifluormethyl)-1H-pyrazol-1-yl]pyridin-2-yl}oxy)pyridin-3-yl |
| 1991 | Pyridazin-4-yl | H | 3-{[3-(Pyridin-2-yl)-1H-pyrazol-1-yl]methyl}phenyl |
| 1992 | Pyridazin-4-yl | H | 1-(2-Brom-4-chlorphenyl)-5-methyl-1H-pyrazol-4-yl |
| 1993 | Pyridazin-4-yl | H | 2,2-Difluor-1,3-benzodioxol-5-yl |
| 1994 | Pyridazin-4-yl | H | 2-[3-(Trifluormethyl)pyridin-2-yl]-1,3-thiazol-4-yl |
| 1995 | Pyridazin-4-yl | H | 4-(Benzylcarbamoyl)phenyl |
| 1996 | Pyridazin-4-yl | H | 4-[(2-Chlorpyrimidin-4-yl)oxy]phenyl |
| 1997 | Pyridazin-4-yl | H | 1-[3-(Trifluormethyl)pyridin-2-yl]-1H-pyrazol-4-yl |
| 1998 | Pyridazin-4-yl | H | 5-Chlor-6-(4-chlorphenoxy)pyridin-3-yl |
| 1999 | Pyridazin-4-yl | H | 3-Cyan-4-(1H-1,2,4-triazol-1-yl)phenyl |
| 2000 | Pyridazin-4-yl | H | 3-Amino-4-(methylsulfonyl)phenyl |
| 2001 | Pyridazin-4-yl | H | 4-Methyl-3-[(2-methylpropanoyl)amino]phenyl |
| 2002 | Pyridazin-4-yl | H | 1-(2,6-Dibromphenyl)-5-(trifluormethyl)-1H-pyrazol-4-yl |
| 2003 | Pyridazin-4-yl | H | 3-{[3-(Trifluormethyl)-1H-1,2,4-triazol-1-yl]methyl}phenyl |
| 2004 | Pyridazin-4-yl | H | 4-[(3,3,3-Trifluorpropanoyl)amino]phenyl |
| 2005 | Pyridazin-4-yl | H | 5-(Pyrazin-2-yl)-1-(2,2,2-trifluorethyl)-1H-pyrazol-4-yl |
| 2006 | Pyridazin-4-yl | H | 1-(2,6-Dichlorphenyl)-5-(dimethoxymethyl)-1H-pyrazol-4-yl |
| 2007 | Pyridazin-4-yl | H | 1-(4-Chlorphenyl)-5-(1,1-dimethoxyethyl)-1H-pyrazol-4-yl |
| 2008 | Pyridazin-4-yl | H | 6-[3-(Trifluormethyl)phenoxy]pyridin-3-yl |
| 2009 | Pyridazin-4-yl | H | 4-({6-[3-(Trifluormethyl)-1H-pyrazol-1-yl]pyridin-2-yl}oxy)phenyl |
| 2010 | Pyridazin-4-yl | H | 4-Methoxy-3-(propan-2-ylcarbamoyl)phenyl |
| 2011 | Pyridazin-4-yl | H | 1-[2-(1-Methoxyethyl)phenyl]-5-methyl-1H-pyrazol-4-yl |
| 2012 | Pyridazin-4-yl | H | 1-Ethyl-3-methyl-1H-pyrazol-4-yl |
| 2013 | Pyridazin-4-yl | H | 4-[(4,6-Dimethoxypyrimidin-2-yl)sulfanyl]phenyl |
| 2014 | Pyridazin-4-yl | H | 6-(4-Methylphenoxy)pyridin-3-yl |
| 2015 | Pyridazin-4-yl | H | 6-(4-Chlor-2-cyanphenoxy)pyridin-3-yl |
| 2016 | Pyridazin-4-yl | H | 1-Propyl-3-(trifluormethyl)-1H-pyrazol-4-yl |
| 2017 | Pyridazin-4-yl | H | 6-(2-Methylphenoxy)pyridin-3-yl |
| 2018 | Pyridazin-4-yl | H | 4-{[4-(Trifluormethyl)-1H-pyrazol-1-yl]methyl}phenyl |
| 2019 | Pyridazin-4-yl | H | 6-[(6-Methylpyridin-3-yl)oxy]pyridin-3-yl |
| 2020 | Pyridazin-4-yl | H | 6-Methoxypyridin-3-yl |
| 2021 | Pyridazin-4-yl | H | 4-[(Ethoxyimino)methyl]phenyl |
| 2022 | Pyridazin-4-yl | H | 4-{[Acetyl(cyclopentyl)amino]methyl}phenyl |
| 2023 | Pyridazin-4-yl | H | 3-{[3,5-Bis(trifluormethyl)-1H-pyrazol-1-yl]methyl}phenyl |
| 2024 | Pyridazin-4-yl | H | 1-(2-Brom-4-chlorphenyl)-1H-pyrazol-4-yl |
| 2025 | Pyridazin-4-yl | H | 6-[4-(1H-1,2,4-Triazol-1-yl)phenoxy]pyridin-3-yl |
| 2026 | Pyridazin-4-yl | H | 6-[4-(Trifluormethyl)phenoxy]pyridin-3-yl |
| 2027 | Pyridazin-4-yl | H | 1-(2-Chlorphenyl)-5-(trifluormethyl)-1H-pyrazol-4-yl |
| 2028 | Pyridazin-4-yl | H | 4-Methoxy-3-nitrophenyl |
| 2029 | Pyridazin-4-yl | H | 1-(2,6-Dibromphenyl)-5-(difluormethyl)-1H-pyrazol-4-yl |
| 2030 | Pyridazin-4-yl | H | 6-[(4-Methoxyphenyl)sulfanyl]pyridin-3-yl |
| 2031 | Pyridazin-4-yl | H | 1-(2,6-Dichlorphenyl)-5-(1-methoxyethyl)-1H-pyrazol-4-yl |
| 2032 | Pyridazin-4-yl | H | 4-[(6-Chlorpyridin-2-yl)oxy]phenyl |
| 2033 | Pyridazin-4-yl | H | 3-Fluor-4-{[3-(trifluormethyl)-1H-pyrazol-1-yl]methyl}phenyl |
| 2034 | Pyridazin-4-yl | H | 4-{[(Difluormethyl)sulfanyl]methyl}phenyl |
| 2035 | Pyridazin-4-yl | H | 6-{[3-(Trifluormethyl)-1H-pyrazol-1-yl]methyl}pyridin-3-yl |
| 2036 | Pyridazin-4-yl | H | 4-{[2-(3,5-Dichlorphenyl)propan-2-yl]carbamoyl}phenyl |
| 2037 | Pyridazin-4-yl | H | 3-(1H-1,2,4-Triazol-1-ylmethyl)phenyl |
| 2038 | Pyridazin-4-yl | H | 2-(3-Chlorpyridin-2-yl)-1,3-thiazol-4-yl |
| 2039 | Pyridazin-4-yl | H | 5-(1-Methoxyethyl)-1-(pyrimidin-2-yl)-1H-pyrazol-4-yl |
| 2040 | Pyridazin-4-yl | H | 1-(3-Chlorpyridin-2-yl)-5-(1,1-dimethoxyethyl)-1H-pyrazol-4-yl |
| 2041 | Pyridazin-4-yl | H | 1-(2-Brom-4-fluorphenyl)-5-methyl-1H-pyrazol-4-yl |
| 2042 | Pyridazin-4-yl | H | 3-(Butanoylamino)-4-methylphenyl |
| 2043 | Pyridazin-4-yl | H | 5-(Methoxymethyl)-1-[3-(trifluormethyl)pyridin-2-yl]-1H-pyrazol-4-yl |
| 2044 | Pyridazin-4-yl | H | 6-[3-(Trifluormethyl)-1H-pyrazol-1-yl]pyridin-3-yl |
| 2045 | Pyridazin-4-yl | H | 6-(3-Chlorphenoxy)pyridin-3-yl |
| 2046 | Pyridazin-4-yl | H | 1-(2,6-Dichlorpyridin-3-yl)-5-(1-methoxyethyl)-1H-pyrazol-4-yl |
| 2047 | Pyridazin-4-yl | H | 6-[(4,6-Dimethylpyrimidin-2-yl)sulfanyl]pyridin-3-yl |
| 2048 | Pyridazin-4-yl | H | 1-[2-(Difluormethyl)phenyl]-5-methyl-1H-pyrazol-4-yl |
| 2049 | Pyridazin-4-yl | H | 2-[2-(Trifluormethyl)phenyl]-1,3-thiazol-5-yl |
| 2050 | Pyridazin-4-yl | H | 2-[2-(Trifluormethyl)pyridin-3-yl]-1,3-thiazol-5-yl |
| 2051 | Pyridazin-4-yl | H | 6-[(4,6-Dimethoxy-1,3,5-triazin-2-yl)sulfanyl]pyridin-3-yl |
| 2052 | Pyridazin-4-yl | H | 2-(2-Chlorpyridin-3-yl)-1,3-thiazol-5-yl |
| 2053 | Pyridazin-4-yl | H | 1-(2,6-Dichlorpyridin-3-yl)-5-methyl-1H-pyrazol-4-yl |
| 2054 | Pyridazin-4-yl | H | 1-(3-Chlorpyridin-2-yl)-5-(methoxymethyl)-1H-pyrazol-4-yl |
| 2055 | Pyridazin-4-yl | H | 6-[(4-Chlorphenyl)sulfanyl]pyridin-3-yl |
| 2056 | Pyridazin-4-yl | H | 6-(Piperidin-1-yl)pyridin-3-yl |
| 2057 | Pyridazin-4-yl | H | 3-[(Cyclopentylcarbonyl)amino]-4-methylphenyl |
| 2058 | Pyridazin-4-yl | H | 6-Ethoxypyridin-3-yl |
| 2059 | Pyridazin-4-yl | H | 2-(2,6-Dichlorphenyl)-1,3-thiazol-4-yl |
| 2060 | Pyridazin-4-yl | H | 5-Chlor-6-[(4-methylpyrimidin-2-yl)sulfanyl]pyridin-3-yl |
| 2061 | Pyridazin-4-yl | H | 1-[4-Chlor-2-(trifluormethyl)phenyl]-5-(1-methoxyethyl)-1H-pyrazol-4-yl |
| 2062 | Pyridazin-4-yl | H | 6-(3-Methylphenoxy)pyridin-3-yl |
| 2063 | Pyridazin-4-yl | H | 4-[Ethyl(methyl)carbamoyl]phenyl |
| 2064 | Pyridazin-4-yl | H | 6-[3-(Trifluormethoxy)phenoxy]pyridin-3-yl |
| 2065 | Pyridazin-4-yl | H | 5-(Difluormethyl)-1-(pyrimidin-2-yl)-1H-pyrazol-4-yl |
| 2066 | Pyridazin-4-yl | H | 4-[(Cyclopropylcarbonyl)amino]phenyl |
| 2067 | Pyridazin-4-yl | H | 1-(3-Brompyridin-2-yl)-5-(1-methoxyethyl)-1H-pyrazol-4-yl |
| 2068 | Pyridazin-4-yl | H | 3-[(Cyclopropylcarbonyl)amino]-4-methoxyphenyl |
| 2069 | Pyridazin-4-yl | H | 4-Methyl-3-[(3-methylbut-2-enoyl)amino]phenyl |
| 2070 | Pyridazin-4-yl | H | 1-[3-(Methoxymethyl)pyridin-2-yl]-1H-pyrazol-4-yl |
| 2071 | Pyridazin-4-yl | H | 4-[Acetyl(methyl)amino]phenyl |
| 2072 | Pyridazin-4-yl | H | 6-(Naphthalen-2-yloxy)pyridin-3-yl |
| 2073 | Pyridazin-4-yl | H | 1-(2-Chlorphenyl)-5-(1-methoxyethyl)-1H-pyrazol-4-yl |
| 2074 | Pyridazin-4-yl | H | 1-[2-(Difluormethyl)phenyl]-5-(1-methoxyethyl)-1H-pyrazol-4-yl |
| 2075 | Pyridazin-4-yl | H | 1-(3-Chlorpyridin-2-yl)-5-methyl-1H-pyrazol-4-yl |
| 2076 | Pyridazin-4-yl | H | 4-{[3-(Trifluormethyl)-1H-1,2,4-triazol-1-yl]methyl}phenyl |
| 2077 | Pyridazin-4-yl | H | 3-[(Cyclopentylcarbonyl)amino]-4-methoxyphenyl |
| 2078 | Pyridazin-4-yl | H | 1-(2,2,2-Trifluorethyl)-5-(trifluormethyl)-1H-pyrazol-4-yl |
| 2079 | Pyridazin-4-yl | H | 6-[4-(Methylsulfanyl)phenoxy]pyridin-3-yl |
| 2080 | Pyridazin-4-yl | H | 2-[3-(Trifluormethyl)pyridin-2-yl]-1,3-thiazol-5-yl |
| 2081 | Pyridazin-4-yl | H | 4-{[3-(Methoxycarbonyl)-1H-1,2,4-triazol-1-yl]methyl}phenyl |
| 2082 | Pyridazin-4-yl | H | 1-[3-(1,1-Difluorethyl)pyridin-2-yl]-1H-pyrazol-4-yl |
| 2083 | Pyridazin-4-yl | H | 6-{[3-(Pyridin-2-yl)-1H-pyrazol-1-yl]methyl}pyridin-3-yl |
| 2084 | Pyridazin-4-yl | H | 3-(Acetylamino)phenyl |
| 2085 | Pyridazin-4-yl | H | 1-(3-Chlorpyridin-2-yl)-5-(trifluormethyl)-1H-pyrazol-4-yl |
| 2086 | Pyridazin-4-yl | H | 2-{[3-(Pyridin-2-yl)-1H-pyrazol-1-yl]methyl}pyridin-4-yl |
| 2087 | Pyridazin-4-yl | H | 3-[(Methoxyacetyl)amino]-4-methylphenyl |
| 2088 | Pyridazin-4-yl | H | 1-(3-Brompyridin-2-yl)-5-(trifluormethyl)-1H-pyrazol-4-yl |
| 2089 | Pyridazin-4-yl | H | 4-[(Methoxyimino)methyl]phenyl |
| 2090 | Pyridazin-4-yl | H | 1-(Pyrimidin-2-yl)-5-(trifluormethyl)-1H-pyrazol-4-yl |
| 2091 | Pyridazin-4-yl | H | 2-(2-Chlorphenyl)-1,3-thiazol-4-yl |
| 2092 | Pyridazin-4-yl | H | 4-Methoxy-3-[(propan-2-ylsulfonyl)amino]phenyl |
| 2093 | Pyridazin-4-yl | H | 6-(3-Cyanphenoxy)pyridin-3-yl |
| 2094 | Pyridazin-4-yl | H | 4-Fluor-3-[(2-methylpropanoyl)amino]phenyl |
| 2095 | Pyridazin-4-yl | H | 1-(2-Bromphenyl)-5-(difluormethyl)-1H-pyrazol-4-yl |
| 2096 | Pyridazin-4-yl | H | 1,3-Dimethyl-1H-pyrazol-4-yl |
| 2097 | Pyridazin-4-yl | H | 5-(1-Methoxyethyl)-1-(2,2,2-trifluorethyl)-1H-pyrazol-4-yl |
| 2098 | Pyridazin-4-yl | H | 1-(2,6-Dichlorphenyl)-5-(1-fluorethyl)-1H-pyrazol-4-yl |
| 2099 | Pyridazin-4-yl | H | 6-(Pyrrolidin-1-yl)pyridin-3-yl |
| 2100 | Pyridazin-4-yl | H | 3-(Acetylamino)-5-(trifluormethyl)phenyl |
| 2101 | Pyridazin-4-yl | H | 2-[3-(Methoxymethyl)pyridin-2-yl]-1,3-thiazol-4-yl |
| 2102 | Pyridazin-4-yl | H | 2-[3-(1-Methoxyethyl)pyridin-2-yl]-1,3-thiazol-5-yl |
| 2103 | Pyridazin-4-yl | H | 1-[3-(Dimethoxymethyl)pyridin-2-yl]-1H-pyrazol-4-yl |
| 2104 | Pyridazin-4-yl | H | 5-(1-Methoxyethyl)-1-(2-methylphenyl)-1H-pyrazol-4-yl |
| 2105 | Pyridazin-4-yl | H | 1-Methyl-1H-benzotriazol-5-yl |
| 2106 | Pyridazin-4-yl | H | 4-[(Butylsulfanyl)methyl]phenyl |
| 2107 | Pyridazin-4-yl | H | 1H-Pyrazol-4-yl |
| 2108 | Pyridazin-4-yl | H | 1-(2-Brom-6-chlorphenyl)-5-(1-methoxyethyl)-1H-pyrazol-4-yl |
| 2109 | Pyridazin-4-yl | H | 5-Chlor-6-[4-(methylsulfonyl)phenoxy]pyridin-3-yl |
| 2110 | Pyridazin-4-yl | H | 1-[3-(1-Methoxyethyl)pyridin-2-yl]-1H-pyrazol-4-yl |
| 2111 | Pyridazin-4-yl | H | 6-[(4,6-Dimethoxypyrimidin-2-yl)sulfanyl]pyridin-3-yl |
| 2112 | Pyridazin-4-yl | H | 4-[(6-Methylpyridin-3-yl)oxy]phenyl |
| 2113 | Pyridazin-4-yl | H | 3-Methoxy-1-methyl-1H-pyrazol-4-yl |
| 2114 | Pyridazin-4-yl | H | 6-(4-tert-Butylphenoxy)pyridin-3-yl |
| 2115 | Pyridazin-4-yl | H | 1-(2,4-Dichlorphenyl)-5-methyl-1H-pyrazol-4-yl |
| 2116 | Pyridazin-4-yl | H | 3-(But-2-enoylamino)-4-methylphenyl |
| 2117 | Pyridazin-4-yl | H | 4-{[4-(Pyrimidin-2-yl)-1H-pyrazol-1-yl]methyl}phenyl |
| 2118 | Pyridazin-4-yl | H | 4-{[6-(1H-Pyrazol-1-yl)pyridin-2-yl]oxy}phenyl |
| 2119 | Pyridazin-4- yl | H | 4-{[3,5-Bis(trifluormethyl)-1H-1,2,4-triazol-1-yl]methyl}-3-chlorphenyl |
| 2120 | Pyridazin-4-yl | H | 4-[(2-Methoxypyrimidin-4-yl)oxy]phenyl |
| 2121 | Pyridazin-4-yl | H | 1-[4-Chlor-2-(trifluormethyl)phenyl]-5-(methoxymethyl)-1H-pyrazol-4-yl |
| 2122 | Pyridazin-4-yl | H | 5-(Trifluormethyl)-1-[2-(trifluormethyl)phenyl]-1H-pyrazol-4-yl |
| 2123 | Pyridazin-4-yl | H | 6-(Dimethylamino)pyridin-3-yl |
| 2124 | Pyridazin-4-yl | H | 2-[5-Chlor-3-(trifluormethyl)pyridin-2-yl]-1,3-thiazol-5-yl |
| 2125 | Pyridazin-4-yl | H | 4-{[4-(3-Chlorpyridin-2-yl)-1H-pyrazol-l-yl]methyl}phenyl |
| 2126 | Pyridazin-4-yl | H | 4-[(2,2,3,3,4,4,4-Heptafluorbutanoyl)(methyl)amino]phenyl |
| 2127 | Pyridazin-4-yl | H | 4-[(Trifluormethyl)sulfanyl]phenyl |
| 2128 | Pyridazin-4-yl | H | 6-[4-(Methylsulfonyl)phenoxy]pyridin-3-yl |
| 2129 | Pyridazin-4-yl | H | 1-(2-Bromphenyl)-5-(methoxymethyl)-1H-pyrazol-4-yl |
| 2130 | Pyridazin-4-yl | H | 6-[(4-Brom-1H-pyrazol-1-yl)methyl]pyridin-3-yl |
| 2131 | Pyridazin-4-yl | H | 3-Chlor-4-{[3-(trifluormethyl)-1H-pyrazol-1-yl]methyl}phenyl |
| 2132 | Pyridazin-4-yl | H | 6-{[4-(Trifluormethyl)-1H-pyrazol-1-yl]methyl}pyridin-3-yl |
| 2133 | Pyridazin-4-yl | H | 1-(3-Brompyridin-2-yl)-5-(difluormethyl)-1H-pyrazol-4-yl |
| 2134 | Pyridazin-4-yl | CH₃ | 1-(2,6-Dichlorpyridin-3-yl)-5-(trifluormethyl)-1H-pyrazol-4-yl |
| 2135 | Pyridazin-4-yl | CH₃ | 5-(1-Methoxyethyl)-1-[3-(trifluormethyl)pyridin-2-yl]-1H-pyrazol-4-yl |
| 2136 | Pyridazin-4-yl | CH₃ | 1-Phenyl-1H-pyrazol-4-yl |
| 2137 | Pyridazin-4-yl | CH₃ | 6-Chlorpyridin-3-yl |
| 2138 | Pyridazin-4-yl | CH₃ | 2-[5-Chlor-3-(trifluormethyl)pyridin-2-yl]-1,3-thiazol-4-yl |
| 2139 | Pyridazin-4-yl | CH₃ | 3-(Trifluormethyl)-1H-pyrazol-4-yl |
| 2140 | Pyridazin-4-yl | CH₃ | 1-[3-(Difluormethyl)pyridin-2-yl]-1H-pyrazol-4-yl |
| 2141 | Pyridazin-4-yl | CH₃ | 5-(Trifluormethyl)-1-[3-(trifluormethyl)pyridin-2-yl]-1H-pyrazol-4-yl |
| 2142 | Pyridazin-4-yl | CH₃ | 1-(2-Bromphenyl)-5-(1-methoxyethyl)-1H-pyrazol-4-yl |
| 2143 | Pyridazin-4-yl | CH₃ | 1-(1,3-Thiazol-2-ylmethyl)-3-(trifluormethyl)-1H-pyrazol-4-yl |
| 2144 | Pyridazin-4-yl | CH₃ | 3-(Trifluormethyl)phenyl |
| 2145 | Pyridazin-4-yl | CH₃ | 2-[3-(1-Methoxyethyl)pyridin-2-yl]-1,3-thiazol-4-yl |
| 2146 | Pyridazin-4-yl | CH₃ | 5-(1-Fluorethyl)-1-(pyrimidin-2-yl)-1H-pyrazol-4-yl |
| 2147 | Pyridazin-4-yl | CH₃ | 6-(4-Cyanphenoxy)pyridin-3-yl |
| 2148 | Pyridazin-4-yl | CH₃ | 1-[2-(Trifluormethyl)phenyl]-1H-pyrazol-4-yl |
| 2149 | Pyridazin-4-yl | CH₃ | 1-[3-Chlor-2-(trifluormethyl)phenyl]-5-(methoxymethyl)-1H-pyrazol-4-yl |
| 2150 | Pyridazin-4-yl | CH₃ | 4-[(2-Cyanethyl)(methyl)carbamoyl]phenyl |
| 2151 | Pyridazin-4-yl | CH₃ | 4-[(Trifluoracetyl)amino]phenyl |
| 2152 | Pyridazin-4-yl | CH₃ | 1-Benzyl-3-(trifluormethyl)-1H-pyrazol-4-yl |
| 2153 | Pyridazin-4-yl | CH₃ | 6-(2,5-Dichlorphenoxy)pyridin-3-yl |
| 2154 | Pyridazin-4-yl | CH₃ | 2-(Pyrimidin-2-yl)-1,3-thiazol-5-yl |
| 2155 | Pyridazin-4-yl | CH₃ | 3-(But-2-enoylamino)-4-methoxyphenyl |
| 2156 | Pyridazin-4-yl | CH₃ | 4-(Dimethylcarbamoyl)phenyl |
| 2157 | Pyridazin-4-yl | CH₃ | 4-Chlor-3-[(cyclopropylcarbonyl)amino]phenyl |
| 2158 | Pyridazin-4-yl | CH₃ | 1-(2,6-Dichlorphenyl)-5-methyl-1H-pyrazol-4-yl |
| 2159 | Pyridazin-4-yl | CH₃ | 1-(2-Chlorpyridin-3-yl)-5-(difluormethyl)-1H-pyrazol-4-yl |
| 2160 | Pyridazin-4-yl | CH₃ | 1-(3-Chlorpyridin-2-yl)-5-(dimethoxymethyl)-1H-pyrazol-4-yl |
| 2161 | Pyridazin-4-yl | CH₃ | 5-(Difluormethyl)-1-(2,2,2-trifluorethyl)-1H-pyrazol-4-yl |
| 2162 | Pyridazin-4-yl | CH₃ | 4-[(Propan-2-ylsulfanyl)methyl]phenyl |
| 2163 | Pyridazin-4-yl | CH₃ | 1-(2-Brom-6-chlorphenyl)-5-(dimethoxymethyl)-1H-pyrazol-4-yl |
| 2164 | Pyridazin-4-yl | CH₃ | 4-Methoxy-3-[3-(pyridin-2-yl)-1H-pyrazol-1-yl]phenyl |
| 2165 | Pyridazin-4-yl | CH₃ | 3-Chlor-4-{[4-(trifluormethyl)-1H-pyrazol-1-yl]methyl}phenyl |
| 2166 | Pyridazin-4-yl | CH₃ | 3-[(4,6-Dimethoxypyrimidin-2-yl)methoxy]phenyl |
| 2167 | Pyridazin-4-yl | CH₃ | 4-Methyl-3-[(2-methylbutanoyl)amino]phenyl |
| 2168 | Pyridazin-4-yl | CH₃ | 4-{[Chlor(difluor)acetyl](methyl)amino}phenyl |
| 2169 | Pyridazin-4-yl | CH₃ | 4-{[3-(3-Chlorpyridin-2-yl)-1H-pyrazol-1-yl]methyl}phenyl |
| 2170 | Pyridazin-4-yl | CH₃ | 6-{[4-(Pyridin-2-yl)-1H-pyrazol-1-yl]methyl}pyridin-3-yl |
| 2171 | Pyridazin-4-yl | CH₃ | 4-Methoxy-3-[3-(pyrimidin-2-yl)-1H-pyrazol-1-yl]phenyl |
| 2172 | Pyridazin-4-yl | CH₃ | 6-{[4-(Trifluormethyl)phenyl]sulfanyl}pyridin-3-yl |
| 2173 | Pyridazin-4-yl | CH₃ | 4-{[3-(Pyridin-2-yl)-1H-pyrazol-1-yl]methyl}phenyl |
| 2174 | Pyridazin-4-yl | CH₃ | 4-Chlor-3-[(2,2-dimethylpropanoyl)amino]phenyl |
| 2175 | Pyridazin-4-yl | CH₃ | 1-(2-Chlorpyridin-3-yl)-5-(1-methoxyethyl)-1H-pyrazol-4-yl |
| 2176 | Pyridazin-4-yl | CH₃ | 2-(3,5-Dichlorpyridin-2-yl)-1,3-thiazol-4-yl |
| 2177 | Pyridazin-4-yl | CH₃ | 6-(Propylamino)pyridin-3-yl |
| 2178 | Pyridazin-4-yl | CH₃ | 5-Chlor-6-(4-cyanphenoxy)pyridin-3-yl |
| 2179 | Pyridazin-4-yl | CH₃ | 6-(3,4-Dichlorphenoxy)pyridin-3-yl |
| 2180 | Pyridazin-4-yl | CH₃ | 6-[(2-Chlorpyrimidin-4-yl)oxy]pyridin-3-yl |
| 2181 | Pyridazin-4-yl | CH₃ | 1-(3-Chlorpyridin-2-yl)-5-(1-methoxyethyl)-1H-pyrazol-4-yl |
| 2182 | Pyridazin-4-yl | CH₃ | 2-[2-(Trifluormethyl)phenyl]-1,3-thiazol-4-yl |
| 2183 | Pyridazin-4-yl | CH₃ | 3-(Propan-2-ylcarbamoyl)phenyl |
| 2184 | Pyridazin-4-yl | CH₃ | 4-(Heptafluorpropyl)phenyl |
| 2185 | Pyridazin-4-yl | CH₃ | 2-(2,6-Dichlorpyridin-3-yl)-1,3-thiazol-4-yl |
| 2186 | Pyridazin-4-yl | CH₃ | 1-Ethyl-5-(trifluormethyl)-1H-pyrazol-4-yl |
| 2187 | Pyridazin-4-yl | CH₃ | 1-[3-(Difluormethyl)pyridin-2-yl]-5-methyl-1H-pyrazol-4-yl |
| 2188 | Pyridazin-4-yl | CH₃ | 1-(2,4-Dichlorphenyl)-1H-pyrazol-4-yl |
| 2189 | Pyridazin-4-yl | CH₃ | 3-Fluor-4-(methylsulfanyl)phenyl |
| 2190 | Pyridazin-4-yl | CH₃ | 2-[4-Chlor-2-(trifluormethyl)phenyl]-1,3-thiazol-5-yl |
| 2191 | Pyridazin-4-yl | CH₃ | 4-[(2-Methylpyrimidin-4-yl)oxy]phenyl |
| 2192 | Pyridazin-4-yl | CH₃ | 6-{[5-(Trifluormethyl)pyridin-2-yl]sulfanyl}pyridin-3-yl |
| 2193 | Pyridazin-4-yl | CH₃ | 4-{[(Propan-2-yloxy)imino]methyl}phenyl |
| 2194 | Pyridazin-4-yl | CH₃ | 4-(Diethylcarbamoyl)phenyl |
| 2195 | Pyridazin-4-yl | CH₃ | 6-[(4-Nitrophenyl)sulfanyl]pyridin-3-yl |
| 2196 | Pyridazin-4-yl | CH₃ | 4-Methyl-3-{[(propan-2-yloxy)carbonyl]amino}phenyl |
| 2197 | Pyridazin-4-yl | CH₃ | 1-[4-Fluor-2-(trifluormethyl)phenyl]-5-(methoxymethyl)-1H-pyrazol-4-yl |
| 2198 | Pyridazin-4-yl | CH₃ | 5-(1,1-Difluorethyl)-1-(pyrimidin-2-yl)-1H-pyrazol-4-yl |
| 2199 | Pyridazin-4-yl | CH₃ | 3-{[3,5-Bis(trifluormethyl)-1H-1,2,4-triazol-1-yl]methyl}phenyl |
| 2200 | Pyridazin-4-yl | CH₃ | 4-{[3,5-Bis(trifluormethyl)-1H-pyrazol-1-yl]methyl}phenyl |
| 2201 | Pyridazin-4-yl | CH₃ | 1-(2-Chlorphenyl)-5-(1,1-dimethoxyethyl)-1H-pyrazol-4-yl |
| 2202 | Pyridazin-4-yl | CH₃ | 1-(3-Chlorpyridin-2-yl)-5-(1,1-difluorethyl)-1H-pyrazol-4-yl |
| 2203 | Pyridazin-4-yl | CH₃ | 1-(2,6-Dichlorpyridin-3-yl)-5-(methoxymethyl)-1H-pyrazol-4-yl |
| 2204 | Pyridazin-4-yl | CH₃ | 6-(Chinolin-6-yloxy)pyridin-3-yl |
| 2205 | Pyridazin-4-yl | CH₃ | 3-(4-Chlor-1H-pyrazol-1-yl)-4-methoxyphenyl |
| 2206 | Pyridazin-4-yl | CH₃ | 2-(2,4-Dichlorphenyl)-1,3-thiazol-4-yl |
| 2207 | Pyridazin-4-yl | CH₃ | 1-(2-Chlorpyridin-3-yl)-5-(1,1-difluorethyl)-1H-pyrazol-4-yl |
| 2208 | Pyridazin-4-yl | CH₃ | 6-{4-[4-(Methylsulfanyl)phenoxy]phenoxy}pyridin-3-yl |
| 2209 | Pyridazin-4-yl | CH₃ | 6-(Chinolin-5-yloxy)pyridin-3-yl |
| 2210 | Pyridazin-4-yl | CH₃ | 1-[4-Chlor-2-(trifluormethyl)phenyl]-5-(difluormethyl)-1H-pyrazol-4-yl |
| 2211 | Pyridazin-4-yl | CH₃ | 1-[2-(1-Methoxyethyl)phenyl]-1H-pyrazol-4-yl |
| 2212 | Pyridazin-4-yl | CH₃ | 4-Methoxy-3-[(2-methylbutanoyl)amino]phenyl |
| 2213 | Pyridazin-4-yl | CH₃ | 6-(2,4-Dichlorphenoxy)pyridin-3-yl |
| 2214 | Pyridazin-4-yl | CH₃ | 4-{[Acetyl(2-methylpropyl)amino]methyl}phenyl |
| 2215 | Pyridazin-4-yl | CH₃ | 6-[(Methylsulfanyl)methyl]pyridin-3-yl |
| 2216 | Pyridazin-4-yl | CH₃ | 2-(2,4-Dichlorphenyl)-1,3-thiazol-5-yl |
| 2217 | Pyridazin-4-yl | CH₃ | 6-[(4,5,6-Trimethylpyrimidin-2-yl)sulfanyl]pyridin-3-yl |
| 2218 | Pyridazin-4-yl | CH₃ | 4-[(4,6-Dimethylpyrimidin-2-yl)sulfanyl]phenyl |
| 2219 | Pyridazin-4-yl | CH₃ | 6-[2-Chlor-4-(methylsulfanyl)phenoxy]pyridin-3-yl |
| 2220 | Pyridazin-4-yl | CH₃ | 1-(2-Brom-6-chlorphenyl)-5-(difluormethyl)-1H-pyrazol-4-yl |
| 2221 | Pyridazin-4-yl | CH₃ | 4-(Propan-2-yloxy)-3-[3-(trifluormethyl)-1H-pyrazol-1-yl]phenyl |
| 2222 | Pyridazin-4-yl | CH₃ | 3-[Ethyl(methyl)carbamoyl]phenyl |
| 2223 | Pyridazin-4-yl | CH₃ | 6-(3,5-Dimethylphenoxy)pyridin-3-yl |
| 2224 | Pyridazin-4-yl | CH₃ | 1-[4-Chlor-2-(trifluormethyl)phenyl]-5-methyl-1H-pyrazol-4-yl |
| 2225 | Pyridazin-4-yl | CH₃ | 4-Fluor-3-(trifluormethyl)phenyl |
| 2226 | Pyridazin-4-yl | CH₃ | 6-[4-(Methoxycarbonyl)phenoxy]pyridin-3-yl |
| 2227 | Pyridazin-4-yl | CH₃ | 3-[(Cyclopropylcarbonyl)amino]-4-methylphenyl |
| 2228 | Pyridazin-4-yl | CH₃ | 1-(4-Chlorphenyl)-1H-pyrazol-4-yl |
| 2229 | Pyridazin-4-yl | CH₃ | 2-[2-(Difluormethyl)phenyl]-1,3-thiazol-5-yl |
| 2230 | Pyridazin-4-yl | CH₃ | 1-Ethyl-3-(trifluormethyl)-1H-pyrazol-4-yl |
| 2231 | Pyridazin-4-yl | CH₃ | 5-Chlor-6-[4-(trifluormethyl)phenoxy]pyridin-3-yl |
| 2232 | Pyridazin-4-yl | CH₃ | 4-Methyl-3-(propanoylamino)phenyl |
| 2233 | Pyridazin-4-yl | CH₃ | 2-(2-Chlorpyridin-3-yl)-1,3-thiazol-4-yl |
| 2234 | Pyridazin-4-yl | CH₃ | 4-[(2-Methylpropanoyl)amino]phenyl |
| 2235 | Pyridazin-4-yl | CH₃ | 3-[(2,2-Dimethylpropanoyl)amino]-4-methylphenyl |
| 2236 | Pyridazin-4-yl | CH₃ | 4-Methoxy-3-{[(propan-2-yloxy)carbonyl]amino}phenyl |
| 2237 | Pyridazin-4-yl | CH₃ | 1-(2,3-Dichlorphenyl)-5-(methoxymethyl)-1H-pyrazol-4-yl |
| 2238 | Pyridazin-4-yl | CH₃ | 3-(Hydroxymethyl)-1-methyl-1H-pyrazol-4-yl |
| 2239 | Pyridazin-4-yl | CH₃ | 1-(Propan-2-yl)-3-(trifluormethyl)-1H-pyrazol-4-yl |
| 2240 | Pyridazin-4-yl | CH₃ | 2-[3-(Difluormethyl)pyridin-2-yl]-1,3-thiazol-5-yl |
| 2241 | Pyridazin-4-yl | CH₃ | 4-(Pyrrolidin-1-ylcarbonyl)phenyl |
| 2242 | Pyridazin-4-yl | CH₃ | 3-(Methoxymethyl)-1-methyl-1H-pyrazol-4-yl |
| 2243 | Pyridazin-4-yl | CH₃ | 2-(3,5-Dichlorpyridin-2-yl)-1,3-thiazol-5-yl |
| 2244 | Pyridazin-4-yl | CH₃ | 1-(2,6-Dichlorpyridin-3-yl)-5-(difluormethyl)-1H-pyrazol-4-yl |
| 2245 | Pyridazin-4-yl | CH₃ | 6-[(4,6-Dimethylpyrimidin-2-yl)oxy]pyridin-3-yl |
| 2246 | Pyridazin-4-yl | CH₃ | 4-[(4,6-Dimethoxy-1,3,5-triazin-2-yl)sulfanyl]phenyl |
| 2247 | Pyridazin-4-yl | CH₃ | 4-(Methylsulfanyl)phenyl |
| 2248 | Pyridazin-4-yl | CH₃ | 4-(Ethylsulfanyl)phenyl |
| 2249 | Pyridazin-4-yl | CH₃ | 6-[4-(Trifluormethoxy)phenoxy]pyridin-3-yl |
| 2250 | Pyridazin-4-yl | CH₃ | 5-Chlor-6-(4-nitrophenoxy)pyridin-3-yl |
| 2251 | Pyridazin-4-yl | CH₃ | 2-[2-(Difluormethyl)phenyl]-1,3-thiazol-4-yl |
| 2252 | Pyridazin-4-yl | CH₃ | 1-(Difluormethyl)-3-(trifluormethyl)-1H-pyrazol-4-yl |
| 2253 | Pyridazin-4-yl | CH₃ | 6-[(6-Chlorpyridin-2-yl)oxy]pyridin-3-yl |
| 2254 | Pyridazin-4-yl | CH₃ | 3-(Butanoylamino)-4-methoxyphenyl |
| 2255 | Pyridazin-4-yl | CH₃ | 4-(Acetylamino)phenyl |
| 2256 | Pyridazin-4-yl | CH₃ | 1-(2-Bromphenyl)-5-(trifluormethyl)-1H-pyrazol-4-yl |
| 2257 | Pyridazin-4-yl | CH₃ | 6-(4-Chlorphenoxy)pyridin-3-yl |
| 2258 | Pyridazin-4-yl | CH₃ | 3-{[3-(Trifluormethyl)-1H-pyrazol-1-yl]methyl}phenyl |
| 2259 | Pyridazin-4-yl | CH₃ | 5-(Difluormethyl)-1-(2-methylphenyl)-1H-pyrazol-4-yl |
| 2260 | Pyridazin-4-yl | CH₃ | 2-[2-(1-Methoxyethyl)phenyl]-1,3-thiazol-5-yl |
| 2261 | Pyridazin-4-yl | CH₃ | 1-(2-Chlorphenyl)-5-(1,1-difluorethyl)-1H-pyrazol-4-yl |
| 2262 | Pyridazin-4-yl | CH₃ | 1-(2-Brom-4-fluorphenyl)-1H-pyrazol-4-yl |
| 2263 | Pyridazin-4-yl | CH₃ | 6-[(Cyclopropylmethyl)amino]pyridin-3-yl |
| 2264 | Pyridazin-4-yl | CH₃ | 6-[(2-Methoxypyrimidin-4-yl)oxy]pyridin-3-yl |
| 2265 | Pyridazin-4-yl | CH₃ | 4-(Methoxycarbonyl)phenyl |
| 2266 | Pyridazin-4-yl | CH₃ | 1-[2-(1,1-Difluorethyl)phenyl]-5-methyl-1H-pyrazol-4-yl |
| 2267 | Pyridazin-4-yl | CH₃ | 3-{[3,5-Bis(trifluormethyl)-1H-1,2,4-triazol-1-yl]methyl}-4-chlorphenyl |
| 2268 | Pyridazin-4-yl | CH₃ | 6-(Morpholin-4-yl)pyridin-3-yl |
| 2269 | Pyridazin-4-yl | CH₃ | 2-(Pyrimidin-2-yl)-1,3-thiazol-4-yl |
| 2270 | Pyridazin-4-yl | CH₃ | 4-[(4,6-Dimethoxypyrimidin-2-yl)oxy]phenyl |
| 2271 | Pyridazin-4-yl | CH₃ | 2-{[3-(Trifluormethyl)-1H-pyrazol-1-yl]methyl}pyridin-4-yl |
| 2272 | Pyridazin-4-yl | CH₃ | 1-(2-Chlorpyridin-3-yl)-5-(trifluormethyl)-1H-pyrazol-4-yl |
| 2273 | Pyridazin-4-yl | CH₃ | 6-(4-Bromphenoxy)pyridin-3-yl |
| 2274 | Pyridazin-4-yl | CH₃ | 4-[(Propylsulfanyl)methyl]phenyl |
| 2275 | Pyridazin-4-yl | CH₃ | 6-(Pyrimidin-2-ylsulfanyl)pyridin-3-yl |
| 2276 | Pyridazin-4-yl | CH₃ | 1-(2-Chlor-4-fluorphenyl)-1H-pyrazol-4-yl |
| 2277 | Pyridazin-4-yl | CH₃ | 4-Methoxy-3-[4-(pyridin-2-yl)-1H-pyrazol-1-yl]phenyl |
| 2278 | Pyridazin-4-yl | CH₃ | 1-(2-Chlorphenyl)-1H-pyrazol-4-yl |
| 2279 | Pyridazin-4-yl | CH₃ | 6-(2,6-Dichlorphenoxy)pyridin-3-yl |
| 2280 | Pyridazin-4-yl | CH₃ | 3-[(4,6-Dimethoxypyrimidin-2-yl)oxy]phenyl |
| 2281 | Pyridazin-4-yl | CH₃ | 6-(4-Nitrophenoxy)pyridin-3-yl |
| 2282 | Pyridazin-4-yl | CH₃ | 1-(2-Chlorphenyl)-5-methyl-1H-pyrazol-4-yl |
| 2283 | Pyridazin-4-yl | CH₃ | 3-[Ethyl(methyl)carbamoyl]-4-methoxyphenyl |
| 2284 | Pyridazin-4-yl | CH₃ | 4-[(Ethylsulfanyl)methyl]phenyl |
| 2285 | Pyridazin-4-yl | CH₃ | 2-[2-(1-Methoxyethyl)phenyl]-1,3-thiazol-4-yl |
| 2286 | Pyridazin-4-yl | CH₃ | 6-{[6-(1H-Pyrazol-1-yl)pyridin-2-yl]oxy}pyridin-3-yl |
| 2287 | Pyridazin-4-yl | CH₃ | 2-[4-Chlor-2-(trifluormethyl)phenyl]-1,3-thiazol-4-yl |
| 2288 | Pyridazin-4-yl | CH₃ | 4-Methoxy-3-(1H-pyrazol-1-yl)phenyl |
| 2289 | Pyridazin-4-yl | CH₃ | 3-(Acetylamino)-4-methylphenyl |
| 2290 | Pyridazin-4-yl | CH₃ | 2-{[4-(Trifluormethyl)-1H-pyrazol-1-yl]methyl}pyridin-4-yl |
| 2291 | Pyridazin-4-yl | CH₃ | 1-(2,4-Difluorphenyl)-1H-pyrazol-4-yl |
| 2292 | Pyridazin-4-yl | CH₃ | 1-(2-Methylphenyl)-5-(trifluormethyl)-1H-pyrazol-4-yl |
| 2293 | Pyridazin-4-yl | CH₃ | 2-[3-(Difluormethyl)pyridin-2-yl]-1,3-thiazol-4-yl |
| 2294 | Pyridazin-4-yl | CH₃ | 6-(3,5-Dichlorphenoxy)pyridin-3-yl |
| 2295 | Pyridazin-4-yl | CH₃ | 1-(Pyrimidin-2-ylmethyl)-3-(trifluormethyl)-1H-pyrazol-4-yl |
| 2296 | Pyridazin-4-yl | CH₃ | 6-Methylpyridin-3-yl |
| 2297 | Pyridazin-4-yl | CH₃ | 6-[(4-Methylphenyl)sulfanyl]pyridin-3-yl |
| 2298 | Pyridazin-4-yl | CH₃ | 1-(2,4-Dichlorphenyl)-5-(methoxymethyl)-1H-pyrazol-4-yl |
| 2299 | Pyridazin-4-yl | CH₃ | 6-{4-[4-(Trifluormethyl)phenoxy]phenoxy}pyridin-3-yl |
| 2300 | Pyridazin-4-yl | CH₃ | 1-(2,6-Dichlorphenyl)-5-(trifluormethyl)-1H-pyrazol-4-yl |
| 2301 | Pyridazin-4-yl | CH₃ | 4-[(4,6-Dimethylpyrimidin-2-yl)oxy]phenyl |
| 2302 | Pyridazin-4-yl | CH₃ | 6-(Naphthalen-1-yloxy)pyridin-3-yl |
| 2303 | Pyridazin-4-yl | CH₃ | 2-(2,6-Dichlorpyridin-3-yl)-1,3-thiazol-5-yl |
| 2304 | Pyridazin-4-yl | CH₃ | 6-{[(4,6-Dimethoxypyrimidin-2-yl)methyl]sulfanyl}pyridin-3-yl |
| 2305 | Pyridazin-4-yl | CH₃ | 1-[3-Chlor-2-(trifluormethyl)phenyl]-5-(1-methoxyethyl)-1H-pyrazol-4-yl |
| 2306 | Pyridazin-4-yl | CH₃ | 3,4-Dimethoxyphenyl |
| 2307 | Pyridazin-4-yl | CH₃ | 4-Methoxy-3-[(methoxyacetyl)amino]phenyl |
| 2308 | Pyridazin-4-yl | CH₃ | 3-{[4-(Trifluormethyl)-1H-pyrazol-1-yl]methyl}phenyl |
| 2309 | Pyridazin-4-yl | CH₃ | 6-(2-Cyanphenoxy)pyridin-3-yl |
| 2310 | Pyridazin-4-yl | CH₃ | 3-[(Methylsulfanyl)methyl]phenyl |
| 2311 | Pyridazin-4-yl | CH₃ | 3-(Dimethoxymethyl)-1-methyl-1H-pyrazol-4-yl |
| 2312 | Pyridazin-4-yl | CH₃ | 1-(Ethoxymethyl)-3-(trifluormethyl)-1H-pyrazol-4-yl |
| 2313 | Pyridazin-4-yl | CH₃ | 1-(3-Brompyridin-2-yl)-5-(methoxymethyl)-1H-pyrazol-4-yl |
| 2314 | Pyridazin-4-yl | CH₃ | 6-(Methylsulfanyl)pyridin-3-yl |
| 2315 | Pyridazin-4-yl | CH₃ | 1-(2-Brom-6-chlorphenyl)-5-(trifluormethyl)-1H-pyrazol-4-yl |
| 2316 | Pyridazin-4-yl | CH₃ | 3-{[4-(Trifluormethyl)phenoxy]methyl}phenyl |
| 2317 | Pyridazin-4-yl | CH₃ | 6-(Heptafluorpropyl)pyridin-3-yl |
| 2318 | Pyridazin-4-yl | CH₃ | 1-Ethenyl-3-(trifluormethyl)-1H-pyrazol-4-yl |
| 2319 | Pyridazin-4-yl | CH₃ | 4-Methyl-3-nitrophenyl |
| 2320 | Pyridazin-4-yl | CH₃ | 3-(4-Brom-1H-pyrazol-1-yl)-4-methoxyphenyl |
| 2321 | Pyridazin-4-yl | CH₃ | 5-Methyl-1-(2-methylphenyl)-1H-pyrazol-4-yl |
| 2322 | Pyridazin-4-yl | CH₃ | 1-[3-(Difluormethyl)pyridin-2-yl]-5-(methoxymethyl)-1H-pyrazol-4-yl |
| 2323 | Pyridazin-4-yl | CH₃ | 6-{ [2-(Trifluormethyl)pyrimidin-5-yl]oxy }pyridin-3-yl |
| 2324 | Pyridazin-4-yl | CH₃ | 2-[2-(Methoxymethyl)phenyl]-1,3-thiazol-5-yl |
| 2325 | Pyridazin-4-yl | CH₃ | 1-(Ethoxymethyl)-5-(trifluormethyl)-1H-pyrazol-4-yl |
| 2326 | Pyridazin-4-yl | CH₃ | 5-Chlor-6-[(4,5,6-trimethylpyrimidin-2-yl)sulfanyl]pyridin-3-yl |
| 2327 | Pyridazin-4-yl | CH₃ | 6-[3,5-Bis(trifluormethyl)phenoxy]pyridin-3-yl |
| 2328 | Pyridazin-4-yl | CH₃ | 4-{[4-(Pyridin-2-yl)-1H-pyrazol-1-yl]methyl}phenyl |
| 2329 | Pyridazin-4-yl | CH₃ | 1-[3-(1,1-Difluorethyl)pyridin-2-yl]-5-methyl-1H-pyrazol-4-yl |
| 2330 | Pyridazin-4-yl | CH₃ | 1-(2,6-Dichlorphenyl)-5-(difluormethyl)-1H-pyrazol-4-yl |
| 2331 | Pyridazin-4-yl | CH₃ | 1-[4-Fluor-2-(trifluormethyl)phenyl]-5-methyl-1H-pyrazol-4-yl |
| 2332 | Pyridazin-4-yl | CH₃ | 4-Chlor-3-[(cyclobutylcarbonyl)amino]phenyl |
| 2333 | Pyridazin-4-yl | CH₃ | 2-[2-(Methoxymethyl)phenyl]-1,3-thiazol-4-yl |
| 2334 | Pyridazin-4-yl | CH₃ | 4-(1H-Pyrazol-1-ylmethyl)phenyl |
| 2335 | Pyridazin-4-yl | CH₃ | 1-(2-Bromphenyl)-5-methyl-1H-pyrazol-4-yl |
| 2336 | Pyridazin-4-yl | CH₃ | 1-(2,3-Difluorphenyl)-1H-pyrazol-4-yl |
| 2337 | Pyridazin-4-yl | CH₃ | 6-{4-[(Trifluormethyl)sulfanyl]phenoxy}pyridin-3-yl |
| 2338 | Pyridazin-4-yl | CH₃ | 4-{[3-(Pentafluorethyl)-1H-1,2,4-triazol-1-yl]methyl}phenyl |
| 2339 | Pyridazin-4-yl | CH₃ | 4-{[3-(Pyrimidin-2-yl)-1H-pyrazol-1-yl]methyl}phenyl |
| 2340 | Pyridazin-4-yl | CH₃ | 1-(2-Chlorphenyl)-5-(difluormethyl)-1H-pyrazol-4-yl |
| 2341 | Pyridazin-4-yl | CH₃ | 5-(Difluormethyl)-1-[2-(trifluormethyl)phenyl]-1H-pyrazol-4-yl |
| 2342 | Pyridazin-4-yl | CH₃ | 6-(4-Methoxyphenoxy)pyridin-3-yl |
| 2343 | Pyridazin-4-yl | CH₃ | 1-[2-(Dimethoxymethyl)phenyl]-1H-pyrazol-4-yl |
| 2344 | Pyridazin-4-yl | CH₃ | 5-(Methoxymethyl)-1-(pyrimidin-2-yl)-1H-pyrazol-4-yl |
| 2345 | Pyridazin-4-yl | CH₃ | 6-tert-Butoxypyridin-3-yl |
| 2346 | Pyridazin-4-yl | CH₃ | 4-[(Cyclopentylimino)methyl]phenyl |
| 2347 | Pyridazin-4-yl | CH₃ | 1-(3-Chlorphenyl)-1H-pyrazol-4-yl |
| 2348 | Pyridazin-4-yl | CH₃ | 3-[(2-Methylpropanoyl)amino]phenyl |
| 2349 | Pyridazin-4-yl | CH₃ | 6-{[3,5-Bis(trifluormethyl)-1H-pyrazol-1-yl]methyl}pyridin-3-yl |
| 2350 | Pyridazin-4-yl | CH₃ | 4-Chlor-3-[(2-methylpropanoyl)amino]phenyl |
| 2351 | Pyridazin-4-yl | CH₃ | 4-(Propylsulfanyl)phenyl |
| 2352 | Pyridazin-4-yl | CH₃ | 1-[2-(Difluormethyl)phenyl]-5-(methoxymethyl)-1H-pyrazol-4-yl |
| 2353 | Pyridazin-4yl | CH₃ | 4-Methoxy-3-[4-(pyrimidin-2-yl)-1H-pyrazol-1-yl]phenyl |
| 2354 | Pyridazin-4-yl | CH₃ | 4-{[(4,6-Dimethylpyrimidin-2-yl)sulfanyl]methyl}phenyl |
| 2355 | Pyridazin-4-yl | CH₃ | 1-(2-Chlorphenyl)-5-(methoxymethyl)-1H-pyrazol-4-yl |
| 2356 | Pyridazin-4-yl | CH₃ | 6-{[3,5-Bis(trifluormethyl)-1H-1,2,4-triazol-1-yl]methyl}pyridin-3-yl |
| 2357 | Pyridazin-4-yl | CH₃ | 5-Chlor-6-[4-(trifluormethoxy)phenoxy]pyridin-3-yl |
| 2358 | Pyridazin-4-yl | CH₃ | 4-[(tert-Butoxyimino)methyl]phenyl |
| 2359 | Pyridazin-4-yl | CH₃ | 2-(3-Chlorpyridin-2-yl)-1,3-thiazol-5-yl |
| 2360 | Pyridazin-4-yl | CH₃ | 1-(2-Fluorphenyl)-1H-pyrazol-4-yl |
| 2361 | Pyridazin-4-yl | CH₃ | 4-(Trifluormethoxy)phenyl |
| 2362 | Pyridazin-4-yl | CH₃ | 6-(2-Chlorphenoxy)pyridin-3-yl |
| 2363 | Pyridazin-4-yl | CH₃ | 3-[(2,2-Dimethylpropanoyl)amino]-5-(trifluormethyl)phenyl |
| 2364 | Pyridazin-4-yl | CH₃ | 6-[(4-Methylpyrimidin-2-yl)sulfanyl]pyridin-3-yl |
| 2365 | Pyridazin-4-yl | CH₃ | 6-{[2-(Morpholin-4-yl)ethyl]amino}pyridin-3-yl |
| 2366 | Pyridazin-4-yl | CH₃ | 3-Formyl-1-methyl-1H-pyrazol-4-yl |
| 2367 | Pyridazin-4-yl | CH₃ | 1-(3-Chlorphenyl)-5-(1,1-dimethoxyethyl)-1H-pyrazol-4-yl |
| 2368 | Pyridazin-4-yl | CH₃ | 4-Nitrophenyl |
| 2369 | Pyridazin-4-yl | CH₃ | 6-{[5-(Methoxycarbonyl)pyridin-3-yl]oxy}pyridin-3-yl |
| 2370 | Pyridazin-4-yl | CH₃ | 3-{[4-(Trifluormethyl)benzyl]oxy}phenyl |
| 2371 | Pyridazin-4-yl | CH₃ | 1-(2,4-Dichlorphenyl)-5-(1,1-dimethoxyethyl)-1H-pyrazol-4-yl |
| 2372 | Pyridazin-4-yl | CH₃ | 4-{[4-Brom-3-(trifluormethyl)-1H-pyrazol-1-yl]methyl}phenyl |
| 2373 | Pyridazin-4-yl | CH₃ | 3-[(Cyclopropylcarbonyl)amino]-4-fluorphenyl |
| 2374 | Pyridazin-4-yl | CH₃ | 6-[4-(Dimethylsulfamoyl)phenoxy]pyridin-3-yl |
| 2375 | Pyridazin-4-yl | CH₃ | 5-Methyl-1-(2-methylpyridin-3-yl)-1H-pyrazol-4-yl |
| 2376 | Pyridazin-4-yl | CH₃ | 1-(2,4-Difluorphenyl)-5-methyl-1H-pyrazol-4-yl |
| 2377 | Pyridazin-4-yl | CH₃ | 3-(Methylsulfanyl)phenyl |
| 2378 | Pyridazin-4-yl | CH₃ | 3-(Acetylamino)-4-chlorphenyl |
| 2379 | Pyridazin-4-yl | CH₃ | 4-[(4-Brom-1H-pyrazol-1-yl)methyl]phenyl |
| 2380 | Pyridazin-4-yl | CH₃ | 4-Methyl-3-[(propan-2-ylcarbamoyl)amino]phenyl |
| 2381 | Pyridazin-4-yl | CH₃ | 1-(2,2-Diethoxyethyl)-3-(trifluormethyl)-1H-pyrazol-4-yl |
| 2382 | Pyridazin-4-yl | CH₃ | 6-[(4,6-Dimethoxypyrimidin-2-yl)oxy]pyridin-3-yl |
| 2383 | Pyridazin-4-yl | CH₃ | 6-(1H-Pyrazol-1-yl)pyridin-3-yl |
| 2384 | Pyridazin-4-yl | CH₃ | 4-(1H-1,2,4-Triazol-1-ylmethyl)phenyl |
| 2385 | Pyridazin-4-yl | CH₃ | 5-(Methoxymethyl)-1-[2-(trifluormethyl)phenyl]-1H-pyrazol-4-yl |
| 2386 | Pyridazin-4-yl | CH₃ | 5-Methylpyridin-3-yl |
| 2387 | Pyridazin-4-yl | CH₃ | 5-Chlor-6-[(4,6-dimethylpyrimidin-2-yl)sulfanyl]pyridin-3-yl |
| 2388 | Pyridazin-4-yl | CH₃ | 3-[(2,2-Dimethylpropanoyl)amino]-4-methoxyphenyl |
| 2389 | Pyridazin-4-yl | CH₃ | 2-[2-(Trifluormethyl)pyridin-3-yl]-1,3-thiazol-4-yl |
| 2390 | Pyridazin-4-yl | CH₃ | 3-Methyl-1H-pyrazol-4-yl |
| 2391 | Pyridazin-4-yl | CH₃ | 5-(Methoxymethyl)-1-(2,2,2-trifluorethyl)-1H-pyrazol-4-yl |
| 2392 | Pyridazin-4-yl | CH₃ | 4-Methoxy-3-(propanoylamino)phenyl |
| 2393 | Pyridazin-4-yl | CH₃ | 4-Methoxy-3-[(2-methylpropanoyl)amino]phenyl |
| 2394 | Pyridazin-4-yl | CH₃ | 6-(Chinolin-7-yloxy)pyridin-3-yl |
| 2395 | Pyridazin-4-yl | CH₃ | 4-{[3-(Trifluormethyl)-1H-pyrazol-1-yl]methyl}phenyl |
| 2396 | Pyridazin-4-yl | CH₃ | 3-[(4,6-Dimethylpyrimidin-2-yl)oxy]phenyl |
| 2397 | Pyridazin-4-yl | CH₃ | 4-{[3,5-Bis(trifluormethyl)-1H-1,2,4-triazol-1-yl]methyl}phenyl |
| 2398 | Pyridazin-4-yl | CH₃ | 3-Fluor-4-{[4-(trifluormethyl)-1H-pyrazol-1-yl]methyl}phenyl |
| 2399 | Pyridazin-4-yl | CH₃ | 2,3-Dihydro-1,4-benzodioxin-6-yl |
| 2400 | Pyridazin-4-yl | CH₃ | 2-[3-(Methoxymethyl)pyridin-2-yl]-1,3-thiazol-5-yl |
| 2401 | Pyridazin-4-yl | CH₃ | 6-({6-[3-(Trifluormethyl)-1H-pyrazol-1-yl]pyridin-2-yl}oxy)pyridin-3-yl |
| 2402 | Pyridazin-4-yl | CH₃ | 3-{[3-(Pyridin-2-yl)-1H-pyrazol-1-yl]methyl}phenyl |
| 2403 | Pyridazin-4-yl | CH₃ | 1-(2-Brom-4-chlorphenyl)-5-methyl-1H-pyrazol-4-yl |
| 2404 | Pyridazin-4-yl | CH₃ | 2,2-Difluor-1,3-benzodioxol-5-yl |
| 2405 | Pyridazin-4-yl | CH₃ | 2-[3-(Trifluormethyl)pyridin-2-yl]-1,3-thiazol-4-yl |
| 2406 | Pyridazin-4-yl | CH₃ | 4-(Benzylcarbamoyl)phenyl |
| 2407 | Pyridazin-4-yl | CH₃ | 4-[(2-Chlorpyrimidin-4-yl)oxy]phenyl |
| 2408 | Pyridazin-4-yl | CH₃ | 1-[3-(Trifluormethyl)pyridin-2-yl]-1H-pyrazol-4-yl |
| 2409 | Pyridazin-4-yl | CH₃ | 5-Chlor-6-(4-chlorphenoxy)pyridin-3-yl |
| 2410 | Pyridazin-4-yl | CH₃ | 3-Cyan-4-(1H-1,2,4-triazol-1-yl)phenyl |
| 2411 | Pyridazin-4-yl | CH₃ | 3-Amino-4-(methylsulfonyl)phenyl |
| 2412 | Pyridazin-4-yl | CH₃ | 4-Methyl-3-[(2-methylpropanoyl)amino]phenyl |
| 2413 | Pyridazin-4-yl | CH₃ | 1-(2,6-Dibromphenyl)-5-(trifluormethyl)-1H-pyrazol-4-yl |
| 2414 | Pyridazin-4-yl | CH₃ | 3-{[3-(Trifluormethyl)-1H-1,2,4-triazol-1-yl]methyl}phenyl |
| 2415 | Pyridazin-4-yl | CH₃ | 4-[(3,3,3-Trifluorpropanoyl)amino]phenyl |
| 2416 | Pyridazin-4-yl | CH₃ | 5-(Pyrazin-2-yl)-1-(2,2,2-trifluorethyl)-1H-pyrazol-4-yl |
| 2417 | Pyridazin-4-yl | CH₃ | 1-(2,6-Dichlorphenyl)-5-(dimethoxymethyl)-1H-pyrazol-4-yl |
| 2418 | Pyridazin-4-yl | CH₃ | 1-(4-Chlorphenyl)-5-(1,1-dimethoxyethyl)-1H-pyrazol-4-yl |
| 2419 | Pyridazin-4-yl | CH₃ | 6-[3-(Trifluormethyl)phenoxy]pyridin-3-yl |
| 2420 | Pyridazin-4-yl | CH₃ | 4-({6-[3-(Trifluormethyl)-1H-pyrazol-1-yl]pyridin-2-yl}oxy)phenyl |
| 2421 | Pyridazin-4-yl | CH₃ | 4-Methoxy-3-(propan-2-ylcarbamoyl)phenyl |
| 2422 | Pyridazin-4-yl | CH₃ | 1-[2-(1-Methoxyethyl)phenyl]-5-methyl-1H-pyrazol-4-yl |
| 2423 | Pyridazin-4-yl | CH₃ | 4-[(4,6-Dimethoxypyrimidin-2-yl)sulfanyl]phenyl |
| 2424 | Pyridazin-4-yl | CH₃ | 6-(4-Methylphenoxy)pyridin-3-yl |
| 2425 | Pyridazin-4-yl | CH₃ | 6-(4-Chlor-2-cyanphenoxy)pyridin-3-yl |
| 2426 | Pyridazin-4-yl | CH₃ | 1-Propyl-3-(trifluormethyl)-1H-pyrazol-4-yl |
| 2427 | Pyridazin-4-yl | CH₃ | 6-(2-Methylphenoxy)pyridin-3-yl |
| 2428 | Pyridazin-4-yl | CH₃ | 4-{[4-(Trifluormethyl)-1H-pyrazol-1-yl]methyl}phenyl |
| 2429 | Pyridazin-4-yl | CH₃ | 6-[(6-Methylpyridin-3-yl)oxy]pyridin-3-yl |
| 2430 | Pyridazin-4-yl | CH₃ | 6-Methoxypyridin-3-yl |
| 2431 | Pyridazin-4-yl | CH₃ | 4-[(Ethoxyimino)methyl]phenyl |
| 2432 | Pyridazin-4-yl | CH₃ | 4-{[Acetyl(cyclopentyl)amino]methyl}phenyl |
| 2433 | Pyridazin-4-yl | CH₃ | 3-{[3,5-Bis(trifluormethyl)-1H-pyrazol-1-yl]methyl}phenyl |
| 2434 | Pyridazin-4-yl | CH₃ | 1-(2-Brom-4-chlorphenyl)-1H-pyrazol-4-yl |
| 2435 | Pyridazin-4-yl | CH₃ | 6-[4-(1H-1,2,4-Triazol-1-yl)phenoxy]pyridin-3-yl |
| 2436 | Pyridazin-4-yl | CH₃ | 6-[4-(Trifluormethyl)phenoxy]pyridin-3-yl |
| 2437 | Pyridazin-4-yl | CH₃ | 1-(2-Chlorphenyl)-5-(trifluormethyl)-1H-pyrazol-4-yl |
| 2438 | Pyridazin-4-yl | CH₃ | 4-Methoxy-3-nitrophenyl |
| 2439 | Pyridazin-4-yl | CH₃ | 1-(2,6-Dibromphenyl)-5-(difluormethyl)-1H-pyrazol-4-yl |
| 2440 | Pyridazin-4-yl | CH₃ | 6-[(4-Methoxyphenyl)sulfanyl]pyridin-3-yl |
| 2441 | Pyridazin-4-yl | CH₃ | 1-(2,6-Dichlorphenyl)-5-(1-methoxyethyl)-1H-pyrazol-4-yl |
| 2442 | Pyridazin-4-yl | CH₃ | 4-[(6-Chlorpyridin-2-yl)oxy]phenyl |
| 2443 | Pyridazin-4-yl | CH₃ | 3-Fluor-4-{[3-(trifluormethyl)-1H-pyrazol-1-yl]methyl}phenyl |
| 2444 | Pyridazin-4-yl | CH₃ | 4-{[(Difluormethyl)sulfanyl]methyl}phenyl |
| 2445 | Pyridazin-4-yl | CH₃ | 6-{[3-(Trifluormethyl)-1H-pyrazol-1-yl]methyl}pyridin-3-yl |
| 2446 | Pyridazin-4-yl | CH₃ | 4-{[2-(3,5-Dichlorphenyl)propan-2-yl]carbamoyl}phenyl |
| 2447 | Pyridazin-4-yl | CH₃ | 3-(1H-1,2,4-Triazol-1-ylmethyl)phenyl |
| 2448 | Pyridazin-4-yl | CH₃ | 2-(3-Chlorpyridin-2-yl)-1,3-thiazol-4-yl |
| 2449 | Pyridazin-4-yl | CH₃ | 5-(1-Methoxyethyl)-1-(pyrimidin-2-yl)-1H-pyrazol-4-yl |
| 2450 | Pyridazin-4-yl | CH₃ | 1-(3-Chlorpyridin-2-yl)-5-(1,1-dimethoxyethyl)-1H-pyrazol-4-yl |
| 2451 | Pyridazin-4-yl | CH₃ | 1-(2-Brom-4-fluorphenyl)-5-methyl-1H-pyrazol-4-yl |
| 2452 | Pyridazin-4-yl | CH₃ | 3-(Butanoylamino)-4-methylphenyl |
| 2453 | Pyridazin-4-yl | CH₃ | 5-(Methoxymethyl)-1-[3-(trifluormethyl)pyridin-2-yl]-1H-pyrazol-4-yl |
| 2454 | Pyridazin-4-yl | CH₃ | 6-[3-(Trifluormethyl)-1H-pyrazol-1-yl]pyridin-3-yl |
| 2455 | Pyridazin-4-yl | CH₃ | 6-(3-Chlorphenoxy)pyridin-3-yl |
| 2456 | Pyridazin-4-yl | CH₃ | 1-(2,6-Dichlorpyridin-3-yl)-5-(1-methoxyethyl)-1H-pyrazol-4-yl |
| 2457 | Pyridazin-4-yl | CH₃ | 1-[2-(Difluormethyl)phenyl]-5-methyl-1H-pyrazol-4-yl |
| 2458 | Pyridazin-4-yl | CH₃ | 2-[2-(Trifluormethyl)phenyl]-1,3-thiazol-5-yl |
| 2459 | Pyridazin-4-yl | CH₃ | 2-[2-(Trifluormethyl)pyridin-3-yl]-1,3-thiazol-5-yl |
| 2460 | Pyridazin-4-yl | CH₃ | 6-[(4,6-Dimethoxy-1,3,5-triazin-2-yl)sulfanyl]pyridin-3-yl |
| 2461 | Pyridazin-4-yl | CH₃ | 2-(2-Chlorpyridin-3-yl)-1,3-thiazol-5-yl |
| 2462 | Pyridazin-4-yl | CH₃ | 1-(2,6-Dichlorpyridin-3-yl)-5-methyl-1H-pyrazol-4-yl |
| 2463 | Pyridazin-4-yl | CH₃ | 1-(3-Chlorpyridin-2-yl)-5-(methoxymethyl)-1H-pyrazol-4-yl |
| 2464 | Pyridazin-4-yl | CH₃ | 6-[(4-Chlorphenyl)sulfanyl]pyridin-3-yl |
| 2465 | Pyridazin-4-yl | CH₃ | 6-(Piperidin-1-yl)pyridin-3-yl |
| 2466 | Pyridazin-4-yl | CH₃ | 3-[(Cyclopentylcarbonyl)amino]-4-methylphenyl |
| 2467 | Pyridazin-4-yl | CH₃ | 6-Ethoxypyridin-3-yl |
| 2468 | Pyridazin-4-yl | CH₃ | 2-(2,6-Dichlorphenyl)-1,3-thiazol-4-yl |
| 2469 | Pyridazin-4-yl | CH₃ | 5-Chlor-6-[(4-methylpyrimidin-2-yl)sulfanyl]pyridin-3-yl |
| 2470 | Pyridazin-4-yl | CH₃ | 1-[4-Chlor-2-(trifluormethyl)phenyl]-5-(1-methoxyethyl)-1H-pyrazol-4-yl |
| 2471 | Pyridazin-4-yl | CH₃ | 6-(3-Methylphenoxy)pyridin-3-yl |
| 2472 | Pyridazin-4-yl | CH₃ | 4-[Ethyl(methyl)carbamoyl]phenyl |
| 2473 | Pyridazin-4-yl | CH₃ | 6-[3-(Trifluormethoxy)phenoxy]pyridin-3-yl |
| 2474 | Pyridazin-4-yl | CH₃ | 5-(Difluormethyl)-1-(pyrimidin-2-yl)-1H-pyrazol-4-yl |
| 2475 | Pyridazin-4-yl | CH₃ | 4-[(Cyclopropylcarbonyl)amino]phenyl |
| 2476 | Pyridazin-4-yl | CH₃ | 1-(3-Brompyridin-2-yl)-5-(1-methoxyethyl)-1H-pyrazol-4-yl |
| 2477 | Pyridazin-4-yl | CH₃ | 3-[(Cyclopropylcarbonyl)amino]-4-methoxyphenyl |
| 2478 | Pyridazin-4-yl | CH₃ | 4-Methyl-3-[(3-methylbut-2-enoyl)amino]phenyl |
| 2479 | Pyridazin-4-yl | CH₃ | 1-[3-(Methoxymethyl)pyridin-2-yl]-1H-pyrazol-4-yl |
| 2480 | Pyridazin-4-yl | CH₃ | 4-[Acetyl(methyl)amino]phenyl |
| 2481 | Pyridazin-4-yl | CH₃ | 6-(Naphthalen-2-yloxy)pyridin-3-yl |
| 2482 | Pyridazin-4-yl | CH₃ | 1-(2-Chlorphenyl)-5-(1-methoxyethyl)-1H-pyrazol-4-yl |
| 2483 | Pyridazin-4-yl | CH₃ | 1-[2-(Difluormethyl)phenyl]-5-(1-methoxyethyl)-1H-pyrazol-4-yl |
| 2484 | Pyridazin-4-yl | CH₃ | 1-(3-Chlorpyridin-2-yl)-5-methyl-1H-pyrazol-4-yl |
| 2485 | Pyridazin-4-yl | CH₃ | 4-{[3-(Trifluormethyl)-1H-1,2,4-triazol-1-yl]methyl}phenyl |
| 2486 | Pyridazin-4-yl | CH₃ | 3-[(Cyclopentylcarbonyl)amino]-4-methoxyphenyl |
| 2487 | Pyridazin-4-yl | CH₃ | 6-[4-(Methylsulfanyl)phenoxy]pyridin-3-yl |
| 2488 | Pyridazin-4-yl | CH₃ | 2-[3-(Trifluormethyl)pyridin-2-yl]-1,3-thiazol-5-yl |
| 2489 | Pyridazin-4-yl | CH₃ | 4-{[3-(Methoxycarbonyl)-1H-1,2,4-triazol-1-yl]methyl}phenyl |
| 2490 | Pyridazin-4-yl | CH₃ | 1-[3-(1,1-Difluorethyl)pyridin-2-yl]-1H-pyrazol-4-yl |
| 2491 | Pyridazin-4-yl | CH₃ | 6-{[3-(Pyridin-2-yl)-1H-pyrazol-1-yl]methyl}pyridin-3-yl |
| 2492 | Pyridazin-4-yl | CH₃ | 3-(Acetylamino)phenyl |
| 2493 | Pyridazin-4-yl | CH₃ | 1-(3-Chlorpyridin-2-yl)-5-(trifluormethyl)-1H-pyrazol-4-yl |
| 2494 | Pyridazin-4-yl | CH₃ | 2-{[3-(Pyridin-2-yl)-1H-pyrazol-1-yl]methyl}pyridin-4-yl |
| 2495 | Pyridazin-4-yl | CH₃ | 3-[(Methoxyacetyl)amino]-4-methylphenyl |
| 2496 | Pyridazin-4-yl | CH₃ | 1-(3-Brompyridin-2-yl)-5-(trifluormethyl)-1H-pyrazol-4-yl |
| 2497 | Pyridazin-4-yl | CH₃ | 4-[(Methoxyimino)methyl]phenyl |
| 2498 | Pyridazin-4-yl | CH₃ | 1-(Pyrimidin-2-yl)-5-(trifluormethyl)-1H-pyrazol-4-yl |
| 2499 | Pyridazin-4-yl | CH₃ | 2-(2-Chlorphenyl)-1,3-thiazol-4-yl |
| 2500 | Pyridazin-4-yl | CH₃ | 4-Methoxy-3-[(propan-2-ylsulfonyl)amino]phenyl |
| 2501 | Pyridazin-4-yl | CH₃ | 6-(3-Cyanphenoxy)pyridin-3-yl |
| 2502 | Pyridazin-4-yl | CH₃ | 4-Fluor-3-[(2-methylpropanoyl)amino]phenyl |
| 2503 | Pyridazin-4-yl | CH₃ | 1-(2-Bromphenyl)-5-(difluormethyl)-1H-pyrazol-4-yl |
| 2504 | Pyridazin-4-yl | CH₃ | 1,3-Dimethyl-1H-pyrazol-4-yl |
| 2505 | Pyridazin-4-yl | CH₃ | 5-(1-Methoxyethyl)-1-(2,2,2-trifluorethyl)-1H-pyrazol-4-yl |
| 2506 | Pyridazin-4-yl | CH₃ | 1-(2,6-Dichlorphenyl)-5-(1-fluorethyl)-1H-pyrazol-4-yl |
| 2507 | Pyridazin-4-yl | CH₃ | 6-(Pyrrolidin-1-yl)pyridin-3-yl |
| 2508 | Pyridazin-4-yl | CH₃ | 3-(Acetylamino)-5-(trifluormethyl)phenyl |
| 2509 | Pyridazin-4-yl | CH₃ | 2-[3-(Methoxymethyl)pyridin-2-yl]-1,3-thiazol-4-yl |
| 2510 | Pyridazin-4-yl | CH₃ | 2-[3-(1-Methoxyethyl)pyridin-2-yl]-1,3-thiazol-5-yl |
| 2511 | Pyridazin-4-yl | CH₃ | 1-[3-(Dimethoxymethyl)pyridin-2-yl]-1H-pyrazol-4-yl |
| 2512 | Pyridazin-4-yl | CH₃ | 5-(1-Methoxyethyl)-1-(2-methylphenyl)-1H-pyrazol-4-yl |
| 2513 | Pyridazin-4-yl | CH₃ | 1-Methyl-1H-benzotriazol-5-yl |
| 2514 | Pyridazin-4-yl | CH₃ | 4-[(Butylsulfanyl)methyl]phenyl |
| 2515 | Pyridazin-4-yl | CH₃ | 1H-Pyrazol-4-yl |
| 2516 | Pyridazin-4-yl | CH₃ | 1-(2-Brom-6-chlorphenyl)-5-(1-methoxyethyl)-1H-pyrazol-4-yl |
| 2517 | Pyridazin-4-yl | CH₃ | 5-Chlor-6-[4-(methylsulfonyl)phenoxy]pyridin-3-yl |
| 2518 | Pyridazin-4-yl | CH₃ | 1-[3-(1-Methoxyethyl)pyridin-2-yl]-1H-pyrazol-4-yl |
| 2519 | Pyridazin-4-yl | CH₃ | 6-[(4,6-Dimethoxypyrimidin-2-yl)sulfanyl]pyridin-3-yl |
| 2520 | Pyridazin-4-yl | CH₃ | 4-[(6-Methylpyridin-3-yl)oxy]phenyl |
| 2521 | Pyridazin-4-yl | CH₃ | 3-Methoxy-1-methyl-1H-pyrazol-4-yl |
| 2522 | Pyridazin-4-yl | CH₃ | 6-(4-tert-Butylphenoxy)pyridin-3-yl |
| 2523 | Pyridazin-4-yl | CH₃ | 1-(2,4-Dichlorphenyl)-5-methyl-1H-pyrazol-4-yl |
| 2524 | Pyridazin-4-yl | CH₃ | 3-(But-2-enoylamino)-4-methylphenyl |
| 2525 | Pyridazin-4-yl | CH₃ | 4-{[4-(Pyrimidin-2-yl)-1H-pyrazol-1-yl]methyl}phenyl |
| 2526 | Pyridazin-4-yl | CH₃ | 4-{[6-(1H-Pyrazol-1-yl)pyridin-2-yl]oxy}phenyl |
| 2527 | Pyridazin-4-yl | CH₃ | 4-{[3,5-Bis(trifluormethyl)-1H-1,2,4-triazol-1-yl]methyl}-3-chlorphenyl |
| 2528 | Pyridazin-4-yl | CH₃ | 4-[(2-Methoxypyrimidin-4-yl)oxy]phenyl |
| 2529 | Pyridazin-4-yl | CH₃ | 1-[4-Chlor-2-(trifluormethyl)phenyl]-5-(methoxymethyl)-1H-pyrazol-4-yl |
| 2530 | Pyridazin-4-yl | CH₃ | 5-(Trifluormethyl)-1-[2-(trifluormethyl)phenyl]-1H-pyrazol-4-yl |
| 2531 | Pyridazin-4-yl | CH₃ | 6-(Dimethylamino)pyridin-3-yl |
| 2532 | Pyridazin-4-yl | CH₃ | 2-[5-Chlor-3-(trifluormethyl)pyridin-2-yl]-1,3-thiazol-5-yl |
| 2533 | Pyridazin-4-yl | CH₃ | 4-{[4-(3-Chlorpyridin-2-yl)-1H-pyrazol-1-yl]methyl}phenyl |
| 2534 | Pyridazin-4-yl | CH₃ | 4-[(2,2,3,3,4,4,4-Heptafluorbutanoyl)(methyl)amino]phenyl |
| 2535 | Pyridazin-4-yl | CH₃ | 4-[(Trifluormethyl)sulfanyl]phenyl |
| 2536 | Pyridazin-4-yl | CH₃ | 6-[4-(Methylsulfonyl)phenoxy]pyridin-3-yl |
| 2537 | Pyridazin-4-yl | CH₃ | 1-(2-Bromphenyl)-5-(methoxymethyl)-1H-pyrazol-4-yl |
| 2538 | Pyridazin-4-yl | CH₃ | 6-[(4-Brom-1H-pyrazol-1-yl)methyl]pyridin-3-yl |
| 2539 | Pyridazin-4-yl | CH₃ | 3-Chlor-4-{[3-(trifluormethyl)-1H-pyrazol-1-yl]methyl}phenyl |
| 2540 | Pyridazin-4-yl | CH₃ | 6-{[4-(Trifluormethyl)-1H-pyrazol-1-yl]methyl}pyridin-3-yl |
| 2541 | Pyridazin-4-yl | CH₃ | 1-(3-Brompyridin-2-yl)-5-(difluormethyl)-1H-pyrazol-4-yl |

### Anwendungsbeispiele

### Beispiel Aw

### Myzus-Test (MYZUPE Spritzbehandlung)

| | |
|---|---|
| Lösungsmittel: | 78 Gewichtsteile Aceton |
| | 1,5 Gewichtsteile Dimethylformamid |
| | |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele eine Wirkung von ≥ 80 % bei einer Aufwandmenge von 500 g / ha :
Bsp Nr : 1-257, 1-258, 1-259, 1-260, 1-261, 1-262, 1-263, 1-264, 1-265, 1-266, 1-268, 1-269, 1-270, 1-271, I-272, I-273, I-274, I-275, I-276, I-277, I-278, 1-279, 1-280, 1-281, 1-282, 1-283, 1-285, 1- 286, I-287, I-288, I-289, I-290, I-291, I-292, I-293, 1-294, 1-295, 1-297, 1-298, 1-299, I-300, I-301, 1-302, 1-303, 1- 305, 1-307, 1-308, 1-310, I-311, 1-312, 1-314, 1-315, 1-316, 1-317, 1-318, 1-319, 1-320, 1-321, I-322, I-323, I-324, I-326, I-327, I-329, I-330, I-331, I-332, I-333, I-334, I-335, I-336, I-337, 1-336

Bei diesem Test zeigen z. B. die folgenden Verbindungen der Herstellungsbeispiele eine Wirkung von ≥ 80 % bei einer Aufwandmenge von 20 g / ha :
Bsp Nr : 1-2

Bei diesem Test zeigen z. B. die folgenden Verbindungen der Herstellungsbeispiele eine Wirkung von ≥ 80 % bei einer Aufwandmenge von 500 ppm :
Bsp Nr : 1-284, 1-296

### Beispiel B

### Myzus-Test; oral; (MYZUPE O)

| | |
|---|---|
| Lösungsmittel: | 80 Gewichtsteile Aceton |

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser auf die gewünschte Konzentration.

Gefäße werden mit allen Stadien der Grünen Pfirsichblattlaus (*Myzus persicae*) besetzt, durch saugen an der Wirkstoffzubereitung der gewünschten Konzentration wird behandelt.

Nach der gewünschten Zeit wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele eine Wirkung von ≥ 80 % bei einer Aufwandmenge von 20 ppm
Bsp Nr : 1-304, 1-306, 1-309, 1-325, 1-328

### Beispiel C

### Boophilus microplus -Test (BOOPMI Injektion)

| | |
|---|---|
| Lösungsmittel: | Dimethylsulfoxid |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration.

Die Wirkstofflösung wird in das Abdomen (*Boophilus microplus*) injiziert, die Tiere werden in Schalen überführt und in einem klimatisierten Raum aufbewahrt. Die Wirkungskontrolle erfolgt auf Ablage fertiler Eier.

Nach der gewünschten Zeit wird die Wirkung in % bestimmt. Dabei bedeutet 100%, dass keine Zecke fertile Eier gelegt hat. Bei diesem Test zeigen z. B. die folgenden Verbindungen der Herstellungsbeispiele eine Wirkung von ≥ 80 % bei einer Aufwandmenge von 20µg /Tier :
Bsp Nr : 1-8, 1-101, 1-135

### Beispiel D

### Myzus - Test ( MYZUPE )

| | |
|---|---|
| Lösungsmittel: | 3 Gewichtsteile Dimethylformamid |
| Emulgator: | 1 Gewichtsteil Polyoxyethylenalkylphenylether |

Zur Herstellung einer zweckmäßigen Wirkstoffzubereitung vermischt man 1 Gewichtsteil Wirkstoff mit der angegebenen Menge.Lösungsmittel und verdünnt das Konzentrat mit Wasser auf die gewünschte Konzentration.

Auberginenpflanzen (*Solanum melongena*) die von allen Stadien der Grünen Pfirsichblattlaus (*Myzus persicae*) befallen sind, werden mit einer Wirkstoffzubereitung der gewünschten Konzentration gespritzt.

Nach der gewünschten Zeit wird die Wirkung in % bestimmt, dabei bedeutet 100 % dass alle Blattläuse abgetötet wurden. 0 % bedeutet, dass keine Blattläuse abgetötet wurden.

Bei diesem Test zeigen z. B. die folgenden Verbindungen der Herstellungsbeispiele eine Wirkung von ≥ 80 % bei einer Aufwandmenge von 500 ppm:
Bsp Nr : 1-5, 1-6, 1-8, 1-9, 1-12, 1-13, 1-14, 1-15, 1-16, 1-17, 1-18, 1-20, 1-21, 1-22, 1-23, 1-25, 1-26, 1-27, I-28, I-29, I-30, I-32, I-33, I-34, I-35, I-36, I-37, 1-38, 1-39, 1-40, 1-41, 1-42, 1-43, 1-47, 1-48, 1-49, I-50, I-51, I-52, I-53, I-54, I-55, I-56, I-57, I-58, I-59, 1-60, 1-61, 1-62, 1-63, 1-64, 1-66, 1-67, 1-68, 1-69, 1-70, I-71, I-73, I-74, I-75, I-76, I-77, I-78, I-79, I-80, I-81, 1-82, 1-83, 1-84, 1-88, 1-89, 1-90, 1-91, 1-92, 1-93, I-96, I-97, I-98, I-99, I-100, I-101, I-102, I-103, I-104, 1-105, 1-106, 1-107, 1-108, 1-109, 1-110, 1-112, I-113, I-114, I-115, I-116, I-117, I-118, I-119, I-123, I-124, 1-128, 1-130, 1-131, 1-132, 1-133, 1-134, 1-135, I-136, I-137, I-138, I-141, I-142, I-144, I-145, I-146, I-147, I-148, I-149, I-152, I-154, I-155, I-156, I-157, I-159, I-161, I-163, I-164, I-165, I-166, I-167, I-168, I-169, I-170, 1-171,1-172,1-173,1-174, I-175, I-176, I-177, I-178 1-179, 1-180, 1-181, 1-182, 1-183, 1-184, 1-185, 1-186, 1-187, 1-188, 1-189, I-190, I-191, I-192 , I-193, I-194, I-195, I-196, I-197, I-200, I-205, I-207, I-208, I-209, I-210, I-212, I-217, I-218, I-219, I-220, I-221, I-222, I-223, 1-224, 1-225, 1-226, 1-227, 1-228, 1-229, 1-230, 1-231, I-232, I-233, I-234, I-236, I-238, I-241, I-244, 1-245, 1-246, 1-247, 1-248, 1-250, 1-251, 1-252, 1-253,1-254,1-255

Bei diesem Test zeigen z. B. die folgenden Verbindungen der Herstellungsbeispiele eine Wirkung von ≥ 80 % bei einer Aufwandmenge von 100 ppm:
Bsp Nr: I-1, 1-10, I-65, I-95

### Beispiel E

### Spodoptera litura - Test ( PRODLI )

| | |
|---|---|
| Lösungsmittel: | 3 Gewichtsteile Dimethylformamid |
| Emulgator: | 1 Gewichtsteil Polyoxyethylenalkylphenylether |

Zur Herstellung einer zweckmäßigen Wirkstoffzubereitung vermischt man 1 Gewichtsteil Wirkstoff mit der angegebenen Menge .Lösungsmittel und verdünnt das Konzentrat mit Wasser auf die gewünschte Konzentration.

Blätter der Süßkartoffel *(Ipomoea batatas )* werden in die Wirkstoffzubereitung der gewünschten Konzentration getaucht und mit Larven des asiatischen Baumwollwurmes (*Spodoptera litura*) besetzt.

Nach der gewünschten Zeit wird die Wirkung in % bestimmt, dabei bedeutet 100 % dass alle Larven abgetötet wurden. 0 % bedeutet, dass keine Larven abgetötet wurden.

Bei diesem Test zeigen z. B. die folgenden Verbindungen der Herstellungsbeispiele eine Wirkung von ≥ 80 % bei einer Aufwandmenge von 500 ppm:
Bsp Nr : 1-86

### Beispiel F

### Tetranychus - Test ( TETRUR )

| | |
|---|---|
| Lösungsmittel: | 3 Gewichtsteile Dimethylformamid |
| Emulgator: | 1 Gewichtsteil Polyoxyethylenalkylphenylether |

Zur Herstellung einer zweckmäßigen Wirkstoffzubereitung vermischt man 1 Gewichtsteil Wirkstoff mit der angegebenen Menge .Lösungsmittel und verdünnt das Konzentrat mit Wasser auf die gewünschte Konzentration. Pflanzen der Buschbohne *(Phaseolus vulgaris )* werden mit gemischter Population der gemeinen Spinnmilbe (*Tetranychus urticae*) infiziert und mit der Wirkstoffzubereitung der gewünschten Konzentration gespritzt. Nach der gewünschten Zeit wird die Wirkung in % bestimmt, dabei bedeutet 100 % dass alle Spinnmilben abgetötet wurden. 0 % bedeutet, dass keine Spinnmilben abgetötet wurden.

Bei diesem Test zeigen z. B. die folgenden Verbindungen der Herstellungsbeispiele eine Wirkung von ≥ 80 % bei einer Aufwandmenge von 500 ppm: Bsp Nr: 1-127

## Patentansprüche

1. Aminopyridin- und Aminopyridazin-Derivate der allgemeinen Formel (I), 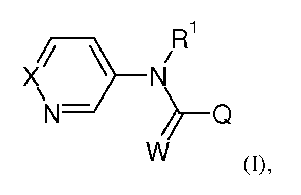 in welcher
R¹ für Wasserstoff, für gegebenenfalls einfach oder mehrfach, gleich oder verschieden substituiertes C₁-C₆-Alkyl, C₂-C₆-Alkenyl, C₂-C₆-Alkinyl oder C₃-C₆-Cycloalkyl steht,
wobei die Substituenten unabhängig voneinander ausgewählt sein können aus Halogen, Cyano, Nitro, Hydroxy, C₁-C₆-Alkyl, C₁-C₆-Alkoxy, C₃-C₆-Cycloalkyl, C₁-C₆- Halogenalkoxy, C₁-C₆-Alkylthio, Aryl, Hetaryl, Arylalkyl, Hetarylalkyl,
wobei die Substituenten Aryl, Hetaryl, Arylalkyl, Hetarylalkyl gegebenenfalls einfach oder mehrfach, gleich oder verschieden durch Halogen, Cyano, Nitro, Hydroxy, C₁-C₆-Alkyl, C₂-C₆-Alkenyl, C₂-C₆-Alkinyl, C₃-C₆-Cycloalkyl, C₁-C₆-Alkoxy, C₁-C₆-Halogenalkyl, C₁- C₆-Halogenalkoxy oder C₁-C₆-Alkylthio substituiert sein können,
X für CH oder N steht,
Q für einen aromatischen, gegebenenfalls durch 1-bis 3 Heteroatome aus der Reihe N, S, O substituierten, 5-6 gliedrigen Ring Q steht, welcher gegebenenfalls durch die Reste R²-R⁴ und den Rest A-Y unabhängig voneinander substituiert sein kann,
falls X für N steht
Q für einen aromatischen, gegebenenfalls durch 1-bis 3 Heteroatome aus der Reihe N, S, O substituierten, 6 gliedrigen Ring Q steht, welcher gegebenenfalls durch die Reste R²-R⁴ und den Rest A-Y unabhängig voneinander substituiert sein kann; für einen aromatischen, gegebenenfalls durch 1-bis 3 Heteroatome aus der Reihe N, S, O substituierten, 5 gliedrigen Ring Q steht, wobei höchstens eines der Heteroatome N ist, welcher gegebenenfalls durch die Reste R²-R⁴ und den Rest A- Y unabhängig voneinander substituiert sein kann;
oder für die folgenden Ringsysteme steht, ausgewählt aus Q₈ₐ-Q_{8d} 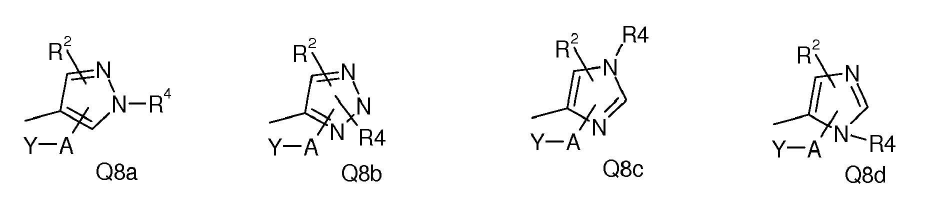
falls X für CH steht:
W für O oder S steht,
R² und R³ unabhängig voneinander für Wasserstoff, Hydroxy, Halogen, Nitro, für gegebenenfalls einfach oder mehrfach, gleich oder verschieden substituiertes Amino, C₁-C₆-Alkyl, C₁-C₆-Alkoxy, C₁-C₆-Alkoxy-C₁-C₆-Alkyl, C₂-C₆-Alkenyl, C₂-C₆-Alkinyl oder C₃-C₆-Cycloalkyl stehen, wobei die Substituenten unabhängig voneinander ausgewählt sein können aus Halogen, C₁-C₆-Alkyl, C₁-C₆- Alkylcarbonyl, C₁-C₆-Alkoxy, C₁-C₆-Halogenalkoxy, C₃-C₆-Cycloalkyl, C₃-C₆- Cycloalkylcarbonyl, Cyano, Nitro, Hydroxy, C₁-C₆-Alkylthio, C₁-C₆-Alkylsulfinyl, C₁-C₆-Alkylsulfonyl, (C₁-C₆-Alkoxy)carbonyl, C₁-C₆-Alkylamino, C₃-C₆- Cycloalkylamino, (C₁-C₆-Alkyl)C₃-C₆-cycloalkylamino oder Aryl, Hetaryl, das gegebenenfalls einfach oder mehrfach gleich oder verschieden durch Halogen, Cyano, Nitro, Hydroxy, C₁-C₆-Alkyl, C₁-C₆-Halogenalkyl, C₂-C₆-Alkenyl, C₂-C₆- Alkinyl, C₃-C₆-Cycloalkyl, C₁-C₆-Alkoxy, C₁-C₆-Halogenalkoxy C₁-C₆-Alkylthio, C₁-C₆-Alkylsulfinyl, C₁-C₆-Alkylsulfonyl substituiert sein kann,
R² und R³ ebenfalls zusammen einen 5-bis 7-gliedrigen, 1-3 Heteroatome aus der Reihe N,S,O enthaltenden, heteroaromatischen Ring ausbilden können, welcher gegebenenfalls ein-oder mehrfach durch C₁-C₆-Alkyl substituiert sein kann,
R⁴ für Wasserstoff, für gegebenenfalls einfach oder mehrfach, gleich oder verschieden substituiertes, C₁-C₆-Alkyl, C₁-C₆-Alkoxy-C₁-C₆-Alkyl, C₂-C₆-Alkenyl, C₂-C₆- Alkinyl, C₃-C₆-Cycloalkyl, Aryl, Hetaryl steht, wobei die Substituenten unabhängig voneinander ausgewählt sein können aus Halogen, C₁-C₆-Alkyl, C₁- C₆-Alkylcarbonyl, C₁-C₆-Alkoxy, C₁-C₆-Halogenalkoxy, C₃-C₆-Cycloalkyl, C₃-C₆- Cycloalkylcarbonyl, Cyano, Nitro, Hydroxy, C₁-C₆-Alkylthio, C₁-C₆-Alkylsulfinyl, C₁-C₆-Alkylsulfonyl, (C₁-C₆-Alkoxy)carbonyl, C₁-C₆-Alkylamino, C₃-C₆- Cycloalkylamino, (C₁-C₆-Alkyl)C₃-C₆-cycloalkylamino, Aryl oder Hetaryl, das gegebenenfalls einfach oder mehrfach gleich oder verschieden durch Halogen, Cyano, Nitro, Hydroxy, C₁-C₆-Alkyl, C₁-C₆-Halogenalkyl, C₂-C₆-Alkenyl, C₂-C₆- Alkinyl, C₃-C₆-Cycloalkyl, C₁-C₆-Alkoxy, C₁-C₆-Halogenalkoxy C₁-C₆-Alkylthio, C₁-C₆-Alkylsulfinyl, C₁-C₆-Alkylsulfonyl substituiert sein kann,
A für eine direkte Bindung oder für C₁-C₆-Alkylen, C₁-C₆-Alkylenoxy, Oxy(C₁-C₆)- alkylen, Thio(C₁-C₆)-alkylen, C₁-C₆-alkylenthio, -O- -S-, C₁-C₄-Alkylencarboxy, Carboxy(C₁-C₄)-Alkylen, -NR⁵, -C=N-O-, C₁-C₄-Alkylencarbamido, Carbamido(C₁-C₄)-Alkylen, -NR⁵(C=O)O-, NR⁵(SO₂)- -NR⁵(C=O)NR⁵-, -(SO₂)- steht,
R⁵ für C₁-C₆-Alkyl, C₃-C₆-Cycloalkyl oder für Wasserstoff steht,
Y für Wasserstoff, für gegebenenfalls ein- oder mehrfach, gleich oder verschieden substituiertes C₁-C₆-Alkyl, C₃-C₆-Cycloalkyl, für ein gegebenenfalls ein- oder mehrfach, gleich oder verschieden substituiertes, 5-10 gliederiges, 0-4 Heteroatome enthaltendes, gegebenenfalls annelliertes Ringsystem steht, wobei die Heteroatome ausgewählt sein können aus der Reihe N,S,O,
wobei die Substituenten ausgewählt sein können aus Halogen, Nitro, Cyano, aus gegebenenfalls einfach oder mehrfach, gleich oder verschieden substituiertem Amino, C₁-C₆-Alkyl, C₁-C₆-Alkoxy, C₁-C₆-Alkoxy-C₁-C₆-Alkyl, C₂-C₆-Alkenyl, C₂-C₆-Alkinyl, C₃-C₆-Cycloalkyl, Aryl, Hetaryl, C₁-C₆-Arylalkyl, C₁-C₆-Hetarylalkyl, Aryloxy, Hetaryloxy, Sulfonyl, C₁-C₆-Thioalkyl, C₁-C₆- Carboxyalkyl,
wobei die Substituenten unabhängig voneinander ausgewählt sein können aus Halogen, C₁-C₆-Alkyl, C₁-C₆-Alkylcarbonyl, C₁-C₆-Halogenalkyl, C₁-C₆-Alkoxy, C₁-C₆-Halogenalkoxy, C₃-C₆-Cycloalkyl, C₃-C₆-Cycloalkylcarbonyl, Cyano, Nitro, C₁-C₆-Alkylthio, C₁-C₆-Alkylsulfinyl, C₁-C₆-Alkylsulfonyl, (C₁-C₆-Alkoxy)carbonyl, C₂-C₆-Alkenyl, C₂-C₆-Alkinyl, C₁-C₆-Alkylamino, C₃-C₆-Cycloalkylamino, (C₁-C₆-Alkyl)C₃-C₆-cycloalkylamino, Di(C₁-C₄)alkylamino,
sowie Salze von Verbindungen der Formel (I).

2. Verbindungen der Formel (I) gemäß Anspruch 1, in welcher
R¹ für
Wasserstoff, für gegebenenfalls einfach oder mehrfach, gleich oder verschieden substituiertes C₁-C₄-Alkyl, C₂-C₄-Alkenyl, C₂-C₄-Alkinyl oder C₃-C₄-Cycloalkyl steht,
wobei die Substituenten unabhängig voneinander ausgewählt sein können aus Halogen, Cyano, Nitro, Hydroxy, C₁-C₄-Alkyl, C₁-C₄-Alkoxy, C₃-C₄-Cycloalkyl, C₁-C₄-Halogenalkoxy, Aryl, Hetaryl, Arylalkyl, Hetarylalkyl,
wobei die Substituenten Aryl, Hetaryl, Arylalkyl, Hetarylalkyl gegebenenfalls einfach oder mehrfach, gleich oder verschieden durch Halogen, Cyano, Nitro, Hydroxy, C₁-C₄-Alkyl, C₂-C₄-Alkenyl, C₂-C₄-Alkinyl, C₃-C₄-Cycloalkyl, C₁-C₄-Alkoxy, C₁-C₄-Halogenalkyl oder C₁-C₄-Halogenalkoxy substituiert sein können,
Q für einen Ring steht, ausgewählt aus der Gruppe Q1 bis Q 14 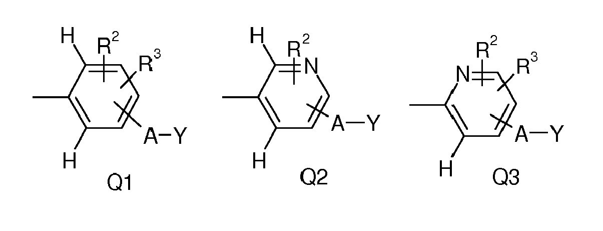 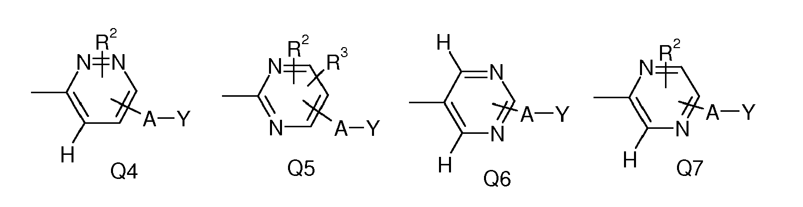 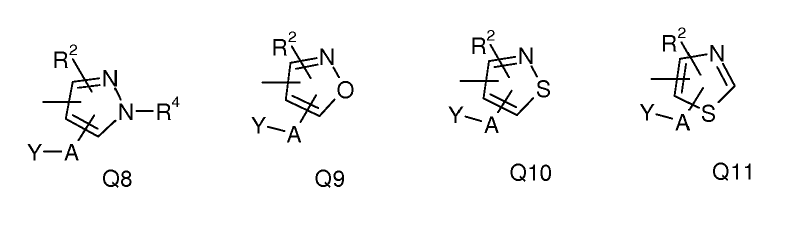 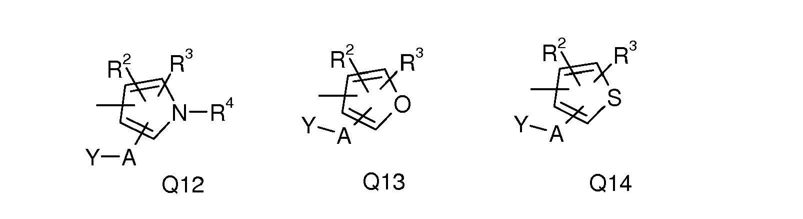
Falls X für N steht,
Q für einen Ring steht, ausgewählt aus der Gruppe Q1 bis Q 14 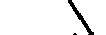 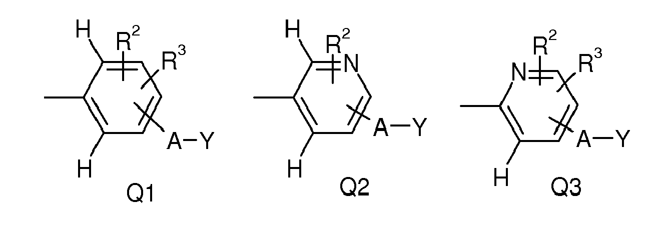 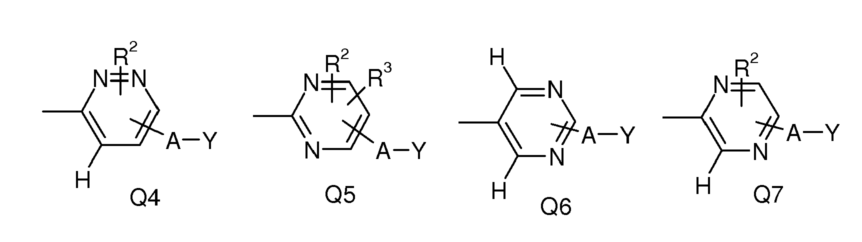 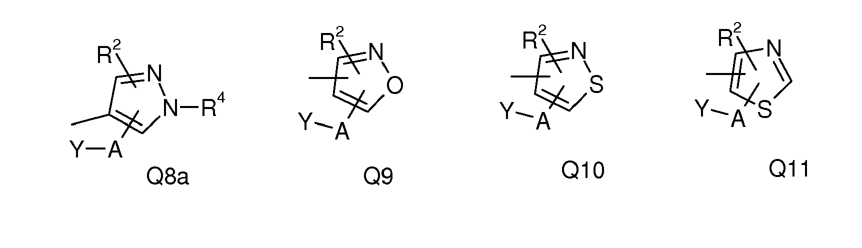 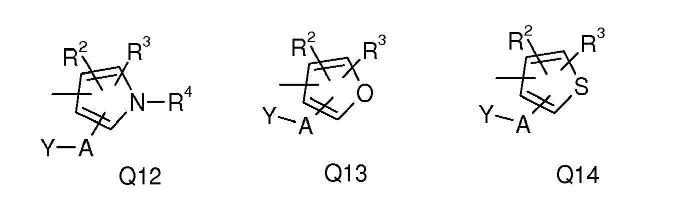
Falls X für CH steht,
R² und R³ unabhängig voneinander für Wasserstoff, Hydroxy, Halogen, Nitro, für gegebenenfalls einfach oder mehrfach, gleich oder verschieden substituiertes Amino, C₁-C₄-Alkyl, C₁-C₄-Alkoxy, C₁-C₄-Alkoxy-C₁-C₄-Alkyl, C₂-C₄-Alkenyl, C₂-C₄-Alkinyl oder C₃-C₄-Cycloalkyl stehen, wobei die Substituenten unabhängig voneinander ausgewählt sein können aus Halogen, C₁-C₄-Alkyl, C₁-C₄- Alkylcarbonyl, C₁-C₄-Alkoxy, C₁-C₄-Halogenalkoxy, C₃-C₄-Cycloalkyl, C₃-C₄- Cycloalkylcarbonyl, Cyano, Nitro, Hydroxy, C₁-C₄-Alkylthio, C₁-C₄-Alkylsulfinyl, C₁-C₄-Alkylsulfonyl, (C₁-C₄-Alkoxy)carbonyl, C₁-C₄-Alkylamino, C₃-C₄- Cycloalkylamino, (C₁-C₄-Alkyl)C₃-C₄-cycloalkylamino oder Aryl, Hetaryl, das gegebenenfalls einfach oder mehrfach gleich oder verschieden durch Halogen, Cyano, Nitro, Hydroxy, C₁-C₄-Alkyl, C₁-C₄-Halogenalkyl, C₂-C₄-Alkenyl, C₂-C₄- Alkinyl, C₃-C₄-Cycloalkyl, C₁-C₄-Alkoxy, C₁-C₄-Halogenalkoxy C₁-C₄-Alkylthio, C₁-C₄-Alkylsulfinyl, C₁-C₄-Alkylsulfonyl substituiert sein kann,
R² und R³ ebenfalls zusammen einen 5-gliedrigen, gegebenenfalls ein-oder mehrfach durch C₁-C₆-Alkyl substituierten, N enthaltenden, heteroaromatischen Ring ausbilden können,
R⁴ für Wasserstoff, für gegebenenfalls einfach oder mehrfach, gleich oder verschieden substituiertes, C₁-C₄-Alkyl, C₁-C₄-Alkoxy-C₁-C₄-Alkyl, C₂-C₄-Alkenyl, C₂-C₄- Alkinyl, C₃-C₄-Cycloalkyl, Aryl, Hetaryl steht, wobei die Substituenten unabhängig voneinander ausgewählt sein können aus Halogen, C₁-C₄-Alkyl, C₁- C₄-Alkoxy, C₁-C₄-Halogenalkoxy, C₃-C₄-Cycloalkyl, Cyano, Nitro, C₁-C₄- Alkylthio, (C₁-C₆-Alkoxy)carbonyl oder Aryl, Hetaryl, das gegebenenfalls einfach oder mehrfach gleich oder verschieden durch Halogen, Cyano, Nitro, C₁-C₄-Alkyl, C₁-C₄-Halogenalkyl, C₃-C₄-Cycloalkyl, C₁-C₄-Alkoxy, C₁-C₄-Halogenalkoxy substituiert sein kann,
A für eine direkte Bindung oder für C₁-C₄-Alkylen, C₁-C₄-Alkylenoxy, Oxy(C₁-C₄)- alkylen, Thio(C₁-C₄)-alkylen, C₁-C₄-alkylenthio, -O- -S-, C₁-C₄-Alkylencarboxy, Carboxy(C₁-C₄)-Alkylen, -NR⁵, -C=N-O-, -NR⁵(C=O)O-, NR⁵(SO₂)-, - NR⁵(C=O)NR⁵-, gegebenenfalls durch R⁵ substituiertes Carbamido, Amidocarbonyl, C₁-C₄-Alkylencarbamido, Carbamido(C₁-C₄)-Alkylen, -(SO₂)- steht,
R⁵ für C₁-C₄-Alkyl, C₃-C₆-Cycloalkyl oder für Wasserstoff steht,
Y für Wasserstoff, für gegebenenfalls ein- oder mehrfach gleich oder verschieden substituiertes C₁-C₄-Alkyl, C₃-C₄-Cycloalkyl, für einen gegebenenfalls ein- oder mehrfach gleich oder verschieden substituierten, 5-6 gliederiges, 0-4 Heteroatome enthaltendes Ringsystem steht, wobei die Heteroatome ausgewählt sein können aus der Reihe N, O, sowie für Naphthalin, Chinolin steht,
wobei die Substituenten ausgewählt sein können aus Halogen, Nitro, Cyano, aus gegebenenfalls einfach oder mehrfach, gleich oder verschieden substituiertem Amino, C₁-C₄-Alkyl, C₁-C₄-Alkoxy, C₁-C₄-Alkoxy-C₁-C₄-Alkyl, C₂-C₄-Alkenyl, C₂-C₄-Alkinyl, C₃-C₄-Cycloalkyl, Aryl, Hetaryl, C₁-C₄-Arylalkyl, C₁-C₄-Hetarylalkyl, Aryloxy, Hetaryloxy, Sulfonyl, C₁-C₄-Thioalkyl, C₁-C₄- Carboxyalkyl,
wobei die Substituenten unabhängig voneinander ausgewählt sein können aus Halogen, C₁-C₄-Alkyl, C₁-C₄-Alkylcarbonyl, C₁-C₄-Halogenalkyl, C₁-C₄-Alkoxy, C₁-C₄-Halogenalkoxy, C₃-C₄-Cycloalkyl, C₃-C₄-Cycloalkylcarbonyl, Cyano, Nitro, (C₁-C₄-Alkoxy)carbonyl, C₁-C₄-Alkylthio, Di(C₁-C₄)alkylamino,
X für CH oder N steht,
W für O oder S steht,

3. Verbindungen der allgemeneinen Formel (I) gemäß einem der Ansprüche 1 oder 2, in welcher
R¹ für
Wasserstoff, für gegebenenfalls einfach oder mehrfach, gleich oder verschieden substituiertes C₁-C₄-Alkyl, C₂-C₄-Alkenyl oder C₂-C₄-Alkinyl steht,
wobei die Substituenten unabhängig voneinander ausgewählt sein können aus Halogen, C₁-C₄-Alkyl, Aryl, Arylalkyl,
wobei die Substituenten Aryl, Arylalkyl gegebenenfalls einfach oder mehrfach, gleich oder verschieden durch Halogen, C₁-C₄-Alkyl, C₁-C₄-Halogenalkyl, C₁-C₄-Halogenalkoxy substituiert sein können,
Q für einen der folgenden Ringe steht, 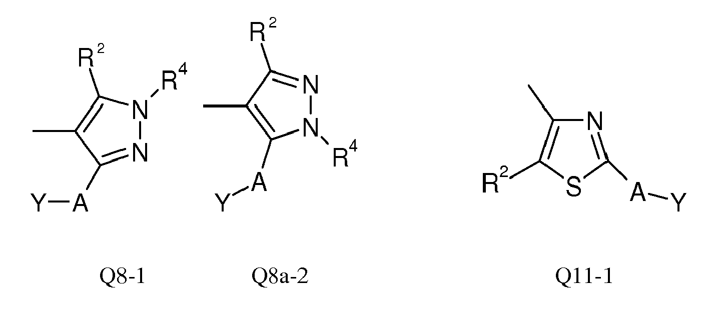 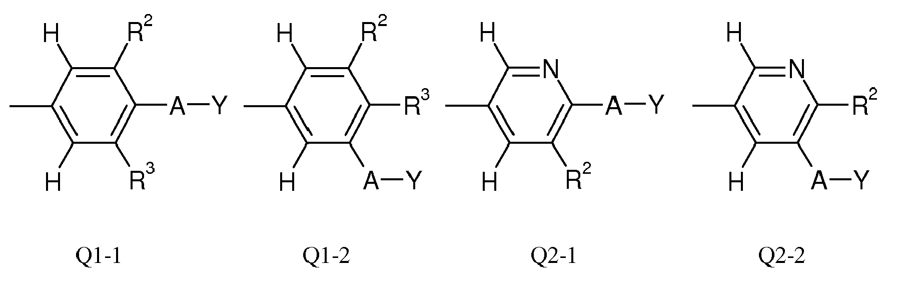
Falls X für N steht
Q für einen der folgenden Ringe steht, 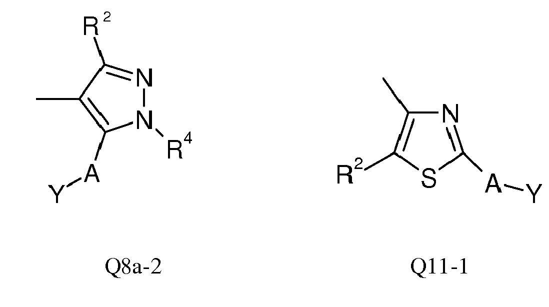 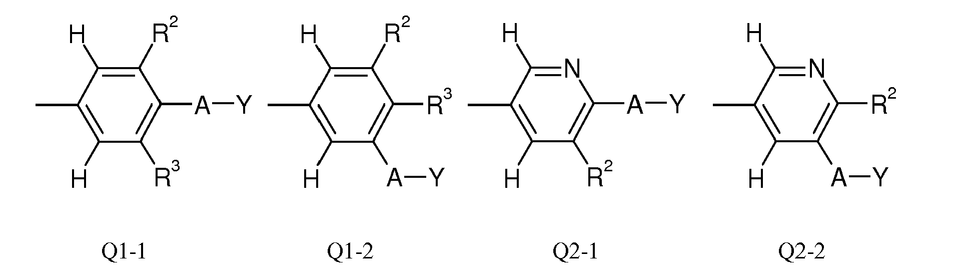
Falls X für CH steht
R² und R³ unabhängig voneinander für Wasserstoff, Halogen, Nitro, für gegebenenfalls einfach oder mehrfach, gleich oder verschieden substituiertes Amino, C₁-C₄-Alkyl, C₁-C₄-Alkoxy, C₁-C₄-Alkoxy-C₁-C₄-Alkyl oder C₃-C₄-Cycloalkyl stehen, wobei die Substituenten unabhängig voneinander ausgewählt sein können aus Halogen, C₁-C₄-Alkyl, C₁-C₄-Alkoxy, C₁-C₄-Halogenalkoxy, C₃-C₄-Cycloalkyl, Cyano, Nitro,
R⁴ für Wasserstoff, für gegebenenfalls einfach oder mehrfach, gleich oder verschieden substituiertes, C₁-C₄-Alkyl, C₁-C₄-Alkoxy-C₁-C₄-Alkyl, C₂-C₄-Alkenyl, C₂-C₄- Alkinyl, C₃-C₄-Cycloalkyl, Aryl, Hetaryl steht, wobei die Substituenten unabhängig voneinander ausgewählt sein können aus Halogen, C₁-C₄-Alkyl, C₁- C₄-Alkoxy, C₁-C₄-Halogenalkoxy, C₃-C₄-Cycloalkyl, Cyano, Nitro, (C₁-C₄- Alkoxy)carbonyl oder Aryl, Hetaryl, das gegebenenfalls einfach oder mehrfach gleich oder verschieden durch Halogen, Cyano, Nitro, C₁-C₄-Alkyl, C₁-C₄- Halogenalkyl, C₁-C₄-Alkoxy, C₁-C₄-Halogenalkoxy substituiert sein kann,
A für eine direkte Bindung oder für C₁-C₄-Alkylen, -CH₂-O-, CH₂-S-, -O- -S-, -O-CH₂-, - S-CH₂-, -C(=O)O-, OC(=O)-, -NR⁵-, -C=N-O-, - R⁵NC(=O)-, - (CH₂)R⁵NC(=O)-, - C(=O)NR⁵-, -NR⁵(C=O)O-, NR⁵(SO₂)-, -NR⁵(C=O)NR⁵-, -(SO₂)- steht,
R⁵ für Methyl, Ethyl, Cyclopentyl, Isobutyl, Wasserstoff steht,
Y für gegebenenfalls ein- oder mehrfach, gleich oder verschieden substituiertes C₁-C₄- Alkyl, C₃-C₄-Cycloalkyl, für einen gegebenenfalls ein- oder mehrfach, gleich oder verschieden substituierten, 5-6 gliederigen, 0-3 Stickstoffatome enthaltenden Ring steht, sowie für Naphthalin, Chinolin steht,
wobei die Substituenten ausgewählt sein können aus Halogen, Nitro, Cyano, aus gegebenenfalls einfach oder mehrfach, gleich oder verschieden substituiertem Amino, C₁-C₄-Alkyl, C₁-C₄-Alkoxy, C₁-C₄-Alkoxy-C₁-C₄-Alkyl, C₃-C₄-Cycloalkyl, Aryl, Hetaryl, C₁-C₄-Arylalkyl, C₁-C₄-Hetarylalkyl, Aryloxy, Hetaryloxy, Sulfonyl, C₁-C₄-Thioalkyl, C₁-C₄₋Carboxyalkyl,
wobei die Substituenten unabhängig voneinander ausgewählt sein können aus Halogen, C₁-C₄-Alkyl, C₁-C₄-Halogenalkyl, C₁-C₄-Alkoxy, C₁-C₄-Halogenalkoxy, C₁-C₄-Alkylthio, C₃-C₄-Cycloalkyl, Di(C₁-C₄)alkylamino,
X für CH oder N steht,
W für O steht.

4. Agrochemische Zusammensetzungen, **dadurch gekennzeichnet, daß** sie mindestens eine Verbindung der Formel (I) gemäß einem der Ansprüche 1-4, sowie Streckmittel und oberflächenaktive Stoffe enthalten.

5. Verfahren zur Bekämpfung tierischer Schädlinge, **dadurch gekennzeichnet, dass** man Verbindungen der Formel (I) gemäß den Ansprüchen 1 bis 3 oder eine Zusammensetzung gemäß Anspruch 4 auf tierische Schädlinge und/oder deren Lebensraum und/oder Saatgut einwirken lässt.

6. Verfahren zur Herstellung agrochemischer Zusammensetzungen, **dadurch gekennzeichnet, dass** man Verbindungen der Formel (I) gemäß den Ansprüchen 1 bis 3 mit Streckmitteln und/oder oberflächenaktiven Stoffen vermischt.

7. Verwendung von Verbindungen der Formel (I) gemäß den Ansprüchen 1 bis 3 oder einer Zusammensetzung gemäß Anspruch 4 zur Bekämpfung von tierischen Schädlingen im Pflanzenschutz, im Materialschutz, im Hygienesektor und/oder im veterinärmedizinischen Sektor.
